# EUROPEAN PATENT APPLICATION

(11) **EP 1 394 252 A2**
(43) Date of publication of application: **03.03.2004**
(21) Application number: 03009752.1
(22) Date of filing: 06.03.1998
(51) Int. Cl.: C12N 15/12, C12N 5/10, C12N 1/21, C07K 14/47, C07K 16/18, C12Q 1/68, G01N 33/50, G01N 33/53, G01N 33/68, A61K 38/17

(54) **70 human secreted proteins**

(30) Priority: 07.03.1997 US 40162 P; 07.03.1997 US 40333 P; 07.03.1997 US 38621 P; 07.03.1997 US 40161 P; 07.03.1997 US 40626 P; 07.03.1997 US 40334 P; 07.03.1997 US 40336 P; 07.03.1997 US 40163 P; 11.04.1997 US 43580 P; 11.04.1997 US 43568 P; 11.04.1997 US 43314 P; 11.04.1997 US 43569 P; 11.04.1997 US 43311 P; 11.04.1997 US 43671 P; 11.04.1997 US 43674 P; 11.04.1997 US 43669 P; 11.04.1997 US 43312 P; 11.04.1997 US 43313 P; 11.04.1997 US 43672 P; 11.04.1997 US 43315 P; 11.04.1997 US 43578 P; 11.04.1997 US 43576 P; 11.04.1997 US 43670 P; 23.05.1997 US 47600 P; 23.05.1997 US 47615 P; 23.05.1997 US 47597 P; 23.05.1997 US 47502 P; 23.05.1997 US 47633 P; 23.05.1997 US 47583 P; 23.05.1997 US 47617 P; 23.05.1997 US 47618 P; 23.05.1997 US 47503 P; 23.05.1997 US 47592 P; 23.05.1997 US 47581 P; 23.05.1997 US 47584 P; 23.05.1997 US 47500 P; 23.05.1997 US 47587 P; 23.05.1997 US 47492 P; 23.05.1997 US 47598 P; 23.05.1997 US 47613 P; 23.05.1997 US 47582 P; 23.05.1997 US 47596 P; 23.05.1997 US 47612 P; 23.05.1997 US 47632 P; 23.05.1997 US 47601 P; 23.05.1997 US 47595 P; 23.05.1997 US 47599 P; 23.05.1997 US 47588 P; 23.05.1997 US 47585 P; 23.05.1997 US 47586 P; 23.05.1997 US 47590 P; 23.05.1997 US 47594 P; 23.05.1997 US 47589 P; 23.05.1997 US 47593 P; 23.05.1997 US 47614 P; 23.05.1997 US 47501 P; 06.06.1997 US 48974 P; 06.06.1997 US 48964 P; 22.08.1997 US 56886 P; 22.08.1997 US 56877 P; 22.08.1997 US 56889 P; 22.08.1997 US 56893 P; 22.08.1997 US 56630 P; 22.08.1997 US 56878 P; 22.08.1997 US 56662 P; 22.08.1997 US 56872 P; 22.08.1997 US 56882 P; 22.08.1997 US 56637 P; 22.08.1997 US 56903 P; 22.08.1997 US 56888 P; 22.08.1997 US 56879 P; 22.08.1997 US 56880 P; 22.08.1997 US 56894 P; 22.08.1997 US 56911 P; 22.08.1997 US 56636 P; 22.08.1997 US 56874 P; 22.08.1997 US 56910 P; 22.08.1997 US 56864 P; 22.08.1997 US 56631 P; 22.08.1997 US 56845 P; 22.08.1997 US 56892 P; 22.08.1997 US 56632 P; 22.08.1997 US 56664 P; 22.08.1997 US 56876 P; 22.08.1997 US 56881 P; 22.08.1997 US 56909 P; 22.08.1997 US 56875 P; 22.08.1997 US 56862 P; 22.08.1997 US 56887 P; 22.08.1997 US 56908 P; 22.08.1997 US 56884 P; 22.08.1997 US 57761 P; 22.08.1997 US 57650 P
(62) Divisional of application: 98905126.3
(71) Applicant: HUMAN GENOME SCIENCES, INC., Rockville, MD 20850-3338 (US)
(72) Inventor: Olsen, Henrik S., Gaithersburg, MD 20878 (US); Yu, Guo-Liang, Berkeley, CA 94705 (US); Endress, Gregory A., Florence, MA 01062 (US); Bednarik, Daniel P., Columbia, MD 21046 (US); Carter, Kenneth C., North Potomac, MD 20878 (US); Feng, Ping, Germantown, MD 20874 (US); Soppet, Daniel R., Centreville, VA 20120 (US); Young, Paul E., Gaithersburg, MD 20878 (US); Duan, D. Roxanne, Gaithersburg, MD 20878 (US); Florence, Kimberly A., Rockville, MD 20851 (US); Greene, John M., Gaithersburg, MD 20878 (US); Fischer, Carrie L., Burke, VA 22015 (US); Hu, Jing-Shan, Mountain View, CA 94040 (US); Ruben, Steven M., Brookeville, MD 20833 (US); Ebner, Reinhard, Gaithersburg, MD 20878 (US); Brewer, Laurie A., St. Paul, MN 55119 (US); Ferrie, Ann M., Painted Post, NY 14870 (US); Moore, Paul A., Germantown, MD 20874 (US); Ni, Jian, Germantown, MD 20874 (US); Shi, Yanggu, Gaithersburg, MD 20878 (US); Lafleur, David W., Washington, DC 20015 (US); Li, Yi, Sunnyvale, CA 94086 (US); Zeng, Zhizhen, Lansdale, PA 19446 (US); Kyaw, Hla, Boonsboro, MD 21713 (US); Rosen, Craig A., Laytonsville, MD 20882 (US)
(74) Representative: VOSSIUS & PARTNER

(57) **Abstract**

The present invention relates to 70 novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein , a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 12301 Park Lawn Drive, Rockville, Maryland 20852,USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA. pH 7.4), 0.5% SDS, 30% formamide, 100 ug/ml salmon sperm blocking DNA; followed by washes at 50°C with 1XSSPE, 0.1 % SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62(1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

The translation product of Gene NO: 1 shares sequence homology with alpha-L-fucosidase which is thought to be important as a lysosomal enzyme that hydrolyzes fucose from fucoglycoconjugates. (See Accession No. gi/178409.) Lysosome fructosidase is involved in certain lysosome storage diseases. (See Biochem. Biophys. Res. Commun., 164(1):439-445 (1989).) Fucosidosis, an autosomal recessive lysosomal storage disorder characterized by progressive neurological deterioration and mental retardation. The disease results from deficient activity of alpha-L-fucosidase, a lysosomal enzyme that hydrolyzes fucose from fucoglycoconjugates. This gene likely encodes a novel fucosidase isoenzyme. Based on homology, it is likely that the translated product of this gene is also involved in lysosome catabolism of molecules and that aberrations in the concentration and/or composition of this product may be causative in lysosome storage disorders. Preferred polypeptide fragments comprise the amino acid sequence PGHLLPHKWENC (SEQ ID NO: 257).

Gene NO: 1 is expressed primarily in stromal cells, and to a lesser extent in human fetal kidney and human tonsils.

Therefore, polynucleotides or polypeptides of the invention are useful as reagents for differential identification of the tissue(s) or cell type(s) present in a biological sample and for diagnosis of diseases and conditions which include, but are not limited to, fucosidosis and other lysosome storage disorders. Similarly, polypeptides and antibodies directed to the polypeptides are useful in providing immunological probes for differential identification of the tissue(s) or cell type(s). For a number of disorders of the above tissues of cells. particularly of the nervous system, expression of this gene at significantly higher or lower levels may routinely be detected in certain tissues and cell types (e.g., stromal cells. kidney, tonsils, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology of Gene NO: 1 to alpha-L-fucosidase indicates that polypeptides and polynucleotides corresponding to Gene NO: 1 are useful for the treatment of fucosidosis and general lysosomal disorders.

Preferred epitopes include those comprising a sequence shown in SEQ ID NO: 134 as residues: Met-1 to Leu-6, Thr-32 to Glu-39, Lys-80 to Lys-85, and Met-90 to Pro-96.

### FEATURES OF PROTEIN ENCODED BY GENE NO: 2

The translation product of Gene No. 2 shares sequence homology with stromal cell-derived factor-2 (SDF-2) which is a novel secreted factor. See, for example, Gene. 176(1-2):211-214, (1996, Oct. 17.) The amino acid sequence of SDF-2 shows similarity to yeast dolichyl phosphate-D-mannose:protein mannosyltransferases, Pmt1p [Strahl-Bolsinger et al. Proc. Natl. Acad. Sci. USA 90, 8164-8168 (1993)] and Pmt2p [Lussier et al. J. Biol. Chem. 270, 2770-2775 (1995)], whose activities have not been detected in higher eukaryotes. Based on the sequence similarity, the translation product of this gene is expected to share certain biological activities with SDF-2, Pmt1p and Pmt2p.

Gene NO: 2 is expressed primarily in immune system tissue and cancerous tissues, such as liver hepatoma, human B-cell lymphoma, spleen in a patient suffering from chronic lymphocytic leukemia, hemangiopericytoma, pharynx carcinoma, breast cancer, thyroid, bone marrow, osteoblasts and to a lesser extent in a few other tissues such as kidney pyramids.

Therefore, polynucleotides or polypeptides of the invention are useful as reagents for differential identification of the tissue(s) or cell type(s) present in a biological sample and for diagnosis of the diseases and conditions which include, but are not limited to, disorders in kidney, liver, and immune organs, particularly cancers. Similarly, polypeptides and antibodies directed to these polypeptides are useful in providing immunological probes for differential identification of the tissue(s) or cell type(s). For a number of disorders of the above tissues or cells, particularly of the kidney, liver, thyroid, and bone marrow expression of this gene at significantly higher or lower levels may routinely be detected in certain tissues and cell types (e.g., liver, spleen, B-cells, pharynx, thyroid, mammary tissue, bone marrow, osteoblasts and kidneys, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution and homology of Gene NO: 2 to stromal cell-derived factor-2 indicates that polypeptides and polynucleotides corresponding to Gene NO: 2 are useful for diagnosis and therapeutic treatment of disorders in kidney, liver, and immune organs since stromal cells play important role in organ function. Stroma carries the blood supply and provides support for the growth of parenchymal cells and is therefore crucial to the growth of a neoplasm. Nucleic acids of the present invention comprise, but preferably do not consist of, and more preferably do not comprise, SEQ ID NO: 3 from US Patent No. 5,576,423, incorporated herein by reference, and shown herein as SEQ ID NO: 258).

Preferred epitopes include those comprising a sequence shown in SEQ ID NO: 135 as residues: His-56 to Gly-65, Ala-74 to Ser-80, Ile-84 to Pro-97, Leu-124 to Glu-129, Glu-135 to Asp-143, Gly-175 to Ser-180, and Ala-194 to Thr-199.

### FEATURES OF PROTEIN ENCODED BY GENE NO: 3

The translation product of Gene NO: 3 shares sequence homology with LZIP-1, LZIP-2 and other leucine zipper proteins, which are thought to be important in nucleic acid binding. This gene has been reported in Mol. Cell. Biol. 17 (9), 5117-5126 (1997) as "Luman". Luman is a cyclic AMP response element (CRE)-binding protein/activating transcription factor 1 protein of the basic leucine zipper superfamily. It binds CREs in vitro and activates CRE-containing promoters when transfected into COS7 cells. The complete amino acid sequence of Luman reported in Mol. Cell. Biol. 17 (9): 5117-5126 (1997) is:







 (SEQ ID
N:259).

Gene NO: 3 is expressed primarily in apoptotic T-cells and Soares senescent cells and to a lesser extent in multiple tissues and cell types, including, multiple sclerosis tissue, and hippocampus.

Therefore, polynucleotides or polypeptides of the invention are useful as reagents for differential identification of the tissue(s) or cell type(s) present in a biological sample and for diagnosis of diseases and conditions which include, but are not limited to, immunologically mediated disorders, transplantation, immunodeficiency, and tumor necrosis. Similarly, polypeptides and antibodies directed to these polypeptides are useful in providing immunological probes for differential identification of the tissue(s) or cell type(s). For a number of disorders of the above tissues or cells, particularly of the immune system and transplantation, expression of this gene at significantly higher or lower levels may routinely be detected in certain tissues (e.g., multiple sclerosis tissue, hippocampus, bone marrow and cancerous and wounded tissues) or bodily fluids (e.g., serum, plasma, urine, synovial fluid or spinal fluid) or another tissue or cell sample taken from an individual having such a disorder, relative to the standard gene expression level. i.e., the expression level in healthy tissue or bodily fluid from an individual not having the disorder.

The tissue distribution and homology of Gene NO: 3 to leucine zipper nucleic acid binding proteins indicates that polypeptides and polynucleotides corresponding to Gene NO: 3 are useful for diagnosis and treatment of immunologically mediated disorders, transplantation, immunodeficiency, and tumor necrosis. The secreted nucleic acid binding protein in the apoptotic tissues may be involved in the disposal of the DNA released by apoptotic cells. Furthermore, the studies conducted in support of Luman suggest that the translation product of this gene may be used to identify transcriptional regulation elements which in turn are useful in modulation of immune function.

Preferred epitopes include those comprising a sequence shown in SEQ ID NO: 136 as residues: Asn-7 to Ser-12, Tyr-32 to Gly-38, Pro-55 to Tyr-60, Glu-70 to Thr-76, and Pro-104 to Leu-110.

### FEATURES OF PROTEIN ENCODED BY GENE NO: 4

The translation product of Gene NO: 4 shares sequence homology with a number of tetraspan transmembrane surface molecules such as human metastasis tumor suppressor gene, CO-029 tumor associated antigen protein, CD53 hematopoietic antigen, human membrane antigen TM4 superfamily protein, metastasis controlling peptide, and human CD9 sequence, which are thought to be important in development of cancer, immune system development and functions.

Gnee NO: 4 is expressed primarily in cancers of several different tissues and to a lesser extent in normal tissue like prostate, skin and kidney.

Therefore, polynucleotides or polypeptides of the invention are useful as reagents for differential identification of the tissue(s) or cell type(s) present in a biological sample and for diagnosis of diseases and conditions which include, but are not limited to, cancers and disorders of the immune system, prostate and kidney. Similarly, polypeptides and antibodies directed to these polypeptides are useful in providing immunological probes for differential identification of the tissue(s) or cell type(s). For a number of disorders of the above tissues or cells, particularly of the kidney, skin, prostate and immune system, expression of this gene at significantly higher or lower levels may routinely be detected in certain tissues (e.g., kidney, skin and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology of Gene NO: 4 to tetraspan transmembrane surface molccules such as human metastasis tumor suppressor gene, CO-029 tumor associated antigen protein, CD53 hematopoietic antigen, human membrane antigen TM4 superfamily protein, metastasis controlling peptide, and human CD9 sequence, indicates that polypeptides and polynucleotides corresponding to Gene NO: 4 are involved with the cellular control of growth and differentiation. Therefore, the translation product of this gene is believed to be useful for diagnosis and treatment of neoplasia and disorders of the kidney, skin and prostate. For example, recombinant protein can be produced in transformed host cells for diagnostic and prognostic applications. Alterations in the protein sequence are indicative of the presence of malignant cancer, or of a predisposition to malignancy, in a subject. Gene therapy can be used to restore the wild-type gene product to a subject. Additionally, the antibodies are a useful tool for the identification of hematopoietic neoplasms, and may prove helpful for identifying morphologically poorly defined cells. Moreover, this protein can be used to isolate cognate receptors and ligands and identify potential agonists and antagonists using techniques known in the art. The protein also has immunomodulatory activity, regulates hematopoiesis and stimulates growth and regeneration as a male/female contraceptive, increases fertility depending on activin and inhibin like activities. Other uses are as a chemotactic agent for lymphocytes, treatment of coagulation disorders, an anti-inflammatory agent, an antimicrobial or analgesic and as a modulator of behavior and metabolism. The DNA can be used in genetic diagnosis or gene therapy, and for the production of recombinant protein. It can also be used to identify protein expressing cells, isolate related sequences, prepare primers for genetic fingerprinting and generate anti-protein or anti-DNA antibodies. In addition, residues 1-71, in the translation product for this gene are believed to be the extracellular domain. Thus, polypeptide comprising residues 1-71 or derivatives (including fragments) or analogs thereof, are useful as a soluble polypeptide which may be routinely used therapeutically to antagonize the activities of the receptor.

Preferred epitopes include those comprising a sequence shown in SEQ ID NO: 137 as residues: Lys-118 to Phe-127, Asn-145 to Ala-160, and Thr-177 to Val-188.

### FEATURES OF PROTEIN ENCODED BY GENE NO: 5

Gene NO: 5 is expressed primarily in human testes.

Therefore, polynucleotides or polypeptides of the invention are useful as reagents for differential identification of the tissue(s) or cell type(s) present in a biological sample and for diagnosis of diseases and conditions which include, but are not limited to, diseases of the testes including cancer and reproductive disorders. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may routinely be detected in certain tissues (e.g., testes and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of Gene NO: 5 indicates that the protein product of this gene is useful for treatment/diagnosis of diseases of the testes, particularly testicular cancer since expression is observed primarily in the testes.

Preferred epitopes include those comprising a sequence shown in SEQ ID NO: 138 as residue: Gly-22 to Gln-30.

### FEATURES OF PROTEIN ENCODED BY GENE NO: 6

The translation product of Gene NO: 6 shares sequence homology with GALNS (N-acetylgalactosamine 6-sulphatase) which is thought to be important in the storage of the glycosaminoglycans, keratan sulfate and chondroitin 6-sulfate. See Genbank accession no. gi1618426. Based on the sequence similarity, the translation product of this gene is expected to share biological activities with GALNS.

Gene NO: 6 is expressed primarily in human bone marrow.

Therefore, polynucleotides or polypeptides of the invention are useful as reagents for differential identification of the tissue(s) or cell type(s) present in a biological sample and for diagnosis of diseases and conditions which include, but are not limited to, storage disorders of glycosaminoglycans, keratan sulfate and chondroitin 6-sulfate, e.g., Morquio A syndrome. Similarly, polypeptides and antibodies directed to these polypeptides are useful in providing immunological probes for differential identification of the tissue(s) or cell type(s). For a number of disorders of the above tissues or cells, particularly involving cell storage disorder, expression of this gene at significantly higher or lower levels may routinely be detected in certain tissues (e.g., bone marrow and cancerous and wounded tissues) or bodily fluids (e.g., serum. plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology of Gene NO: 6 to N-acetylgalactosamine 6-sulphatase indicates that polypeptides and polynucleotides corresponding to Gene NO: 6 are useful for the treatment and diagnosis of storage disorders of glycosaminoglycans, keratan sulfate and chondroitin 6-sulfate. Such disorders are known in the art and include, e.g., Morquio A syndrome which is caused by an error of mucopolysaccharide metabolism with excretion of keratan sulfate in urine. Morquio A syndrome is characterized by severe skeletal defects with short stature, severe deformity of spine and thorax, long bones with irregular epiphyses but with shafts of normal length, enlarged joints, flaccid ligaments, and waddling gait; autosomal recessive inheritance.

Preferred epitopes include those comprising a sequence shown in SEQ ID NO: 139 as residues: Gly-29 to Pro-36 and Glu-57 to Leu-64.

### FEATURES OF PROTEIN ENCODED BY GENE NO: 7

The translation product of Gene NO: 7 shares sequence homology with carboxy peptidase E and H (carboxypeptidase E is thought to be important in the biosynthesis of numerous peptide hormones and neurotransmitters). The translation product of this gene also shares sequence homology with bone-related carboxypeptidase "OSF-5" from the mouse. See European patent application EP-588118-A. Based on the sequence similarity to OSF-5, the translation product of this gene will hereinafter sometimes be referred to as "human-OSF-5" or "hOSF-5".

Gene NO: 7 is expressed primarily in tumor cell lines derived from connective tissues including chondrosarcoma, synovial sarcoma. Wilm's tumor and rhabdomyosarcoma and to a lesser extent in a myeloid progenitor cell line, bone marrow, and placenta.

Therefore, polynucleotides or polypeptides of the invention are useful as reagents for differential identification of the tissue(s) or cell type(s) present in a biological sample and for diagnosis of diseases and conditions which include, but are not limited to, various cancers involving the skeletal system and connective tissues in general, in particular at cartilage interfaces. Similarly, polypeptides and antibodies directed to these polypeptides are useful in providing immunological probes for differential identification of the tissue(s) or cell type(s). For a number of disorders of the above tissues or cells, particularly of the skeletal system and various other tumor tissues, expression of this gene at significantly higher or lower levels may routinely be detected in certain tissues (e.g., connective tissues and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The restricted tissue distribution and homology of Gene NO: 7 to carboxypeptidase E and mouse OSF-5 indicates that polypeptides and polynucleotides corresponding to Gene NO: 7 are for processing of peptides to their mature form that may have various activities similar to the activities of neuropeptides but in the periphery. In addition the abundance of expression in cancer tissues indicates that aberrant expression and subsequent processing may play a role in the progression of malignancies, e.g., growth factor and/or adhesion factor activities. In particular, the expression of this gene is restricted to connective tissues and embryonic tissues. Furthermore, it is overexpressed in cancers of these same tissues (i.e., in sarcomas). Moreover, hOSF-5 shares very strong sequence similarity with mOSF-5 which is a known bone growth factor and is thought to be useful in obtaining products for the diagnosis and treatment of bone metabolic diseases, e.g., osteoporosis and Paget's disease. Like OSF-5, the translation product of this gene is believed to be a bone-specific carboxypeptidase which acts as an adhesion molecule/growth factor and takes part in osteogenesis at the site of bone induction. hOSF-5 can, therefore, be used to treat bone metabolic diseases, osteoporosis, Paget's disease, osteomalacia, hyperostosis or osteopetrosis. Furthermore, hOSF-5 can be used to stimulate the regeneration of bone at the site of mechanical damage, e.g., accidentally or surgically caused fractures.

Preferred epitopes include those comprising a sequence shown in SEQ ID NO: 140 as residues: Leu-24 to Val-30, Ala-89 to Lys-94, Phe-150 to Trp-157, Leu-162 to Asp-167, Asp-187 to Ser-199, His-241 to Asp-254, and Pro-362 to Asp-376.

### FEATURES OF PROTEIN ENCODED BY GENE NO: 8

Gene NO: 8 is expressed primarily in bone marrow, and to a lesser extent in an erythroleukemia cell line.

Therefore, polynucleotides or polypeptides of the invention are useful as reagents for differential identification of the tissue(s) or cell type(s) present in a biological sample and for diagnosis of diseases and conditions which include, but are not limited to, hematological disorders including cancer and anemia. Similarly, polypeptides and antibodies directed to these polypeptides are useful in providing immunological probes for differential identification of the tissue(s) or cell type(s). For a number of disorders of the above tissues or cells, particularly of the immune and hematologic systems, expression of this gene at significantly higher or lower levels may routinely be detected in certain tissues (e.g., bone marrow, kidney, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 8 are useful as a growth factor for hematopoietic stem cells or progenitor cells, e.g., in the treatment of bone marrow stem cell loss in chemotherapy patients and in the treatment of kidney disease.

Preferred epitopes include those comprising a sequence shown in SEQ ID NO: 141 as residues: Gly-30 to Lys-35.

### FEATURES OF PROTEIN ENCODED BY GENE NO: 9

Gene NO: 9 is expressed primarily in neutrophils.

Therefore, polynucleotides or polypeptides of the invention are useful as reagents for differential identification of the cell type present in a biological sample and for diagnosis of diseases and conditions which include, but are not limited to, inflammatory diseases. Similarly, polypeptides and antibodies directed to these polypeptides are useful in providing immunological probes for differential identification of the cell type indicated. For a number of disorders of the above tissues or cells, particularly of the immune system, expression of this gene at significantly higher or lower levels may routinely be detected in certain tissues or cell types (e.g., neutrophils, bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 9 are useful for immune modulation or as a growth factor to stimulate neutrophil differentiation or proliferation that may be useful in the treatment of neutropenia.

Preferred epitopes include those comprising a sequence shown in SEQ ID NO: 142 as residues: Thr-22 to Pro-37.

### FEATURES OF PROTEIN ENCODED BY GENE NO: 10

Gene NO: 10 is expressed primarily in the epidermis.

Therefore, polynucleotides or polypeptides of the invention are useful as reagents for differential identification of the tissue(s) or cell type(s) present in a biological sample and for diagnosis of diseases and conditions which include, but are not limited to, diseases of the epidermis such as psoriasis or eczema or may be involved in the normal proliferation or differentiation of the epithelial cells or fibroblasts constituting the skin. Similarly, polypeptides and antibodies directed to these polypeptides are useful in providing immunological probes for differential identification of the tissue(s) or cell type(s). For a number of disorders of the above tissues or cells, particularly of the skin, expression of this gene at significantly higher or lower levels may routinely be detected in certain tissues (e.g., epidermis and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 10 are useful for diagnosis and treatment of skin conditions and as an aid in the healing of various epidermal injuries including wounds, and diabetic ulcers.

Preferred epitopes include those comprising a sequence shown in SEQ ID NO: 143 as residues: Ser-3 to Ser-9 and Trp-27 to Glu-32.

### FEATURES OF PROTEIN ENCODED BY GENE NO: 11

The translation product of Gene NO: 11 shares sequence homology with phosphatidylcholine 2-acylhydrolase (PLA2). See, for example, Genbank accession no. gil190004. PLA2 is involved in inflammation, where it is responsible for the conversion of cell membrane phospholipids into arachidonic acid. Arachidonic acid in turn feeds into both the lipoxygenase and cyclooxygenase pathways to produce leukotrienes (involved in chemotaxis, vasoconstriction, bronchoconstriction, and increased vascular permeability) and prostaglandins (responsible for vasodilation, potentiate edema, and increased pain). Diseases in which PLA2 is implicated as a major factor include rheumatoid arthritis, sepsis, ischemia, and thrombosis. The inventors refer to the translation product of this gene as PLA2-like protein based on the sequence similarity. Furthermore, owing to the sequence similarity PLA2 and PLA2-like protein are expected to share certain biological activities.

Gene NO: 11 is expressed primarily in human cerebellum and in T-cells.

Therefore, polynucleotides or polypeptides of the invention are useful as reagents for differential identification of the tissue(s) or cell type(s) present in a biological sample and for diagnosis of diseases and conditions which include, but are not limited to, cerebellum disorders, rheumatoid arthritis, sepsis, ischemia, and thrombosis. Similarly, polypeptides and antibodies directed to these polypeptides are useful in providing immunological probes for differential identification of the tissue(s) or cell type(s). For a number of disorders of the above tissues or cells, particularly of the cerebellum and Purkinje cells, expression of this gene at significantly higher or lower levels may routinely be detected in certain tissues and cell types (e.g., brain, bone marrow, T-cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 11 are useful for diagnosis and treatment of cerebellum disorders, rheumatoid arthritis, sepsis, ischemia, and thrombosis. This gene is also useful as a chromosome marker. It is believed to map to Chr.15, D15S118-D15S123.

### FEATURES OF PROTEIN ENCODED BY GENE NO: 12

Gene NO: 12 is expressed primarily in highly vascularized tissues such as placenta, uterus, tumors, fetal liver, fetal spleen and also in the C7MCF7 cell line treated with estrogen.

Therefore, polynucleotides or polypeptides of the invention are useful as reagents for differential identification of the tissue(s) or cell type(s) present in a biological sample and for diagnosis of diseases and conditions which include, but are not limited to, endometriosis, endometritis, endometrial carcinoma, primary hepatocellular carcinoma, and spleen-related diseases. Similarly, polypeptides and antibodies directed to these polypeptides are useful in providing immunological probes for differential identification of the tissue(s) or cell type(s). For a number of disorders of the above tissues or cells, particularly of the endometrium, liver and spleen, expression of this gene at significantly higher or lower levels may routinely be detected in certain tissues (e.g., endometrium, liver, and spleen, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 12 are useful for diagnosis and treatment of diseases of the endometrium (such as endometrial carcinoma, endometriosis, and endometritis), liver diseases (such as primary hepatocellular carcinoma), and spleen-related diseases.

SEQ ID NO: 145 as residues: Ala-29 to Leu-35, Leu-50 to Ser-57, Glu-96 to Glu-105, Asp-140 to Asp-148, and Asn-191 to Ser-197.

### FEATURES OF PROTEIN ENCODED BY GENE NO: 13

Gene NO: 13 is expressed primarily in B cell lymphoma and to a lesser extent in other tissues.

Therefore, polynucleotides or polypeptides of the invention are useful as reagents for differential identification of the tissue(s) or cell type(s) present in a biological sample and for diagnosis of diseases and conditions which include, but are not limited to, B cell lymphoma; hematopoietic disorders; immune dysfunction. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., bone marrow and B-cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

Enhanced expression of this gene product in B cell lymphoma indicates that it may play a role in the proliferation of hematopoietic cells. It is also believed to be involved in the survival and/or differentiation of various hematopoietic lineages. Expression in lymphoma also indicates that it may be involved in other cancers and abnormal cellular proliferation. The tissue distribution, therefore, indicates that polypeptides and polynucleotides corresponding to Gene NO: 13 are useful for the diagnosis and/or therapeutic treatment of hematopoietic disorders, particularly B cell lymphoma. Furthermore, since overexpression of this gene is associated with the development of B cell lymphoma, antagonists of this protein are useful to interfere with the progression of the disease. This protein is useful in assays for identifying such antagonists. Assays for identifying antagonists are known in the art and are described briefly elsewhere herein. Preferred antagonists include antibodies and antisense nucleic acid molecules. Preferred are antagonists which inhibit B-cell proliferation.

### FEATURES OF PROTEIN ENCODED BY GENE NO: 14

The translation product of Gene NO: 14 shares sequence homology with very low density lipoprotein receptor which is thought to be important in transport of lipoproteins. Owing to the sequence similarity the translation product of this gene is believed to share certain biological activities with VLDL receptors. Assaying such activity may be achieved by assays known in the art and set forth elsewhere herein.

This gene is expressed primarily in human synovium, umbilical vein endothelial cells, CD34+ cells, Jurkat cells, and HL60 cells, and to a lesser extent in thymus, meningioma, hypothalmus, adult testis, and fetal liver and spleen.

Therefore, polynucleotides or polypeptides of the invention are useful as reagents for differential identification of the tissue(s) or cell type(s) present in a biological sample and for diagnosis of diseases and conditions which include, but are not limited to, atherosclerosis, ataxia malabsortion, vascular damage, hyperlipidemia, and other cardiovascular diseases. Similarly, polypeptides and antibodies directed to these polypeptides are useful in providing immunological probes for differential identification of the tissue(s) or cell type(s). For a number of disorders of the above tissues or cells, particularly of the cardiovascular and hematological systems, expression of this gene at significantly higher or lower levels may routinely be detected in certain tissues (e.g., endothelium, thymus meningioma, hypothalmus, testes, liver, and spleen and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in the vascular endothelial cells and homology to VLDL receptors indicates that polypeptides and polynucleotides corresponding to Gene NO: 14 are useful for diagnosis and treatment of atherosclerosis, ataxia malabsortion, and hyperlipidemia. These and other factors often result in other cardiovascular diseases. Additionally, the presence of the gene product in cells of blood lineages indicates that it may be useful in hematopoietic regulation and hemostasis.

Preferred epitopes include those comprising a sequence shown in SEQ ID NO: 147 as residues: Pro-39 to Ser-52, Trp-71 to Thr-76, and Pro-94 to His-100.

### FEATURES OF PROTEIN ENCODED BY GENE NO: 15

The translation product of Gene NO: 15 shares sequence homology with kallikrein which is thought to be important in blood pressure and renal secretion. Furthermore, this gene has now been characterized as a novel hepatitis B virus X binding protein that inhibits viral replication. See, for example, J. Virol. 72 (3), 1737-1743 (1998).

This gene is expressed primarily in kidney, placenta, lung, aorta and other endothelial cells, caudate nucleus and to a lesser extent in melanocytes, liver, adipose tissue.

Therefore, polynucleotides or polypeptides of the invention are useful as reagents for differential identification of the tissue(s) or cell type(s) present in a biological sample and for diagnosis of diseases and conditions which include, but are not limited to, renovascular hypertension, renal secretion, electrolyte metabolism, toxemia of pregnancy. Similarly, polypeptides and antibodies directed to these polypeptides are useful in providing immunological probes for differential identification of the tissue(s) or cell type(s). For a number of disorders of the above tissues or cells, particularly of the renovascular or respiratory vascular systems, expression of this gene at significantly higher or lower levels may routinely be detected in certain tissues and cell types (e.g., kidney, placenta, lung, endothelial cells, melanocytes, liver, and adipose tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to kallikrein indicates that polypeptides and polynucleotides corresponding to Gene NO: 15 are useful for treating renovascular hypertension, renal secretion, electrolyte metabolism, toxemia of pregnancy and hydronephrosis. The protein expression in the organs like kidney, lung and vascular endothelial cells indicates the gene involvement in hemodynamic regulatory functions. The translation product of this gene is also useful in the treatment of viral infection, particularly liver infection, and particularly hepatitis B virus(es).

Preferred epitopes include those comprising a sequence shown in SEQ ID NO: 148 as residues: Leu-9 to Asn-15 and Thr-56 to Asp-61.

### FEATURES OF PROTEIN ENCODED BY GENE NO: 16

The translation product of Gene NO: 16 shares sequence homology with secretory component protein, immunoglobulins and their receptors which are thought to be important in immunological functions. The amino acid sequence of secretory component protein can be accessed as accession no. pirlA02112, incorporated herein by reference.

Gene NO: 16 is expressed primarily in macrophages, monocytes and dendritic cells and to a lesser extent in placenta and brain.

Therefore, polynucleotides or polypeptides of the invention are useful as reagents for differential identification of the tissue(s) or cell type(s) present in a biological sample and for diagnosis of diseases and conditions which include, but are not limited to, inflammation and tumors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues or cells (e.g., macrophages, monocytes, dendritic cells, plancenta and brain, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to immunoglobulins and secretory component protein indicates that polypeptides and polynucleotides corresponding to Gene NO: 16 are useful for diagnosis and treatment of inflammation and bacterial infection, and other diseases where immunomodulation would be beneficial.

Preferred epitopes include those comprising a sequence shown in SEQ ID NO: 149 as residues: Pro-37 to Cys-51, Gln-53 to Cys-60, Asn-99 to Gly-106, Gly-145 to Glu-151, and Ile-159 to Ser-164.

### FEATURES OF PROTEIN ENCODED BY GENE NO: 17

The translation product of Gene NO: 17 is evolutionarily conserved and shares sequence homology with proteins from yeast and *C. elegans.* See, for example, Genbank accession no.gil746540. As is known in the art, strong sequence similarity to a secreted protein from C. elegans is predictive of cellular location of human proteins.

Gene NO: 17 is expressed primarily in colon carcinoma cell lines, messangial cells, many tumors like T cell lymphoma, osteoclastoma, Wilm's tumor, adrenal gland tumor, testes tumor, synovial sarcoma, and to a lesser extent in placenta, lung and brain.

Therefore, polynucleotides or polypeptides of the invention are useful as reagents for differential identification of the tissue(s) or cell type(s) present in a biological sample and for diagnosis of diseases and conditions which include, but are not limited to, rapidly growing/dividing cells such as cancerous tissue, including, colon carcinoma, lymphomas, and sarcomas. Similarly, polypeptides and antibodies directed to these polypeptides are useful in providing immunological probes for differential identification of the tissue(s) or cell type(s). For a number of disorders of the above tissues or cells, particularly of the gastrointestinal, hematological and immune systems, expression of this gene at significantly higher or lower levels may routinely be detected in certain tissues and cell types (e.g.. placenta, lung, brain, colon, messangial cells. adrenal gland, T-cells, testes, and lymph tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in colon cancer and many other tumors indicates that the polynucleotides and polypeptides of Gene NO: 17 are useful for cancer diagnosis and therapeutic targeting. The extracellular nature may contribute to solid tumor immunosuppression, angiogenesis and cell growth stimulation. The tissue distribution of this gene in cells of the immune system indicates that polypeptides and polynucleotides corresponding to Gene NO: 17 are useful for treatment, prophylaxis and diagnosis of immune and autoimmune diseases, such as lupus, transplant rejection, allergic reactions, arthritis, asthma, immunodeficiency diseases, leukemia, and AIDS. Its expression predominantly in hematopoietic cells also indicates that the gene could be important for the treatment and/or detection of hematopoietic disorders such as graft versus host reaction, graft versus host disease, transplant rejection, myelogenous leukemia, bone marrow fibrosis, and myeloproliferative disease. The protein can also be used to enhance or protect proliferation, differentiation and functional activation of hematopoietic progenitor cells such as bone marrow cells, which could be useful for cancer patients undergoing chemotherapy or patients undergoing bone marrow transplantation. The protein may also be useful to increase the proliferation of peripheral blood leukocytes, which could be useful in the combat of a range of hematopoietic disorders including immunodeficiency diseases, leukemia, and septicemia.

Preferred epitopes include those comprising a sequence shown in SEQ ID NO: 150 as residues: Val-131 to Asn-136.

### FEATURES OF PROTEIN ENCODED BY GENE NO: 18

The translation product of Gene NO: 18 shares sequence homology with immunoglobulin, which is thought to be important in immunoreactions.

Gene NO: 18 is expressed primarily in macrophage.

Therefore, polynucleotides or polypeptides of the invention are useful as reagents for differential identification of the tissue(s) or cell type(s) present in a biological sample and for diagnosis of diseases and conditions which include, but are not limited to, inflammation. Similarly, polypeptides and antibodies directed to these polypeptides are useful in providing immunological probes for differential identification of the tissue(s) or cell type(s). For a number of disorders of the above tissues or cells, particularly of the immune system. expression of this gene at significantly higher or lower levels may routinely be detected in certain tissues and cell types (e.g., macrophage and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in macrophages and the weak homology to immunoglobin indicates that polypeptides and polynucleotides corresponding to Gene NO: 18 are useful for diagnosing and treating immune response disorders, including inflammation, antigen presentation and immunosurveillance.

### FEATURES OF PROTEIN ENCODED BY GENE NO: 19

The translation product of Gene NO: 19 shares sequence homology with proline rich proteins which are thought to be important in protein-protein interaction.

This gene has a wide range of tissue distribution, but is expressed primarily in normal prostate, synovial fibroblasts, brain amygdala depression, fetal bone and fetal cochlea, and to a lesser extent in adult retina, umbilical vein endothelial cells, atrophic endometrium, osteoclastoma, melanocytes, pancreatic carcinoma and smooth muscle.

Therefore, polynucleotides or polypeptides of the invention are useful as reagents for differential identification of the tissue(s) or cell type(s) present in a biological sample and for diagnosis of diseases and conditions which include, but are not limited to, cancer metastasis, wound healing, tissue repair. Similarly, polypeptides and antibodies directed to these polypeptides are useful in providing immunological probes for differential identification of the tissue(s) or cell type(s). For a number of disorders of the above tissues or cells, particularly of the skeletal, connective tissues, reproductive and central nervous system, expression of this gene at significantly higher or lower levels may routinely be detected in certain tissues and cell types (e.g., brain. prostrate, fibroblasts, bone, cochlea, retina, endothelial cells, endometrium, pancreas and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to proline-rich proteins indicates that the protein is a extracellular matrix protein or an ingredient of bodily fluid. Polypeptides and polynucleotides corresponding to Gene NO: 19 are useful for cancer metastasis intervention, tissue culture additive, bone modeling, wound healing and tissue repair.

### FEATURES OF PROTEIN ENCODED BY GENE NO: 20

Gene NO: 20 is expressed primarily in prostate cancer, leukocytes, meningima, adult liver, pancreas, brain, and to a lesser extent in lung.

Therefore, polynucleotides or polypeptides of the invention are useful as reagents for differential identification of the tissue(s) or cell type(s) present in a biological sample and for diagnosis of diseases and conditions which include, but are not limited to, prostate cancers. Similarly, polypeptides and antibodies directed to these polypeptides are useful in providing immunological probes for differential identification of the tissue(s) or cell type(s). For a number of disorders of the above tissues or cells, particularly of the prostate and brain, expression of this gene at significantly higher or lower levels may routinely be detected in certain tissues and cell types (e.g., prostate, leukocytes, memingima, liver, brain, pancreas and lung, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

Prostate cancer cell lines are known to be responsive to estrogen and androgen. The protein expression of Gene NO: 20 appears to be influenced by both estrogen and androgen levels. The prostate cancer tissue distribution indicates that polypeptides and polynucleotides corresponding to Gene NO: 20 are is useful in the intervention and detection of prostate hyperplasia and prostate cancer.

### FEATURES OF PROTEIN ENCODED BY GENE NO: 21

The translation product of Gene NO: 21 is identical to the human wnt-7a gene. Wnt-7a is a secreted signaling molecule, thought to be important in signaling and the regulation of cell fate and pattern formation during embryogenesis. Specifically, knock out studies in mice have demonstrated that wnt7a plays a critical role in the development of the dorsal-ventral patterning in the developing limb, and to a lesser extent plays a role in the development of anterior-posterior patterning. Overexpression of wnt7a can induce transformation of cultured mammary cells, suggesting that it is an oncogene.

Expression of Gene NO: 21 has only been observed in testes.

Therefore, polynucleotides or polypeptides of the invention are useful as reagents for differential identification of the tissue(s) or cell type(s) present in a biological sample and for diagnosis of diseases and conditions which include, but are not limited to, testicular cancer; abnormal limb development. Similarly, polypeptides and antibodies directed to these polypeptides are useful in providing immunological probes for differential identification of the testes or developing embryo. For a number of disorders of the above tissues or cells, particularly of the developing embryo, expression of this gene at significantly higher or lower levels may routinely be detected in the developing embryo or amniotic fluid taken from a pregnant individual and compared relative to the standard gene expression level, i.e., the expression level in healthy tissue or bodily fluid from an individual not having the disorder. Also, expression of this gene at significantly higher or lower levels may routinely be detected in the testes of patient suffering from testicular cancer and compared relative to the standard gene expression level, i.e., the expression level in healthy tissue or bodily fluid from an individual not having the disorder.

The tissue distribution and homology to mouse wnt7a indicates that polypeptides and polynucleotides corresponding to Gene NO: 21 are useful to restore abnormal limb development in an affected individual. Furthermore, its oncogenic potential and tissue distribution indicates that it could serve as a diagnostic for testicular cancer.

Preferred epitopes include those comprising a sequence shown in SEQ ID NO: 154 as residues: Gly-22 to Arg-28.

### FEATURES OF PROTEIN ENCODED BY GENE NO: 22

Gene NO: 22 is expressed primarily in fetal liver/spleen, breast, testes and placenta and to a lesser extent in brain, and a series of cancer tissues.

Therefore, polynucleotides or polypeptides of the invention are useful as reagents for differential identification of the tissue(s) or cell type(s) present in a biological sample and for diagnosis of diseases and conditions which include, but are not limited to, immune disorders, brain diseases, male infertility, and disposition to pregnant miscarriages. Similarly, polypeptides and antibodies directed to these polypeptides are useful in providing immunological probes for differential identification of the tissue(s) or cell type(s). For a number of disorders of the above tissues or cells, particularly of the immune system, hematopoietic system, and sexual organs, expression of this gene at significantly higher or lower levels may routinely be detected in certain tissues (e.g., liver, spleen, testes, placenta. and brain, and cancerous and wounded tissues) or bodily fluids (e.g., serum, plasma, urine, synovial fluid or spinal fluid) or another tissue or cell sample taken from an individual having such a disorder, relative to the standard gene expression level, i.e.. the expression level in healthy tissue or bodily fluid from an individual not having the disorder.

The tissue distribution of this gene indicates that polypeptides and polynucleotides corresponding to Gene NO: 22 are useful as a marker for non-differentiated, dividing cells and hence could serve as an oncogenic marker. Its high expression in fetal liver, suggests an involvement in hematopoiesis and/or the immune system. Hence it is useful as a factor to enhance an individuals immune system, e.g., in individuals with immune disorders. It is also thought to affect the survival, proliferation, and differentiation of a number of hematopoietic cell lineages, including hematopoietic stem cells. Its disruption, e.g., mutation or altered expression, may also be a marker of immune disorder. Its expression in the testes, suggests it may be important in controlling male fertility. Expression of this gene in breast further reflects a role in immune function and immune surveillance (breast lymph node). This gene is believed to be useful as a marker for breast cancer.

Preferred epitopes include those comprising a sequence shown in SEQ ID NO: 155 as residues: Gln-57 to Lys-70 and Ala-91 to Pro-100.

### FEATURES OF PROTEIN ENCODED BY GENE NO: 23

Gene NO: 23 is expressed primarily in bone marrow and brain (whole and fetal).

Therefore, polynucleotides or polypeptides of the invention are useful as reagents for differential identification of the tissue(s) or cell type(s) present in a biological sample and for diagnosis of diseases and conditions which include, but are not limited to, neurological, immune and hematopoietic disorders. Similarly, polypeptides and antibodies directed to these polypeptides are useful in providing immunological probes for differential identification of the tissue(s) or cell type(s). For a number of disorders of the above tissues or cells, particularly of the central nervous and hematopoietic systems, expression of this gene at significantly higher or lower levels may routinely be detected in certain tissues (e.g., bone marrow, brain, and cancerous and wounded tissues) or bodily fluids (e.g., serum, plasma, urine, synovial fluid or spinal fluid) or another tissue or cell sample taken from an individual having such a disorder, relative to the standard gene expression level. i.e., the expression level in healthy tissue or bodily fluid from an individual not having the disorder.

The tissue distribution indicates that polypeptides and polynucleotides corresponding to Gene NO: 23 are useful in the diagnosis and treatment of disorders related to the central nervous system (e.g. neuro-degenerative conditions, trauma, and behavior abnormalities) and hematopoiesis. In addition, the expression in fetal brain indicates a role for this gene product in diagnosis of predisposition to developmental defects of the brain.

Preferred epitopes include those comprising a sequence shown in SEQ ID NO: 156 as residues: Thr-23 to Tyr-29.

### FEATURES OF PROTEIN ENCODED BY GENE NO: 24

Gene NO: 24 is expressed primarily in smooth muscle, placenta, prostate, and osteoblasts.

Therefore, polynucleotides or polypeptides of the invention are useful as reagents for differential identification of the tissue(s) or cell type(s) present in a biological sample and for diagnosis of diseases and conditions which include, but are not limited to, cardiovascular pathologies. Similarly, polypeptides and antibodies directed to these polypeptides are useful in providing immunological probes for differential identification of the tissue(s) or cell type(s). For a number of disorders of the above tissues or cells, particularly of the cardiovascular, reproductive and skeletal systems, expression of this gene at significantly higher or lower levels may routinely be detected in certain tissues and cell types (e.g., placenta, smooth muscle, prostrate, and osteoblasts, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 24 are useful for detection and treatment of neoplasias and developmental abnormalities associated with these tissues.

Preferred epitopes include those comprising a sequence shown in SEQ ID NO: 157 as residues: Asn-21 to Thr-26.

### FEATURES OF PROTEIN ENCODED BY GENE NO: 25

The translation product of Gene NO: 25 shares sequence homology with Pregnancy Associated Mouse Protein (PAMP)-1. (See. FEBS Lett 1993 May 17;322(3):219-222). Based on the sequence similarity the translation product of this gene is expected to share certain biological activities with PAMP-1.

Gene NO: 25 is expressed primarily in 12-week-old human embryos and prostate.

Therefore, polynucleotides or polypeptides of the invention are useful as reagents for differential identification of the tissue(s) or cell type(s) present in a biological sample and for diagnosis of diseases and conditions which include, but are not limited to, prostate disorders (cancer). Similarly, polypeptides and antibodies directed to these polypeptides are useful in providing immunological probes for differential identification of the tissue(s) or cell type(s). For a number of disorders of the above tissues or cells, particularly of the prostate, expression of this gene at significantly higher or lower levels may routinely be detected in certain tissues (e.g., embryonic tissue, and prostate, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 25 are useful for the diagnosis and treatment of prostate disorders (such as cancer) and developmental abnormalities and fetal deficiencies. The homology to PAMP-1 indicates that this gene and gene product are useful in detecting pregnancy.

Preferred epitopes include those comprising a sequence shown in SEQ ID NO: 158 as residues: Pro-23 to Glu-28 and Ser-44 to Gly-55.

### FEATURES OF PROTEIN ENCODED BY GENE NO: 26

Gene NO: 26 is expressed primarily in testes and to a lesser extent in epididymis.

Therefore, polynucleotides or polypeptides of the invention are useful as reagents for differential identification of the tissue(s) or cell type(s) present in a biological sample and for diagnosis of diseases and conditions which include, but are not limited to, reproductive and endocrine disorders. as well as testicular cancer. Similarly, polypeptides and antibodies directed to these polypeptides are useful in providing immunological probes for differential identification of the tissue(s) or cell type(s). For a number of disorders of the above tissues or cells, particularly of the male reproductive and endocrine systems, expression of this gene at significantly higher or lower levels may routinely be detected in certain tissues (e.g., testes, and epididymis, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 26 are useful for the treatment and diagnosis of conditions concerning proper testicular function (e.g., endocrine function, sperm maturation), as well as cancer. Therefore, this gene product is useful in the treatment of male infertility and/or impotence. This gene product is also useful in assays designed to identify binding agents as such agents (antagonists) are useful as male contraceptive agents. Similarly, the protein is believed to by useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications.

Preferred epitopes include those comprising a sequence shown in SEQ ID NO: 159 as residues: Pro-24 to Gly-33 and Arg-70 to Gly-76.

### FEATURES OF PROTEIN ENCODED BY GENE NO: 27

The translation product of Gene NO: 27 shares sequence homology with salivary protein precursors which are thought to be important in immune response and production of secreted proteins.

Gene NO: 27 is expressed primarily in salivary gland tissue.

Therefore, polynucleotides or polypeptides of the invention are useful as reagents for differential identification of the tissue(s) or cell type(s) present in a biological sample and for diagnosis of diseases and conditions which include, but are not limited to, immune disorders, diseases of the salivary gland. Similarly. polypeptides and antibodies directed to these polypeptides are useful in providing immunological probes for differential identification of the tissue(s) or cell type(s). For a number of disorders of the above tissues or cells, particularly of the immune system, digestive system, expression of this gene at significantly higher or lower levels may routinely be detected in certain tissues (e.g., salivary gland, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to salivary secreted protein indicates that polypeptides and polynucleotides corresponding to Gene NO: 27 are useful for treatment of immune disorders and diagnostic uses related to secretion of protein in disease states. For example, the gene product can be used as an anti-microbial agent, an ingredient for oral or dental hygiene, treatment of xerostomia, sialorrhea, intervention for inflammation including parotitis, and an indication for tumors in the salivary gland (adenomas, carcinomas).

Preferred epitopes include those comprising a sequence shown in SEQ ID NO: 160 as residues: Asp-21 to Gly-28, Asp-30 to Glu-43, Glu-49 to Glu-62, and Thr-75 to Pro-83.

### FEATURES OF PROTEIN ENCODED BY GENE NO: 28

Gene NO: 28 is expressed primarily in human fetal heart tissue and to a lesser extent in olfactory tissue.

Therefore, polynucleotides or polypeptides of the invention are useful as reagents for differential identification of the tissue(s) or cell type(s) present in a biological sample and for diagnosis of diseases and conditions which include, but are not limited to, immune, olfactory and cardiovascular disorders. Similarly, polypeptides and antibodies directed to these polypeptides are useful in providing immunological probes for differential identification of the tissue(s) or cell type(s). For a number of disorders of the above tissues or cells, particularly of the immune, olfactory and vascular systems, expression of this gene at significantly higher or lower levels may routinely be detected in certain tissues (e.g., olfactory tissue, and heart, and cancerous and wounded tissues) or bodily fluids (e.g., serum, plasma, urine, synovial fluid or spinal fluid) or another tissue or cell sample taken from an individual having such a disorder, relative to the standard gene expression level, i.e.. the expression level in healthy tissue or bodily fluid from an individual not having the disorder.

The tissue distribution indicates that polypeptides and polynucleotides corresponding to Gene NO: 28 are useful for diagnosis and treatment of immune, olfactory and vascular disorders.

Preferred epitopes include those comprising a sequence shown in SEQ ID NO: 161 as residues: Cys-33 to Gly-44, Arg-71 to Arg-78, Ser-130 to Gly-142, Lys-150 to Gly-157, and Thr-159 to Asp-177.

### FEATURES OF PROTEIN ENCODED BY GENE NO: 29

Gene NO: 29 is expressed primarily in brain and to a lesser degree in activated macrophages, endothelial and smooth muscle cells, and some bone cancers.

Therefore, polynucleotides or polypeptides of the invention are useful as reagents for differential identification of brain and endothelial present in a biological sample and for diagnosis of diseases and conditions which include, but are not limited to, neurodegeneration, inflammation and other immune disorders, fibrotic conditions. Similarly, polypeptides and antibodies directed to these polypeptides are useful in providing immunological probes for differential identification brain, smooth muscle, and endothelium. For a number of disorders of the above tissues or cells, particularly of the brain and endothelium, expression of this gene at significantly higher or lower levels may routinely be detected in certain tissues or cell types (e.g., brain, endothelial cells, macrophages, smooth muscle, and bone, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

Tissue distribution suggests polypeptides and polynucleotides corresponding to Gene NO: 29 are useful in study and treatment of neurodegenerative and immune disorders.

Preferred epitopes include those comprising a sequence shown in SEQ ID NO: 162 as residues: Asn-18 to Glu-20, Ser-33 to Gln-48, Cys-55 to Ser-56, Pro-67 to Cys-69.

### FEATURES OF PROTEIN ENCODED BY GENE NO: 30

Gene NO: 30 is expressed primarily in early stage human brain and to a lesser extent in cord blood, heart, and some tumors.

Therefore, polynucleotides or polypeptides of the invention are useful as reagents for differential identification of developing CNS tissue present in a biological sample and for diagnosis of diseases and conditions which include, but are not limited to, cardiovascular and neurodegenerative disorders. Similarly, polypeptides and antibodies directed to these polypeptides are useful in providing immunological probes for differential identification of the tissue(s) or cell type(s). For a number of disorders of the above tissues or cells, particularly of the nervous and immune systems, expression of this gene at significantly higher or lower levels may routinely be detected in certain tissues (e.g., brain and heart, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that that polypeptides and polynucleotides corresponding to Gene NO: 30 are useful for the treatment of cancer and of neurodegenerative and cognitive disorders.

### FEATURES OF PROTEIN ENCODED BY GENE NO: 31

Gene NO: 31 is expressed primarily in brain and thymus and to a lesser extent in several other organs and tissues including the hematopoictic system, liver skin and bone

Therefore, polynucleotides or polypeptides of the invention are useful as reagents for differential identification of the tissue(s) or cell type(s) present in a biological sample and for diagnosis of diseases and conditions which include, but are not limited to, CNS disorders, hematopoietic system disorders, disorders of the endocrine system, bone, and skin. Similarly, polypeptides and antibodies directed to these polypeptides are useful in providing immunological probes for differential identification of the tissue(s) or cell type(s). For a number of disorders of the above tissues or cells, particularly CNS disorders, hematopoietic system disorders, disorders of the endocrine system, bone, and skin, expression of this gene at significantly higher or lower levels may routinely be detected in certain tissues and cell types (e.g., hematopoietic cells, brain, thymus, liver, bone, and epidermis, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 31 are useful for treatment and diagnosis of CNS disorders, hematopoietic system disorders, disorders of the endocrine system, and of bone and skin.

Preferred epitopes include those comprising a sequence shown in SEQ ID NO: 164 as residues: Thr-35 to Arg-40, Pro-55 to His-75, Pro-93 to Ala-98, Ala-111 to Pro-119, and Pro-132 to Glu-138.

### FEATURES OF PROTEIN ENCODED BY GENE NO: 32

Gene NO: 32 is expressed primarily in organs and tissue of the nervous system and to a lesser extent in various developing tissues and organs.

Therefore, polynucleotides or polypeptides of the invention are useful as reagents for differential identification of the tissue(s) or cell type(s) present in a biological sample and for diagnosis of diseases and conditions which include, but are not limited to, disorders of the central nervous system and disorders of developing and growing tissues and organs. Similarly, polypeptides and antibodies directed to these polypeptides are useful in providing immunological probes for differential identification of the tissue(s) or cell type(s). For a number of disorders of the above tissues or cells, particularly disorders of the CNS, expression of this gene at significantly higher or lower levels may routinely be detected in certain tissues (e.g., tissue of the nervous system and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 32 are useful for diagnosis and treatment of disorders of the central nervous system, general neurological diseases and neoplasias.

Preferred epitopes include those comprising a sequence shown in SEQ ID NO: 165 as residues: Ser-33 to Lys-41 and Glu-86 to Glu-91.

### FEATURES OF PROTEIN ENCODED BY GENE NO: 33

Residues 141-156 in the translation product for Gene NO: 33 as shown in the sequence listing matches phosphopantetheine binding site motifs. Phosphopantetheine (or pantetheine 4' phosphate) is the prosthetic group of acyl carrier proteins (ACP) in some multienzyme complexes where it serves as a 'swinging arm' for the attachment of activated fatty acid and amino-acid groups. Phosphopantetheine is attached to a serine residue in these proteins. ACP proteins or domains have been found in various enzyme systems which are listed below. Fatty acid synthetase (FAS), which catalyzes the formation of long-chain fatty acids from acetyl-CoA, malonyl-CoA and NADPH. Bacterial and plant chloroplast FAS are composed of eight separate subunits which correspond to the different enzymatic activities; ACP is one of these polypeptides. Fungal FAS consists of two multifunctional proteins. FAS 1 and FAS2; the ACP domain is located in the N-terminal section of FAS2. Vertebrate FAS consists of a single multifunctional enzyme; the ACP domain is located between the beta-ketoacyl reductase domain and the C-terminal thioesterase domain. Based on the presence of a phosphopantetheine binding site in the translation product of this gene, it is believed to share activities fatty acid synthetase polypeptides. Such activities may be assayed by methods known in the art.

This gene is expressed primarily in developing and rapidly growing tissues like placenta fetal heart and endometrial tumor and to a lesser extent in B and T cell lymphoma tissues

Therefore, polynucleotides or polypeptides of the invention are useful as reagents for differential identification of the tissue(s) or cell type(s) present in a biological sample and for diagnosis of diseases and conditions which include, but are not limited to, cancer and disorders of developing tissues and organs. Similarly, polypeptides and antibodies directed to these polypeptides are useful in providing immunological probes for differential identification of the tissue(s) or cell type(s). For a number of disorders of the above tissues or cells, particularly of the hematopoietic tissues and developing organs and tissues, expression of this gene at significantly higher or lower levels may routinely be detected in certain tissues and cell types (e.g., embryonic tissue, endometrium, B-cells, and T-cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 33 are useful for treatment and diagnosis of cancer in the hematopoietic system developing organs and tissues. It may also be useful for induction of cell growth in disorders of the hematopoietic system and other tissue and organs. The homology to fatty acid synthetases indicates that this gene product is useful in the diagnosis and treatment of lipid metabolism disorders such as hyperlipidemia.

Preferred epitopes include those comprising a sequence shown in SEQ ID NO: 166 as residues: Arg-27 to Glu-34.

### FEATURES OF PROTEIN ENCODED BY GENE NO: 34

Gene NO: 34 is expressed primarily in breast and testes tissues and to a lesser extent in hematopoietic tissues including tonsils, T cells and monocytes.

Therefore, polynucleotides or polypeptides of the invention are useful as reagents for differential identification of the tissue(s) or cell type(s) present in a biological sample and for diagnosis of diseases and conditions which include, but are not limited to, diseases of the reproductive organs and systems, including cancer, autoimmune diseases and inflammatory diseases. Similarly, polypeptides and antibodies directed to these polypeptides are useful in providing immunological probes for differential identification of the tissue(s) or cell type(s). For a number of disorders of the above tissues or cells, particularly of the reproductive organs and hematopoietic tissues, expression of this gene at significantly higher or lower levels may routinely he detected in certain tissues and cell types (e.g., hemotopoietic cells. T-cells and monocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Nucleic acids comprising sequence of this gene are also useful as chromosome markers since this gene maps to Chr.15, D15S118-D15S123.

The tissue distribution indicates that polypeptides and polynucleotides corresponding to Gene NO: 34 are useful for treatment of diseases of the reproductive organs and hematopoietic system including cancer, autoimmune diseases and inflammatory diseases .

Preferred epitopes include those comprising a sequence shown in SEQ ID NO: 167 as residues: Phe-81 to Lys-86.

### FEATURES OF PROTEIN ENCODED BY GENE NO: 35

The translation product of Gene NO: 35 shares sequence similarity with the mouse cytokine-inducible inhibitor of signaling. See, e.g., Nature 1997 Jun 26;387(6636):917-921. Cytokines are secreted proteins that regulate important cellular responses such as proliferation and differentiation. Key events in cytokine signal transduction are well defined: cytokines induce receptor aggregation, leading to activation of members of the JAK family of cytoplasmic tyrosine kinases. In turn, members of the STAT family of transcription factors are phosphorylated, dimerize and increase the transcription of genes with STAT recognition sites in their promoters. Less is known of how cytokine signal transduction is switched off. Expression of the mouse SOCS-1 protein inhibited both interleukin-6- induced receptor phosphorylation and STAT activation. We have also cloned two relatives of SOCS-1, named SOCS-2 and SOCS-3, which together with the previously described CIS form a new family of proteins. Transcription of all four SOCS genes is increased rapidly in response to interleukin-6. in vitro and in vivo, suggesting they may act in a classic negative feedback loop to regulate cytokine signal transduction. The translation product of this gene is believed to have similar biological activities as this family of mouse genes. The biological activity of the translation product of this gene may be assayed by methods shown in Nature 1997 Jun 26:387(6636): 917-921, which is incorporated herein by reference in its entirety.

Gene NO: 35 is expressed primarily in tissues of hematopoietic origin including activated monocytes, neutrophils, activated T-cells and to a lesser extent in breast, adipose tissue and dendritic cells.

Therefore, polynucleotides or polypeptides of the invention are useful as reagents for differential identification of the tissue(s) or cell type(s) present in a biological sample and for diagnosis of diseases and conditions which include, but are not limited to, diseases of the hematopoietic system including cancer autoimmune diseases and inflammatory diseases. Similarly, polypeptides and antibodies directed to these polypeptides are useful in providing immunological probes for differential identification of the tissue(s) or cell type(s). For a number of disorders of the above tissues or cells, particularly of the hematopoietic system expression of this gene at significantly higher or lower levels may routinely be detected in certain tissues and cell types (e.g., hematopoietic cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to cytokine inducible inhibitor of signaling indicates that polypeptides and polynucleotides corresponding to Gene NO: 35 are useful for diagnosis and treatment of diseases of the hematopoietic system including autoimmune diseases, inflammatory diseases, infectious diseases and neoplasia. For example, administration of, or upregulation of this gene could by used to decrease the response of immune-system to lymphokines and cytokines.

Preferred epitopes include those comprising a sequence shown in SEQ ID NO: 168 as residues: Arg-23 to His-30, Ala-35 to Gly-42.

### FEATURES OF PROTEIN ENCODED BY GENE NO: 36

Gene NO: 36 is expressed primarily in infant brain and to a lesser extent in osteoclastoma, placenta, and a wide variety of other tissues.

Therefore, polynucleotides or polypeptides of the invention are useful as reagents for differential identification of the tissue(s) or cell type(s) present in a biological sample and for diagnosis of diseases and conditions which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may routinely be detected in certain tissues and cell types (e.g., osteoclastoma, placenta, and tissue of the central nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 36 are useful for diagnosis and treatment of neurologic disorders.

Preferred epitopes include those comprising a sequence shown in SEQ ID NO: 169 as residues: Gln-31 to Ser-37, He-49 to Gly-54, Tyr-57 to Asp-67, Gln-141 to Pro-151, and Val-207 to Thr-219.

### FEATURES OF PROTEIN ENCODED BY GENE NO: 37

Gene NO: 37 is expressed primarily in osteoclastoma stromal cells, dendritic cells, liver, and placenta.

Therefore, polynucleotides or polypeptides of the invention are useful as reagents for differential identification of the tissue(s) or cell type(s) present in a biological sample and for diagnosis of diseases and conditions which include, but are not limited to, cancer, wound, pathological conditions. Similarly, polypeptides and antibodies directed to these polypeptides are useful in providing immunological probes for differential identification of the tissue(s) or cell type(s). For a number of disorders of the above tissues or cells, expression of this gene at significantly higher or lower levels may routinely be detected in certain tissues or cell types (e.g., stromal cells, dendritic cells, liver, and placenta and, cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 37 are useful for fundamental role in basic growth and development of human.

Preferred epitopes include those comprising a sequence shown in SEQ ID NO: 170 as residues: Leu-32 to Thr-37 and Arg-48 to Pro-55.

### FEATURES OF PROTEIN ENCODED BY GENE NO: 38

The translation product of Gene NO: 38 shares sequence homology with a yeast protein, Lpe10p, which may be involved in mRNA processing. (See Accession Nos. 2104457 and 1079682.) It is likely that an upstream signal sequence exists, other than the predicted sequence described in Table 1. Preferred polypeptide fragments comprise the open reading frame upstream from the predicted signal sequence, as well as polynucleotide fragments encoding these polypeptide fragments.

This gene is expressed primarily in skin, and to a lesser extent in embryonic tissues, and fetal liver.

Therefore, polynucleotides or polypeptides of the invention are useful as reagents for differential identification of the tissue(s) or cell type(s) present in a biological sample and for diagnosis of diseases and conditions which include, but are not limited to, defects of the skin. Similarly, polypeptides and antibodies directed to these polypeptides are useful in providing immunological probes for differential identification of the tissue(s) or cell type(s). For a number of disorders of the above tissues or cells, particularly of the skin, expression of this gene at significantly higher or lower levels may routinely be detected in certain tissues (e.g., epidermis, liver, and embryanic tissues, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 38 are useful for diagnosis and treatment of defects of the skin.

### FEATURES OF PROTEIN ENCODED BY GENE NO: 39

Gene NO: 39 is expressed primarily in Amygdala, activated monocytes, testis, and fetal liver. Moreover, this gene is mapped to chromosome 4. Thus, polynucleotides of the present invention can be used in linkage analysis as markers for chromosome 4.

Therefore, polynucleotides and polypeptides of the invention are useful as reagents for differential identification of the tissue(s) or cell type(s) present in a biological sample and for diagnosis of diseases and conditions which include, but are not limited to, defects of the brain, immune system and testis. Similarly, polypeptides and antibodies directed to these polypeptides are useful in providing immunological probes for differential identification of the tissue(s) or cell type(s). For a number of disorders of the above tissues or cells, particularly of the brain, immune system and testis, expression of this gene at significantly higher or lower levels may routinely be detected in certain tissues and cell types (e.g., Amygdala, monocytes, testes, and liver and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 39 are useful for detecting defects of the brain, immune system and testis because of its abundance in these tissues.

### FEATURES OF PROTEIN ENCODED BY GENE NO: 40

The translation product of Gene NO: 40 shares sequence homology with lymphoma 3-encoded protein (bcl-3) which is thought to contribute to leukemogenesis when abnormally expressed.

This gene is expressed primarily in Human Neutrophils, and to a lesser extent in Human Osteoclastoma Stromal Cells (unamplified), Hepatocellular Tumor, and Human Neutrophils, (Activated).

Therefore, polynucleotides or polypeptides of the invention are useful as reagents for differential identification of the tissue(s) or cell type(s) present in a biological sample and for diagnosis of diseases and conditions which include, but are not limited to, chronic lymphocytic leukemia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., neutrophils, osteoclastoma, and kidney, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to lymphoma 3-encoded protein (bcl-3) indicates that polypeptides and polynucleotides corresponding to Gene NO: 40 are useful for treatment of lymphoma and related cancers.

### FEATURES OF PROTEIN ENCODED BY GENE NO: 41

Gene NO: 41 is expressed primarily in ovary tumor, and to a lesser extent in endometrial stromal cells and fetal brain.

Therefore, polynucleotides or polypeptides of the invention are useful as reagents for differential identification of the tissue(s) or cell type(s) present in a biological sample and for diagnosis of diseases and conditions which include, but are not limited to, ovarian or endometrial cancer. Similarly, polypeptides and antibodies directed to these polypeptides are useful in providing immunological probes for differential identification of the tissue(s) or cell type(s). For a number of disorders of the above tissues or cells, particularly of the female reproductive system and the developing central nervous system, expression of this gene at significantly higher or lower levels may routinely be detected in certain tissues (e.g., ovary, endometrium and brain, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 41 are useful for development of factors involved in ovarian or endometrial and general reproductive organ disorders.

Preferred epitopes include those comprising a sequence shown in SEQ ID NO: 174 as residues: Glu-22 to Trp-31, Asn-84 to Asp-90, and Ser-144 to Asp-151.

### FEATURES OF PROTEIN ENCODED BY GENE NO: 42

The translation product of Gene 42 has sequence identity with a gene designated PTHrP(B). The PTHrP(B) polypeptide inhibits parathyroid hormone related peptide (PTHrP) activity.

This gene is expressed primarily in adult testis, and to a lesser extent in pituitary.

Therefore polynucleotides or polypeptides of the invention are useful as reagents for differential identification of the tissue(s) or cell type(s) present in a biological sample and for diagnosis of male reproductive disorders. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may routinely be detected in certain tissues (e.g., testes, and pituitary, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Furthermore, based in part on sequence identity with PTHrP(B), nucleic acids and polypeptides of the present invention may be used to diagnose or treat such conditions as hypercalcemia, osteoporosis, and disorders related to calcium metabolism.

The tissue distribution indicates that polypeptides and polynucleotides corresponding to Gene NO: 42 are useful for treatment of male reproductive disorders, hypercalcemia, osteoporosis. and other disorders related to calcium metabolism.

Preferred epitopes include those comprising a sequence shown in SEQ ID NO: 175 as residues: Tyr-81 to Met-86, Gly-103 to Scr-108, Glu-127 to Pro-128, Pro-175 to Ser-180, Glu-196 to Lys-203, Pro-235 to Ser-241, and Ala-249 to Ser-264.

### FEATURES OF PROTEIN ENCODED BY GENE NO: 43

The translation product of Gene NO: 43 shares sequence homology with brevican, which is thought to be important as a proteoglycan core protein of the aggrecan/versican family. The translation product of this gene may also contain a hyaluronan (HA)-binding region domain in frame with, but downstream of, the predicted open reading frame (Barta, et al., Biochem. J. 292:947-949 (1993)). The HA-binding domain, also termed the link domain, is found in proteins of vertebrates that are involved in the assembly of extracellular matrix, cell adhesion, and migration. It is about 100 amino acids in length. The structure has been shown to consist of two alpha helices and two antiparallel beta sheets arranged around a large hydrophobic core similar to that of C-type lectin. This domain typically contains four conserved cysteines involved in two disulfide bonds.

This gene is expressed primarily in early stage human brain and to a lesser extent in frontal cortex and epileptic tissues.

Therefore, polynucleotides or polypeptides of the invention are useful as reagents for differential identification of the tissue(s) or cell type(s) present in a biological sample and for diagnosis of disorders associated with, or observed during, neuronal development. Similarly, polypeptides and antibodies directed to these polypeptides are useful as immunological probes for differential identification of neuronal and associated tissues and cell types. For a number of disorders of the above tissues or cells, particularly for those of the nervous system, expression of this gene at significantly higher or lower levels may routinely be detected in certain tissues (e.g., brain and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to brevican indicates that polypeptides and polynucleotides corresponding to Gene NO: 43 are useful for neuronal regulation and signaling. The uses include directing or inhibiting axonal growth for the treatment of neuro-fibromatosis and in detection of glioses.

Preferred epitopes include those comprising a sequence shown in SEQ ID NO: 176 as residues: Asp-28 to Arg-33 and Arg-126 to Arg-131.

### FEATURES OF PROTEIN ENCODED BY GENE NO: 44

Gene NO: 44 is the human homolog of Notch-2 (Accession No. 477495) and mouse EGF repeat transmembrane protein (Accession No. 1336628), both genes are important in differentiation and development of an organism. The EGF repeat transmembrane protein is regulated by insulin like growth factor Type I receptor. These proteins are involved in cell-cell signaling and cell fate determination. Based on homology, it is likely that this gene products also involved in cell differentiation and development. Although the predicted signal sequence is indicated in Table 1, it is likely that a second signal sequence is located further upstream. Moreover, further translated coding regions are likely found downstream from the disclosed sequence, which can easily be obtained using standard molecular biology techniques. A frameshift occurs somewhere around nucleotide 714, causing a frame shift in amino acid sequence from frame +2 to frame +3. However, using the homology of Notch-2 and EGF repeat transmembrane protein, the complete open reading frame can be elucidated. Preferred polynucleotide fragments comprise nucleotides 146-715, 281-715, and 714-965. Other preferred polypeptide fragments comprise the following EGF-like motifs: CRCASGFTGEDC (SEQ ID NO:260), CTCQVGFTGKEC (SEQ ID NO:261), CLNLPGSYQCQC (SEQ ID NO:262), CKCLTGFTGQKC (SEQ ID NO:263), and

CQCLQGFTGQYC (SEQ ID NO:264).

Gene NO: 44 is expressed primarily in placenta and to a lesser extent in stromal and immune cells.

Therefore, polynucleotides and polypeptides of the invention are useful as reagents for differential identification of the tissue(s) or cell type(s) present in a biological sample and for diagnosis of diseases and conditions which include, but are not limited to, hemophelia and other blood disorders, central nervous system disorders, muscle disorders, and any other disorder resulting from abnormal development. Similarly, polypeptides and antibodies directed to these polypeptides are useful in providing immunological probes for differential identification of the tissue(s) or cell type(s). For a number of disorders of the above tissues or cells, particularly of the immune, hematopoietic and vascular systems, expression of this gene at significantly higher or lower levels may routinely be detected in certain tissues and cell types (e.g., placenta, stromal and immune cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Notch-2 indicates that polypeptides and polynucleotides corresponding to Gene NO: 44 are useful for diagnosing and treating disorders relating to abnormal regulation of cell fate, induction, and differentiation of cells (e.g., cancer), epidermal growth factors, axonal pathfinding, and hematopoiesis.

Preferred epitopes include those comprising a sequence shown in SEQ ID NO: 177 as residues: Gln-27 to Tyr-32, His-45 to Glu-55, Tyr-61 to Gly-77, Glu-99 to Ser-106, Ser-125 to Cys-131, and Thr-138 to Trp-144.

### FEATURES OF PROTEIN ENCODED BY GENE NO: 45

The translation product of this gene shares sequence homology with Laminin A which is thought to be important in the binding of epithelial cells to basement membrane and is associated with tumor invasion. Moreover, the translated protein is homologous to the *Drosophila* LAMA gene (Accession No. 1314864), a gene expressed in the first optic ganglion of *Drosophila.* Thus, it is likely that the gene product from this gene is involved in the development of the eye. Nucleotide fragments comprising nucleotides 822-1223, 212-475, 510-731, and 1677-1754 are preferred. Also preferred are the polypeptide fragments encoded by these polynucleotide fragments. It is likely that a frame shift occurs somewhere between nucleotides 475 to 510, shifting the open reading frame from +2 to +3. However, the open reading frame can be clarified using known molecular biology techniques.

This gene is expressed primarily in human testes tumor and to a lesser extent in placenta and activated monocytes.

Therefore, polynucleotides or polypeptides of the invention are useful as reagents for differential identification of the tissue(s) or cell type(s) present in a biological sample and for diagnosis of diseases and conditions which include, but are not limited to, invasive cancers or tumors of the epithelium, as well as disorders relating to eye development. Similarly, polypeptides and antibodies directed to these polypeptides are useful as immunological probes for differential identification of the tissue(s) or cell type(s). For a number of disorders of the above tissues or cells, particularly of neoplastic conditions, expression of this gene at significantly higher or lower levels may routinely be detected in certain tissues and cell types (e.g., testes, placenta, and monocytes and cancerous and wounded tissues) or bodily fluids (e.g., serum, plasma. urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Laminin A indicates that polypeptides and polynucleotides corresponding to Gene NO: 45 are useful for study and diagnosis of malignant or benign tumors, fibrotic disorders, and eye disorders.

Preferred epitopes include those comprising a sequence shown in SEQ ID NO: 178 as residues: Met-1 to Gly-8, Glu-32 to Ala-37, Met-113 to Asn-119, and Glu-139 to Gln-153.

### FEATURES OF PROTEIN ENCODED BY GENE NO: 46

The translation product of Gene NO: 46 is novel and shares sequence homology with the product of the *Drosophila* tissue polarity gene frizzled. In vertebrates, it appears that there is a family of proteins that represent frizzled gene homologs. (See, e.g., Accession Nos. 1946343 and AFO17989.) The *Drosophila* frizzled protein is thought to transmit polarity signals across the plasma membrane of epidermal cells. The structure of frizzled proteins suggest that they may function as a G-protein-coupled receptor. The frizzled proteins are thought to represent receptors for Wnt gene products - secreted proteins that control tissue differentiation and the development of embryonic and adult structures. Inappropriate expression of Wnts has also been demonstrated to contribute to tumor formation. Moreover, mammalian secreted frizzled related proteins are thought to regulate apoptosis. (See Accession No. AFO17989.) The human homolog has also been recently cloned by other groups. (See Accession No. H2415415.) Thus, the protein encoded by this gene plays a role in mediating tissue differentiation, proliferation, tumorigenesis and apoptosis. Preferred polypeptide fragments lack the signal sequence as described in Table 1, as well as N-terminal and C-terminal deletions. Preferred polynucleotide fragments encode these polypeptide fragments.

Gene NO: 46 is expressed primarily in fetal tissues - particularly fetal lung - and adult cancers, most notably pancreas tumor and Hodgkin's lymphoma. Together, this distribution is consistent with expression in tissues undergoing active proliferation. The gene is also expressed to a lesser extent in other organs, including stomach, prostate, and thymus.

Therefore. polynucleotides and polypeptides of the invention are useful as reagents for differential identification of the tissue(s) or cell type(s) present in a biological sample and for diagnosis of diseases and conditions which include, but are not limited to, cancer (particularly pancreatic cancer and/or Hodgkin's lymphoma). as well as other forms of aberrant cell proliferation. Similarly, polypeptides and antibodies directed to these polypeptides are useful in providing immunological probes for differential identification of the tissue(s) or cell type(s). For a number of disorders of the above tissues or cells, particularly of the immune system and hyperproliferative disorders, expression of this gene at significantly higher or lower levels may routinely be detected in certain tissues (e.g., fetal tissue, pancreas, and tissue of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to frizzled indicates that polypeptides and polynucleotides corresponding to Gene NO: 46 are useful for influencing cell proliferation, differentiation, and apoptosis. The full-length protein or a truncated domain could potentially bind to and regulate the function of specific factors, such as Wnt proteins or other apoptotic genes, and thereby inhibit uncontrolled cellular proliferation. Expression of this protein within a cancer - such as via gene therapy or systemic administration - could effect a switch from proliferation to differentiation, thereby arresting the progression of the cancer.

Preferred epitopes include those comprising a sequence shown in SEQ ID NO: 179 as residues: Pro-31 to Arg-37.

### FEATURES OF PROTEIN ENCODED BY GENE NO: 47

The translation product of Gene NO: 47 shares sequence homology with members of the Rh/T2/S-glycoprotein family of ribonuclease-encoding genes. These ribonuclease proteins are found predominantly in fungi, plants, and bacteria and have been implicated in a number of functions, including phosphate-starvation response. self-incompatibility, and responses to wounding. A second group has recently cloned this same gene, calling it a ribonuclease 6 precursor. (See Accession No. 2209029.) This group also mapped the gene to chromosome 6, thus, the polynucleotides of the present invention can be used in linkage analysis as a marker for chromosome 6.

Gene NO: 47 is expressed primarily in hematopoietic cells and tissues, including macrophages, eosinophils, CD34 positive cells, T-cells, and spleen. It is also expressed to a lesser extent in brain and spinal cord.

Therefore, polynucleotides and polypeptides of the invention are useful as reagents for differential identification of the tissue(s) or cell type(s) present in a biological sample and for diagnosis of diseases and conditions which include, but are not limited to, tumors of a hematopoictic origin, graft rejection, wounding, inflammation, and allergy. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., hematopoietic cells, and tissues and cells of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the Rh/T2/S-glycoprotein family of ribonuclease-encoding genes indicates that polypeptides and polynucleotides corresponding to Gene NO: 47 are useful as a cytotoxin that could be directed against specific cell types (e.g. cancer cells; HIV- infected cells), and that would be well tolerated by the human immune system.

Preferred epitopes include those comprising a sequence shown in SEQ ID NO: 180 as residues: Ala-24 to Asp-30, Ile-51 to Tyr-61, Pro-69 to Ser-78, Pro-105 to Phe-110, Asn-129 to Phe-135, Pro-187 to Glu-192, Lys-205 to Gln-224, and Pro-250 to His-256.

### FEATURES OF PROTEIN ENCODED BY GENE NO: 48

The translation product of Gene NO: 48 shares sequence homology with dolichyl-phosphate glucosyltransferase, a transmembrane-bound enzyme of the endoplasmic reticulum which is thought to be important in N-linked glycosylation, by catalyzing the transfer of glucose from UDP-glucose to dolichyl phosphate. (See Accession No. 535141.) Based on homology, it is likely that this gene product also play a role similar in humans. Preferred polynucleotide fragments comprise nucleotides 132-959. Also preferred are the polypeptide fragments encoded by this nucleotide fragment.

Gene NO: 48 is expressed primarily in endothelial cells and to a lesser extent in hematopoietic cells and brain.

Therefore, polynucleotides and polypeptides of the invention are useful as reagents for differential identification of the tissue(s) or cell type(s) present in a biological sample and for diagnosis of diseases and conditions which include, but are not limited to, defects in proper N-linked glycosylation of proteins, such as Wiskott-Aldrich syndrome: tumors of an endothelial cell origin. Similarly, polypeptides and antibodies directed to these polypeptides are useful in providing immunological probes for differential identification of the tissue(s) or cell type(s). For a number of disorders of the above tissues or cells, particularly of the vascular and hematopoietic systems, as well as brain, expression of this gene at significantly higher or lower levels may routinely be detected in certain tissues and cell tpes (e.g., endothelial cells, hematopoietic cells, and brain, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to dolichyl-phosphate glucosyltransferase indicates that polypeptides and polynucleotides corresponding to Gene NO: 48 are useful in diagnosing and treating defects in N-linked glycosylation pathways that contribute to disease conditions and/or pathologies.

Preferred epitopes include those comprising a sequence shown in SEQ ID NO: 181 as residues: Lys-50 to Thr-55, Ser-73 to Arg-79, Glu-92 to Pro-99, Asp-110 to Ser-117, Gln-125 to Lys-131, Gly-179 to Asn-188, Ile-231 to Cys-236, and Glu-318 to Asn-324.

### FEATURES OF PROTEIN ENCODED BY GENE NO: 49

Gene NO: 49 is expressed primarily in brain, most notably in the hypothalamus and amygdala. This gene is also mapped to chromosome X, and therefore, can be used in linkage analysis as a marker for chromosome X.

Therefore, polynucleotides and polypeptides of the invention are useful as reagents for differential identification of the tissue(s) or cell type(s) present in a biological sample and for diagnosis of diseases and conditions which include, but are not limited to, tumors of a brain origin: neurodegenerative disorders, and sex-linked disorders. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may routinely be detected in certain tissues (e.g., brain and cancerous and wounded tissues) or bodily fluids (e.g., serum, plasma, urine, synovial fluid or spinal fluid) or another tissue or cell sample taken from an individual having such a disorder, relative to the standard gene expression level. i.e., the expression level in healthy tissue or bodily fluid from an individual not having the disorder.

The tissue distribution indicates that polypeptides and polynucleotides corresponding to Gene NO: 49 are useful for the diagnosis of tumors of a brain origin, and the treatment of neurodegenerative disorders, such as Parkinson's disease, and sex-linked disorders.

### FEATURES OF PROTEIN ENCODED BY GENE NO: 50

The translation product Gene NO: 50 shares sequence homology with canine phospholemman, a major plasma membrane substrate for cAMP-dependent protein kinases A and C. (See Accession No. M63934; see also Accession No. A40533.) In fact, a group also recently cloned the human phospholemman gene, and mapped this gene to chromosome 19. (See Accession No.1916010.) Phospholemman is a type I integral membrane protein that gets phosphorylated in response to specific extracellular stimuli such as insulin and adrenalin. Phospholemman forms ion channels in the cell membrane and appears to regulate taurine transport, suggesting an involvement in cell volume regulation. It has been proposed that phospholemman is a member of a superfamily of membrane proteins, characterized by single transmembrane domains, which function in transmembrane ion flux. They are capable of linking signal transduction to the regulation of such cellular processes as the control of cell volume.

Gene No 50 is expressed primarily in fetal liver and to a lesser extent in adult brain and kidney, as well as other organs.

Therefore, polynucleotides and polypeptides of the invention are useful as reagents for differential identification of the tissue(s) or cell type(s) present in a biological sample and for diagnosis of diseases and conditions which include, but are not limited to, insulin and/or adrenalin defects; diabetes; aberrant ion channel signaling; defective taurine transport; and defects in cell volume regulation. Similarly, polypeptides and antibodies directed to these polypeptides are useful in providing immunological probes for differential identification of the tissue(s) or cell type(s). For a number of disorders of the above tissues or cells, particularly of the brain and/or immune system, expression of this gene at significantly higher or lower levels may routinely be detected in certain tissues (e.g., liver, brain, and kidney, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to phospholemman indicates that polypeptides and polynucleotides corresponding to Gene NO: 50 are useful for treatment of disorders involving the transport of ions and small molecules, in particular taurine. It could also be beneficial for control of pathologies or diseases wherein aberrancies in the control of cell volume are a distinguishing feature, due to the predicted role for phospholemman in the normal control of cell volume. It also may play a role in disorders involving abnormal circulating levels of insulin and/or adrenalin - along with other active secreted molecules - as revealed by its phosphorylation upon stimulation with insulin or adrenalin.

Preferred epitopes include those comprising a sequence shown in SEQ ID NO: 183 as residues: Ala-20 to Gln-34, Arg-58 to Thr-79, and Leu-87 to Arg-92.

### FEATURES OF PROTEIN ENCODED BY GENE NO: 52

Gene NO: 52 is expressed primarily in metastic melanoma and to a lesser extent in infant brain.

Therefore, polynucleotides or polypeptides of the invention are useful as reagents for differential identification of the tissue(s) or cell type(s) present in a biological sample and for diagnosis of diseases and conditions which include, but are not limited to, cancer and cancer metastasis. Similarly, polypeptides and antibodies directed to these polypeptides are useful in providing immunological probes for differential identification of the tissue(s) or cell type(s). For a number of disorders of the above tissues or cells, expression of this gene at significantly higher or lower levels may routinely be detected in certain tissues (e.g., epidermis, and brain, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 52 are useful for diagnosis and treatment of melanoma.

### FEATURES OF PROTEIN ENCODED BY GENE NO: 53

The translation product of Gene NO: 53 shares sequence homology with mucin which is thought to be important cell surface molecule. It also exhibits sequence identity with a calcium channel blocker of Agelenopsis aperta. In particular, with those calcium channel blockers which affect neuronal and muscle cells.

Gene NO: 53 is expressed primarily in prostate, endothelial cells, smooth muscle and fetal tissues and to a lesser extent in T cells and placenta.

Therefore, polynucleotides or polypeptides of the invention are useful as reagents for differential identification of the tissue(s) or cell type(s) present in a biological sample and for diagnosis of diseases and conditions which include, but are not limited to, prostate cancer, immune disorders, angina, hypertension, cardiomyopathics, supraventricular arrhythmia, oesophogeal achalasia, premature labour, and Raynaud's disease. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues or cell types (e.g., prostrate, and tissue and cells of the immune system, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution and homology to mucin indicates that polypeptides and polynucleotides corresponding to Gene NO: 53 are useful as a surface antigen for diagnosis of diseases such as prostate cancer and as tumor vaccine.

### FEATURES OF PROTEIN ENCODED BY GENE NO: 54

Gene NO: 54 encodes a polypeptide which exhibits sequence identity with the rab receptor and VAMP-2 receptor proteins. (Martincic, et al., J. Biol. Chem. 272 (1997).)

Gene NO: 54 is expressed primarily in placenta, fetal liver, osteoclastoma and smooth muscle and to a lesser extent in T cell, fetal lung and colon cancer.

Therefore, polynucleotides or polypeptides of the invention are useful as reagents for differential identification of the tissue(s) or cell type(s) present in a biological sample and for diagnosis of diseases and conditions which include, but are not limited to, cancers, osteoporosis and immuno-related diseases. Similarly, polypeptides and antibodies directed to these polypeptides are useful in providing immunological probes for differential identification of the tissue(s) or cell type(s). For a number of disorders of the above tissues or cells, particularly of the immune system, hematopoiesis system and bone system, expression of this gene at significantly higher or lower levels may routinely be detected in certain tissues and cell types (e.g.. placenta, liver, osteoclastama, smooth muscle. T-cells, and lung, and colon, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 54 are useful for treating cancer, osteoporosis and immuno-disorders.

Preferred epitopes include those comprising a sequence shown in SEQ ID NO: 187 as residues: Pro-16 to Phe-21, Pro-24 to Arg-35, Arg-92 to Pro-98, Asn-143 to Lys-151, and Leu-169 to Ile-176.

### FEATURES OF PROTEIN ENCODED BY GENE NO: 55

Gene NO: 55 encodes a protein having sequence identity to the rat galanin receptor GALR2.

Gene NO: 55 is expressed primarily in ovarian cancer.

Therefore, polynucleotides or polypeptides of the invention are useful as reagents for differential identification of the tissue(s) or cell type(s) present in a biological sample and for diagnosis of ovarian cancer. Similarly, polypeptides and antibodies directed to those polypeptides are useful in providing immunological probes for differential identification of the tissue(s) or cell type(s). For a number of disorders of the above tissues or cells, particularly of the immune system and reproductive system, expression of this gene at significantly higher or lower levels may routinely be detected in certain tissues (e.g., ovary, and tissues and cells of the immune system, and cancerous and wounded tissues) or bodily fluids (e.g.. serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder. GALR2 antagonists can be used to treat obesity, bulimia, or Alzheimer's disease, while GALR2 agonists can be used to treat anorexia or pain, or to decrease nociception (claimed). Agonists and antagonists can also be used to treat numerous other disorders, including cognitive disorders, sensory disorders, motion sickness, convulsion/epilepsy, hypertension, diabetes, glaucoma, reproductive disorders, gastric and intestinal ulcers, inflammation, immune disorders, and anxiety.

The tissue distribution indicates that polypeptides and polynucleotides corresponding to Gene NO: 55 are useful for diagnosis and treatment of ovarian cancer.

### FEATURES OF PROTEIN ENCODED BY GENE NO: 56

As indicated in Table 1, the predicted signal sequence of Gene NO: 56 relates to an open reading frame that is homologous to the mouse major histocompatibility locus class III. (See Accession No. 2564953.) Any frame shift mutations that alter the correct open reading frame can easily be clarified using known molecular biology techniques. Moreover, in the opposite orientation, a second translated product is disclosed. This second translation product of this contig is identical in sequence to intracellular protein lysophosphatidic acid acyltranslerase. The nucleotide and amino acid sequences of this translated product have since been published by Stamps and colleagues (Biochem. J. 326 (Pt 2), 455-461 (1997)), West and coworkers (DNA Cell Biol. 6, 691-701 (1997)), Rowan (GenBank Accession No. U89336), and Soyombo and Hofmann (GenBank Accession No. AF020544). This gene is thought to enhance cytokine signaling response in cells. It is likely that a signal peptide is located upstream from this translated product. Preferred polypeptide fragments comprise the amino acid sequence: 
GLACWLAGVIFIDRKRTGDAISVMSEVAQTLLTQDVXVWVFPEGTRNHNGSML
PFKRGAFHLAVQAQVPIVPIVMSSYQDFYCKKERRFTSGQCQVRVLPPVPTEGL
TPDVPALADRVRHSMLHCF(SEQ ID NO: 265);
 
PSAKYFFKMAFYNGWILFLAVLAIPVCAVRGRNVENMKILRLMLLHIKYLYGI
RVEVRGAHHFPPSQPYVVVSNHQSSLDLLGMMEVLPGRCVPIAKR (SEQ ID
NO:266); TVFREISTD (SEQ ID NO:267); or LWAGSAGWPAG (SEQ ID NO: 268).
 Also provided are polynucleotide fragments encoding these polypeptide fragments.

Gene NO: 56 is expressed primarily in infant adrenal gland, hypothalamus, 7 week old embryonic tissue, fetal lung, osteoclastoma stromal cells, and to a lesser extent in a large number of additional tissues.

Therefore, polynucleotides or polypeptides of the invention are useful as reagents for differential identification of the tissue(s) or cell type(s) present in a biological sample and for diagnosis of developmental disorders and osteoclastoma. Similarly, polypeptides and antibodies directed to these polypeptides are useful in providing immunological probes for differential identification of the tissue(s) or cell type(s) in which it is highly expressed. For a number of disorders of the above tissues or cells, particularly during development or of the nervous or bone systems, expression of this gene at significantly higher or lower levels may routinely be detected in certain tissues and cell types (e.g., adrenal, embryonic tissue, lung, and osteoclastomal stromal cells, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level. i.e., the expression level in healthy tissue or bodily fluid from an individual not having the disorder. Further, expression of this protein can be used to alter the fatty acid composition of a given cell or membrane type.

The tissue distribution indicates that polypeptides and polynucleotides corresponding to Gene NO: 56 are useful for diagnosis and treatment of osteoclastoma and other bone and non-bone-related cancers, as well as for the diagnosis and treatment of developmental disorders.

Preferred epitopes include those comprising a sequence shown in SEQ ID NO: 189 as residues: Gly-29 to Gly-36 and Tyr-49 to Tyr-58.

### FEATURES OF PROTEIN ENCODED BY GENE NO: 57

The translation product of Gene NO: 57 shares sequence homology with longevity-assurance protein-1. (See Accession No. g1123105.) Preferred polynucleotide fragments comprise nucleotides 6-125 and 118-432, as well as the polypeptides encoded by these polynucleotides. It is likely that a second signal sequence exists upstream from the predicted signal sequence in Table 1. Moreover, a frame shift likely occurs between nucleotides 118-125, which can be elucidated using standard molecular biology techniques.

Gene NO: 57 is expressed primarily in fetal liver, kidney, brain, thymus, and bone marrow.

Therefore, polynucleotides or polypeptides of the invention are useful as reagents for differential identification of the tissue(s) or cell type(s) present in a biological sample and for diagnosis of diseases and conditions which include, but are not limited to, immunological diseases and hyperproliferative disorders. Similarly, polypeptides and antibodies directed to these polypeptides are useful in providing immunological probes for differential identification of the tissue(s) or cell type(s). For a number of disorders of the above tissues or cells, particularly of the fetal liver, kidney, brain, thymus, and bone marrow expression of this gene at significantly higher or lower levels may routinely be detected in certain tissues (e.g., liver, kidney, brain, thymus, and bone marrow, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution and homology to longevity-assurance protein suggest that Gene NO: 57 encodes a protein useful in increasing life span and in replacement therapy for those suffering from immune system disorders or hyperproliferative disorders caused by underexpression or overexpression of this gene.

Preferred epitopes include those comprising a sequence shown in SEQ ID NO: 190 as residues: Val-29 to Arg-46 and Gly-50 to Gly-56.

### FEATURES OF PROTEIN ENCODED BY GENE NO: 58

Domains of the Gene NO: 58 product are homologous to porcine surfactant protein-A receptor. (See Accession No. B485 16.) The bovine gene binds surfactant protein-A receptor, modulating the secretion of alveolar surfactant. Based on this homology, the gene product encoded by this gene will likely have activity similar to the porcine gene. Preferred polynucleotide fragments comprise nucleotides 887-1039, as well as the polypeptide fragments encoded by this nucleotide fragment.

Gene NO: 58 is expressed primarily in brain and to a lesser extent in endothelial cells.

Therefore, polynucleotides or polypeptides of the invention are useful as reagents for differential identification of the tissue(s) or cell type(s) present in a biological sample and for diagnosis of diseases and conditions which include, but are not limited to, diseases of the central nervous system including dimentia, stroke, neurological disorders, respiratory distress, and diseases affecting the endothelium including inflammatory diseases, restenosis, and vascular diseases. Similarly, polypeptides and antibodies directed to these polypeptides are useful in providing immunological probes for differential identification of the tissue(s) or cell type(s). For a number of disorders of the above tissues or cells, particularly of the placenta, liver, endothelial cells, prostate, thymus, and lung, expression of this gene at significantly higher or lower levels may routinely be detected in certain tissues and cell types (e.g., brain, and endothelial cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology indicates that polypeptides and polynucleotides corresponding to Gene NO: 58 are useful for the diagnosis and /or treatment of diseases on the central nervous system, such as a factor that promote neuronal survival or protection, in the treatment of inflammatory disorders of the endothelium, or in disorders of the lung. In addition this protein may inhibit or promote angiogenesis and therefore is useful in the treatment of vascular disorders.

Preferred epitopes include those comprising a sequence shown in SEQ ID NO: 191 as residues: His-66 to Pro-80, Gly-139 to Ser-146 and Ser-262 to Pro-267.

### FEATURES OF PROTEIN ENCODED BY GENE NO: 59

The translation product of Gene NO: 59 is homologous to the rat hypertension-induced protein which is thought to be important in hypertension, and found expressed mainly in kidneys. (Sec Accession No. B61209.) Thus, it is likely that this gene product is involved in hypertension in humans. Preferred polypeptide fragments comprise the short chain dehydrogenase/reductase motif SILGIISVPLSIGYCASKHALRGFFNGLR (SEQ ID NO:269), as well as polynucleotides encoding this polypeptide fragment. Also preferred are polynucleotide fragments of 337-639, as well as the polypeptide fragments encoded by this polynucleotide fragment.

Gene NO: 59 is expressed primarily in liver, spleen, lung, brain, and prostate.

Therefore, polynucleotides and polypeptides of the invention are useful as reagents for differential identification of the tissue(s) or cell type(s) present in a biological sample and for diagnosis of diseases and conditions which include, but are not limited to, cardiovascular, immunological, and renal disorders. Similarly, polypeptides and antibodies directed to these polypeptides are useful in providing immunological probes for differential identification of the tissue(s) or cell type(s). For a number of disorders of the above tissues or cells, particularly of the cardiovascular, renal, and immune, expression of this gene at significantly higher or lower levels may routinely be detected in certain tissues (e.g., liver, spleen, lung, brain, and prostrate, and cancerous and wounded tissues) or bodily fluids (e.g., serum, plasma, urine, synovial fluid or spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution and homology to hypertension-induced protein indicates that polypeptides and polynucleotides corresponding to Gene NO: 59 are useful for treating hypertension.

Preferred epitopes include those comprising a sequence shown in SEQ ID NO: 192 as residues: Gln-40 to Glu-45, Glu-96 to Glu-102, Asn-256 to Thr-266, and Asp-308 to Asp-317.

### FEATURES OF PROTEIN ENCODED BY GENE NO: 60

Gene NO: 60 is expressed primarily in activated T-cell and jurkat cell and to a lesser extent in apoptic T-cell and CD34+ cell. It is likely that alternative open reading frames provide the full length amino acid sequence, which can be verified using standard molecular biology techniques.

Therefore, polynucleotides and polypeptides of the invention are useful as reagents for differential identification of the tissue(s) or cell type(s) present in a biological sample and for diagnosis of diseases and conditions which include, but are not limited to, T lymphocyte related diseases or hematopoiesis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., T-cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 60 are useful for diagnosis or treatment of immune system disorders.

### FEATURES OF PROTEIN ENCODED BY GENE NO: 61

The translation product of Gene NO: 61, a vacuolar proton-ATPase, shares sequence homology with a *Caenorhabditis elegans* protein which is thought to be important in development. This protein may be a human secretory homologue that may also influence embryo development. Ludwig, J., also recently cloned this gene from chromaffin granules. (See, Accession No. 2584788.) Although Table 1 indicates the predicted signal peptide sequence, the translated product of this gene may in fact start with the upstream methionine, beginning with the amino acid sequence MAYHGLTV (SEQ ID NO:270). Thus. polypeptides comprising this upstream sequence. as well as N-terminus deletions. are also contemplated in the present invention.

Gene NO: 61 is expressed primarily in human placenta, liver, and Hodgkin's Lymphoma and to a lesser extent in bone marrow. Modest levels of expression were also observed in dendritic cells.

Therefore, polynucleotides and polypeptides of the invention are useful as reagents for differential identification of the tissue(s) or cell type(s) present in a biological sample and for diagnosis of diseases and conditions which include, but are not limited to, hyperproliferative disorders, defects in embryonic development, and diseases or disorders caused by defects in chromaffin granules. Similarly, polypeptides and antibodies directed to these polypeptides are useful in providing immunological probes lor differential identification of the tissue(s) or cell type(s). For a number of disorders of the above tissues or cells, particularly cancer, expression of this gene at significantly higher or lower levels may routinely be detected in certain tissues (e.g., placenta, liver, lymph tissue, and bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to *Caenorhabditis elegans* indicates that polypeptides and polynucleotides corresponding to Gene NO: 61 are useful for diagnostic or therapeutic modalities for hyperproliferative disorders, embryonic development disorders, and chromaffin granules disorders.

### FEATURES OF PROTEIN ENCODED BY GENE NO: 62

The translation product of Gene NO: 62 shares sequence homology with the murine LAG3 gene which is thought to be important in the mediation of natural killer cell (NK cell) activity as previously determined by experiments in mice containing null mutations of LAG3. The similarity of this gene to the CD4 receptor may imply that the gene product may be a secreted, soluble receptor and immune mediator.

Gene NO: 62 is expressed primarily in human fetal heart, meningima, and to a lesser extent in tonsils. This gene also is expressed in the breast cancer cell line MDA 36.

Therefore, polynucleotides or polypeptides of the invention are useful as reagents for differential identification of the tissue(s) or cell type(s) present in a biological sample and for diagnosis of diseases and conditions which include, but are not limited to, lymphomas, leukemias, breast cancer and any immune system dysfunction, including those dysfunctions which involve natural killer cell activities. Similarly, polypeptides and antibodies directed to these polypeptides are useful in providing immunological probes for differential identification of the tissue(s) or cell type(s). For a number of disorders of the above tissues or cells, particularly of the immune system or breast cancer, expression of this gene at significantly higher or lower levels may routinely be detected in certain tissues (e.g., heart, meningima, and tonsils and cancerous and wounded tissues) or bodily fluids (e.g., serum, plasma, urine, synovial fluid or spinal fluid) or another tissue or cell sample taken from an individual having such a disorder, relative to the standard gene expression level. i.e., the expression level in healthy tissue or bodily fluid from an individual not having the disorder.

The tissue distribution and homology to the LAG3 gene (murine) indicates that the polynucleotides and polypeptides corresponding to Gene NO: 62 are useful for diagnostic and/or therapeutic modalities directed at abnormalities or disease states involving defective immune systems, preferably involving natural killer cell activity, as well as breast cancer.

Preferred epitopes include those comprising a sequence shown in SEQ ID NO: 195 as residues: Pro-10 to Trp-17, Cys-58 to Pro-67, Thr-76 to Glu-85, and Arg-93 to Asn-101.

### FEATURES OF PROTEIN ENCODED BY GENE NO: 63

The translation product of Gene NO: 63 shares sequence homology with a *Caenorhabditis elegans* alpha-collagen gene (Clg), which is thought to be important in organism development, as well as other collagen genes. Thus, based on sequence homology, polypeptides of this gene are expected to have activity similar to collagen, including involvement in organ development.

Gene NO: 63 is expressed primarily in human B-Cell Lymphoma, and to a lesser extent in human pituitary tissue. This gene has also demonstrated expression in keratinocytes.

Therefore, polynucleotides or polypeptides of the invention are useful as reagents for differential identification of the tissue(s) or cell type(s) present in a biological sample and for diagnosis of diseases and conditions which include, but are not limited to, B-Cell Lymphoma, other lymphomas, leukemias, and other cancers, as well as disorders related to development. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., tissue and/or cells of the immune system, and pituitary, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to *Caenorhabditis elegans* alpha-collagen gene indicates that polypeptides and polynucleotides corresponding to Gene NO: 63 are useful for development of diagnostic and/or therapeutic modalities directed at the detection and/or treatment of cancer, specifically B-Cell Lymphomas, leukemias, or diseases related to development.

Preferred epitopes include those comprising a sequence shown in SEQ ID NO: 196 as residues: Thr-22 to Arg-27 and Ser-29 to Thr-39.

### FEATURES OF PROTEIN ENCODED BY GENE NO: 64

The translation product of Gene NO: 64 shares sequence homology with human extracellular molecule olfactomedin, which is thought to be important in the maintenance, growth, or differentiation of chemosensory cilia on the apical dendrites of olfactory neurons. Based on this sequence homology, it is likely that polypeptides of this gene have activity similar to the olfactomedin, particularly the differentiation or proliferation of neurons.

Gene NO: 64 is expressed primarily in fetal lung tissue.

Therefore, polynucleotides or polypeptides of the invention are useful as reagents for differential identification of the tissue(s) or cell type(s) present in a biological sample and for diagnosis of diseases and conditions which include, but are not limited to, diseases of the lung as well as neural development, particularly of the lung. Similarly, polypeptides and antibodies directed to these polypeptides are useful in providing immunological probes for differential identification of the tissue(s) or cell type(s). For a number of disorders of the above tissues or cells, particularly of the pulmonary system, expression of this gene at significantly higher or lower levels may routinely be detected in certain tissues (e.g., lungs and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the olfactomedin family indicates that polypeptides and polynucleotides corresponding to Gene NO: 64 are useful for the development of diagnostic and/or therapeutic modalities directed at detection and/or treatment of pulmonary disease states, e.g., cystic fibrosis.

Preferred epitopes include those comprising a sequence shown in SEQ ID NO: 197 as residues: Gly-17 to Gln-23, Gln-45 to Arg-50, Arg-56 to Lys-61, Glu-70 to Leu-76, Asp-88 to Glu-93, Pro-117 to Met-131, Asp-161 to Glu-167, Arg-224 to Asn-237, Asp-302 to Trp-312, Pro-315 to Asn-320, and Thr-337 to Ser-341.

### FEATURES OF PROTEIN ENCODED BY GENE NO: 65

The translation product of Gene NO: 65 shares sequence homology with *Saccharomyces cerevisiae* hypothetical protein YKL166 (Accession No. gi/687880) which is thought to be important in secretory and/or vesicular transport mechanisms. Based on this homology, it is likely that the gene product would have similar activity to YKL166, particularly secretory or transport mechanisms. Preferred polypeptide fragments of this gene include those fragments starting with the amino acid sequence ISAARV (SEQ ID NO:271). Other polypeptide fragments include the former fragment, which ends with the amino acid sequence PDVSEFMTRLF (SEQ ID NO:272). Further preferred fragments include those polypeptide fragments comprising the amino acid sequence FDPVRVDITSKGKMRAR (SEQ ID NO:273). Also preferred are polypeptide fragments having exogenous signal sequences fused to the polypeptide.

Gene No 65 is expressed primarily in placenta, testis, osteoclastoma and to a lesser extent in adrenal gland.

Therefore, polynucleotides or polypeptides of the invention are useful as reagents for differential identification of the tissue(s) or cell type(s) present in a biological sample and for diagnosis of diseases and conditions which include, but are not limited to, cancer and/or diseases involving defects in protein secretion. Similarly, polypeptides and antibodies directed to these polypeptides are useful in providing immunological probes for differential identification of the tissue(s) or cell type(s). For a number of disorders of the above tissues or cells, particularly of the reproductive system, cartilage and bone, expression of this gene at significantly higher or lower levels may routinely be detected in certain tissues and cell types (e.g., placenta, testis, adrenal gland, and osteoclastoma, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the yeast YKLIGG protein indicates that polypeptides and polynucleotides corresponding to Gene NO: 65 are useful for the development of therapeutic and/or diagnostic modalities targeted at cancer or secretory anomalies, such as genetically caused secretory diseases.

Preferred epitopes include those comprising a sequence shown in SEQ ID NO: 198 as residues: Ser-18 to Ser-29 and Lys-53 to Arg-74.

### FEATURES OF PROTEIN ENCODED BY GENE NO: 66

The translation product of Gene NO: 66 shares sequence homology with the human papilloma virus (HPV) E5 ORF region which is thought to be important as a secreted growth factor. Although this is described as a viral gene product, it is believed to have several cellular secretory homologues. Therefore, based on the sequence similarity between the HPV E5 ORF and the translated product of this gene, this gene product is likely to have activity similar to HPV E5 ORF.

Gene NO: 66 is expressed primarily in activated T-Cells, monocytes, cerebellum and to a lesser extent in infant brain.

Therefore, polynucleotides or polypeptides of the invention are useful as reagents for differential identification of the tissue(s) or cell type(s) present in a biological sample and for diagnosis of diseases and conditions which include, but are not limited to, cancer and/or human papilloma virus infection. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., brain, lymph tissue, monocytes, and T-cells and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Moreover, polynucleotides of this gene have been mapped to chromosome 1. Therefore, polynucleotides of the present invention can be used in linkage analysis as a marker for chromosome 1.

The tissue distribution and homology to human papilloma virus E5 region indicates that polypeptides and polynucleotides corresponding to Gene NO: 66 are useful for development of diagnostic and/or therapeutic modalities directed at the diagnosis and/or treatment of cancer and/or human papilloma virus infection (HPV).

Preferred epitopes include those comprising a sequence shown in SEQ ID NO: 199 as residues: Asn-31 to Arg-36 and Leu-102 to Ser-112.

### FEATURES OF PROTEIN ENCODED BY GENE NO: 67

The translation product of Gene NO: 67 shares sequence homology with the 8hs20 protein precursor [*Mus musculus*] which is thought to be important in B-Cell mu chain assembly. (See, Accession No. PID/d1002996; Shiraswa, T., EMBO. J. 12(5):1827-1834 (1993).) A polypeptide fragment starting at amino acid 53 is preferred, as well as 1-20 amino acid N-terminus and/or C-terminus deletions. Based on the sequence similarity between 8hs20 protein and the translation product of this gene, the two polypeptides are expected to share certain biological activities, particularly immunologic activities.

Gene NO: 67 is expressed primarily in human B-cells and to a lesser extent in Hodgkin's Lymphoma. It is also likely that the polypeptide will be expressed in B-cell specific cells, bone marrow, and spleen, as is observed with 8hs20.

Therefore, polynucleotides or polypeptides of the invention are useful as reagents for differential identification of the tissue(s) or cell type(s) present in a biological sample and for diagnosis of diseases and conditions which include, but are not limited to, Hodgkin's Lymphoma, Common Variable Immunodeficiency, and/or other B-cell lymphomas. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may routinely be detected in certain tissues and cell types (e.g., bone marrow, spleen, lymph tissue, and B-cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to 8hs20 protein precursor [*Mus musculus*], indicates that polypeptides and polynucleotides corresponding to Gene NO: 67 are useful for therapeutic and/or diagnostic purposes, targeting Hodgkin's Lymphoma, B-cell lymphomas, Common Variable Immunodeficiency, or other immune disorders.

Preferred epitopes include those comprising a sequence shown in SEQ ID NO: 200 as residues: Asp-51 to Trp-56, Arg-72 to Asp-85, and Gln-106 to Asp-112.

### FEATURES OF PROTEIN ENCODED BY GENE NO: 68

Gene NO: 68 is expressed primarily in fetal liver/spleen, rhabdomyosarcoma, and to a lesser extent in 9 week-old early stage human embryo and bone marrow.

Therefore, polynucleotides or polypeptides of the invention are useful as reagents for differential identification of the tissue(s) or cell type(s) present in a biological sample and for diagnosis of diseases and conditions which include, but are not limited to, rhabdomyosarcoma and other cancers, hematopoietic disorders, and immune dysfunction. Similarly, polypeptides and antibodies directed to these polypeptides are useful in providing immunological probes for differential identification of the tissue(s) or cell type(s). For a number of disorders of the above tissues or cells, particularly of the immune system. expression of this gene at significantly higher or lower levels may routinely be detected in certain tissues (e.g., embryonic tissue, striated muscle, liver, spleen, and bone marrow, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that theprotein product of Gene NO: 68 is useful for diagnostic and/or therapeutic purposes directed to cancer, preferably rhabdomyosarcoma. Enhanced expression of this gene in fetal liver, spleen, and bone marrow indicates that this gene plays an active role in hematopoiesis. Polypeptides or polynucleotides of the present invention may therefore help modulate survival, proliferation, and/or differentiation of various hematopoietic lineages, including the hematopoietic stem cell. Thus, polynucleotides or polypeptides can be used treat various hematopoietic disorders and influence the development and differentiation of blood cell lineages, including hematopoeitic stem cell expansion. The polypeptide does contain a thioredoxin family active site at amino acids 64-82. Polypeptides comprising this thioredoxin active site are contemplated.

### FEATURES OF PROTEIN ENCODED BY GENE NO: 69

Gene NO: 69 is expressed primarily in liver and kidney and to a lesser extent in macrophages, uterus, placenta, and testes.

Therefore, polynucleotides or polypeptides of the invention are useful as reagents for differential identification of the tissue(s) or cell type(s) present in a biological sample and for diagnosis of diseases and conditions which include, but are not limited to, renal disorders, neoplasms (e.g., soft tissue cancer, hepatacellular tumors), immune disorders, endocrine imbalances, and reproductive disorders. Similarly, polypeptides and antibodies directed to these polypeptides are useful in providing immunological probes for differential identification of the tissue(s) or cell type(s). For a number of disorders of the above tissues or cells, particularly of the hepatic, urogenital, immune, and reproductive systems, expression of this gene at significantly higher or lower levels may routinely be detected in certain tissues and cell types (e.g., liver, kidney, uterus, placenta, testes, and macrophages and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polypeptides and polynucleotides corresponding to Gene NO: 69 are useful for diagnosis and treatment of disorders in the hepatic, urogenital, immune, and reproductive systems.

Preferred epitopes include those comprising a sequence shown in SEQ ID NO: 202 as residues: Arg-41 to Ser-50, Glu-138 to Asn-148, Scr-155 to Arg-172, Pro-219 to Glu-228.

### FEATURES OF PROTEIN ENCODED BY GENE NO: 70

Gene NO: 70 is expressed primarily in the immune system, including macrophages, T-cells, and dendritic cells and to a lesser extent in fetal tissue.

Therefore, polynucleotides or polypeptides of the invention are useful as reagents for differential identification of the tissue(s) or cell type(s) present in a biological sample and for diagnosis of diseases and conditions which include, but are not limited to, immune disorders, inflammatory diseases, lymph node disorders, fetal development, and cancers. Similarly, polypeptides and antibodies directed to these polypeptides are useful in providing immunological probes for differential identification of the tissue(s) or cell type(s). For a number of disorders of the above tissues or cells, particularly of the immune and hematopoietic systems expression of this gene at significantly higher or lower levels may routinely be detected in certain tissues and certain cell types (e.g., macrophages, T-cells, dendritic cells, and fetal tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. There is some evidence that the polynucleotide is mapped to chromosome 19. Thus, the polynucleotide can be a marker for genetic analysis for chromosome 19.

The tissue distribution indicates that polypeptides and polynucleotides corresponding to Gene NO: 70 are useful for treatment, prophylaxis, and diagnosis of immune and autoimmune diseases, such as lupus, transplant rejection, allergic reactions, arthritis, asthma, immunodeficiency diseases, leukemia, and AIDS. The polypeptides or polynucleotides of the present invention are also useful in the treatment, prophylaxis, and detection of thymus disorders, such as Graves Disease, lymphocytic thyroiditis, hyperthyroidism, and hypothyroidism. The expression observed predominantly in hematopoietic cells also indicates that the polynucleotides or polypeptides are important in treating and/or detecting hematopoietic disorders, such as graft versus host reaction, graft versus host disease, transplant rejection, myelogenous leukemia, bone marrow fibrosis, and myeloproliferative disease. The polypeptides or polynucleotides are also useful to enhance or protect proliferation, differentiation, and functional activation of hematopoietic progenitor cells (e.g.. bone marrow cells), useful in treating cancer patients undergoing chemotherapy or patients undergoing bone marrow transplantation. The polypeptides or polynucleotides are also useful to increase the proliferation of peripheral blood leukocytes, which can be used in the combat of a range of hematopoietic disorders, including immunodeficiency diseases, leukemia, and septicemia.

Preferred epitopes include those comprising a sequence shown in SEQ ID NO: 203 as residues: Thr-21 to Ser-27, Pro-33 to Ser-38, and Arg-73 to Lys-84.

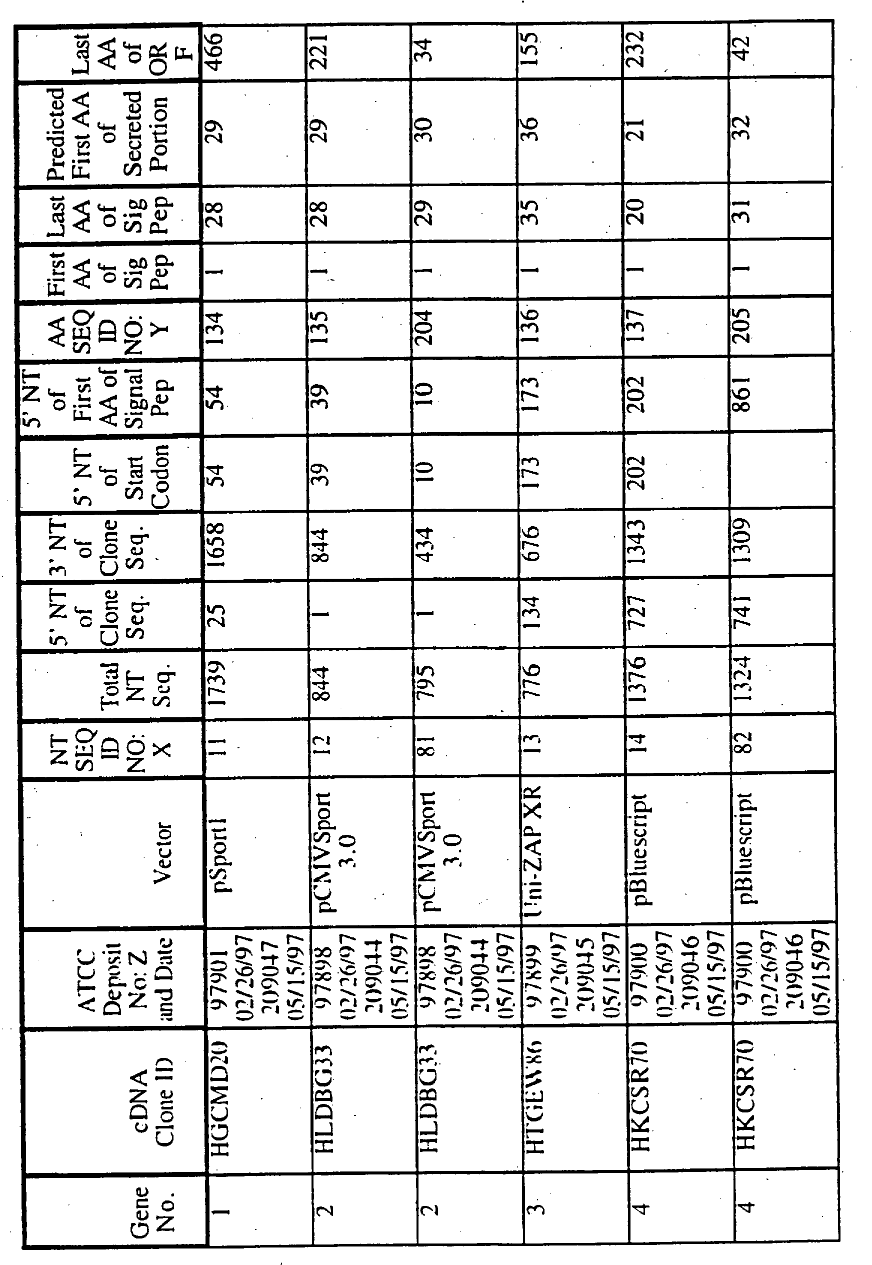

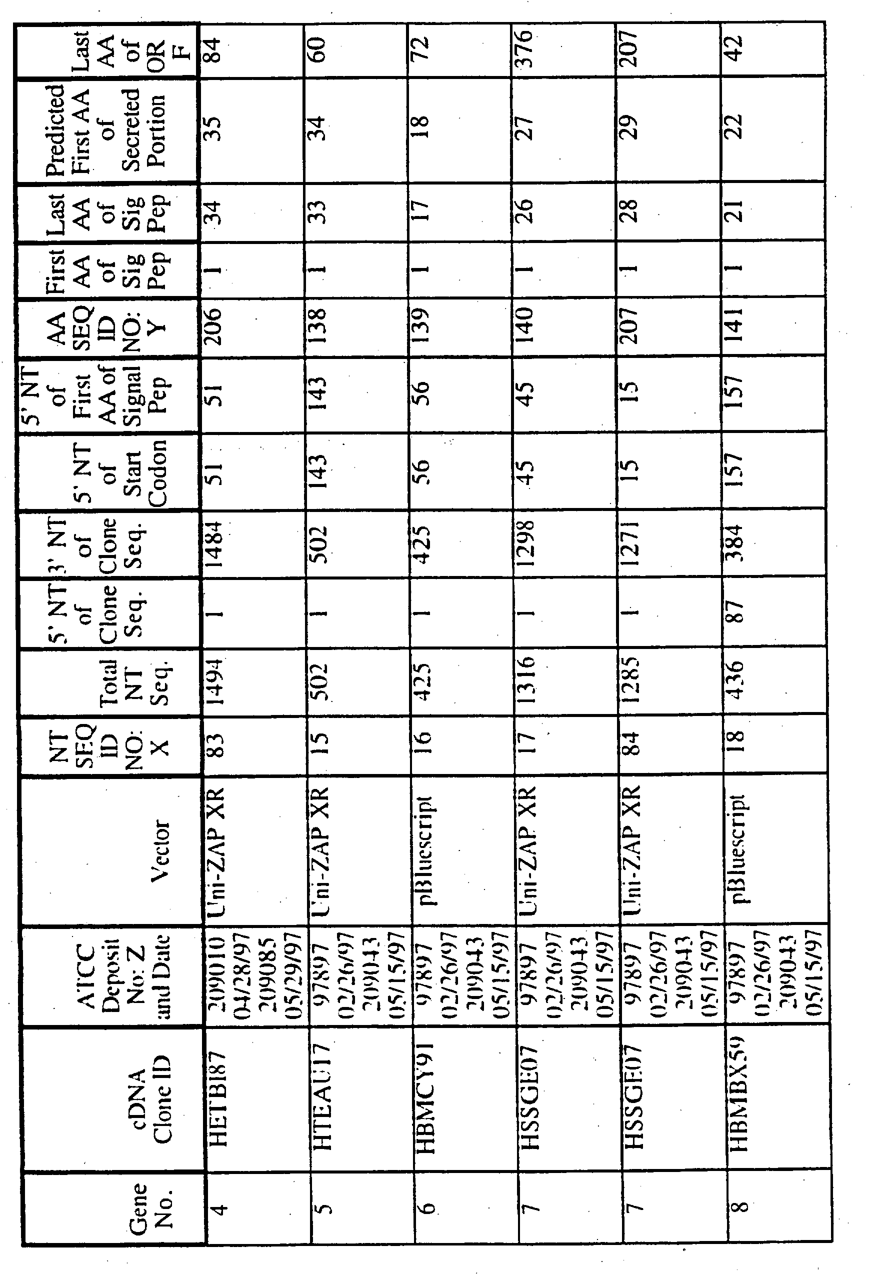

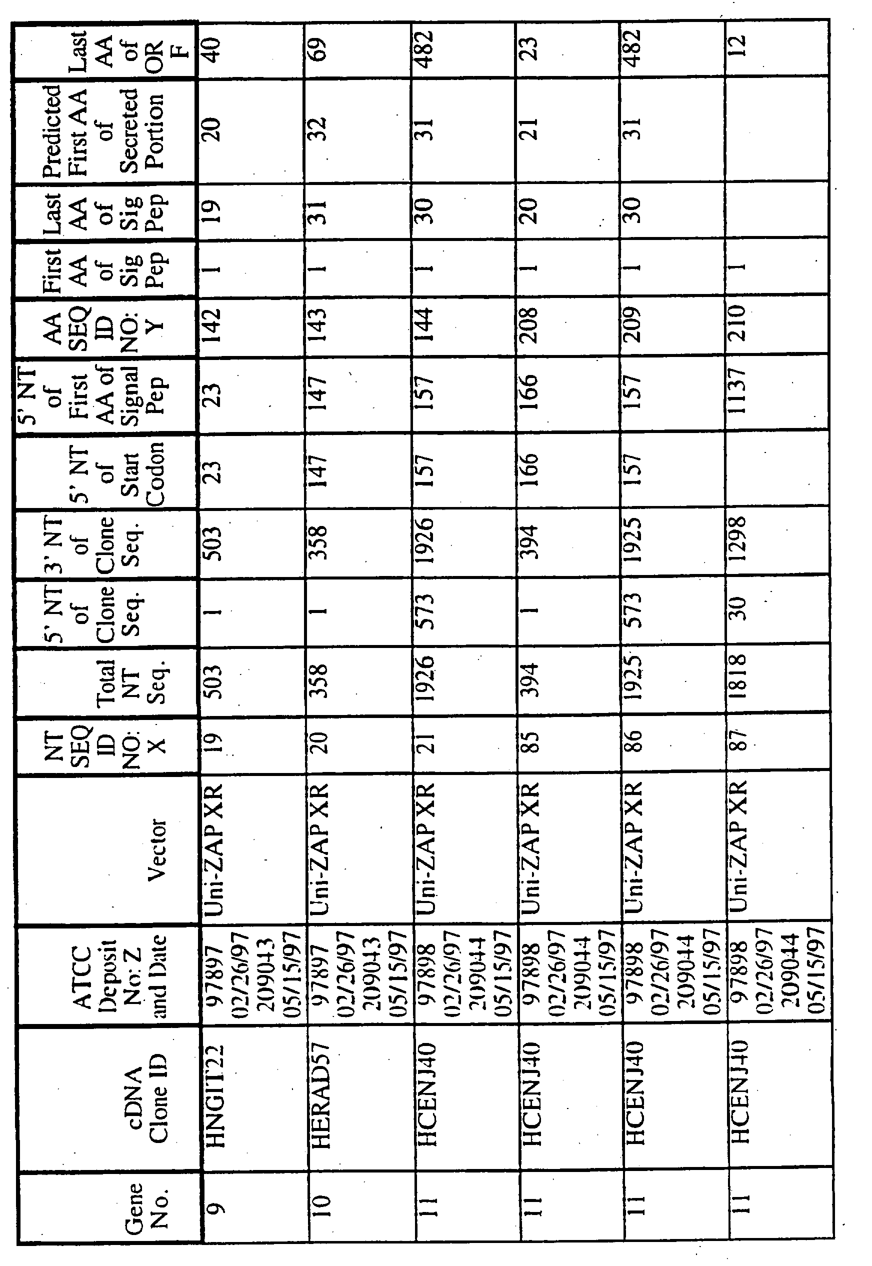

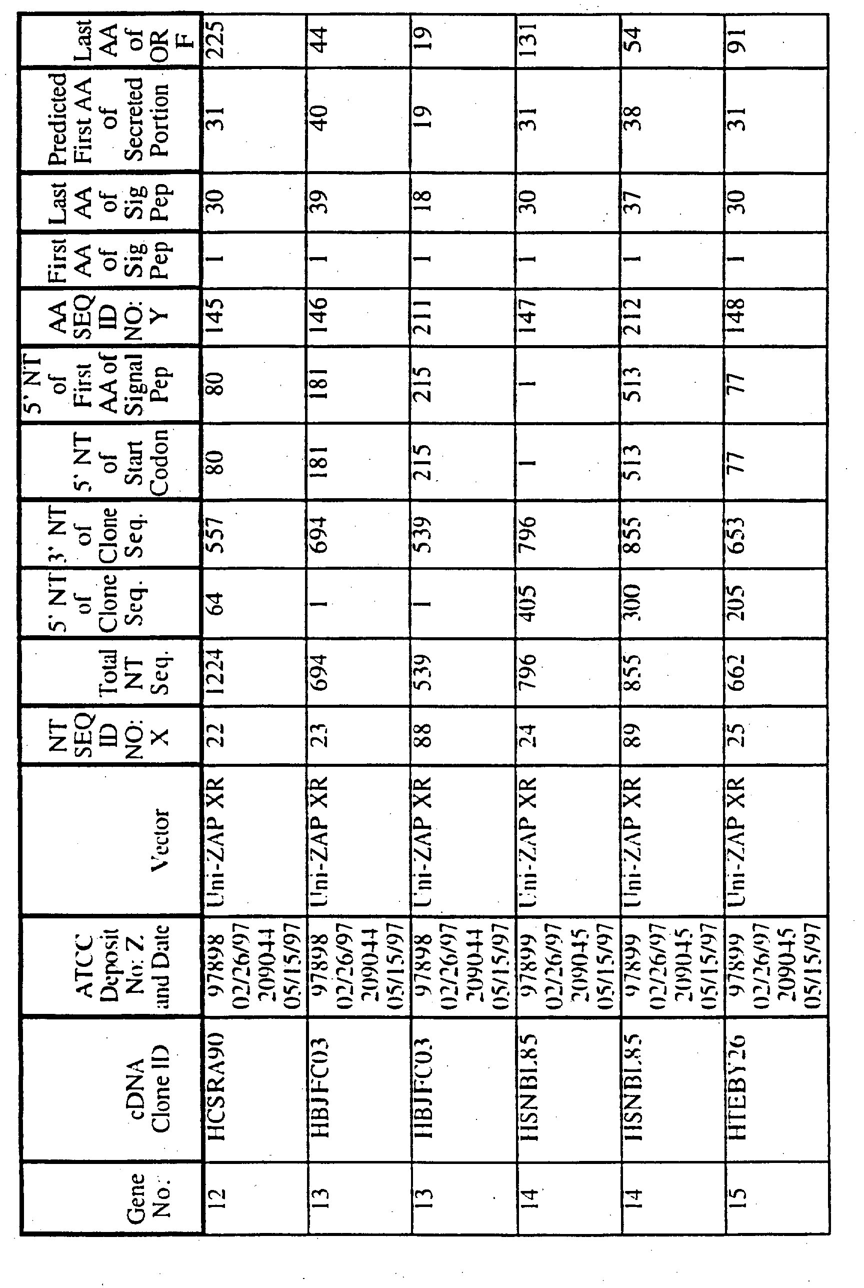

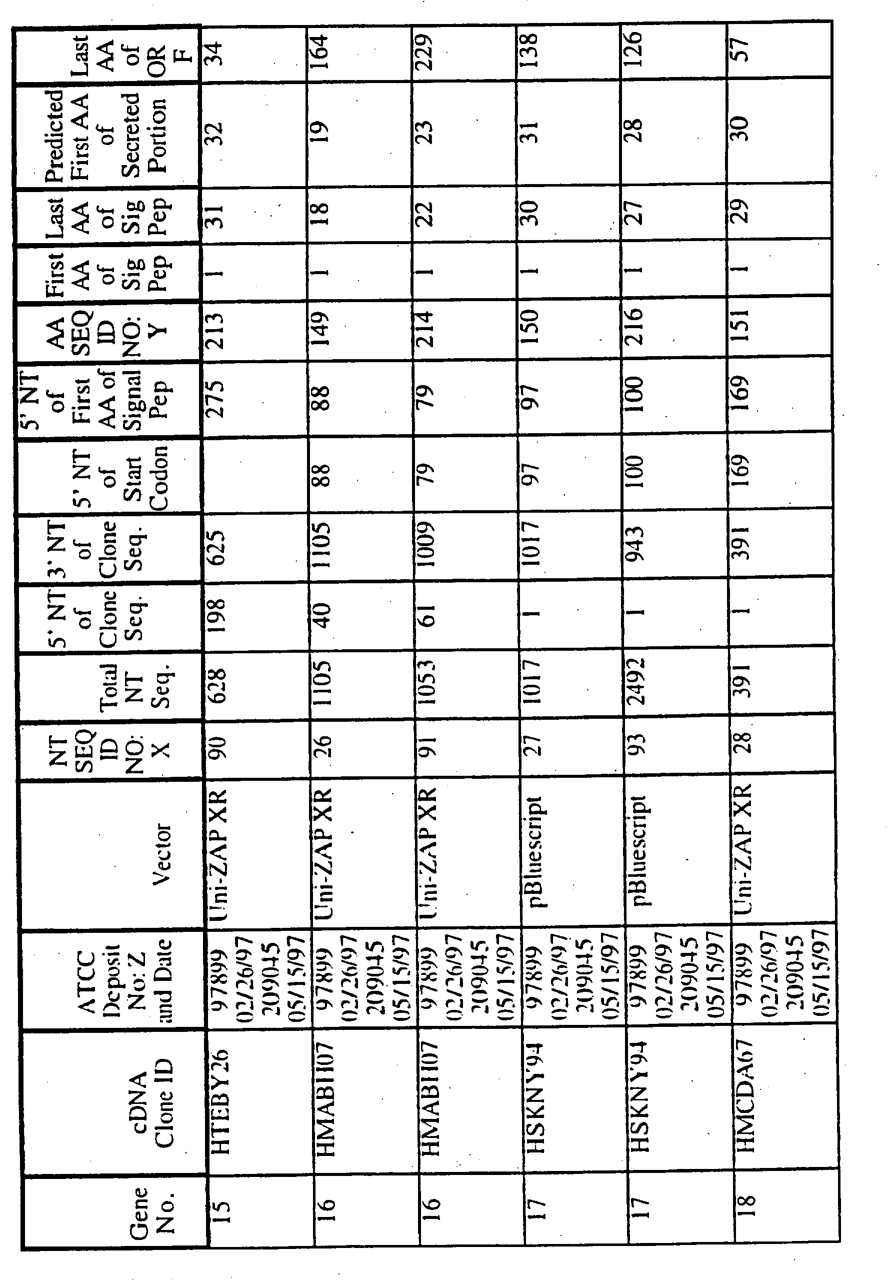

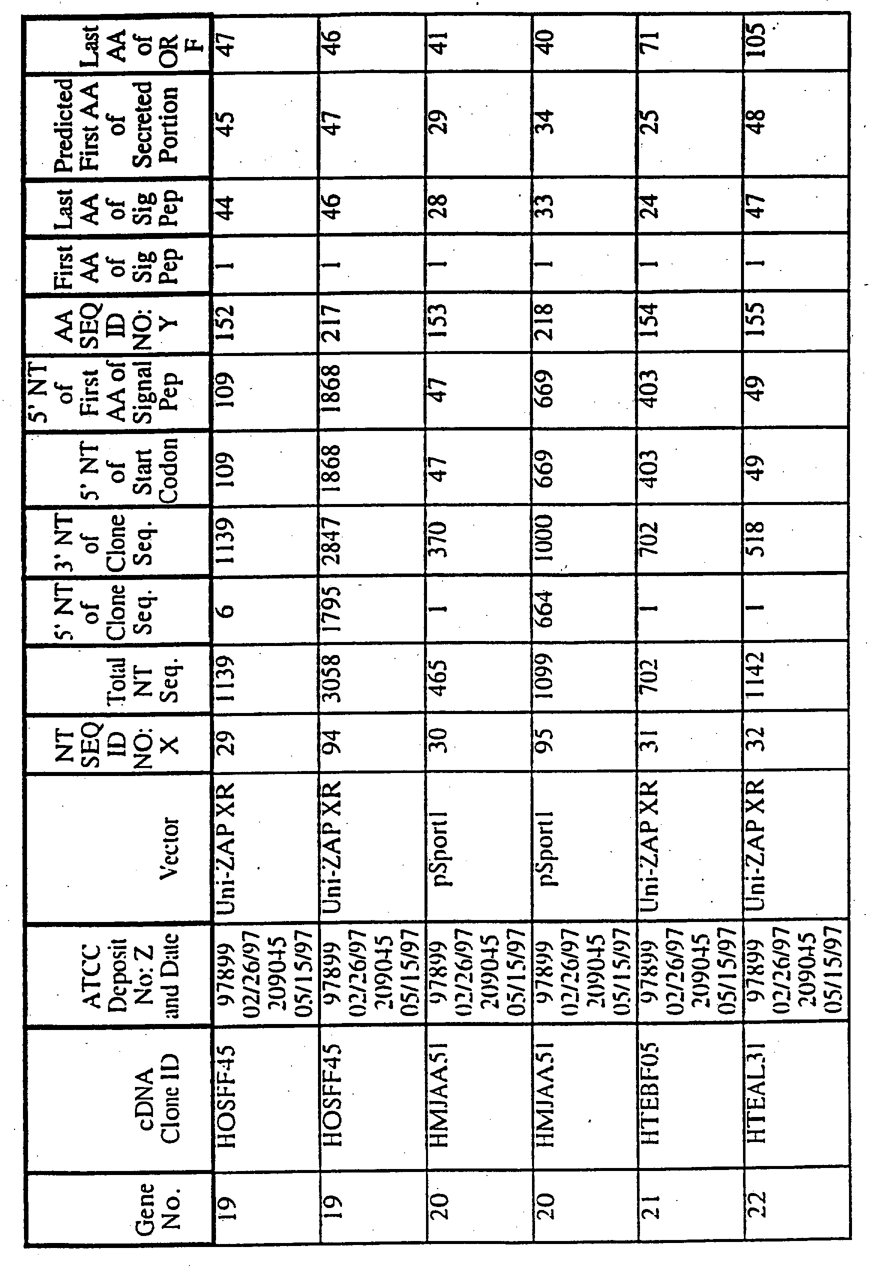

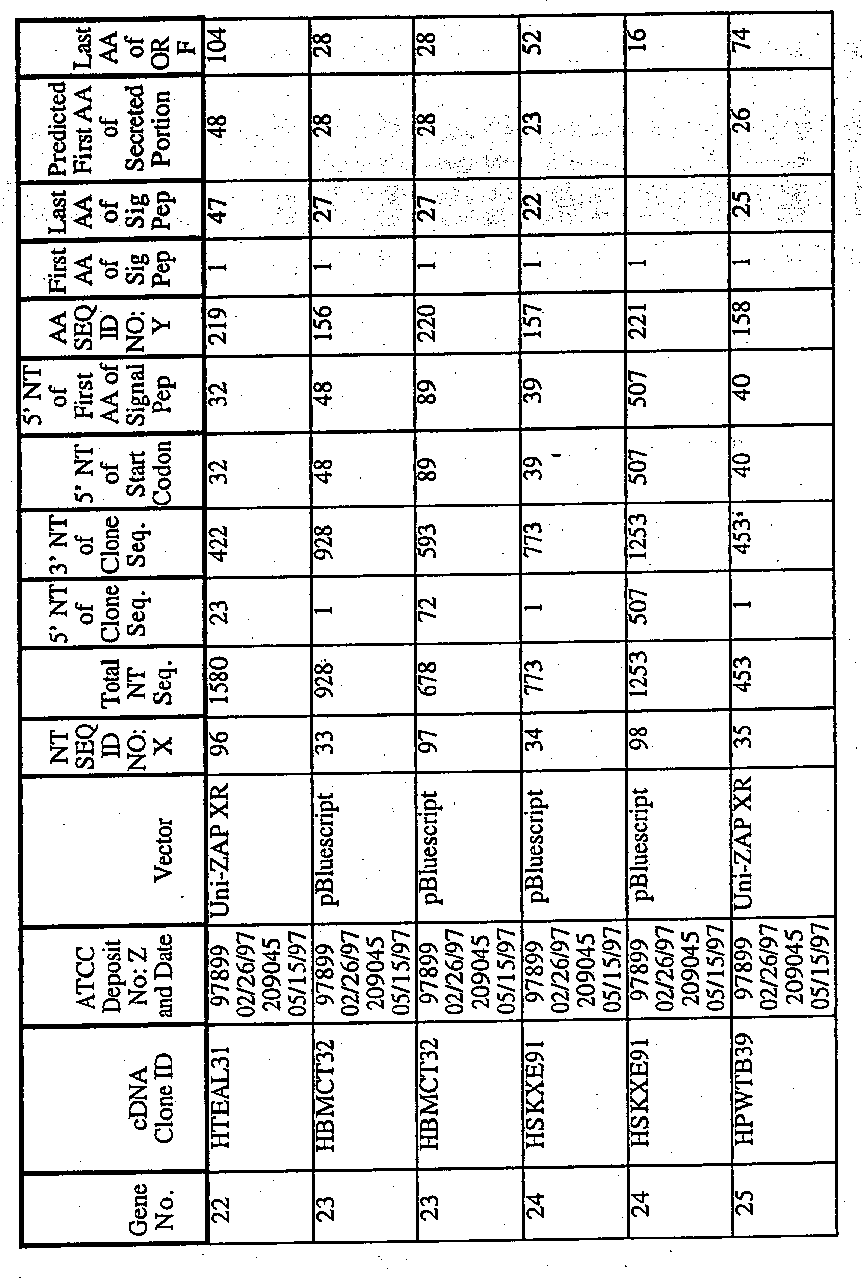

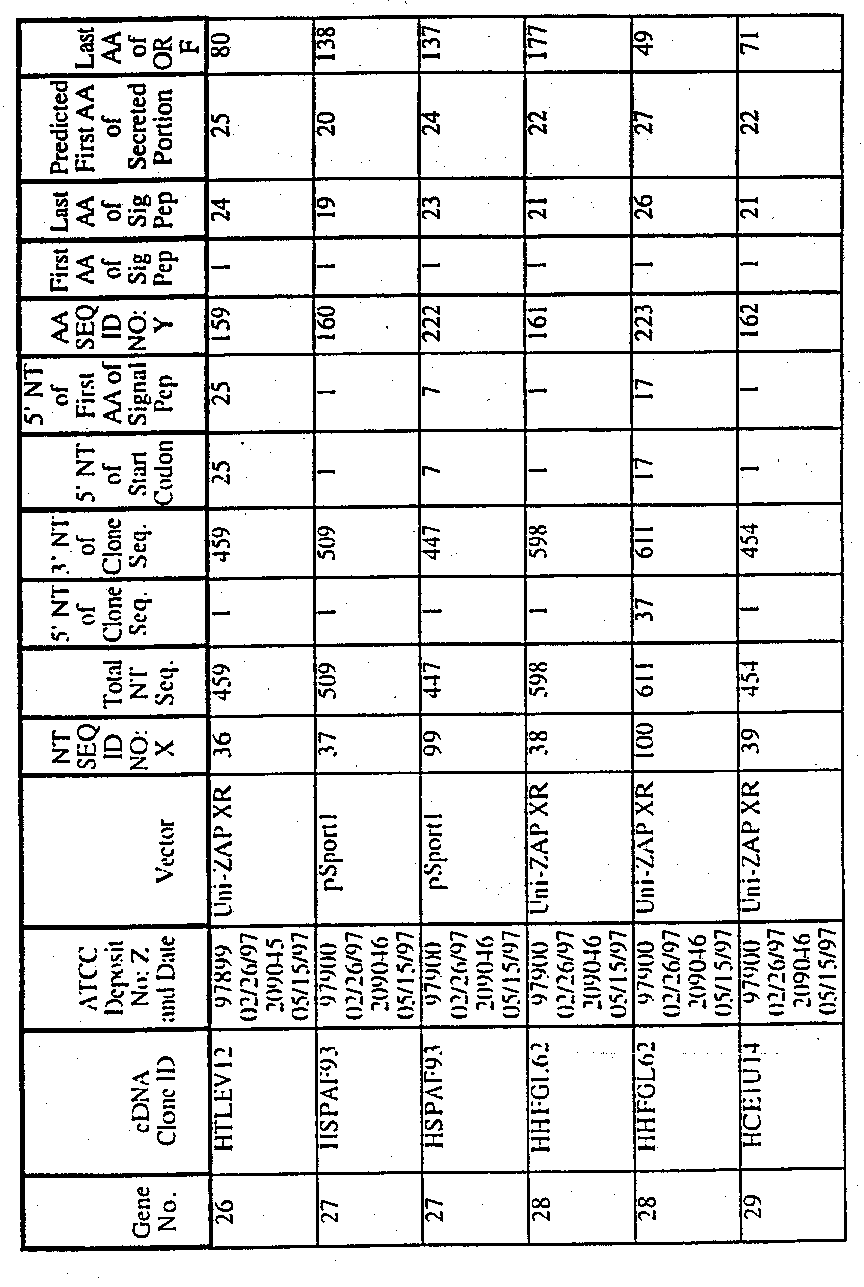

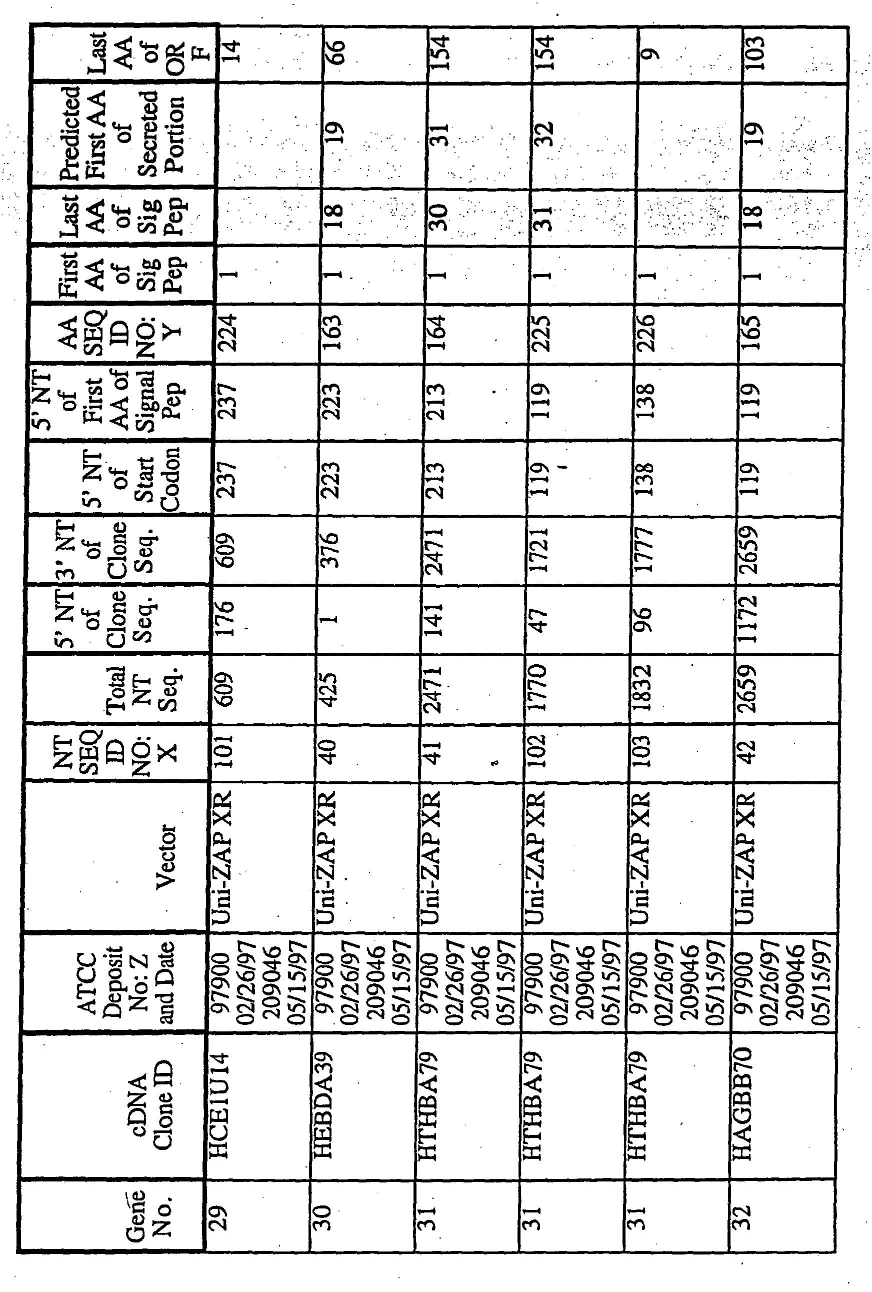

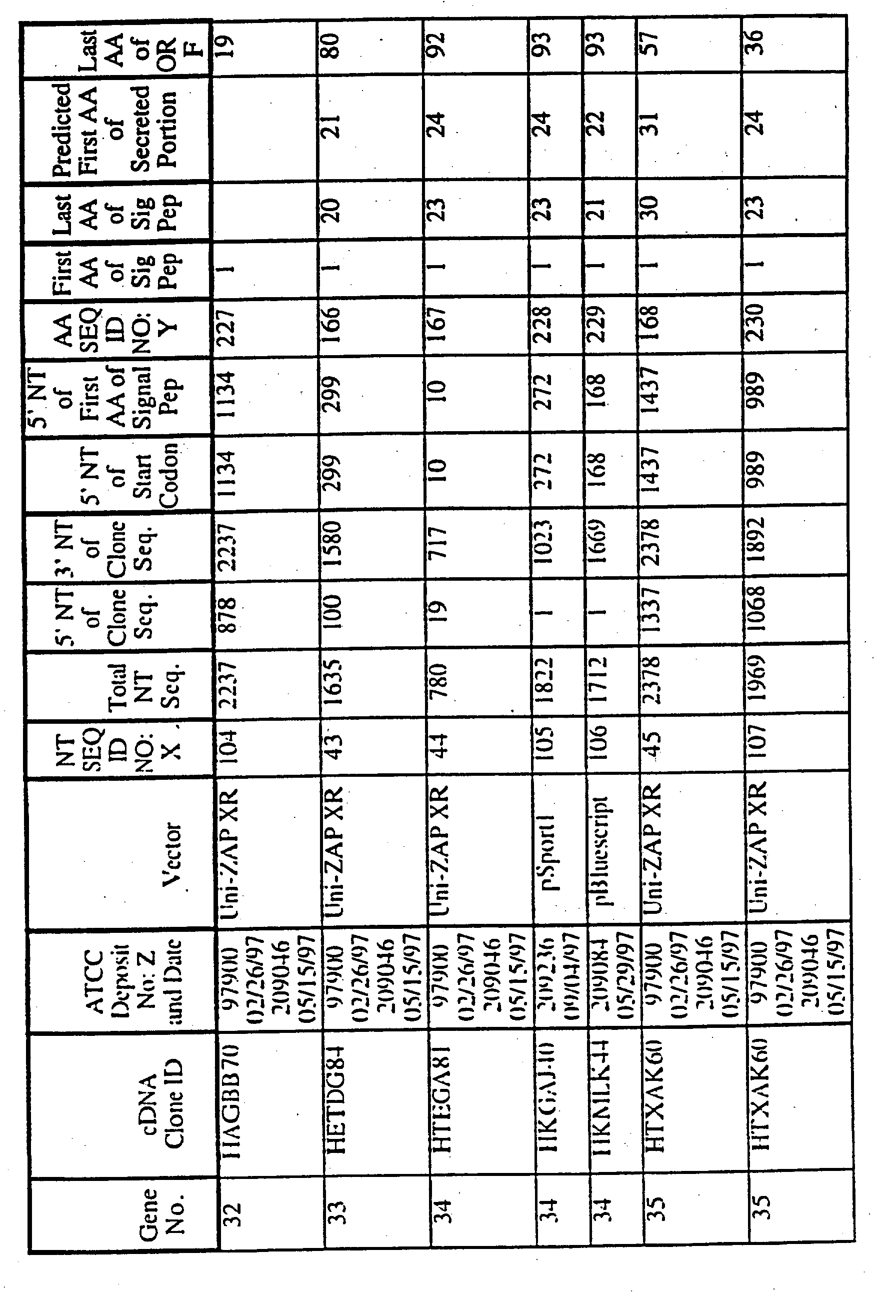

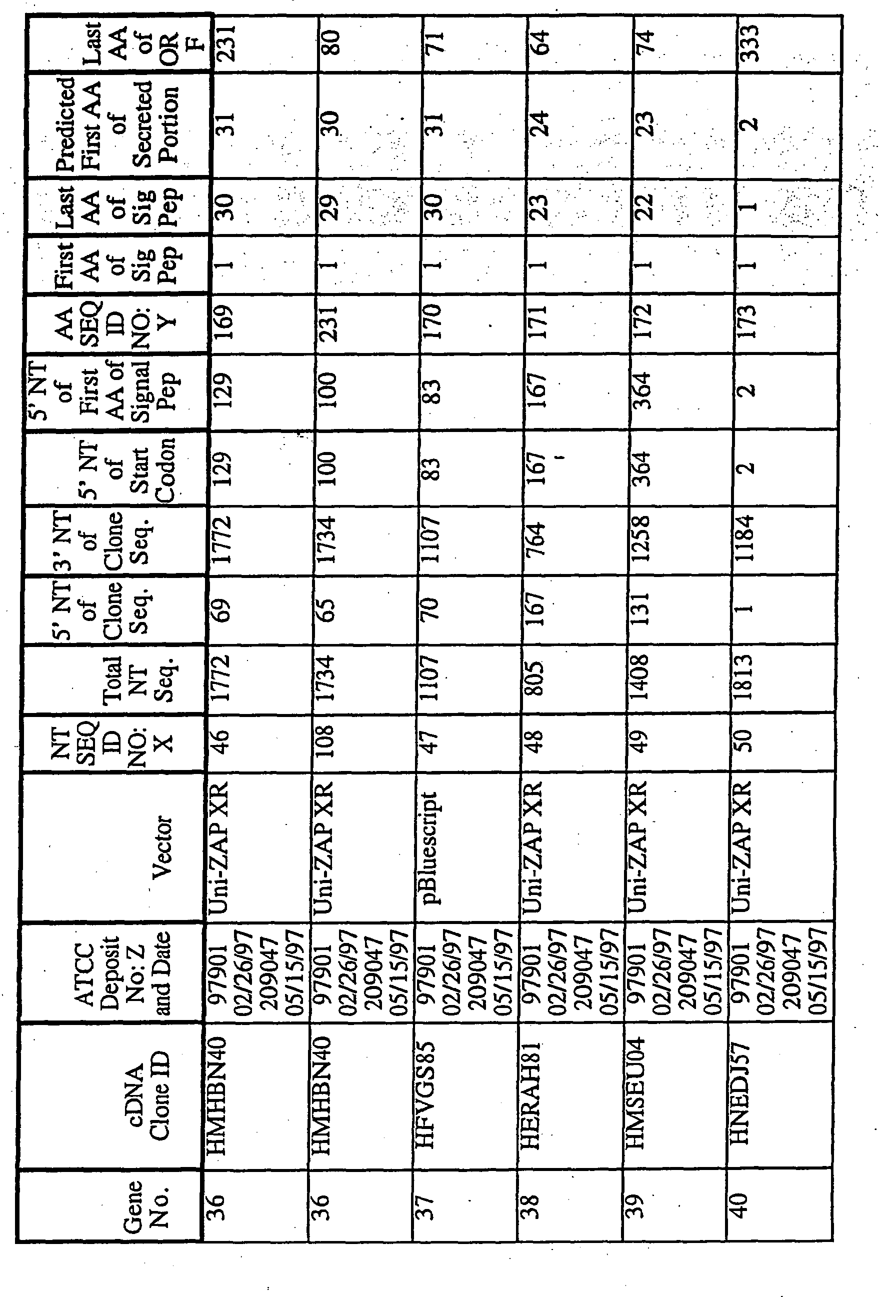

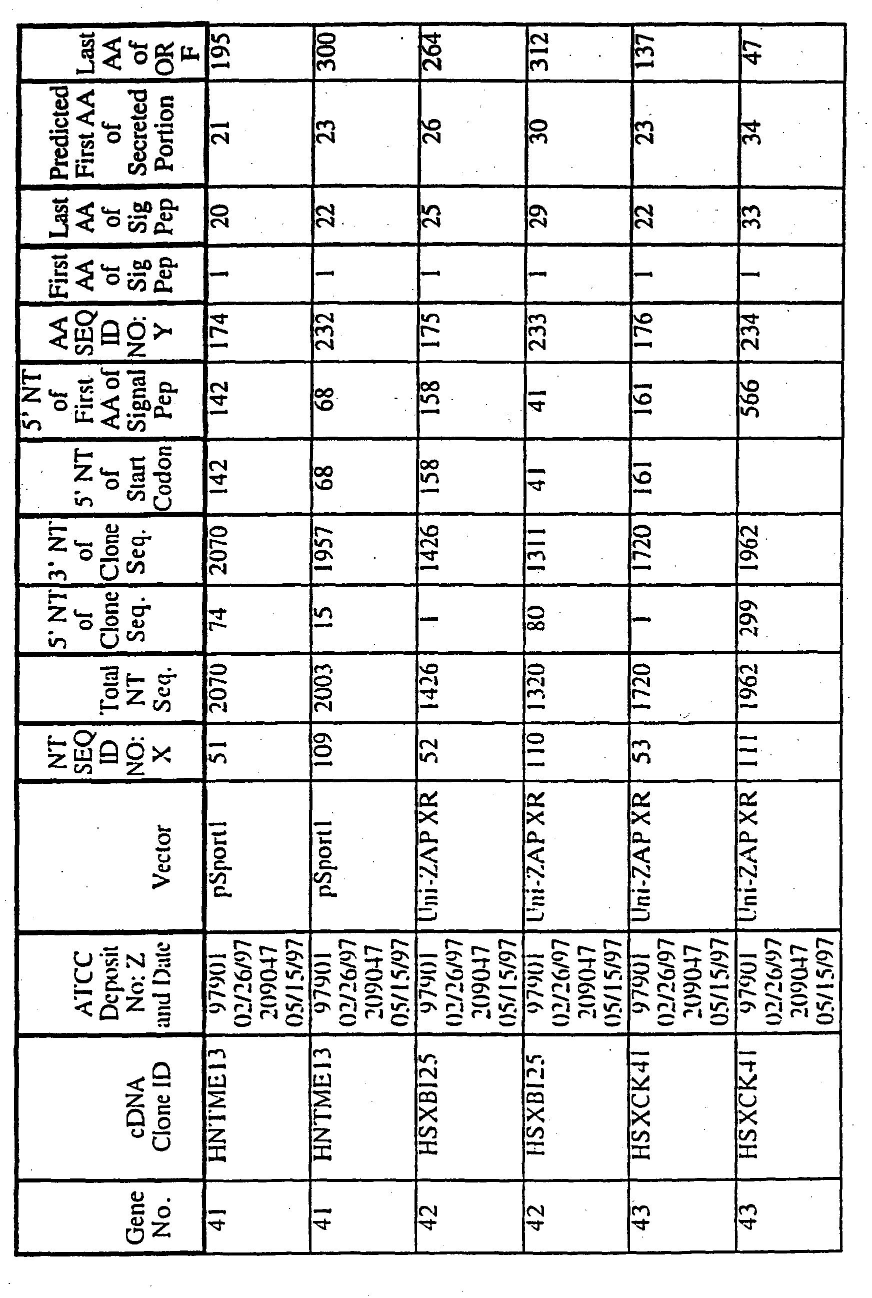

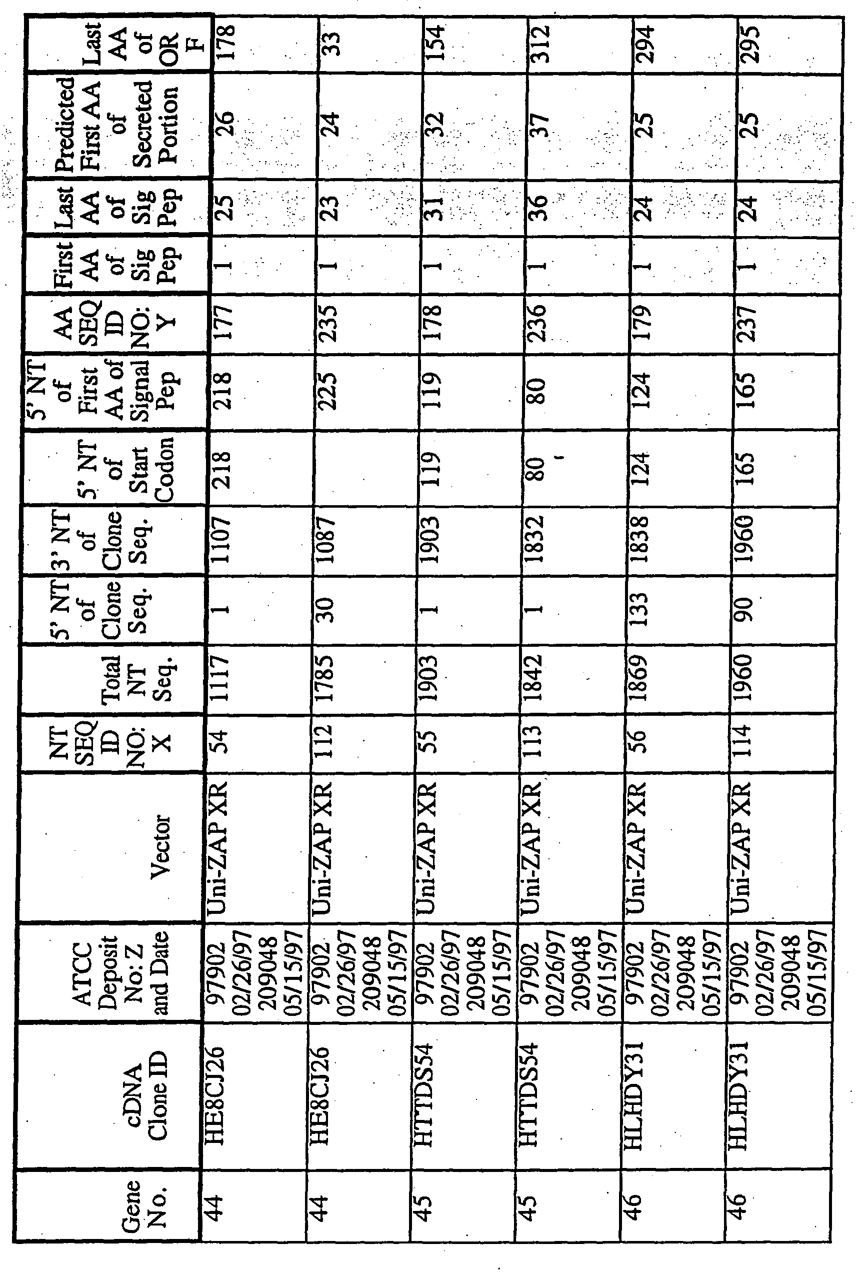

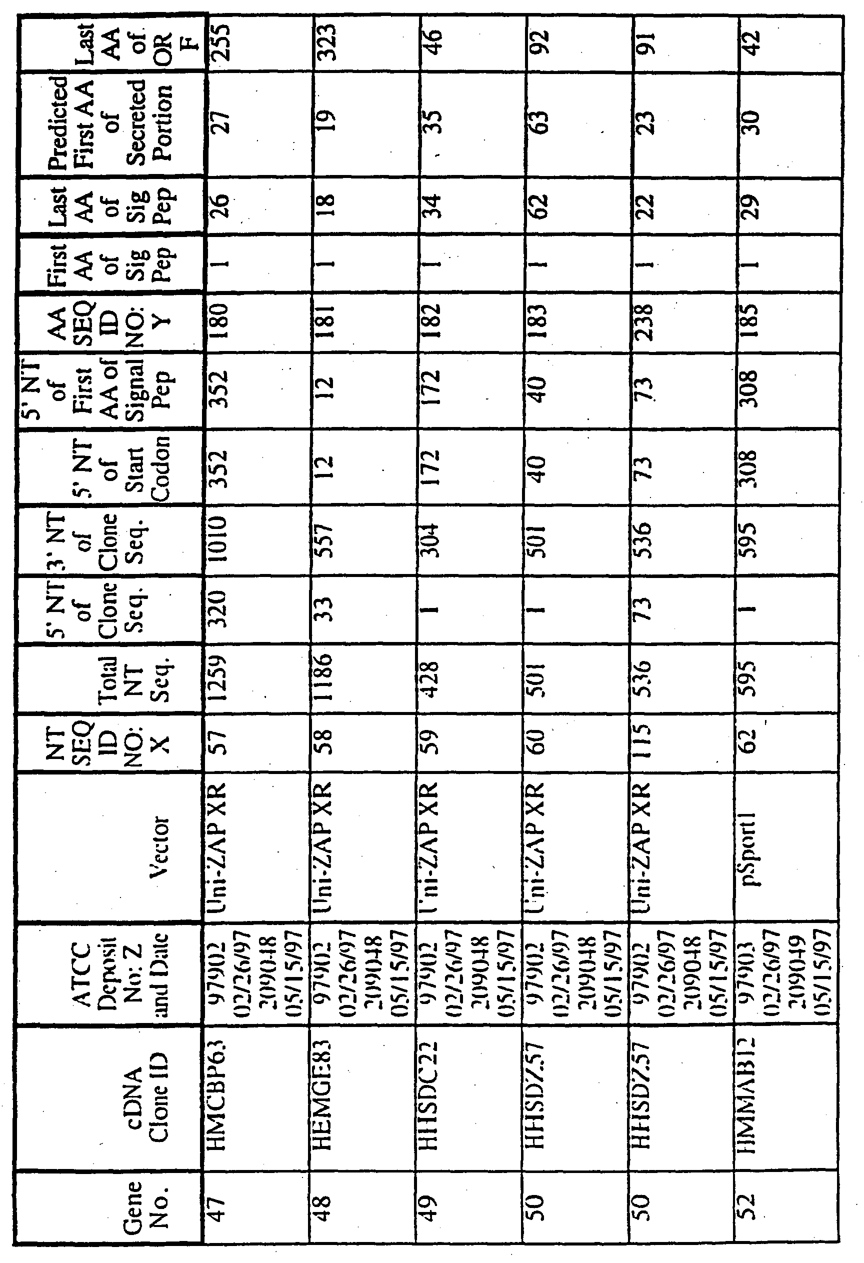

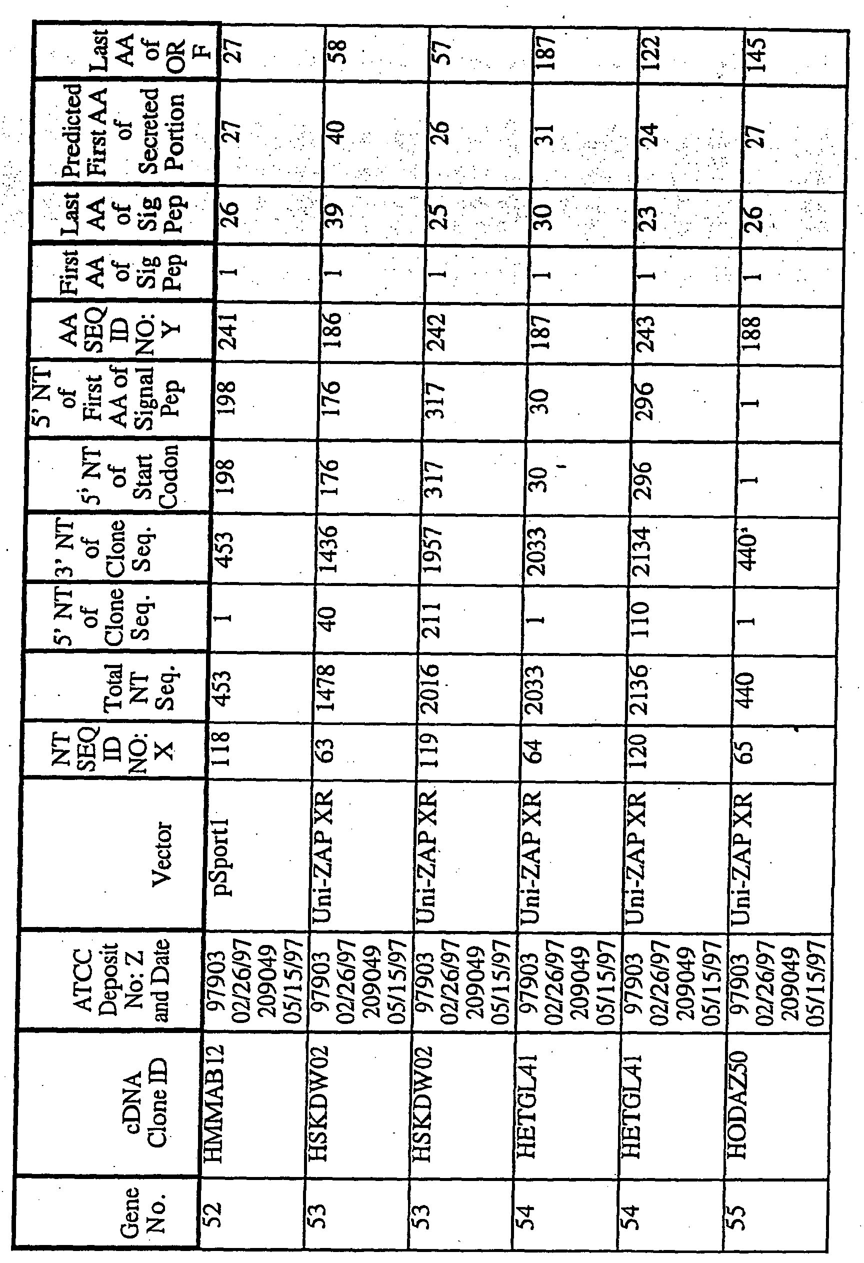

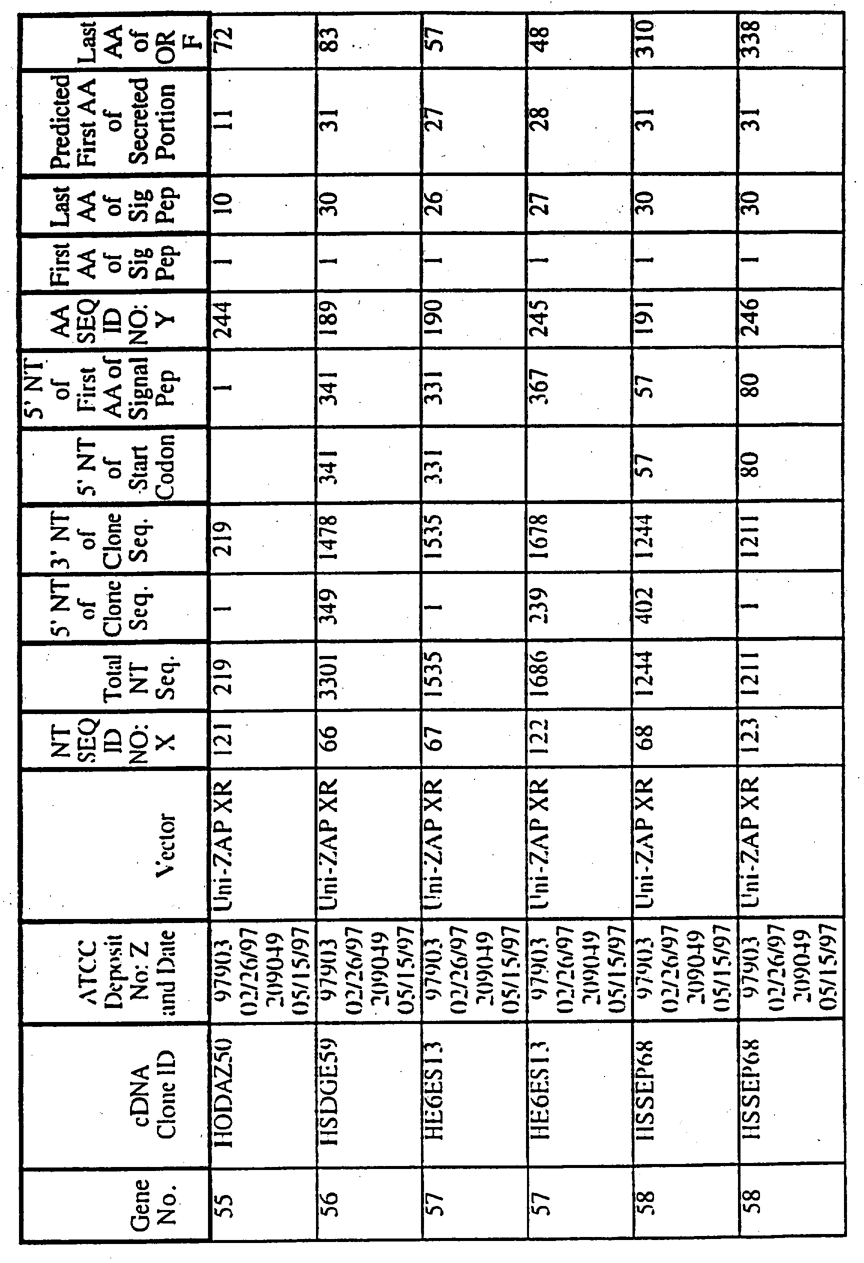

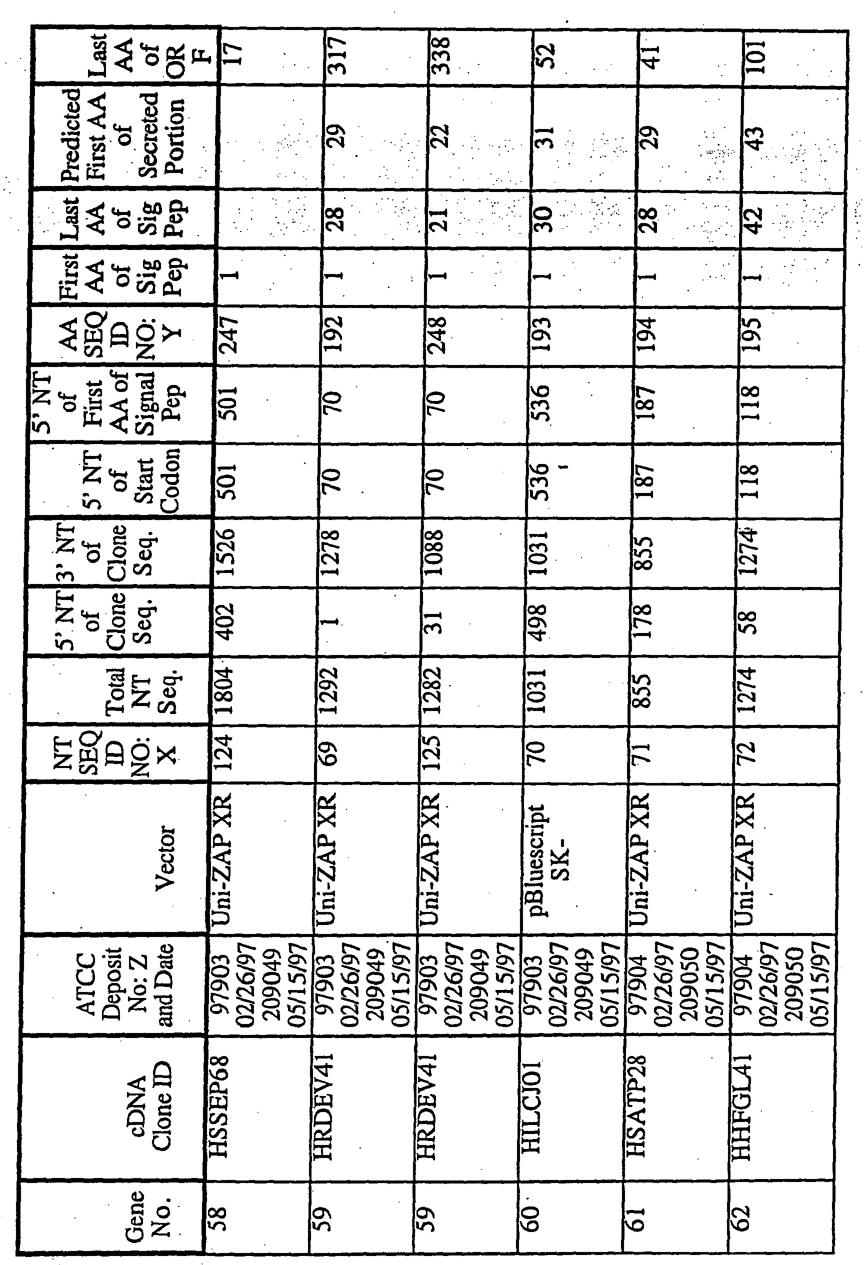

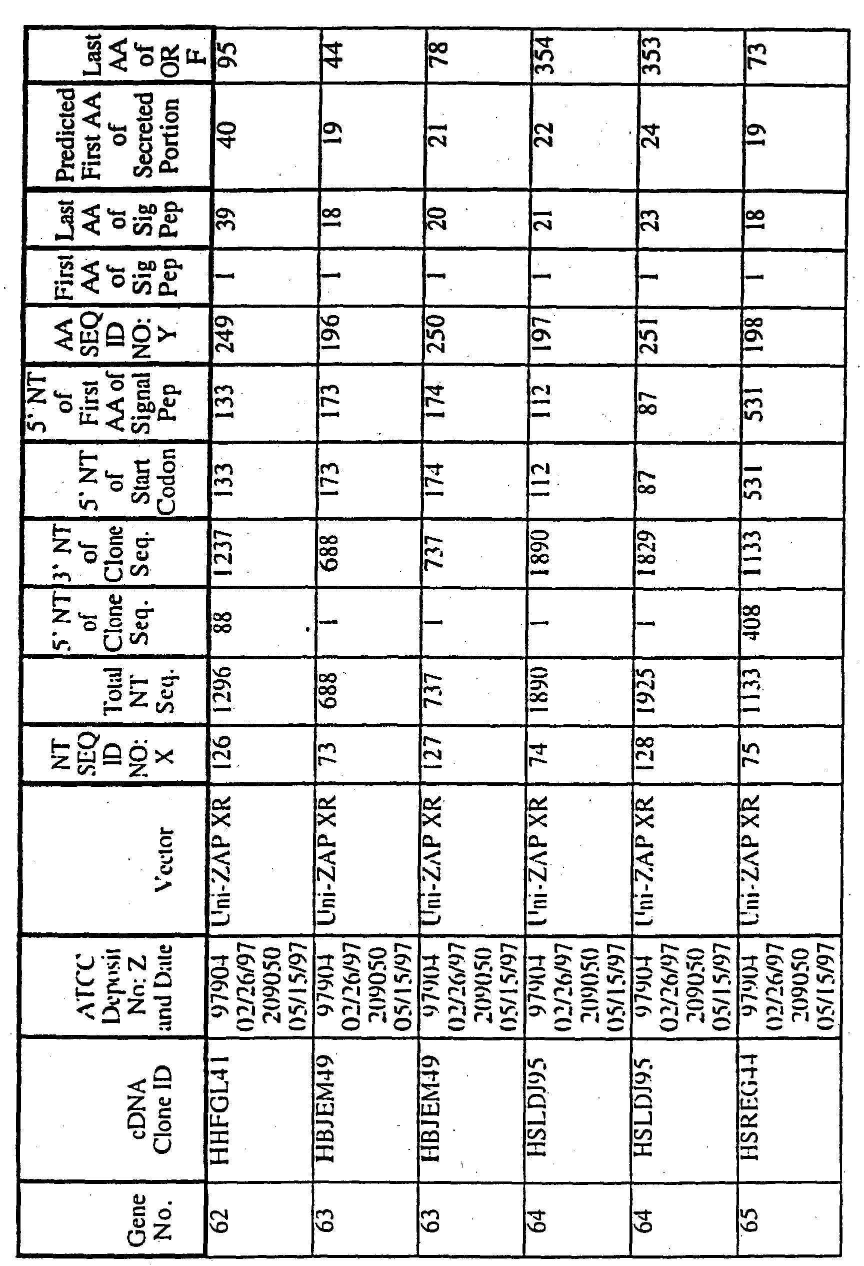

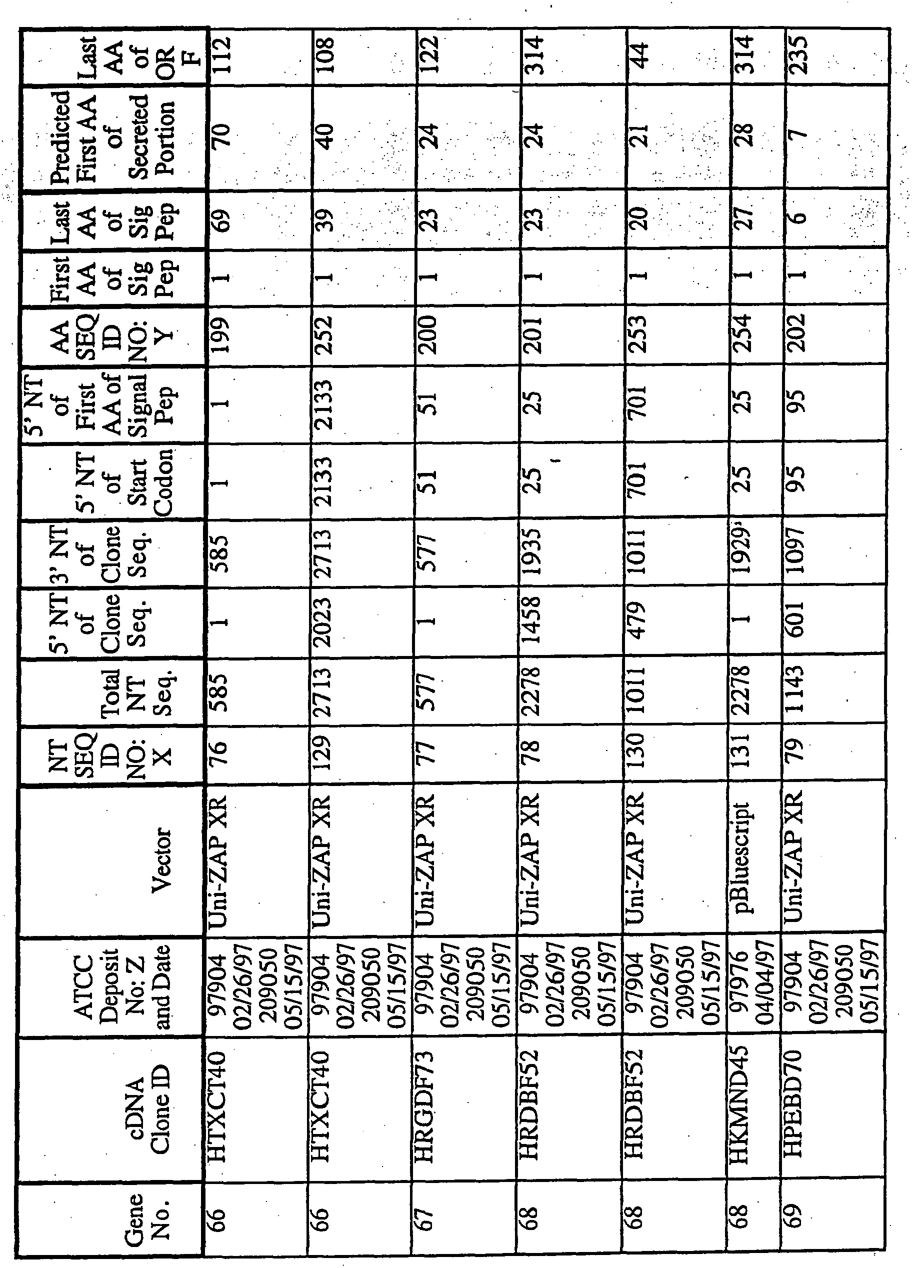

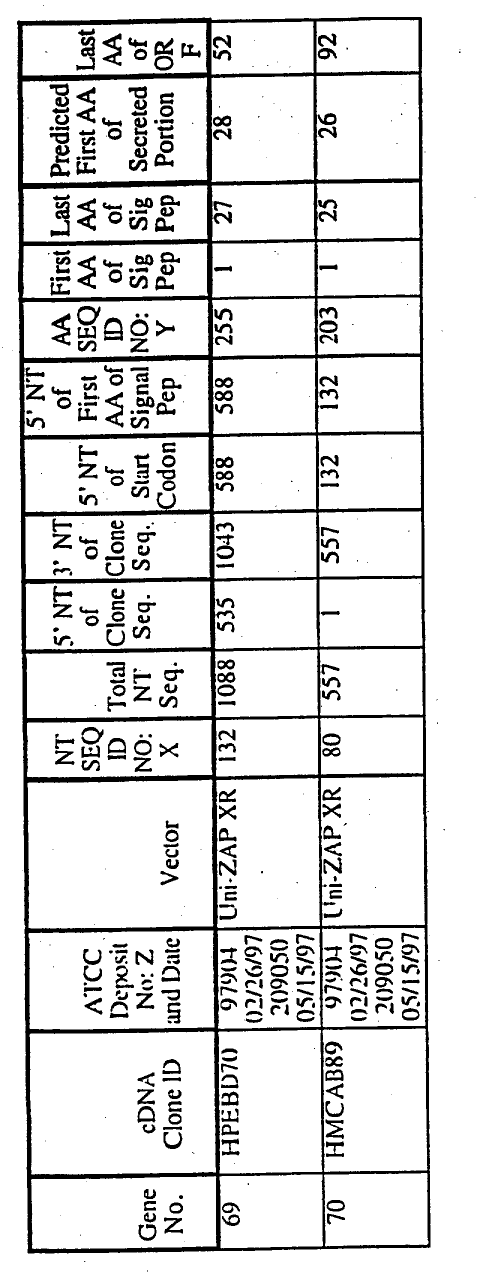

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly, the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification, such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988).
Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and. in many regions, identical to the polynucleotide or polypeptide of the present invention.

"Identity" per se has an art-recognized meaning and can be calculated using published techniques. (See, e.g.: (COMPUTATIONAL MOLECULAR BIOLOGY. Lesk. A.M.. ed., Oxford University Press, New York. (1988): BIOCOMPUTING: INFORMATICS AND GENOME PROJECTS, Smith, D.W., ed., Academic Press. New York, (1993); COMPUTER ANALYSIS OF SEQUENCE DATA, PART I, Griffin, A.M., and Griffin, H.G., eds.. Humana Press. New Jersey, (1994); SEQUENCE ANALYSIS IN MOLECULAR BIOLOGY. von Heinje. G., Academic Press, (1987); and SEQUENCE ANALYSIS PRIMER, Gribskov. M. and Devereux. J., eds., M Stockton Press, New York. (1991).) While there exists a number of methods to measure identity between two polynucleotide or polypeptide sequences. the term "identity" is well known to skilled artisans. (Carillo, H., and Lipton, D., SIAM J Applied Math 48:1073 (1988).) Methods commonly employed to determine identity or similarity between two sequences include, but are not limited to, those disclosed in "Guide to Huge Computers." Martin J. Bishop, ed., Academic Press, San Diego, (1994), and Carillo, H., and Lipton, D., SIAM J Applied Math 48:1073 (1988). Methods for aligning polynucleotides or polypeptides are codified in computer programs, including the GCG program package (Devereux, J., et al., Nucleic Acids Research (1984) 12(1):387 (1984)), BLASTP, BLASTN, FASTA (Atschul, S.F. et al., J. Molec. Biol. 215:403 (1990), Bestfit program (Wisconsin Sequence Analysis Package, Version 8 for Unix, Genetics Computer Group, University Research Park, 575 Science Drive, Madison, WI 53711 (using the local homology algorithm of Smith and Waterman, Advances in Applied Mathematics 2:482-489 (1981).)

When using any of the sequence alignment programs to determine whether a particular sequence is, for instance, 95% identical to a reference sequence, the parameters are set so that the percentage of identity is calculated over the full length of the reference polynucleotide and that gaps in identity of up to 5% of the total number of nucleotides in the reference polynucleotide are allowed.

A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245 (1990).) The term "sequence" includes nucleotide and amino acid sequences. In a sequence alignment the query and subject sequences are either both nucleotide sequences or both amino acid sequences. The result of said global sequence alignment is in percent identity. Preferred parameters used in a FASTDB search of a DNA sequence to calculate percent identiy are: Matrix=Unitary, k-tuple=4, Mismatch Penalty=1, Joining Penalty=30, Randomization Group Length=0, and Cutoff Score=1, Gap Penalty=5, Gap Size Penalty 0.05, and Window Size=500 or query sequence length in nucleotide bases, whichever is shorter. Preferred parameters employed to calculate percent identity and similarity of an amino acid alignment are: Matrix=PAM 150, k-tuple=2, Mismatch Penalty=1, Joining Penalty=20, Randomization Group Length=0, Cutoff Score=1, Gap Penalty=5, Gap Size Penalty=0.05, and Window Size=500 or query sequence length in amino acid residues, whichever is shorter.

As an illustration, a polynucleotide having a nucleotide sequence of at least 95% "identity" to a sequence contained in SEQ ID NO:X or the cDNA contained in the deposited clone, means that the polynucleotide is identical to a sequence contained in SEQ ID NO:X or the cDNA except that the polynucleotide sequence may include up to five point mutations per each 100 nucleotides of the total length (not just within a given 100 nucleotide stretch). In other words, to obtain a polynucleotide having a nucleotide sequence at least 95% identical to SEQ ID NO:X or the deposited clone, up to 5% of the nucleotides in the sequence contained in SEQ ID NO:X or the cDNA can be deleted, inserted, or substituted with other nucleotides. These changes may occur anywhere throughout the polynucleotide.

Further embodiments of the present invention include polynucleotides having at least 85% identity, more preferably at least 90% identity, and most preferably at least 95%, 96%, 97%, 98% or 99% identity to a sequence contained in SEQ ID NO:X or the cDNA contained in the deposited clone. Of course, due to the degeneracy of the genetic code, one of ordinary skill in the art will immediately recognize that a large number of the polynucleotides having at least 85%, 90%, 95%, 96%, 97%, 98%, or 99% identity will encode a polypeptide identical to an amino acid sequence contained in SEQ ID NO:Y or the expressed protein produced by the deposited clone.

Similarly, by a polypeptide having an amino acid sequence having at least, for example, 95% "identity" to a reference polypeptide, is intended that the amino acid sequence of the polypeptide is identical to the reference polypeptide except that the polypeptide sequence may include up to five amino acid alterations per each 100 amino acids of the total length of the reference polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

Further embodiments of the present invention include polypeptides having at least 80% identity, more preferably at least 85% identity, more preferably at least 90% identity, and most preferably at least 95%, 96%, 97%, 98% or 99% identity to an amino acid sequence contained in SEQ ID NO:Y or the expressed protein produced by the deposited clone. Preferably, the above polypeptides should exhibit at least one biological activity of the protein.

In a preferred embodiment, polypeptides of the present invention include polypeptides having at least 90% similarity, more preferably at least 95% similarity, and still more preferably at least 96%, 97%, 98%, or 99% similarity to an amino acid sequence contained in SEQ ID NO:Y or the expressed protein produced by the deposited clone.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain.a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, and 701 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, and 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a scquence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved phatmacokinetic properties. (EP-A 0232 262.) Alternatively, deleting the Fe part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the claimed invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979): Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 1311, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention could be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the slopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace. or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Dresophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1: and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table I and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid scquence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ a NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVsport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR' (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems. Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories. Inc.). according to manufacturer's protocol number PT120U-1. The purified labeled probe is then used to examine various human tissues for mRNA expression.

Multiple Tissue Northern (MTN) blots containing various human tissues ( 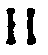 ) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCI by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details sec: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can he successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4°C or frozen at -80°C.

In addition to the above expression vector, the present invention further includes an expression vector comprising phage operator and promoter elements operatively linked to a polynucleotide of the present invention, called pHE4a. (ATCC Accession Number XXXXXX.) This vector contains: 1) a neomycinphosphotransferase gene as a selection marker. 2) an E. coli origin of replication, 3) a T5 phage promoter sequence, 4) two lac operator sequences, 5) a Shine-Delgarno sequence, and 6) the lactose operon repressor gene (lac1q). The origin of replication (oriC) is derived from pUC19 (LT1, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, Xhol, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidies, Corp. or APV Gaulin, Inc.) twice at 4000-6000 psi. The homogenate is then mixed with NaCI solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30.000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCI, 50 mM sodium acetate, pH 6.0 to 1.0 M NaCI, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB 101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired. 6 hours later the medium is removed and is replaced with SF900 Il medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978): Hamlin, J. L. and Ma, C., Biochem. et Biophys. Acta, 1097:107-143 (1990): Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology. 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC

ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1.000 U/ml of penicillin, and about 100 µg/ml of streptomycin.

The splenocytes of such mice are extracted and fused with a suitable myeloma cell line. Any suitable mycloma cell line may be employed in accordance with the present invention; however, it is preferable to employ the parent mycloma cell line (SP20), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4.816,567; Taniguchi et al., EP 171496; Morrison et al., EP 173494; Neuberger et al., WO 8601533; Robinson et al., WO 8702671; Boulianne et al., Nature 312:643(1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectaminc/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (see below) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1 %BSA for 45 hours or CHO-5 for 72 hours.

On day four, using a 300ul multichannel pipetter, aliquot 600ul in one 1 ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### HGS-CHO-5 medium formulation:

### Inorganic Salts

| | |
|---|---|
| CaCl2 (anhyd) | 116.6 mg/L |
| CuSO₄-5H₂O | 0.00130 |
| Fe(NO₃)₃-9H₂O | 0.050 |
| FeSO₄-7H₂O | 0.417 |
| KCl | 311.80 |
| MgCl₂ | 28.64 |
| MgSO₄ | 48.84 |
| NaCI | 6995.50 |
| NaHCO₃ | 2400.0 |
| NaH₂PO₄-H₂O | 62.50 |
| Na₂HPO4 | 71.02 |
| ZnSO₄-7H₂O | .4320 |

### Lipids

| | |
|---|---|
| Arachidonic Acid | .002 mg/L |
| Cholesterol | 1.022 |
| DL-alpha-Tocopherol-Acetate | .070 |
| Linoleic Acid | 0.0520 |
| Linolenic Acid | 0.010 |
| Myristic Acid | 0.010 |
| Oleic Acid | 0.010 |
| Palmitric Acid | 0.010 |
| Palmitic Acid | 0.010 |
| Pluronic F-68 | 100 |
| Stearic Acid | 0.010 |
| Tween 80 | 2.20 |

### Carbon Source

| | |
|---|---|
| D-Glucose | 4551 mg/L |

### Amino Acids

| | |
|---|---|
| L- Alanine | 130.85 mg/ml |
| L-Arginine-HCL | 147.50 |
| L-Asparagine-H₂0 | 7.50 |
| L-Aspartic Acid | 6.65 |
| L-Cystine-2HCL-H₂0 | 29.56 |
| L-Cystine-2HCL | 31.29 |
| L-Glutamic Acid | 7.35 |
| L-Glutamine | 365.0 |
| Glycine | 18.75 |
| L-Histidine-HCL-H₂0 | 52.48 |
| L-Isoleucine | 106.97 |
| L-Leucine | 111.45 |
| L-Lysine HCL | 163.75 |
| L-Methionine | 32.34 |
| L-Phenylalainine | 68.48 |
| L-Proline | 40.0 |
| L-Serine | 26.25 |
| L-Threonine | 101.05 |
| L-Tryptophan | 19.22 |
| L-Tryrosine-2Na-2H₂0 | 91.79 |
| L-Valine | 99.65 |

### Vitamins

| | |
|---|---|
| Biotin | 0.0035 mg/L |
| D-Ca Pantothenate | 3.24 |
| Choline Chloride | 11.78 |
| Folic Acid | 4.65 |
| i-Inositol | 15.60 |
| Niacinamide | 3.02 |
| Pyridoxal HCL | 3.00 |
| Pyridoxine HCL | 0.031 |
| Riboflavin | 0.319 |
| Thiamine HCL | 3.17 |
| Thymidine | 0.365 |
| Vitamin B₁₂, | 0.680 |

### Other Components

| | |
|---|---|
| HEPES Buffer | 25 mM |
| Na Hypoxanthine | 2.39 mg/L |
| Lipoic Acid | 0.105 |
| Sodium Putrescine-2HCL | 0.081 |
| Sodium Pyruvate | 55.0 |
| Sodium Selenite | 0.0067 |
| Ethanolamine | 20uM |
| Ferric Citrate | 0.122 |
| Methyl-B-Cyclodextrin complexed with Linoleic Acid | 41.70 |
| Methyl-B-Cyclodextrin complexed with Oleic Acid | 33.33 |
| Methyl-B-Cyclodextrin complexed with Retinal Acetate | 10 |

### Adjust osmolarity to 327 mOsm

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2. Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |
| | | | | | | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |
| | | | | | | |

| g-C family | | | | | | |
|---|---|---|---|---|---|---|
| IL-2 (lymphocytes) | - | + | - | + | 1,3,5 | GAS |
| IL-4 (lymph/mycloid) | - | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |
| | | | | | | |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |
| | | | | | | |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |
| | | | | | | |

| Receptor Tyrosine Kinases | | | | | | |
|---|---|---|---|---|---|---|
| EGF | ? | + | + | - | 1.3 | GAS (IRF1) |
| PDGF | ? | + | + | - | 1.3 | |
| CSF-1 | ? | + | + | - | 1.3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologics)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0. 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAF-Dextran, 8 ug GAS-SEAP2 plasmid DNA, 140 mM NaCl, 5 mM KCI, 375 uM Na₃HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x 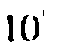 cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 95-well plate, and allowed to air dry for 2 hr.

PC12 cells are routinely grown in RPM1-1640 medium (Bio Whittaker) containing 10% horse serum (JRH BIOSCIENCES. Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC 12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin. and by expression of certain viral gene products. As a transcription factor. NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I-κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an Xhol site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5';GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using Xhol and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes Sall and NotI, and inserted into a vector containing neomycin resistance. Particularly, the NF-κB/SV40/SEAP cassette was inserted into pGFP-1 (Clontech), replacing the GFP gene, after restricting pGFP-1 with Sall and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm: and (6) Sample addition is 50 ul. Increased emission at 530 nm indicates an extracellular signaling even which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11. the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for I hr at RT. The protein G plates are then treated with 2 commercial monoclonal antibodies (100ng/well) against Erk- 1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products is then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals is identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect soluble polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36.676; EP 88.046; EP 143.949; EP 142.641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml. at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin, is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is being produced.

The engineered fibroblasts are then transplanted onto the host, either alone or after having been grown to confluence on cytodex 3 microcarrier beads. It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

## Claims

1. A nucleic acid molecule comprising a polynucleotide having a nucleotide sequence selected from the group consisting of:
(a) a polynucleotide having the entire nucleotide sequence of SEQ ID NO: 91;
(b) a polynucleotide having the entire cDNA sequence of a clone included in ATCC Deposit No. 97899, which is hybridizable to SEQ ID NO: 91;
(c) a polynucleotide fragment of SEQ ID NO: 91;
(d) a polynucleotide fragment of the cDNA sequence of a clone included in ATCC Deposit No. 97899, which is hybridizable to SEQ ID NO: 91;
(e) a polynucleotide encoding a polypeptide fragment of SEQ ID NO: 214;
(f) a polynucleotide encoding a polypeptide fragment encoded by the cDNA sequence of a clone included in ATCC Deposit No. 97899, which is hybridizable to SEQ ID NO: 91;
(g) a polynucleotide encoding a polypeptide fragment of SEQ ID NO: 214 or a polypeptide fragment encoded by the cDNA sequence of a clone included in ATCC Deposit No. 97899, having biological activity;
(h) a polynucleotide encoding a polypeptide fragment of SEQ ID NO: 214 or a polypeptide fragment encoded by the cDNA sequence of a clone included in ATCC Deposit No. 97899, which is secreted;
(i) a polynucleotide encoding a full length polypeptide of SEQ ID NO: 214 or being encoded by the cDNA sequence of a clone included in ATCC Deposit No. 97899;
(j) a polynucleotide encoding a polypeptide domain of SEQ ID NO: 214;
(k) a polynucleotide encoding a polypeptide domain encoded by the cDNA sequence of a clone included in ATCC Deposit No. 97899, which is hybridizable to SEQ ID NO: 91;
(l) a polynucleotide encoding a polypeptide epitope of SEQ ID NO: 214;
(m) a polynucleotide encoding a polypeptide epitope encoded by the cDNA sequence of a clone included in ATCC Deposit No. 97899, which is hybridizable to SEQ ID NO: 91;
(n) a polynucleotide which is a variant of SEQ ID NO: 91;
(o) a polynucleotide encoding a variant of SEQ ID NO: 214;
(p) a polynucleotide which is an allelic variant of SEQ ID NO: 91;
(q) a polynucleotide encoding an allelic variant of SEQ ID NO: 214;
(r) a polynucleotide which encodes a species homologue of SEQ ID NO: 214;
(s) a polynucleotide which is at least 90% identical to a polynucleotide as defined in any one of (a) to (r);
(t) a polynucleotide encoding a polypeptide having an amino acid sequence at least 90% identical to the amino acid sequence of a polypeptide encoded by a polynucleotide of any one of (a) to (s); and
(u) a polynucleotide capable of hybridizing under stringent conditions to any one of the polynucleotides specified in (a) to (t), wherein said polynucleotide does not hybridize under stringent conditions to a nucleic acid molecule having a nucleotide sequence of only A residues or of only T residues;
or the complementary strand of polynucleotide (a) to (u).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. The gene corresponding to the cDNA sequence of SEQ ID NO: 91 or encoding the polypeptide having the amino acid sequence of SEQ ID NO: 214.

5. A vector comprising the nucleic acid molecule of any one of claims 1 to 3.

6. A method of making a recombinant host cell comprising introducing the nucleic acid molecule of any one of claims 1 to 3, or the vector of claim 5.

7. A recombinant host cell containing the nucleic acid molecule of any one of claims 1 to 3 or the vector of claim 5 or produced by the method of claim 6.

8. The recombinant host cell of claim 7 which expresses a polypeptide encoded by the nucleic acid molecule of any one of claims 1 to 3.

9. A method of making a polypeptide encoded by the nucleic acid molecule of any one of claims 1 to 3 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3 or obtainable by the method of claim 9.

11. A polypeptide comprising a polypeptide selected from the group consisting of:
(a) a polypeptide fragment of SEQ ID NO: 214;
(b) a polypeptide fragment of SEQ ID NO: 214 having biological activity;
(c) a polypeptide fragment of SEQ ID NO: 214, which is secreted;
(d) a full length polypeptide of SEQ ID NO: 214;
(e) a polypeptide domain of SEQ ID NO: 214;
(f) a polypeptide epitope of SEQ ID NO: 214;
(g) a polypeptide variant of SEQ ID NO: 214;
(h) an allelic variant of SEQ ID NO: 214;
(i) a species homologue of the SEQ ID NO: 214; and
(j) a polypeptide which is at least 90% identical to a polypeptide as defined in any one of (a) to (i).

12. An antibody that binds specifically to the polypeptide of claim 10 or 11.

13. An antagonist of the polypeptide of claim 10 or 11 comprising a molecule closely related to the natural ligand of said polypeptide or a polypeptide related to the natural receptor of the polypeptide of claim 10 or 11 or a fragment thereof.

14. An agonist of the polypeptide of claim 10 or 11 comprising a molecule closely related to the natural ligand of said polypeptide or a polypeptide related to the natural receptor of the polypeptide of claim 10 or 11 or a fragment thereof.

15. A method for identifying antagonists or agonists of the polypeptide of claim 10 or 11 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

16. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

17. An in vitro method of diagnosing a pathological condition or a susceptibility to a pathological condition in a subject comprising:
(a) determining the presence or amount of expression of the polypeptide of claim 10 or 11 in a biological sample; and
(b) diagnosing a pathological condition or a susceptibility to a pathological condition based on the presence or amount of expression of the polypeptide.

18. A method for identifying a binding partner to the polypeptide of claim 10 or 11 comprising:
(a) contacting the polypeptide of claim 10 or 11 with a binding partner; and
(b) determining whether the binding partner effects an activity of the polypeptide.

19. A method of identifying an activity in a biological assay, wherein the method comprises:
(a) expressing the nucleic acid molecule of any one of claims 1 to 3 in a cell;
(b) isolating the supernatant;
(c) detecting an activity in a biological assay; and
(d) identifying the protein in the supernatant having the activity.

20. The product produced by the method of claim 19 or identified by the method of claim 15 or 18.

21. A pharmaceutical composition comprising the nucleic acid molecule of any one of claims 1 to 3, the polypeptide of claim 10 or 11, a DNA encoding and capable of expressing said polypeptide in vivo, the antibody of claim 12, the antagonist of claim 13 or the agonist of claim 14, or the product of claim 20 and optionally a pharmaceutically acceptable carrier.

22. Use of the nucleic acid molecule of any one of claims 1 to 3, the polypeptide of claim 10 or 11, the antibody of claim 12, the antagonist of claim 13 or the agonist of claim 14, or the product of claim 20 or a DNA encoding and capable of expressing said polypeptide in vivo for the preparation of a pharmaceutical composition for preventing, treating, or ameliorating a medical condition.

23. A diagnostic composition comprising the nucleic acid molecule of any one of claims 1 to 3 or the antibody of claim 12.

24. A method for the production of a pharmaceutical composition comprising the steps of the method of claim 15 and (d) formulating the compound identified in step (c) in a pharmaceutically acceptable form.


(1) GENERAL INFORMATION:

(i) APPLICANTS: Human Genome Sciences, Inc. et al.

(ii) TITLE OF INVENTION: 70 Human Secreted Proteins

(iii) NUMBER OF SEQUENCES: 273

(iv) CORRESPONDENCE ADDRESS:

(A) ADDRESSEE: Human Genome Sciences, Inc.

(B) STREET: 9410 Key West Avenue

(C) CITY: Rockville

(D) STATE: Maryland

(E) COUNTRY: USA

(F) ZIP: 20850

(v) COMPUTER READABLE FORM:

(A) MEDIUM TYPE: Diskette, 3.50 inch, 1.4Mb storage

(B) COMPUTER: HP Vectra 486/33

(C) OPERATING SYSTEM: MSDOS version 6.2

(D) SOFTWARE: ASCII Text

(vi) CURRENT APPLICATION DATA:

(A) APPLICATION NUMBER:

(B) FILING DATE: March 6, 1998

(C) CLASSIFICATION:

(vii) PRIOR APPLICATION DATA:

(A) APPLICATION NUMBER:

(B) FILING DATE:

(viii) ATTORNEY/AGENT INFORMATION:

(A) NAME: A. Anders Brookes

(B) REGISTRATION NUMBER: 36,373

(C) REFERENCE/DOCKET NUMBER: PS001PCT

(vi) TELECOMMUNICATION INFORMATION:

(A) TELEPHONE: (301) 309-8504

(B) TELEFAX: (301) 309-8439

(2) INFORMATION FOR SEQ ID NO: 1:

(i) SEQUENCE CHARACTERISTICS:

(A) LENGTH: 733 base pairs

(B) TYPE: nucleic acid

(C) STRANDEDNESS: double

(D) TOPOLOGY: linear

(xi) SEQUENCE DESCRIPTION: SEQ ID NO: 1:

GGGATCCGGA GCCCAAATCT TCTGACAAAA CTCACACATG CCCACCGTGC CCAGCACCTG 60

AATTCGAGGG TGCACCGTCA GTCTTCCTCT TCCCCCCAAA ACCCAAGGAC ACCCTCATGA 120

TCTCCCGGAC TCCTGAGGTC ACATGCGTGG TGGTGGACGT AAGCCACGAA GACCCTGAGG 180

TCAAGTTCAA CTGGTACGTG GACGGCGTGG AGGTGCATAA TGCCAAGACA AAGCCGCGGG 240

AGGAGCAGTA CAACAGCACG TACCGTGTGG TCAGCGTCCT CACCGTCCTG CACCAGGACT 300

GGCTGAATGG CAAGGAGTAC AAGTGCAAGG TCTCCAACAA AGCCCTCCCA ACCCCCATCG 360

AGAAAACCAT CTCCAAAGCC AAAGGGCAGC CCCGAGAACC ACAGGTGTAC ACCCTGCCCC 420

CATCCCGGGA TGAGCTGACC AAGAACCAGG TCAGCCTGAC CTGCCTGGTC AAAGGCTTCT 480

ATCCAAGCGA CATCGCCGTG GAGTGGGAGA GCAATGGGCA GCCGGAGAAC AACTACAAGA 540

CCACGCCTCC CGTGCTGGAC TCCGACGGCT CCTTCTTCCT CTACAGCAAG CTCACCGTGG 600

ACAAGAGCAG GTGGCAGCAG GGGAACGTCT TCTCATGCTC CGTGATGCAT GAGGCTCTGC 660

ACAACCACTA CACGCAGAAG AGCCTCTCCC TGTCTCCGGG TAAATGAGTG CGACGGCCGC 720

GACTCTAGAG GAT 733

(2) INFORMATION FOR SEQ ID NO: 2:

(i) SEQUENCE CHARACTERISTICS:

(A) LENGTH: 5 amino acids

(B) TYPE: amino acid

(D) TOPOLOGY: linear

(xi) SEQUENCE DESCRIPTION: SEQ ID NO: 2:

Trp Ser Xaa Trp Ser
1               5

(2) INFORMATION FOR SEQ ID NO: 3:

(i) SEQUENCE CHARACTERISTICS:

(A) LENGTH: 86 base pairs

(B) TYPE: nucleic acid

(C) STRANDEDNESS: double

(D) TOPOLOGY: linear

(xi) SEQUENCE DESCRIPTION: SEQ ID NO: 3:

GCGCCTCGAG ATTTCCCCGA AATCTAGATT TCCCCGAAAT GATTTCCCCG AAATGATTTC 60

CCCGAAATAT CTGCCATCTC AATTAG 86

(2) INFORMATION FOR SEQ ID NO: 4:

(i) SEQUENCE CHARACTERISTICS:

(A) LENGTH: 27 base pairs

(B) TYPE: nucleic acid

(C) STRANDEDNESS: double

(D) TOPOLOGY: linear

(xi) SEQUENCE DESCRIPTION: SEQ ID NO: 4:

GCGGCAAGCT TTTTGCAAAG CCTAGGC 27

(2) INFORMATION FOR SEQ ID NO: 5:

(i) SEQUENCE CHARACTERISTICS:

(A) LENGTH: 271 base pairs

(B) TYPE: nucleic acid

(C) STRANDEDNESS: double

(D) TOPOLOGY: linear

(xi) SEQUENCE DESCRIPTION: SEQ ID NO: 5:

CTCGAGATTT CCCCGAAATC TAGATTTCCC CGAAATGATT TCCCCGAAAT GATTTCCCCG 60

AAATATCTGC CATCTCAATT AGTCAGCAAC CATAGTCCCG CCCCTAACTC CGCCCATCCC 120

GCCCCTAACT CCGCCCAGTT CCGCCCATTC TCCGCCCCAT GGCTGACTAA TTTTTTTTAT 180

TTATGCAGAG GCCGAGGCCG CCTCGGCCTC TGAGCTATTC CAGAAGTAGT GAGGAGGCTT 240

TTTTGGAGGC CTAGGCTTTT GCAAAAAGCT T 271

(2) INFORMATION FOR SEQ ID NO: 6:

(i) SEQUENCE CHARACTERISTICS:

(A) LENGTH: 32 base pairs

(B) TYPE: nucleic acid

(C) STRANDEDNESS: double

(D) TOPOLOGY: linear

(xi) SEQUENCE DESCRIPTION: SEQ ID NO: 6:

GCGCTCGAGG GATGACAGCG ATAGAACCCC GG 32

(2) INFORMATION FOR SEQ ID NO: 7:

(i) SEQUENCE CHARACTERISTICS:

(A) LENGTH: 31 base pairs

(B) TYPE: nucleic acid

(C) STRANDEDNESS: double

(D) TOPOLOGY: linear

(xi) SEQUENCE DESCRIPTION: SEQ ID NO: 7:

GCGAAGCTTC GCGACTCCCC GGATCCGCCT C 31

(2) INFORMATION FOR SEQ ID NO: 8:

(i) SEQUENCE CHARACTERISTICS:

(A) LENGTH: 12 base pairs

(B) TYPE: nucleic acid

(C) STRANDEDNESS: double

(D) TOPOLOGY: linear

(xi) SEQUENCE DESCRIPTION: SEQ ID NO: 8:

GGGGACTTTC CC 12

(2) INFORMATION FOR SEQ ID NO: 9:

(i) SEQUENCE CHARACTERISTICS:

(A) LENGTH: 73 base pairs

(B) TYPE: nucleic acid

(C) STRANDEDNESS: double

(D) TOPOLOGY: linear

(xi) SEQUENCE DESCRIPTION: SEQ ID NO: 9:

GCGGCCTCGA GGGGACTTTC CCGGGGACTT TCCGGGGACT TTCCGGGACT TTCCATCCTG 60

CCATCTCAAT TAG 73

(2) INFORMATION FOR SEQ ID NO: 10:

(i) SEQUENCE CHARACTERISTICS:

(A) LENGTH: 256 base pairs

(B) TYPE: nucleic acid

(C) STRANDEDNESS: double

(D) TOPOLOGY: linear

(xi) SEQUENCE DESCRIPTION: SEQ ID NO: 10:

CTCGAGGGGA CTTTCCCGGG GACTTTCCGG GGACTTTCCG GGACTTTCCA TCTGCCATCT 60

CAATTAGTCA GCAACCATAG TCCCGCCCCT AACTCCGCCC ATCCCGCCCC TAACTCCGCC 120

CAGTTCCGCC CATTCTCCGC CCCATGGCTG ACTAATTTTT TTTATTTATG CAGAGGCCGA 180

GGCCGCCTCG GCCTCTGAGC TATTCCAGAA GTAGTGAGGA GGCTTTTTTG GAGGCCTAGG 240

CTTTTGCAAA AAGCTT 256

(2) INFORMATION FOR SEQ ID NO: 11:

(i) SEQUENCE CHARACTERISTICS:

(A) LENGTH: 1739 base pairs

(B) TYPE: nucleic acid

(C) STRANDEDNESS: double

(D) TOPOLOGY: linear

(xi) SEQUENCE DESCRIPTION: SEQ ID NO: 11:

GCGCTCCCGA GGCCGCGGGA CCTGCAGAGA GGACAGCCGG CCTGCGCCGG GACATGCGGC 60

CCCAGGAGCT CCCCAGGCTC GCGTTCCCGT TGCTGCTGTT GCTGTTGCTG CTGCTGCCGC 120

CGCCGCCGTG CCCTGCCCAC AGCGCCACGC GTTTCGACCC CACCTGGGAG TCCCTGGACG 180

CCCGCCAGCT GCCCGCGTGG TTTGACCAGG CCAAGTTCGG CATCTTCATC CACTGGGGAG 240

TGTTTTCCGT GCCCAGCTTC GGTAGCGAGT GGTTCTGGTG GTATTGGCAA AAGGAAAAGA 300

TACCGAAGTA TGTGGAATTT ATGAAAGATA ATTACCCTCC TARTTTCAAA TATGAAGATT 360

TTGGACCACT ATTTACAGCA AAATTTTTTA ATGCCAACCA RTGGGCARAT ATTTTYCAGG 420

CCTCTGGTGC CAAATACATT GTCTTAACTT CCAAACATCA TGAAGGCTTT ACCTTGTGGG 480

GGTCAGAATA TTCGTGGAAC TGGAATGCCA TAGATGAGGG GCCCAAGAGG GACATTGTCA 540

AGGAACTTGA GGTAGCCATT AGGAACAGAA CTGACCTGCG TTTTGGACTG TACTATTCCC 600

TTTTTGAATG GTTTCATCCG CTCTTCCTTG AGGATGAATC CAGTTCATTC CATAAGCGGC 660

AATTTCCAGT TTCTAAGACA TTGCCAGAGC TCTATGAGTT AGTGAACAAC TATCAGCCTG 720

AGGTTCTGTG GTCGGATGGT GACGGAGGAG CACCGGATCA ATACTGGAAC ANCACAGGCT 780

TCTTGGCCTG GTTATATAAT GAAAGCCCAG TTCGGGGCAC AGTAGTCACC AATGATCGTT 840

GGGGAGCTGG TAGCATCTGT AAGCATGGTG GCTTCTATAC CTGCAGTGAT CGTTATAACC 900

CAGGACATCT TTTGCCACAT AAATGGGAAA ACTGCATGAC AATAGACAAA CTGTCCTGGG 960

GCTATAGGAG GGAAGCTGGA ATCTCTGACT ATCTTACAAT TGAAGAATTG GTGAAGCAAC 1020

TTGTAGAGAC AGTTTCATGT GGAGGAAATC TTTTGATGAA TATTGGGCCC ACACTAGATG 1080

GCACCATTTC TGTAGTTTTT GAGGAGCGAC TGAGGCAAAT GGGGTCCTGG CTAAAAGTCA 1140

ATGGAGAAGC TATTTATGAA ACCCATACCT GGCGATCCCA GAATGACACT GTCACCCCAG 1200

ATGTGTGGTA CACATCCAAG CCTAAAGAAA AATTAGTCTA TGCCATTTTT CTTAAATGGC 1260

CCACATCAGG ACAGCTGTTC CTTGGCCATC CCAAAGCTAT TCTGGGGGCA ACAGAGGTGA 1320

AACTACTGGG CCATGGACAG CCACTTAACT GGATTTCTTT GGAGCAAAAT GGCATTATGG 1380

TAGAACTGCC ACAGCTAACC ATTCATCAGA TGCCGTGTAA ATGGGGCTGG GCTCTAGCCC 1440

TRACTAATGT GATCTAAAGT GCAGCAGAGT GGCTGATGCT GCAAGTTATG TCTAAGGCTA 1500

GGAACTATCA GGTGTCTATA ATTGTAGCAC ATGGAGAAAG CAAATGTAAA ACTGGATAAG 1560

AAAATTATTT TGGCAGTTCA GCCCTTTCCC TTTTTCCCAC TAAATTTTTT CTTAAATTAC 1620

CCATGTAACC ATTTTAACTC TCCAGTGCAC TTTGCCATTA AAGTCTCTTC ACATTGAAAA 1680

AAAAAAAAAA AAAAACCCCG GGGGGGGGGC CCGGGNACCC CATTTCGCCC NTAAAGGGG 1739

(2) INFORMATION FOR SEQ ID NO: 12:

(i) SEQUENCE CHARACTERISTICS:

(A) LENGTH: 844 base pairs

(B) TYPE: nucleic acid

(C) STRANDEDNESS: double

(D) TOPOLOGY: linear

(xi) SEQUENCE DESCRIPTION: SEQ ID NO: 12:

GGCCCCTGGG CCCGAGGGGC TGGAGCCGGG CCGGGGCGAT GTGGAGCGCG GGCCGCGGCG 60

GGGCTGCCTG GCCGGTGCTG TTGGGGCTGC TGCTGGCGCT GTTAGTGCCG GGCGGTGGTG 120

CCGCCAAGAC CGGTGCGGAG CTCGTGACCT GCGGGTCGGT GCTGAAGCTG CTCAATACGC 180

ACCACCGCGT GCGGCTGCAC TCGCACGACA TCAAATACGG ATCCGGCAGC GGCCAGCAAT 240

CGGTGACCGG CGTAGAGGCG TCGGACGACG CCAATAGCTA CTGGCGGATC CGCGGCGGCT 300

CGGAGGGCGG GTGCCGCCGC GGGTCCCCGG TGCGCTGCGG GCAGGCGGTG AGGCTCACGC 360

ATGTGCTTAC GGGCAAGAAC CTGCACACGC ACCACTTCCC GTCGCCGCTG TCCAACAACC 420

AGGAGGTGAG TGCCTTTGGG GAAGACGGCG AGGGCGACGA CCTGGACCTA TGGACAGTGC 480

GCTGCTCTGG ACAGCACTGG GAGCGTGAGG CTGCTGTGCG CTTCCAGCAT GTGGGCACCT 540

CTGTGTTCCT GTCAGTCACG GGTGAGCAGT ATGGAAGCCC CATCCGTGGG CAGCATGAGG 600

TCCACGGCAT GCCCAGTGCC AACACGCACA ATACGTGGAA GGCCATGGAA GGCATCTTCA 660

TCAAGCCTAG TGTGGAGCCC TCTGCAGGTC ACGATGAACT CTGAGTGTGT GGATGGATGG 720

GTGGATGGAG GGTGGCAGGT GGGGCGTCTG CAGGGCCACT CTTGGCAGAG ACTTTGGGTT 780

TGTAGGGGTC CTCAAGTGCC TTTGTGATTA AAGAATGTTG GTCTATGAAA AAAAAAAAAA 840

AAAA 844

(2) INFORMATION FOR SEQ ID NO: 13:

(i) SEQUENCE CHARACTERISTICS:

(A) LENGTH: 776 base pairs

(B) TYPE: nucleic acid

(C) STRANDEDNESS: double

(D) TOPOLOGY: linear

(xi) SEQUENCE DESCRIPTION: SEQ ID NO: 13:

TTCGAAATAA AAGATCTGCT CAAGAGAGCC GCAGAAAAAG AAGGTGTATG TTGGGGGTTT 60

AGAGAGCAGG GTCTTGAAAT ACACAGCCCA GAATATGGAG CTTCAGAACA AAGTACAGCT 120

TCTGGAGGAA CAGAATTTGT CCCTTCTAGA TCAACTGAGG AAACTCCAGG CCATGGTGAT 180

TGAGATATCA AACAAAACCA GCAGCAGCAG CACCTGCATC TTGGTCCTAC TAGTCTCCTT 240

CTGCCTCCTC CTTGTACCTG CTATGTACTC CTCTGACACA AGGGGGAGCC TGCCAGCTGA 300

GCATGGAGTG TTGTCCCGCC AGCTTCGTGC CCTCCCCAGT GAGGACCCTT ACCAGCTGGA 360

GCTGCCTGCC CTGCAGTCAG AAGTGCCGAA AGACAGCACA CACCAGTGGT TGGACGGCTC 420

AGACTGTGTA CTCCAGGCCC CTGGCAACAC TTCCTGCCTG CTGCATTACA TGCCTCAGGC 480

TCCCAGTGCA GAGCCTCCCC TGGAGTGGCC ATTCCCTGAC CTCTTCTCAG AGCCTCTCTG 540

CCGAGGTCCC ATCCTCCCCC TGCAGGCAAA TCTCACAAGG AAGGGAGGAT GGCTTCCTAC 600

TGGTAGCCCC TCTGTCATTT TGCAGGACAG ATACTCAGGC TAGATATGAG GATATGTGGG 660

GGGTCTCAGC AGGAGCCTGG GGGGCTCCCC ATCTGTGTCC AAATAAAAAG CGGTGGGCAA 720

GGGCTGGCCG CAGCTCCTGT GCCCTGTCAG GACGACTGAG GGCTCAAACA CACCAC 776

(2) INFORMATION FOR SEQ ID NO: 14:

(i) SEQUENCE CHARACTERISTICS:

(A) LENGTH: 1376 base pairs

(B) TYPE: nucleic acid

(C) STRANDEDNESS: double

(D) TOPOLOGY: linear

(xi) SEQUENCE DESCRIPTION: SEQ ID NO: 14:

GAATTCGGCA CGAGGCGCCT ACCCTGCCTG CAGGTGAGCA GTGGTGTGTG AGAGCCAGGC 60

GTCCCTCTGC CTGCCCACTC AGTGGCAACA CCCGGGAGCT GTTTTGTCCT TTGTGGAGCC 120

TCAGCAGTTC CCTCTTTCAG AACTCACTGC CAAGAGCCCT GAACAGGAGC CACCATGCAG 180

TGCTTCAGCT TCATTAAGAC CATGATGATC CTCTTCAATT TGCTCATCTT TCTGTGTGGT 240

GCAGCCCTGT TGGCAGTGGG CATCTGGGTG TCAATCGATG GGGCATCCTT TCTGAAGATC 300

TTCGGGCCAC TGTCGTCCAG TGCCATGCAG TTTGTCAACG TGGGCTACTT CCTCATCGCA 360

GCCGGCGTTG TGGTCTTTGC TCTTGGTTTC CTGGGCTGCT ATGGTGCTAA GACTGAGAGC 420

AAGTGTGCCC TCGTGACGTT CTTCTTCATC CTCCTCCTCA TCTTCATTGC TGAGGTTGCA 480

GCTGCTGTGG TCGCCTTGGT GTACACCACA ATGGCTGAGC ACTTCCTGAC GTTGCTGGTA 540

GTGCCTGCCA TCAAGAAAGA TTATGGTTCC CAGGAAGACT TCACTCAAGT GTGGAACACC 600

ACCATGAAAG GGCTCAAGTG CTGTGGCTTC ACCAACTATA CGGATTTTGA GGACTCACCC 660

TACTTCAAAG AGAACAGTGC CTTTCCCCCA TTCTGTTGCA ATGACAACGT CACCAACACA 720

GCCAATGAAA CCTGCACCAA GCAAAAGGCT CACGACCAAA AAGTAGAGGG TTGCTTCAAT 780

CAGCTTTTGT ATGACATCCG AACTAATGCA GTCACCGTGG GTGGTGTGGC AGCTGGAATT 840

GGGGGCCTCG AGCTGGCTGC CATGATTGTG TCCATGTATC TGTACTGCAA TCTACAATAA 900

GTCCACTTCT GCCTCTGCCA CTACTGCTGC CACATGGGAA CTGTGAAGAG GCACCCTGGC 960

AAGCAGCAGT GATTGGGGGA GGGGACAGGA TCTAACAATG TCACTTGGGC CAGAATGGAC 1020

CTGCCCTTTC TGCTCCAGAC TTGGGGCTAG ATAGGGACCA CTCCTTTTAN GCGATGCCTG 1080

ACTTTCCTTC CATTGGTGGG TGGATGGGTG GGGGGCATTC CAGAGCCTCT AAGGTAGCCA 1140

GTTCTGTTGC CCATTCCCCC AGTCTATTAA ACCCTTGATA TGCCCCCTAG GCCTAGTGGT 1200

GATCCCAGTG CTCTACTGGG GGATGAGAGA AAGGCATTTT ATAGCCTGGG CATAAGTGAA 1260

ATCAGCAGAG CCTCTGGGTG GATGTGTAGA AGGCACTTCA AAATGCATAA ACCTGTTACA 1320

ATGTTRAAAA AAAAAAAAAA AAAAAAAAAA AAAAAAYTCG AGGGGGGTCC CGTACC 1376

(2) INFORMATION FOR SEQ ID NO: 15:

(i) SEQUENCE CHARACTERISTICS:

(A) LENGTH: 502 base pairs

(B) TYPE: nucleic acid

(C) STRANDEDNESS: double

(D) TOPOLOGY: linear

(xi) SEQUENCE DESCRIPTION: SEQ ID NO: 15:

TAAAACAGTG CCTGCCTCAA AGGGAGGACT CAGTCAATAT CTGTTGAATG AATGAATGAA 60

TAATTGCCTG GGTCAACGAA TGAATGGCTG AATGAATGAT TTCTCCTTTC CCTCGGCACT 120

GTCTGGAGTC CCCAGGACAG GCATGGGCAG CAGTCGCTGG TCTGTGGCCT GTCCCACTGG 180

ACTTGGGGTT CTCATGCTTG GTCTGGGCGG AGATCACCCA CCAGGCTCCC AGGTCGATCC 240

TCTGCTCATG GGAARCTGCG TCCGGCCCNA GCTGCCAGAA CTCACTGCAS GGTGGAGGGA 300

ARARCAGGRA CGATCTGCGA GCGCCTGAAC AGCGCACAAG AGCCGAGGAG CCGCTGCTTA 360

AAATGCAGGC GTTGAGAGGA GTTTCGCCTC CTTTTTTGAG TTGAATATGA GATTTCCGAG 420

CAGCCATGAC GAGTTGGGTT GGTGGAAGTG GGGAGTCCGT TCCTCAGTCA GATGGAGGAG 480

GGGGTCCCCT TGGATCTCCT CT 502

(2) INFORMATION FOR SEQ ID NO: 16:

(i) SEQUENCE CHARACTERISTICS:

(A) LENGTH: 425 base pairs

(B) TYPE: nucleic acid

(C) STRANDEDNESS: double

(D) TOPOLOGY: linear

(xi) SEQUENCE DESCRIPTION: SEQ ID NO: 16:

ATCTCTAGTG GTGGCTGCCG TCGCTCCAGA CAATCGGAAT CCTGCCTTCA CCACCATGGG 60

CTGGCTTTTT CTAAAGGTTT TGTTGGCGGG AGTGAGTTTC TCAGGATTTC TTTATCCTCT 120

TGTGGATTTT TGCATCAGTG GGAAAACAAG AGGACAGAAG CCAAACTTTG TGATTATTTT 180

GGCCGATGAC ATGGGGTGGG GTGACTGGGG AGCAAACTGG GCAGAAACAA AGGACACTGC 240

CAACCTTGAT AAGATGGCTT CGGAGGGAAT GARGTGARTC TTGARATGCC ARGCCAGCTT 300

TCTTTGGAWG TCTTACTCCC GTTCTTGAAA AGGGAAAGGG GCGTGCAAAG CACTTAARGA 360

WTCATKGATG GACCCATGTG ATTTARTTAA TTTATTAATT AATTTGGTTT GGAARCCAGC 420

ATAGC 425

(2) INFORMATION FOR SEQ ID NO: 17:

(i) SEQUENCE CHARACTERISTICS:

(A) LENGTH: 1316 base pairs

(B) TYPE: nucleic acid

(C) STRANDEDNESS: double

(D) TOPOLOGY: linear

(xi) SEQUENCE DESCRIPTION: SEQ ID NO: 17:

GGCACGAGGA GCTGGGGGAG CCTGAGGTGC GCTACGTGGC TGGCATGCAT GGGAACGAGG 60

CCCTGGGGCG GGAGTTGCTT CTGCTCCTGA TGCAGTTCCT GTGCCATGAG TTCCTGCGAG 120

GGAACCCACG GGTGACCCGG CTGCTCTCTG AGATGCGCAT TCACCTGCTG CCCTCCATGA 180

ACCCTGATGG CTATGAGATC GCCTACCACC GGGGTTCAGA GCTGGTGGGC TGGGCCGAGG 240

GCCGCTGGAA CAACCAGAGC ATCGATCTTA ACCATAATTT TGCTGACCTC AACACACCAC 300

TGTGGGAAGC ACAGGACGAT GGGAAGGTGC CCCACATCGT CCCCAACCAT CACCTGCCAT 360

TGCCCACTTA CTACACCCTG CCCAATGCCA CCGTGGCTCC TGAAACGCGG GCAGTAATCA 420

AGTGGATGAA GCGGATCCCC TTTGTGCTAA GTGCCAACCT CCACGGGGGT GAGCTCGTGG 480

TGTCCTACCC ATTCGACATG ACTCGCACCC CGTGGGCTGC CCGCGAGCTC ACGCCCACAC 540

CAGATGATGC TGTGTTTCGC TGGCTCAGCA CTGTCTATGC TGGCAGTAAT CTGGCCATGC 600

AGGACACCAG CCGCCGACCC TGCCACAGCC AGGACTTCTC CGTGCACGGC AACATCATCA 660

ACGGGGCTGA CTGGCACACG GTCCCCGGGA GCATGAATGA CTTCAGCTAC CTACACACCA 720

ACTGCTTTGA GGTCACTGTG GAGCTGTCCT GTGACAAGTT CCCTCACGAG AATGAATTGC 780

CCCAGGAGTG GGAGAACAAC AAAGACGCCC TCCTCACCTA CCTGGAGCAG GTGCGCATGG 840

GCATTGCAGG AGTGGTGAGG GACAAGGACA CGGAGCTTGG GATTGCTGAC GCTGTCATTG 900

CCGTGGATGG GATTAACCAT GACGTGACCA CGGCGTGGGG CGGGGATTAT TGGCGTCTGC 960

TGACCCCAGG GGACTACATG GTGACTGCCA GTGCCGAGGG CTACCATTCA GTGACACGGA 1020

ACTGTCGGGT CACCTTTGAA GAGGGCCCCT TCCCCTGCAA TTTCGTGCTC ACCAAGACTC 1080

CCAAACAGAG GCTGCGCGAG CTGCTGGCAG CTGGGGCCAA GGTGCCCCCG GACCTTCGCA 1140

GGCGCCTGGA GCGGCTAAGG GGACAGAAGG ATTGATACCT GCGGTTTAAG AGCCCTAGGG 1200

CAGGCTGGAC CTGTCAAGAC GGGAAGGGGA AGAGTAGAGA GGGAGGGACA AAGTGAGGAA 1260

AAGGTGCTCA TTAAAGCTAC CGGGCACCTT AAAAAAAAAA AAAAAAAAAA AAAAAA 1316

(2) INFORMATION FOR SEQ ID NO: 18:

(i) SEQUENCE CHARACTERISTICS:

(A) LENGTH: 436 base pairs

(B) TYPE: nucleic acid

(C) STRANDEDNESS: double

(D) TOPOLOGY: linear

(xi) SEQUENCE DESCRIPTION: SEQ ID NO: 18:

AAAAAAATTC AATGGATATT ATGAAAATAA GAGAGTATTT CCAGAAGTAT GGATATAGTC 60

CACGTGTCAA GAAAAATTCA GTACACGAGC AAGAAGCCAT TAACTCTGAC CCAGAGTTGT 120

CTAATTGTGA AAATTTTCAG AAGACTGATG TGAAAGATGA TCTGTCTGAT CCTCCTGTTG 180

CAAGCAGTTG TATTTCTGAG AAGTCTCCAC GTAGTCCACA ACTTTCAGAT TTTGGACTTG 240

AGCGGTACAT CGTATCCCAA GTTCTACCAA ACCCTCCACA GGCAGTGAAC AACTATAAGG 300

AAGAGCCCGT AATTGTAACC CCACCTACCA AACAATCACT AGTAAAAGTA CTAAAAACTC 360

CAAAATGTGC ACTAAAATGG ATGATTTTGA GTGTGTACTC CTAAATTAGA ACACTTTGGT 420

ATCTCTGAAT ATACTA 436

(2) INFORMATION FOR SEQ ID NO: 19:

(i) SEQUENCE CHARACTERISTICS:

(A) LENGTH: 503 base pairs

(B) TYPE: nucleic acid

(C) STRANDEDNESS: double

(D) TOPOLOGY: linear

(xi) SEQUENCE DESCRIPTION: SEQ ID NO: 19:

TGTGCATATC CTGGGGAAAA AAATGGTACA TGTTTTAGAA ATTTTACTGT TTATAACAAT 60

GCAGGCAGTC AGTTTCCCGT TTCAAACACA GATAGATACA TGCAACACTC AAGATCCTGC 120

AGAGAGGCAG CCAGCATCTA TTGTTTAAAA AGGTTTCAAA AAGAATTCGG ATTGCTCKTT 180

TCTCTTTTGA ATCTGTGTGC CAAATGACAG GGACCAATAT TCGTCTTCTT TTTCKGTAAA 240

AYTCAGAAAG AMACATGAAA GAACCCAGAA TGCATTTCTT AAAGGGATTT AGTGCAGTTA 300

TTTTAAATAA TTTATGCACG CACACACACA TACATATATC CCCCGAGTAC ATATTTTTTC 360

CCTTTTTACT TGTGTGCAAT CAGTAGCTAC AATGACTGAA ATCCACTTCT TTGGGACTGT 420

GACATTTAAG CAAATCTTGT NTCTAGAAAN CGAAATGCCA NANTCTCGCA CAAAGCTGCT 480

CCGTCTGGGG CAACAAATCC ACA 503

(2) INFORMATION FOR SEQ ID NO: 20:

(i) SEQUENCE CHARACTERISTICS:

(A) LENGTH: 358 base pairs

(B) TYPE: nucleic acid

(C) STRANDEDNESS: double

(D) TOPOLOGY: linear

(xi) SEQUENCE DESCRIPTION: SEQ ID NO: 20:

GGGCTGTCTC CCCAGTAGTA ACTTGCTGGC CCTGCCCTTG AAGTGGGGAA ACTGTGAAGG 60

GCTCCTTGAT CAAGCTTGTC CTCTTTTCTT ACCTCTTCCT CTCTTCTGTT TCCGCTGCAG 120

CTGAACAGGC CAGCAGGCAA CCTGCCATGG GGTCCTGCTC CAAGAACCGG TCCTTCTTCT 180

GGATGACTGG GCTCCTGGTA TTCATCAGCC TCCTCCTCAG TGAGTGGCAG GGTCCCTGGG 240

AAGGGAGGGC AATTGGAGAG GGCTGGGCTA GCTGGGCTCT GACCAACGGG TGGGCTGTTC 300

AACTTCTGAT GTCTTTGGGC AACAACACAG AAAAACACTC TGTTATGATT TACGAAAN 358

(2) INFORMATION FOR SEQ ID NO: 21:

(i) SEQUENCE CHARACTERISTICS:

(A) LENGTH: 1926 base pairs

(B) TYPE: nucleic acid

(C) STRANDEDNESS: double

(D) TOPOLOGY: linear

(xi) SEQUENCE DESCRIPTION: SEQ ID NO: 21:

AGTGAAGGGA GCTGGCCGTG CGACTGGGCT TCGGGCCCTG TGCCAGAGGA GCANGCCTTC 60

CTGAGCAGGA GGAAGCAGGT GGTGGCCGCG GCCTTGAGGC AGGCCCTGCA GCTGGATGGA 120

GACCTGCAGG AGGATGAGAT CCCAGTGGTA GCTATTATGG CCACTGGTGG TGGGATCCGG 180

GCAATGACTT CCCTGTATGG GCAGCTGGCT GGCCTGAAGG AGCTGGGCCT CTTGGATTGC 240

KTCTCCTACA TCACCGGGGC CTCGGGCTCC ACCTGGGCCT TGGCCAACCT TTATAAGGAC 300

CCAGAGTGGT CTCAGAAGGA CCTGGCAGGG CCCACTGAGT TGCTGAAGAC CCAGGTGACC 360

AAGAACAAGC TGGGTGTGCT GGCCCCCAGC CAGCTGCAGC GGTACCGGCA GGAGCTGGCC 420

GAGCGTGCCC GCTTGGGCTA CCCAAGCTGC TTCACCAACC TGTGGGCCCT CATCAACGAG 480

GCGCTGCTGC ATGATGAGCC CCATGATCAC AAGCTCTCAG ATCAACGGGA GGCCCTGAGT 540

CATGGCCAGA ACCCTCTGCC CATCTACTGT GCCCTCAACA CCAAAGGGCA GAGCCTGACC 600

ACTTTTGAAT TTGGGGAGTG GTGCGAGTTC TCTCCCTACG AGGTCGGCTT CCCCAAGTAC 660

GGGGCCTTCA TCCCCTCTGA GCTCTTTGGC TCCGAGTTCT TTATGGGGCA GCTGATGAAG 720

AGGCTTCCTG AGTCCCGCAT CTGCTTCTTA GAAGGTATCT GGAGCAACCT GTATGCAGCC 780

AACCTCCAGG ACAGCTTATA CTGGGCCTCA GAGCCCAGCC AGTTCTGGGA CCGCTGGGTC 840

AGGAACCAGG CCAACCTGGA CAAGGAGCAG GTCCCCCTTC TGAAGATAGA AGAACCACCC 900

TCAACAGCCG GCAGAATAGC TGAGTTTTTC ACCGATCTTC TGACGTGGCG TCCACTGGCC 960

CAGGCCACAC ATAATTTCCT GCGTGGCCTC CATTTCCACA AAGACTACTT TCAGCATCCT 1020

CACTTCTCCA CATGGAAAGC TACCACTCTG GATGGGCTCC CCAACCAGCT GACACCCTCG 1080

GAGCCCCACC TGTGCCTGCT GGATGTTGGC TACCTCATCA ATACCAGCTG CCTGCCCCTC 1140

CTGCAGCCCA CTCGGGACGT GGACCTCATC CTGTCATTGG ACTACAACCT CCACGGAGCC 1200

TTCCAGCAGT TGCAGCTCCT GGGCCGGTTC TGCCAGGAGC AGGGGATCCC GTTCCCACCC 1260

ATCTCGCCCA GCCCCGAAGA GCAGCTCCAG CCTCGGGAGT GCCACACCTT CTCCGACCCC 1320

ACCTGCCCCG GAGCCCCTGC GGTGCTGCAC TTTCCTCTGG TCAGCGACTC CTTCCGGGAG 1380

TACTCGGCCC CTGGGGTCCG GCGGACACCC GAGGAGGCGG CAGCTGGGGA GGTGAACCTG 1440

TCTTCATCGG ACTCTCCCTA CCACTACACG AAGGTGACCT ACAGCCAGGA GGACGTGGAC 1500

AAGCTGCTGC ACCTGACACA TTACAATGTC TGCAACAACC AGGAGCAGCT GCTGGAGGCT 1560

CTGCGCCAGG CAGTGCAGCG GAGGCGGCAG CGCAGGCCCC ACTGATGGCC GGGGCCCCTG 1620

CCACCCCTAA CTCTCATTCA TTCCCTGGCT GCTGAGTTGC AGGTGGGAAC TGTCATCACG 1680

CAGTGCTTNC AGAGCCTCGG GCTCAGGTGG CACTGTCCCA GGGTCCAGGC TGAGGGCTGG 1740

GAGCTCCCTT GCGCCTCAGC AGTTTGCAGT GGGGTAAGGA GGCCAAGCCC ATTTGTGTAA 1800

TCACCCAAAA CCCCCCGGCC TGTGCCTGTT TTCCCTTCTG CGCTACCTTG AGTAGTTGGA 1860

GCACTTGATA CATCACAGAC TCATACAAAT GTGAGGCGCT GAGAAAAAAA AAAAAAAAAA 1920

ACTCGA 1926

(2) INFORMATION FOR SEQ ID NO: 22:

(i) SEQUENCE CHARACTERISTICS:
(A) LENGTH: 1224 base pairs
(B) TYPE: nucleic acid
(C) STRANDEDNESS: double
(D) TOPOLOGY: linear

(xi) SEQUENCE DESCRIPTION: SEQ ID NO: 22:

CCGCCGAAGC TCCGTCCCGC CCGCGGCCGG CTCCGCCTCA CCTCCCGGCC GCGGCTGCCC 60

TCTGCCCGGG TTGTCCAAGA TGGAGGGCGC TCCACCGGGG TCGCTCGCCC TCCGGCTCCT 120

GCTGTTCGTG GCGCTACCCG CCTCCGGCTG GCTGACGACG GGCGCCCCCG AGCCGCCGCC 180

GCTGTCCGGA GCCCCACAGG ACGGCATCAG AATTAATGTA ACTACACTGA AAGATGATGG 240

GGACATATCT AAACAGCAGG TTGTTCTTAA CATAACCTAT GAGAGTGGAC AGGTGTATGT 300

AAATGACTTA CCTGTAAATA GTGGTGTAAC CCGAATAAGC TGTCAGACTT TGATAGTGAA 360

GAATGAAAAT CTTGAAAATT TGGAGGAAAA AGAATATTTT GGAATTGTCA GTGTAAGGAT 420

TTTAGTTCAT GAGTGGCCTA TGACATCTGG TTCCAGTTTG CAACTAATTG TCATTCAAGA 480

AGAGGTAGTA GAGATTGATG GAAAACAAGT TCAGCAAAAG GATGTCACTG AAATTGATAT 540

TTTAGTTAAG AACCGGGGAG TACTCAGACA TTCAAACTAT ACCCTCCCTT TGGAAGAAAG 600

CATGCTCTAC TCTATTTCTC GAGACAGTGA CATTTTATTT ACCCTTCCTA ACCTCTCCAA 660

AAAAGAAAGT GTTAGTTCAC TGCAAACCAC TAGCCAGTAT CTTATCAGGA ATGTGGAAAC 720

CACTGTAGAT GAAGATGTTT TACCTGGGCA AGTTACCTGA AACTCCTCTC AGAGCAGAGC 780

CGCCATCTTC ATATAAGGTA ATGTGTCAGT GGATGGAAAA GTTTAGAAAA GATCTGTGTA 840

GGTTCTGGAG CAACGTTTTC CCAGTATTCT TTCAGTTTTT GAACATCATG GTGGTTGGAA 900

TTACAGGAGC AGCTGTGGTA ATAACCATCT TAAAGGTGTT TTTCCCAGTT TCTGAATACA 960

AAGGAATTCT TCAGTTGGAT AAAGTGGACG TCATACCTGT GACAGCTATC AACTTATATC 1020

CAGATGGTCC AGAGAAAAGA GCTGAAAACC TTGAAGATAA AACATGTATT TAAAACGCCA 1080

TCTCATATCA TGGACTCCGA AGTAGCCTGT TGCCTCCAAA TTTGCCACTT GAATATAATT 1140

TTCTTTAAAT CGTTAAGAAT CAGTTTATAC ACTAGAGAAA TTGCTAAACT CTAAGACTGC 1200

CTGAAAATTG ACCTTTACAG TGCC 1224

(2) INFORMATION FOR SEQ ID NO: 23:

(i) SEQUENCE CHARACTERISTICS:
(A) LENGTH: 694 base pairs
(B) TYPE: nucleic acid
(C) STRANDEDNESS: double
(D) TOPOLOGY: linear

(xi) SEQUENCE DESCRIPTION: SEQ ID NO: 23:

GGCACGAGTC TTATTGTGCA CTGTAGCCTG AATCCCCCAG GGTAATTAAT ATGAAGTGCA 60

AAAAGTTGAA TGTTCCAGTC TAAAAGGCAG TGGGAGAAAT TACATAGCAT GGAAATAATA 120

AAATGAACTC TTATTAATGA GAACGAGGCT CTTGCAGTGG CAAGTTCTGC TGGTCACCCG 180

ATGGGGATGG GAGCCTTTCA AGCTTTTTTT TGGGTAATAC TCACAGTTTC CAACGTCTGT 240

GTACTTTTCA AAATGAGCTT GTTCTTCCTT CTGACACTCA TCTCAAAGCT CCATGGTGAC 300

GCAGAGGTCT GTTGAAGGTC ACAGGTCCTC GCTTGCATTG GCATACGGTC CTGTAGCATC 360

ACTTGTTAGC CCACTGCTGC TTGAAGGAAC TAAGAGTATT CAGGGATAGA GAGCTGAAAA 420

TAGGATTAAT TCCTTCCTTT TGACTCTCCC CTCAAGATGT CCTTGCTTTG GTCTGAAAAC 480

CTCTCCTGAC AACTTTTGCC CAAAGCAAAC CATCTGCCTT TTCTGAACTC TGAGTGAATA 540

TATTAGCATC TTCCCTTCTG AGCCCTCGTA CTGCCANGTT TGTTTGTTTG TTTGTTTCCA 600

AGAGACTGTG TCTTGCTCTG TCACCCAGGA GTTTGAAACC AGCCTGGCAA CATAGCAAGA 660

CCCTATCTCT ACAAAAAAAA AAAAAAAAAA AAAA 694

(2) INFORMATION FOR SEQ ID NO: 24:

(i) SEQUENCE CHARACTERISTICS:
(A) LENGTH: 796 base pairs
(B) TYPE: nucleic acid
(C) STRANDEDNESS: double
(D) TOPOLOGY: linear

(xi) SEQUENCE DESCRIPTION: SEQ ID NO: 24:

ATGAGCGGCG GTTGGATGGC GCAGGTTGGA GCGTGGCGAA CAGGGGCTCT GGGCCTGGCG 60

CTGCTGCTGC TGCTCGGCCT CGGACTAGGC CTGGAGGCGC CGCGAGCCCG CTTTCCACCC 120

CGACCTCTGC CCAGGCCGCA CCCGAGCTCA GGCTCGTGCC CACCCACCAA GTTCCAGTGC 180

CGCACCAGTG GCTTATGCGT GCCCCTCACC TGGCGCTGCG ACAGGACTTG GACTGCAGCG 240

ATGGCAGCGA TGAGGAGGAG TGCAGGATTG AGCCATGTAC CCAGAAAGGG CAATGCCCAC 300

CGCCCCCTGG CCTCCCCTGC CCCTGCACCG GCGTCAGTGA CTGCTCTGGG GGAACTGACA 360

AGAAACTGCG CAACTGCAGC CGCCTGGCCT GCCTAGCAGS GRAGSKCMCG WKGCACGCTG 420

AGCGATGACT GCATTCCACT CACGTGGCGC TGCGACGGCC ACCCAGACTG TCCCGACTCC 480

AGCGACGAGC TCGGCTGTGG AACCAATGAG ATCCTCCCGG AAGGGGATGC CACAACCATG 540

GGGCCCCCTG TGACCCTGGA GAGTGTCACC TCTCTCAGGA ATGCCACAAC CATGGGGCCC 600

CCTGTGACCC TGGAGAGTGT CCCCTCTGTC GGGAATGCCA CATCCTCCTC TGCCGGAGAC 660

CAGTCTGGAA GCCCAACTGC CTATGGGGTT ATTGCAGCTG CTGCGGTGCT CAGTGCAAGC 720

CTGGTCACCG CCACCCTCCT CCTTTTGTCC TGGCTCCGAG CCCAGGAGCG CCTCCGCCCA 780

CTGGGGTTAC TGGTGG 796

(2) INFORMATION FOR SEQ ID NO: 25:

(i) SEQUENCE CHARACTERISTICS:
(A) LENGTH: 662 base pairs
(B) TYPE: nucleic acid
(C) STRANDEDNESS: double
(D) TOPOLOGY: linear

(xi) SEQUENCE DESCRIPTION: SEQ ID NO: 25:

TAATTCGGCA CGAGGCTGTG GTGGAGAAGG ACGTGCCGTG CCGCTGGGTT CTGAGCCGGA 60

GTGGTCGGTG GGTGGGATGG AGGCGACCTT GGAGCAGCAC TTGGAAGACA CAATGAAGAA 120

TCCCTCCATT GTTGGAGTCC TGTGCACAGA TTCACAAGGA CTTAATCTGG GTTGCCGCGG 180

GACCCTGTCA GATGAGCATG CTGGAGTGAT ATCTGTTCTA GCCCAGCAAG CAGCTAAGCT 240

AACCTCTGAC CCCACTGATA TTCCTGTGGT GTGTCTAGAA TCAGATAATG GGAACATTAT 300

GATCCAGAAA CACGATGGCA TCACGGTGGC AGTGCACAAA ATGGCCTCTT GATGCTCATA 360

TCTGTTCTTC AGCAGCCTGT CATAGGAACT GGATCCTACC TATGTTAATT ACCTTATAGA 420

ACTACTAAAG TTCCAGTAGT TAGGCCATTC ATTTAATGTG CATTAGGCAC TTTTCTGTTT 480

ATTTAAGAGT CAATTGCTTT CTAATGCTCT ATGGACCGAC TATCAAGATA TTAGTAAGAA 540

AGGATCATGT TTTGAAGCAG CAGGTCCAGG TCACTTTGTA TATAGAATTT TGCTGTATTC 600

AATAAATCTG TTTGGAGGAA AAAAAAAAAA AAAAAAATTA CTGCGGNCCG ACAAGGGAAT 660

TC 662

(2) INFORMATION FOR SEQ ID NO: 26:

(i) SEQUENCE CHARACTERISTICS:
(A) LENGTH: 1105 base pairs
(B) TYPE: nucleic acid

(C) STRANDEDNESS: double
(D) TOPOLOGY: linear

(xi) SEQUENCE DESCRIPTION: SEQ ID NO: 26:

CCTGATCCTC TCTTTTCTGC AGTTCAAGGG AAAGACGAGA TCTTGCACAA GGCACTCTGC 60

TTCTGCCCTT GGCTGGGGAA GGGTGGCATG GAGCCTCTCC GGCTGCTCAT CTTACTCTTT 120

GTCACAGAGC TGTCCGGAGC CCACAACACC ACAGTGTTCC AGGGCGTGGC GGGCCAGTCC 180

CTGCAGGTGT CTTGCCCCTA TGACTCCATG AAGCACTGGG GGAGGCGCAA GGCCTGGTGC 240

CGCCAGCTGG GAGAGAAGGG CCCATGCCAG CGTGTGGTCA GCACGCACAA CTTGTGGCTG 300

CTGTCCTTCC TGAGGAGGTG GAATGGGAGC ACAGCCATCA CAGACGATAC CCTGGGTGGC 360

ACTCTCACCA TTACGCTGCG GAATCTACAA CCCCATGATG CGGGTCTCTA CCAGTGCCAG 420

AGCCTCCATG GCAGTGAGGC TGACACCCTC AGGAAGGTCC TGGTGGAGGT GCTCGCAGAC 480

CCCCTGGATC ACCGGGATGC TGGAGATCTC TGGTTCCCCG GGGAGTCTGA GAGCTTCGAG 540

GATGCCCATG TGGAGCACAG CATCTCCAGG AGCTCTTCKT AGGAAAGGCC GCAAATTCCC 600

ATTCCTTCCC CTCTTGCCTA TCYTTCTCCT CCAAGAYCTG CATCTTTCTC ATCAAGATTC 660

TAGCAGCCAG CGCCCTCTGG GCTGCAGCCT GGCATGGACA GAAGCCAGGG ACACATCCAC 720

CCAGTGAACT GGACTGTGGC CATGACCCAG GGTATCAGCT CCAAACTCTG CCAGGGCTGA 780

GAGACACGTG AAGGAAGATG ATGGGAGGAA AAGCCCAGGA GAAGTCCCAC CAGGGACCAG 840

CCCAGCCTGC ATACTTGCCA CTTGGCCACC AGGACTCCTT GTTCTGCTCT GGCAAGAGAC 900

TACTCTGCCT GAACACTGCT TCTCCTGGAC CCTGGAAGCA GGGACTGGTT GAGGGAGTGG 960

GGAGGTGGTA AGAACACCTG ACAACTTCTG AATATTGGAC ATTTTAAACA CTTACAAATA 1020

AATCCAAGAC TGTCATATTT AAAAAAAAAA AAAAAAAAMA AAARRRRRRC CCCGGTACCC 1080

AATTCGCCCT ATAGTGAGTC GTATA 1105

(2) INFORMATION FOR SEQ ID NO: 27:

(i) SEQUENCE CHARACTERISTICS:
(A) LENGTH: 1017 base pairs
(B) TYPE: nucleic acid
(C) STRANDEDNESS: double
(D) TOPOLOGY: linear

(xi) SEQUENCE DESCRIPTION: SEQ ID NO: 27:

CTCGCCTGGG CTGTTTCCCG GCTTCATTTC TCCCGACTCA GCTTCCCACC CTGGGCTTTC 60

CGAGGTGCTT TCGCCGCTGT CCCCACCACT GCAGCCATGA TCTCCTTAAC GGACACGCAG 120

AAAATTGGAA TGGGATTAAC AGGATTTGGA GTGTTTTTCC TGTTCTTTGG AATGATTCTC 180

TTTTTTGACA AAGCACTACT GGCTATTGGA AATGTTTTAT TTGTAGCCGG CTTGGCTTTT 240

GTAATTGGTT TAGAAAGAAC ATTCAGATTC TTCTTCCAAA AACATAAAAT GAAAGCTACA 300

GGTTTTTTTC TGGGTGGTGT ATTTGTAGTC CTTATTGGTT GGCCTTTGAT AGGCATGATC 360

TTCGAAATTT ATGGATTTTT TCTCTTGTTC AGGGGCTTCT TTCCTGTCGT TGTTGGCTTT 420

ATTAGAAGAG TGCCAGTCCT TGGATCCCTC CTAAATTTAC CTGGAATTAG ATCATTTGTA 480

GATAAAGTTG GAGAAAGCAA CAATATGGTA TAACAACAAG TGAATTTGAA GACTCATTTA 540

AAATATTGTG TTATTTATAA AGTCATTTGA AGAATATTCA GCACAAAATT AAATTACATG 600

AAATAGCTTG TAATGTTCTT TACAGGAGTT TAAAACGTAT AGCCTACAAA GTACCAGCAG 660

CAAATTAGCA AAGAAGCAGT GAAAACAGGC TTCTACTCAA GTGAACTAAG AAGAAGTCAG 720

CAAGCAAACT GAGAGAGGTG AAATCCATGT TAATGATGCT TAAGAAACTC TTGAAGGCTA 780

TTTGTGTTGT TTTTCCACAA TGTGCGAAAC TCAGCCATCC TTAGAGAACT GTGGTGCCTG 840

TTTCTTTTCT TTTTATTTTG AAGGCTCAGG AGCATCCATA GGCATTTGCT TTTTAGAAAT 900

GTCCACTGCA ATGGCAAAAA TATTTCCAGT TGCACTGTAT CTCTGGAAGT GATGCATGAA 960

TTCGATTGGA TTGTGTCATT TTAAAGTATT AAAACCAAGG GAAACCCCAA AAAAAAA 1017

(2) INFORMATION FOR SEQ ID NO: 28:

(i) SEQUENCE CHARACTERISTICS:
(A) LENGTH: 391 base pairs
(B) TYPE: nucleic acid
(C) STRANDEDNESS: double
(D) TOPOLOGY: linear

(xi) SEQUENCE DESCRIPTION: SEQ ID NO: 28:

CCCTGGAAAG AGGAACTGAT GTTTGAGGGG ACAGATGTGG GTCACTTTCC CTGGCAGTGC 60

CCTCTAGCCT TGCTGCCTTG GCTTTCTGAC CCCTTCCAGG CTTCAGGGGC CTGGGAGATC 120

TCATGCCTCA GCCCAGGAAA CATTTAATAG GGAAAGCAGA GACATGTCAT GTCAGCCCCA 180

CAGACAAGAA TTTCTAGAGC ACTTGTCCTG TTGTTCCTTG CCCCGACATT ACTCAGTCTG 240

GGCCATGGAA TCCATCCAAT AAACACAGCA ACACCCTATG NTACTGACCA AGCAAAGCTT 300

GCCCCTGGTA CCAAAGAGCT AAATCATGAC CAAAGTGTGA CATGAATGTA ACTGAAATGC 360

GGGTTAGTTG CTCAATGTAT GCAAAGTCCC A 391

(2) INFORMATION FOR SEQ ID NO: 29:

(i) SEQUENCE CHARACTERISTICS:
(A) LENGTH: 1139 base pairs
(B) TYPE: nucleic acid
(C) STRANDEDNESS: double
(D) TOPOLOGY: linear

(xi) SEQUENCE DESCRIPTION: SEQ ID NO: 29:

GGTGATATCT TCATAGTGGG CTATTACAGG CAGGAAAATG TTTTAACTGG TTTACAAAAT 60

CCATCAATAC TTGTGTCATT CCCTGTAAAA GGCAGGAGAC ATGTGATTAT GATCAGGAAA 120

CTGCACAAAA TTATTGTTTT CAGCCCCCGT GTTATTGTCC TTTTGAACTG TTTTTTTTTT 180

ATTAAAGCCA AATTTGTGTT GTATATATTC GTATTCCATG TGTTAGATGG AAGCATTTCC 240

TATCCAGTGT GAATAAAAAG AACAGTTGTA GTAAATTATT ATAAAGCCGA TGATATTTCA 300

TGGCAGGTTA TTCTACCAAG CTGTGCTTGT TGGTTTTTCC CATGACTGTA TTGCTTTTAT 360

AAATGTACAA ATAGTTACTG AAATGACGAG ACCCTTGTTT GCACAGCATT AATAAGAACC 420

TTGATAAGAA CCATATTCTG TTGACAGCCA GCTCACAGTT TCTTGCCTGA AGCTTGGTGC 480

ACCCTCCAGT GAGACACAAG ATCTCTCTTT TACCAAAGTT GAGAACAGAG CTGGTGGATT 540

AATTAATAGT CTTCGATATC TGGCCATGGG TAACCTCATT GTAACTATCA TCAGAATGGG 600

CAGAGATGAT CTTGAAGTGT CACATACACT AAAGTCCAAA CACTATGTCA GATGGGGGTA 660

AAATCCATTA AAGAACAGGA AAAAATAATT ATAAGATGAT AAGCAAATGT TTCAGCCCAA 720

TGTCAACCCA GTTAAAAAAA AAATTAATGC TGTGTAAAAT GGTTGAATTA GTTTGCAAAC 780

TATATAAAGA CATATGCAGT AAAAAGTCTG TTAATGCACA TCCTGTGGGA ATGGAGTGTT 840

CTAACCAATT GCCTTTTCTT GTTATCTGAG CTCTCCTATA TTATCATACT CAGATAACCA 900

AATTAAAAGA ATTAGAATAT GATTTTTAAT ACACTTAACA TTAAACTCTT CTAACTTTCT 960

TCTTTCTGTG ATAATTCAGA AGATAGTTAT GGATCTTCAA TGCCTCTGAG TCATTGTTAT 1020

AAAAAATCAG TTATCACTAT ACCATGCTAT AGGAGACTGG GCAAAACCTG TACAATGACA 1080

ACCCTGGAAG TTGCTTTTTT TAAAAAAATA ATAAATTTCT TAAATCAAAA AAAAAAAAA 1139

(2) INFORMATION FOR SEQ ID NO: 30:

(i) SEQUENCE CHARACTERISTICS:
(A) LENGTH: 465 base pairs
(B) TYPE: nucleic acid
(C) STRANDEDNESS: double
(D) TOPOLOGY: linear

(xi) SEQUENCE DESCRIPTION: SEQ ID NO: 30:

CCACGCGTCC GCGGACGCGT GGGGAAGGTT TGTGCCAGTA GACATTATGT TACTAAATCA 60

GCACTTTAAA ATCTTTGGTT CTCTAATTCA TATGAATTTG CTGTTTGCTC TAATTTCTTT 120

GGGCTCTTCT AATTTGAGTG GAGTACAATT TTGTTGTGAA ACAGTCCAGT GAAACTGTGC 180

AGGGAAATGA AGGTAGAATT TTGGGAGGTA ATAATGATGT GAAACATAAA GATTTAATAA 240

TTACTGTCCA ACACAGTGGA GCAGCTTGTC CACAAATATA GTAATTACTA TTTATTGCTC 300

TAAGGAAGAT TAAAAAAAGA TAGGGAAAAG GGGGAAACTT CTTTGAAAAA TGAAACATCT 360

GTTACATTAA TGTCTAATTA TAAAATTTTA ATCCTTACTG CATTTCTTCT GTTCCTACAA 420

ATGTATTAAA CATTCAGTTT AACTGGTAAA AAAAAAAAAA AAAAA 465

(2) INFORMATION FOR SEQ ID NO: 31:

(i) SEQUENCE CHARACTERISTICS:
(A) LENGTH: 702 base pairs
(B) TYPE: nucleic acid
(C) STRANDEDNESS: double
(D) TOPOLOGY: linear

(xi) SEQUENCE DESCRIPTION: SEQ ID NO: 31:

GCAACAAGCG GCCCACCTTC CTGAAGATCA AGAAGCCACT GTCGTACCGC AAGCCCATGG 60

ACACGGACCT GGTGTACATC GAGAAGTCGC CCAACTACTG CGAGGAGGAC CCGGTGACCG 120

GCAGTGTGGG CACCCAGGGC CGCGCCTGCA ACAAGACGGC TCCCCAGGCC AGCGGCTGTG 180

ACCTCATGTG CTGTGGGCGT GGCTACAACA CCCACCAGTA CGCCCGCGTG TGGCAGTGCA 240

ACTGTAAGTT CCACTGGTGC TGCTATGTCA AGTGCAACAC GTGCAGCGAG CGCACGGANG 300

ATGTACACGT GCAAGTGAGC CCCGTGTGCA CACCACCCTC CCGCTGCAAG TCAGATTGCT 360

GGGAGGACTG GACCGTTTCC AAGCTGCGGG CTCCCTGGCA GGATGCTGAG CTTGTCTTTT 420

CTGCTGAGGA GGGTACTTTT CCTGGGTTTC CTGCAGGCAT CCGTGGGGGA AAAAAAATCT 480

CTCAGAGNCC TCAACTATTC TGTTCCACAC CCAATGCTGS TCCACCCTCC CCCAGACACA 540

GCCCAGGTCC CTCCGCGGCT GGAGCGAAGC CTTCTGCAGC AGGAACTCTG GACCCCTGGG 600

CCTCATCACA GCAATATTTA ACAATTTATT CCTGATAAAA ATAATATTAA TTTATTTAAT 660

TAAAAAGAAT TCTTCCAAAA AAAAAAAAAA AAAAAAACNT CG 702

(2) INFORMATION FOR SEQ ID NO: 32:

(i) SEQUENCE CHARACTERISTICS:

(A) LENGTH: 1142 base pairs
(B) TYPE: nucleic acid
(C) STRANDEDNESS: double
(D) TOPOLOGY: linear

(xi) SEQUENCE DESCRIPTION: SEQ ID NO: 32:

CGGCACGAGG AAGAAATGGC AGAGACTGGA ATCTCTCTTC ATGAAAAAAT GCAGCCCCTT 60

AACTTCAGTT CGACAGAGTG CAGCTCCTTC TCTCCACCCA CCACAGTGAT TCTCCTTATC 120

CTGCTGTGCT TTGAGGGCCT GCTCTTCCTC ATTTTCACAT CAGTGATGTT TGGGACCCAG 180

GTGCACTCCA TCTGCACAGA TGAGACGGGA ATAGAACAAT TGAAAAAGGA AGAGAGAAGA 240

TGGGCTAAAA AAACAAAATG GATGAACATG AAAGCCGTTT TTGGCCACCC CTTCTCTCTA 300

GGCTGGGCCA GCCCCTTTGC CACGCCAGAC CAAGGGAAGG CAGACCCGTA CCAGTATGTG 360

GTCTGAAGGA CCCCGACCGG CATGGCCACT CAGACACAAG TCCACACCAC AGCACTACCG 420

TCCCATCCGT TCTCATGAAT GTTTAAATCG AAAAAGCAAA ACAACTACTC TTAAAACTTT 480

TTTTATGTCT CAAGTAAAAT GGCTGAGCAT TGCAGAGARA AAAAAAAGTC CCCACATTTT 540

ATTTTTTAAA AACCATCCTT TCGATTTCTT TTGGTGACCG AAGCTGCTCT CTTTTCCTTT 600

TAAAATCACT TCTCTGGCCT CTGGTTTCTC TCTGCTGTCT GTCTGGCATG ACTAATGTAG 660

AGGGCGCTGT CTCGCGCTGT GCCCATTCTA CTAACTGAGT GAGACATGAC GCTGTGCTGG 720

GATGGAATAG TCTGGACACC TGGTGGGGGA TGCATGGGAA AGCCAGGAGG GCCCTGACCT 780

TCCCACTGCC CAGGAGGCAG TGGCGGGCTC CCCGATGGGA CATAAAACCT CACCGAAGAT 840

GGATGCTTAC CCCTTGAGGC CTGAGAAGGG CAGGATCAGA AGGGACCTTG GCACAGCGAC 900

CTCATCCCCC AAGTGGACAC GGTTTGCCTG CTAACTCGCA AAGCAATTGC CTGCCTTGTA 960

CTTTATGGGC TTGGGGTGTG TAGAATGATT TTGCGGGGGA GTGGGGGAGA AAGATGAAAG 1020

AGGTCTTATT TGTATTCTGA ATCAGCAATT ATATTCCCTG TGATTATTTG GAAGAGTGTG 1080

TAGGAAAGAC GTTTTTCCAG TTCAAAATGC CTTATACAAT CAAGAGGAAA AAAAAAAAAA 1140

AG 1142

(2) INFORMATION FOR SEQ ID NO: 33:

(i) SEQUENCE CHARACTERISTICS:
(A) LENGTH: 928 base pairs
(B) TYPE: nucleic acid
(C) STRANDEDNESS: double
(D) TOPOLOGY: linear

(xi) SEQUENCE DESCRIPTION: SEQ ID NO: 33:

GGCACGAGGT CTAATGAGGG CTCTCTTGTT TGCTAGAGAT GAGAGAAATG TATACTAATC 60

ATTTTAATTT GTACTTAAAA TACATTTTAC TAATCATATT GATTTTAAAT ATGACAAATT 120

CTTCTAGTAG ATACTAATCT TTCTTGTTTA TCATATTGTC CTAGAGAAGC CTAGGTAAAA 180

ATGGGTTCCA CCTAGTCTGT TTGTATAACA CCTTCCCCCG TCCCCTCTCC ATCCCTGCCA 240

ATTGGGCTCT ATGCATATTG ACAAGCAAAT AAGAAAACCT TAGGTTCTTG TATTTGAATT 300

TCCAAAACAA TAAAAGGTTT TGACTCAAGA TTTGCATTCA AGAAGAGGCA GAAATTTTGT 360

CTTATCTTTT TATCATTTTG TGAACTTGTG TTTCTCTGTA TGCTTAGAAA ATTTACACAC 420

AAGGAATGTT TGAAAAAGTG AGAATTTTAG AGTGCTTGGG TGGTTTTTAT TTGGTCAGTG 480

CTGATGTGTT AGGTGTTTAG GGAAATAATG CTTCAGGACC TTTTTGACAA CACAGCTTCA 540

TGAATGACTG GGGGATATTT ATGTTTGTGC TGAGAAAAGG GAGGGAGTGG GCAGGTTGGA 600

GTGGGGACCT TTCCATTGAA AGCAGTGCAG TCAGCTGTTT CGTAGATGCA TTTTTTCTTT 660

ATGCTTGTAA CATTGTTCTT GTGTCCATAA TTGACTGAAA TGTCAAGCTC CAGGAATGCA 720

AGGCATTTAT CAGGTGACCA GAAGTAGAAC CTTGTTGATT ATGAAATGGA AGAATAATGT 780

CAAGGTAGTG GGGGTAAAAT GACAAATAAG ATTTTACTGG TGAATTTCCA TGCTTAGTAT 840

GTACATTAAC CTCTTTTTAA GTTGCATGTT AATCTGGTAT AACGTATTGT GTCTGGTTTA 900

TGCTTTGAGT AAAAAAAAAA AAAAAAAA 928

(2) INFORMATION FOR SEQ ID NO: 34:

(i) SEQUENCE CHARACTERISTICS:
(A) LENGTH: 773 base pairs
(B) TYPE: nucleic acid
(C) STRANDEDNESS: double
(D) TOPOLOGY: linear

(xi) SEQUENCE DESCRIPTION: SEQ ID NO: 34:

GGCACGAGTT CTGGCCTCTC ATTTCCTTAC ACTCTGACAT GAATGAATTA TTATTATTTT 60

TCTTTTTCTT TTTTTTTTTT ACATTTTGTA TAGAAACAAA TTCATTTAAA CAAACTTATT 120

ATTATTATTT TTTACAAAAT ATATATATGG AGATGCTCCC TCCCCCTGTG AACCCCCCAG 180

TGCCCCCGTG GGGCTGAGTC TGTGGGCCCA TTCGGCCAAG CTGGATTCTG TGTACCTAGT 240

ACACAGGCAT GACTGGGATC CCGTGTACCG AGTACACGAC CCAGGTATGT ACCAAGTAGG 300

CACCCTTGGG CGCACCCACT GGGGCCAGGG GTCGGGGGAT GTTGGGAGCC TCCTCCCCAC 360

CCCACCTCCC TCACTTCACT GCATTCCAGA TTGGACATGT TCCATAGCCT TGCTGGGGAA 420

GGGCCCACTG CCAACTCCCT CTGCCCCAGC CCCACCCTTG GCCATCTCCC TTTGGGAACT 480

AGGGGGCTGC TGGTGGGAAA TGGGAGCCAG GGCAGATGTA TGCATTCCTT TATGTCCCTG 540

TAAATGTGGG ACTACAAGAA GAGGAGCTGC CTGAGTGGTA CTTTCTCTTC CTGGTAATCC 600

TCTGGCCCAG CCTTATGGCA GAATAGAGGT ATTTTTAGGC TATTTTTGTA ATATGGCTTC 660

TGGTCAAAAT CCCTGTGTAG CTGAATTCCC AAGCCCTGCA TTGTACAGCC CCCCACTCCC 720

CTCACCACCT AATAAAGGAA TAGTTAACAC TCAAAAAAAA AAAAAAAAAA AAA 773

(2) INFORMATION FOR SEQ ID NO: 35:

(i) SEQUENCE CHARACTERISTICS:
(A) LENGTH: 453 base pairs
(B) TYPE: nucleic acid
(C) STRANDEDNESS: double
(D) TOPOLOGY: linear

(xi) SEQUENCE DESCRIPTION: SEQ ID NO: 35:

TAAAATGTTA CACGCTTGTC ATATTCCAGG CACTGCACTA TGTATGCCGT TTATCAACAG 60

TTAGCTCAGC TAACCCTCAT GGTAACCTTG TTAGCCCCGA TTTTGCCAGA TGAGCAAAGT 120

GAGGTTTTTG AGGCCTTAAG TAACTTGCCC AAGGTCACGT GGCTGGGAAG TAACTCTCCC 180

AGTTCTGAGA TGCCCGAGCC TGGACGCTTT GTCATTGTAC ACCATCAACT CAGTGCTGCC 240

AGTCATTCCA GCAGCCAGCT AGCGTAGTCA AGGTTTCTCC ACCTTAGCAC TGTTGACATT 300

TCGAGCCAGA TAATTCTCTG TGGTGAGGAG CTGTCCTATG CCTTGTAGGA TATACAACAG 360

CATCYTGGCT TTACCCACCA GATGYTGGAA CACCTCCCCA GTCGTGACAG CCCAAAATGT 420

CTATAGACGT TGCCACGTAT ACCCAGGGGT TCC 453

(2) INFORMATION FOR SEQ ID NO: 36:

(i) SEQUENCE CHARACTERISTICS:
(A) LENGTH: 459 base pairs
(B) TYPE: nucleic acid
(C) STRANDEDNESS: double
(D) TOPOLOGY: linear

(xi) SEQUENCE DESCRIPTION: SEQ ID NO: 36:

GTGACTGCCG CCCTGCCCGC AGCCATGTGG CCCCCGCTGT TGCTGCTGCT GCTGCTGCTC 60

CCGGCCGCCC CGGTCCCCAC CGCCAAAGCC GCTCCCCACC CGGATGCTAA CACCCAGGAA 120

GGCCTTCAGA ACCTGCTCCA AGGAGTCGGG GCTGGCGGAG ACGGAGAGCT GCGGGCAGAC 180

TCACACCTGG CCCCGGGCTC TGGCTGTATT GATGGGGCTG TGGTGGCCAC GCGACCAGAA 240

AGCCGGGGAG GAAGACCTGC GGTTCCGTGA GAGGCGTCCA GGGCTGCAGG CCACGGCGAC 300

AGGCTCCGGG GAACATGGGG CTTTCCCTGT CCACTCCCAA GGAGTGTGGG CCTCAACGCA 360

TTGGCAGGGG ACGGCCGTGT GCCCTCTYCA GACCCCACCC CCAGATGCAT TTATTAGAAA 420

TAATAAATTC TTTCTTAGCT AAAAAAAAAA AAAAAAAAT 459

(2) INFORMATION FOR SEQ ID NO: 37:

(i) SEQUENCE CHARACTERISTICS:
(A) LENGTH: 509 base pairs
(B) TYPE: nucleic acid
(C) STRANDEDNESS: double
(D) TOPOLOGY: linear

(xi) SEQUENCE DESCRIPTION: SEQ ID NO: 37:

ATGAAATTTA CCACTCTCCT CTTCTTGGCA GCTGTAGCAG GGGCCCTGGT CTATGCTGAA 60

GATGCCTCCT CTGACTCGAC GGGTGCTGAT CCTGCCCAGG AAGCTGGGAC CTCTAAGCCT 120

AATGAAGAGA TCTCAGGTCC AGCAGAACCA GCTTCACCCC CAGAGACAAC CACAACAGCC 180

CAGGAGACTT CGGCGGCAGC AGTTCAGGGG ACAGCCAAGG TCACCTCAAG CAGGCAGGAA 240

CTAAACCCCC TGAAATCCAT AGTGGAGAAA AGTATCTTAC TAACAGAACA AGCCCTTGCA 300

AAAGCAGGAA AAGGAATGCA CGGAGGCGTG CCAGGTGGAA AACAATTCAT CGAAAATGGA 360

AGTGAATTTG CACAAAAATT ACTGAAGAAA TTCAGTCTAT TAAAACCATG GGCATGAGAA 420

GCTGAAAAGA ATGGGATCAT TGGACTTAAA GCCTTAAATA CCCTTGTAGC CCAGAGCTAT 480

TAAAACGAAA GCATCCAAAA AAAAAAAAA 509

(2) INFORMATION FOR SEQ ID NO: 38:

(i) SEQUENCE CHARACTERISTICS:
(A) LENGTH: 598 base pairs
(B) TYPE: nucleic acid
(C) STRANDEDNESS: double
(D) TOPOLOGY: linear

(xi) SEQUENCE DESCRIPTION: SEQ ID NO: 38:

ATGTTGGGCT GTGGGATCCC AGCGCTGGGC CTGCTCCTGC TGCTGCAGGG CTCGGCAGAC 60

GGAAATGGAA TCCAGGGATT CTTCTACCCA TGGAGCTGTG AGGGTGACAT ATGGGACCGG 120

GAGAGCTGTG GGGGCCAGGC GGCCATCGAT AGCCCCAACC TCTGCCTGCG TCTCCGGTGC 180

TGCTACCGCA ATGGGGTCTG CTACCACCAG CGTCCAGACG AAAACGTGCG GAGGAAGCAC 240

ATGTGGGCGC TGGTCTGGAC GTGCAGCGGC CTCCTCCTCC TGAGCTGCAG CATCTGCTTG 300

TTCTGGTGGG CCAAGCGCCG GGACGTGCTG CATATGCCCG GTTTCCTGGC GGGTCCGTGT 360

GACATGTCCA AGTCCGTCTC GCTGCTCTCC AAGCACCGAG GGACCAAGAA GACGCCGTCC 420

ACGGGCAGCG TGCCAGTCGC CCTGTCCAAA GAGTCCAGGG ATGTGGAGGG AGGCACCGAG 480

GGGGAAGGGA CGGAGGAGGG TGAGGAGACA GAGGGCGAGG AAGAGGAGGA TTAGGGGAGT 540

CCCCGGGGGA CTGGTCAATA CAGATACGGT GGACGGAAAA AAAAAAAAAA AAAAAAAA 598

(2) INFORMATION FOR SEQ ID NO: 39:

(i) SEQUENCE CHARACTERISTICS:
(A) LENGTH: 454 base pairs
(B) TYPE: nucleic acid
(C) STRANDEDNESS: double
(D) TOPOLOGY: linear

(xi) SEQUENCE DESCRIPTION: SEQ ID NO: 39:

ATGGAGGCTG TTTTTACAGT TTTTTTTTTT GTTGTTGTTT TGTTTTTAAA GAATACAGAA 60

GGAGCCAAGC TTTTTTGCAC TTTGTATCCA GCTGCAAGCT CAGGGCAGAG TCAAGGGCCT 120

GGGTTGGAAA AACCTGACTC ACAGGAATGC ATAATTGACC CTTGCAGCTA CCCAATAGCC 180

CTTGGAGCTG GCACTGAACC AGGCTGCAAG ATTTGACTGC CTTAAAAACA CAAGGCCCTC 240

TAGGCCTGGC AGGGATGTCC CTGTGCCCAG CACTGGGGGC TCGAAGACTG GTTTCTAGCA 300

CTACCGGTCA CGGCCATGTC GTCCTAGAAG GGTCCAGAAG ATTATTTTAC GTTGAGTCCA 360

TTTTTAATGT TCTGATCACC TGACAGGGCA CCCCAAACCC CCAACTCCCA ATAAAAGCCG 420

TGACGTTCGG ACAAAAAAAA AAAAAAAAAA AAAA 454

(2) INFORMATION FOR SEQ ID NO: 40:

(i) SEQUENCE CHARACTERISTICS:
(A) LENGTH: 425 base pairs
(B) TYPE: nucleic acid
(C) STRANDEDNESS: double
(D) TOPOLOGY: linear

(xi) SEQUENCE DESCRIPTION: SEQ ID NO: 40:

GCTAAAGGCC ATTCCCTCCG CAGGGCATTT GGCGTCGGGT GGGAGGGGAA AACGCATCTT 60

GTTAATTATT TTTAATCTTA TTTATTGTAC ATACCTGGGG CAGGGGCTTG GGGAGGTGGA 120

GGGGGRAGAA GGGTCCCCTC TCTCTGCCCC TCCCACTCCT TTTCTACGGC GATTTGTCTG 180

TGTCTGGCCC CCACCCACTG MCCATCCCCC ATTGTTGTCT GGATGTGGTT CTATTTTTTA 240

TCGGTCTCCT TTCCCCTCCT CCCCGTTYTC GCCCCCGMCC CACCCCCTGC TCCCACTACC 300

CTTTGTCTCT TGCTCTTTCT TGGGYTTCTG TACAACTCAA CTTGTATACA CTGTGTACAC 360

ACAACCAGYC WAACGCAAAA CCCAACGGCA AACACTTTAA AAAAAAAAAA AAAAAACTGG 420

GGGGT 425

(2) INFORMATION FOR SEQ ID NO: 41:

(i) SEQUENCE CHARACTERISTICS:
(A) LENGTH: 2471 base pairs
(B) TYPE: nucleic acid
(C) STRANDEDNESS: double
(D) TOPOLOGY: linear

(xi) SEQUENCE DESCRIPTION: SEQ ID NO: 41:

GGCACGAGTA TGGCTTCCCG TGGACTCAGC CTCTTCCCCG ANTCCTGGCA CGAGGGGGCT 60

TCGCGTCTGT GCTTCCTGTG GCTGACGTCA TCTGGAGGAG ATTTGCTTTC TTTTTCTCCA 120

AAAGGGGAGG AAATTGAAAC TGAGTGGCCC ACGATGGGAA GAGGGGAAAG CCCAGGGGTA 180

CAGGAGGCCT CTGGGTGAAG GCAGAGGCTA ACATGGGGTT CGGAGCGACC TTGGCCGTTG 240

GCCTGACCAT CTTTGTGCTG TCTGTCGTCA CTATCATCAT CTGCTTCACC TGCTCCTGCT 300

GCTGCCTTTA CAAGACGTGC CGCCGACCAC GTCCGGTTGT CACCACCACC ACATCCACCA 360

CTGTGGTGCA TGCCCCTTAT CCTCAGCCTC CAAGTGTGCC GCCCAGCTAC CCTGGACCAA 420

GCTACCAGGG CTACCACACC ATGCCGCCTC AGCCAGGGAT GCCAGCAGCA CCCTACCCAA 480

TGCAGTACCC ACCACCTTAC CCAGCCCAGC CCATGGGCCC ACCGGCCTAC CACGAGACCC 540

TGGCTGGAGA GCAGCCGCGC CCTACCCCGC CAGCCAGCCT CCTTACAACC CGGCCTACAT 600

GGATGCCCCG AAGGCGGCCC TCTGAGCATT CCCTGGCCTC TCTGGCTGCC ACTTGGTTAT 660

GTTGTGTGTG TGCGTGAGTG GTGTGCAGGC GCGGTTCCTT ACGCCCCATG TGTGCTGTGT 720

GTGTCCAGGC ACGGTTCCTT ACGCCCCATG TGTGCTGTGT GTGTCCTGCC TGTATATGTG 780

GCTTCCTCTG ATGCTGACAA GGTGGGGAAC AATCCTTGCC AGAGTGGGCT GGGACCAGAC 840

TTTGTTCTCT TCCTCACCTG AAATTATGCT TCCTAAAATC TCAAGCCAAA CTCAAAGAAT 900

GGGGTGGTGG GGGGCACCCT GTGAGGTGGC CCCTGAGAGG TGGGGGCCTC TCCAGGGCAC 960

ATCTGGAGTT CTTCTCCAGC TTACCCTAGG GTGACCAAGT AGGGCCTGTC ACACCAGGGT 1020

GGCGCAGCTT TCTGTGTGAT GCAGATGTGT CCTGGTTTCG GCAGCGTACC AGCTGCTGCT 1080

TGAGGCCATG GCTCCGTCCC CGGAGTTGGG GGTACCCGTT GCAGAGCCAG GGACATGATG 1140

CAGGCGAAGT TGGGGATCTG GCCAAGTTGG ACTTTGATCC TTTGGGCAGA TGTCCCATTG 1200

CTCCCTGGAG CCTGTCATGC CTGTTGGGGA TCAGGCAGCC TCCTGATGCC AGAACACCTC 1260

AGGCAGAGCC CTACTCAGCT GTACCTGTCT GCCTGGACTG TCCCCTGTCC CCGCATCTCC 1320

CCTGGGACCA GCTGGAGGGC CACATGCACA CACAGCCTAG CTGCCCCCAG GGAGCTCTGC 1380

TGCCCTTGCT GGCCCTGCCC TTCCCACAGG TGAGCAGGGC TCCTGTCCAC CAGCACACTC 1440

AGTTCTCTTC CCTGCAGTGT TTTCATTTTA TTTTAGCCAA ACATTTTGCC TGTTTTCTGT 1500

TTCAAACATG ATAGTTGATA TGAGACTGAA ACCCCTGGGT TGTGGAGGGA AATTGGCTCA 1560

GAGATGGACA ACCTGGCAAC TGTGAGTCCC TGCTTCCCGA CACCAGCCTC ATGGAATATG 1620

CAACAACTCC TGTACCCCAG TCCACGGTGT TCTGGCAGCA GGGACACCTG GGCCAATGGG 1680

CCATCTGGAC CAAAGGTGGG GTGTGGGGCC CTGGATGGCA GCTCTGGCCC AGACATGAAT 1740

ACCTCGTGTT CCTCCTCCCT CTATTACTGT TTCACCAGAG CTGTCTTAGC TCAAATCTGT 1800

TGTGTTTCTG AGTCTAGGGT CTGTACACTT GTTTATAATA AATGCAATCG TTTGGAAAAA 1860

AAAAAAAAAA AAACTCGTAG GGGGGGCCCG TACCCAATGG GCYCMMARAT AGTAGARWAC 1920

RAAAAYAMCA ANTGCAACCA AAGAGGGGCC AGGGGANTTT TAAGAGGGCC CCCTTTTGGG 1980

GGNATCCANT TTAGCCGGGG TTNTTAAGGG AAGTTGCNTG GCGGGGGTTA GGGCCCSGTT 2040

KYTWCTTCCA ACCAAGGGTT YTYGTGGTTA GGCCGGGTTG GGCCCMATGG GCTGGGCTGG 2100

GTAAAGTGGT GGGTMAYTGC MATTGGGTAG GGTGCTGCTG GCATTCCTGG CTGAGGCGGC 2160

ATGGTGTGGT AGCCCTGGTA GCTTGGTCCA GGGTAGCTGG GCGGCACACT TGGAGGCTGA 2220

GGATAAGGGG CATGCACCCA CAGTGGTGGA TGTGGTGGTG GTGACAACCG GACGTGGTCG 2280

GCGGCACGTC TTGTAAAGGC AGCAGCAGGA GCAGGTGAAG CAGATGATGA TAGTGACGAC 2340

AGACAGCACA AAGATGGTCC AGCCAACGGC CAAGGTCGCT CCGAACCCCA TGTTAGCCTC 2400

TGCCTTCACC CAGAGGCCTC CTGTACCCCT GGGCTTTCCC CTCTTCCCAT CGTGGGCCAC 2460

TCACTCGTGC C 2471

(2) INFORMATION FOR SEQ ID NO: 42:

(i) SEQUENCE CHARACTERISTICS:
(A) LENGTH: 2659 base pairs
(B) TYPE: nucleic acid
(C) STRANDEDNESS: double
(D) TOPOLOGY: linear

(xi) SEQUENCE DESCRIPTION: SEQ ID NO: 42:

GGCACGAGCT TTTCTCTAGA GTCTGAAAGA TGCTAGAAAG AAATAAAATT TAACTTACTT 60

AAGAGAATTA TGGATCTTTT ATTAATAAAA ATTAACTTGA TGATTTGAAC TAACAGTTAT 120

GATAATTCTG GTATTTATAG CTTTTTTTAT TCCCCTGCAG AAAACCATAG GCAAAATTGC 180

AACATGCTTG GAATTGCGAA GTGCAGCTTT ACAGTCCACA CAGTCTCAAG AAGAATTTAA 240

ACTGGAGGAC CTGAAGAAGC TAGAACCAAT CCTAAAGAAT ATTCTTACAT ATAATAAAGA 300

ATTCCCATTT GATGTTCAGC CTGTCCCATT AAGAAGAATT TTGGCACCTG GTGAAGAAGA 360

GAATTTGGAA TTTGAAGAAG ATGAAGAAGA GGGTGGTGCT GGAGCAGGTC TCCTGATTCT 420

TTCCTGCTAG AGTTCCCGGT ACTTTATTAC CAAGGTTGCC ATCGGAACCA GGAATGACAT 480

TACTCACTAT CAGAATTGAG AAAATTGGTT TGAAAGATGC TGGGCAGTGC ATCGATCCCT 540

ATATTACAGT TAGTGTAAAG GATCTGAATG GCATAGACTT AACTCCTGTG CAAGATACTC 600

CTGTGGCTTC AAGAAAAGAA GATACATATG TTCATTTTAA TGTGGACATT GAGCTCCAGA 660

AGCATGTTGA AAAATTAACC AAAGGTGCAG CTATCTTCTT TGAATTCAAA CACTACAAGC 720

CTAAAAAAAG GTTTACCAGC ACCAAGTGTT TTGCTTTCAT GGAGATGGAT GAAATTAAAC 780

CTGGGCCAAT TGTAATAGAA CTATACAAGA AACCCACTGA CTTTAAAAGA AAGAAATTGC 840

AATTATTGAC CAAGAAACCA CTTTATCTTC ATCTACATCA AACTTTGCAC AAGGAATGAT 900

CCTGACATGA TGAACCTGGA ACTTCTGTGA ATTTTACCAC TCAGTAGAAA CCATCATAGC 960

TCTGTGTAGC ATATTCACCC TTCAACAGGC AGGAAGCAAG CCGTACCCAG ACCAGTAGGC 1020

CGGACGGAGT CAAATGCAAA GCTGTACCAC AGAATTCAGA GTCCAGCACA TCACACTGAC 1080

GTATAGGACT CCTTGGGATA CAGGTTTATT GTAGATTTTG AAACATGTTT TTACTTTTCT 1140

ATTAATTGTG CAATTAATAG TCTATTTTCT AATTTACCAC TACTCCTACC CTGCTTCCTG 1200

GAACAATACT GTTGTGGGTA GGATGTGCTC ATCTTCAGAC TTAATACAGC AATAAGAATG 1260

TGCTAGAGTT TACACATCTG TTCACTTTTG CTCCAATATG CTCTTTTGAC TTAACGTCAA 1320

GCTTTGGGTT GATGTGGGTA GGGTAGTGTC AAACTGCTTT GAGAGGAATG GGACCAGTTC 1380

TGCTGCCTAA GAAGGTCTGT CTGGATGTTT ATAGGCAGCA CCTCTGAAGT GGCCTAAATT 1440

CACCCTGATC TGATAGTTTT CCTGCTTAGA AAGTGTGCCT TGGCCAGATC AGTATCCCAC 1500

ATGGGAGTGT TCCCTAGGTT GTAGCTGTGA TTGTTTCCAG ATGACCAGAT TGTTTTTCTG 1560

AAAATGAGCA TATTTTTAGT CATGTCGATT AGCTGTTCTT CTACATCACA TTGTTACTCT 1620

TTCTGATGAT GATTCTAGGG TTAACATTGG AACCATCTCA AAATAATTAC AAAGTTTTAG 1680

ATGGGTTTAC AATGTCTTCT AAACAATGTA ATCTAAAAAT AATTGAGTCA GATGCTAACG 1740

AGATACTGCA GGCATAACTG CTGTTTTTCT GACAACTGAT TGTGAAACCT TAAAACCTGC 1800

ATACCTCTTC TTACAGTGAG GAGTATGCAA AATCTGGAAA GATATTCTAT TTTTTTTATA 1860

TAGGTAGATA GGATCGCCAT TTATTTCCTA TTTAGATATA CTGACATTCA TCCATATGAA 1920

AATATGCAGG TCATTAGCTT ACTATAATTT ACTTTTGACT TAATGGGGCA TAAATAAAAC 1980

TTTCATAGTA CACATGAGGT GGATATTTGA TACACAGAAC ATTTGCGGTG GGCTTTCTGT 2040

GGGTTAGATG TAAAGCCCAC ATATTTTAAT ATTCACTATT TTAAATGAGC AATGCATGAG 2100

GGGAATGCAG TGTCAGTACC TGGCCTATTT TTAAACTAGT GTAATCACCC TAGTCATACC 2160

ATTCAGTATG TTTGCTTTTT AAAATAAGTA ACCACAATTA AGTTGTTGTA GCCCTTGCAC 2220

TTCAAGAGAT CTAGTCTTTA CTTTCAGTTG TCTGTTAGGT CCATTCTGTT TACTAGACGG 2280

ATGTTAATAA AAACTATGCG AGCCTGGAAT GGAATTCTCC AGCCAAATTT TAGTCTTGTC 2340

CTCTCCATCT TGATTGGATT AATTCCAAAT TCTAAAATGA TTCAGTCCAC AATAGCTCTA 2400

GGGGATGAAG AATTTGCCTT ACTTTGCCCA GTTCCTAAGA CTGTGAGTTG TCAAATCCCT 2460

AGACTGTAAG CTCTTCAAGG AGCAAGAGGC GCATTTTCTC CGTGTCATGT AATTTTTCTA 2520

AGGTGTTTGG CAGCACTCTG TACCCTGTGG AGTACTCAGT ACCTTTTGTT TGATGTTGCT 2580

GACAAGACCT GAAAAAAAAT CCCTTAAAAA AAAAACCCAT TAAAGTGTAG CAAAACCGAA 2640

AWAAAAAAAA AAAAAAAAA 2659

(2) INFORMATION FOR SEQ ID NO: 43:

(i) SEQUENCE CHARACTERISTICS:
(A) LENGTH: 1635 base pairs
(B) TYPE: nucleic acid
(C) STRANDEDNESS: double
(D) TOPOLOGY: linear

(xi) SEQUENCE DESCRIPTION: SEQ ID NO: 43:

CGAGGAGGTC ATGAACAAGG AGGCGGGAGA GGTGGACGTG GTGGCTATGA CCATGGTGGC 60

CGAGGGGGAG GAAGAGGAAA TAAGCATCAA GGAGGCTGGA CAGATGGAGG GAGTGGTGGA 120

GGAGGTGGCT ACCAAGATGG TGGTTATCGA GATTCAGGTT TCCAGCCAGG TGGCTATCAT 180

GGTGGCCACA GCAGTGGTGG CTATCAAGGC GGAGGTTATG GTGGCTTCCA AACATCTTCT 240

TCATATACAG GAAGTGGATA CCAGGGTGGT GGCTACCAGC AGGACAATAG ATACCAAGAT 300

GGCGGGCACC ATGGTGATCG TGGTGGTGGT CGTGGTGGGC GAGGTGGTCG TGGAGGCCGA 360

GGTGGTCGTG CAGGCCAGGG AGGAGGCTGG GGAGGAAGAG GGAGCCAGAA TTATCACCAA 420

GGGGGTCAAT TTGAACAGCA TTTCCAGCAT GGAGGTTATC AGTATAATCA TTCTGGATTT 480

GGACAGGGAA GACATTACAC TAGTTGAGGC TACCGAACCT TACATTTTGC TAGAGCTCAA 540

GTAATAGAAA CTTAGTTTCA GAATCCTGAA TTCAGCACCT ATTTTGAATT AATGTGAGAC 600

CACAGGTGGC AGGCAGATTC CTGCTTGGCA TAAGCATTTG TAGGTCTTCA TTCAATTCTG 660

TTAGATTTTT TTATTGGACT TACATAATGC CGTTTATTTG AGAAACACAT AACATCTCTC 720

CTTTCTATGA AAAATTTTTT AAAAGGTGGT TAAAATTGCC TTTAATTGCC CAGTAGACTA 780

ATTCCACAGT CAGAACATGC AAACTTTTTT GAAGAAATTA CTTGAATAAG TAGTTTTCAT 840

GTTTTCAATA TGCAGTTTTG AAAATGAGGA TTCACCTAGA CTTTTTTAGA TTTACTACYA 900

GGAAACCTTC CYCATATGAA TAACCATTTA TATGTGTTTT GCTTAAAGTA TTCCAATGCC 960

TATTTTCCAA GCACAGTTCT GCCCCCCGGT TGACTTTTAT GCCACGTGTG CTTCATGATG 1020

GAACTTTTAG GTCAGTTCCT ATTAAATGAG CTCTTYTGCA GATAGCACAT TCAGTAGCCT 1080

TATTTTGTTG ATGGAATACT GTATCATATG CTCAACTCTG AAAACCTTGA ACACGGCCAA 1140

AATCCATAAA GATTATAAAA GCAAACTAAG TTGTGAAGCT ATAGTACATG TAGGCATTTA 1200

GTTAAGTATA GCAATTCAAA CTGACCTGCA TCCATCCAAA ACAAATTCCT CCTTCAACCT 1260

TATTTTTACT TGAAATTTGC TAGAAGAAAT AGCAAACCGA AATTTGTTTT ATGCATGAGT 1320

TAATACCACT GGCTCAGCAA ATACAAGTTA GTTTGCTTTA AGCAGGTAAC TTTTTTTGTA 1380

ATGGAAGAAA TGCACTACAA AGTTAAGACA GATTTTTGCT AAGTGCAGGA GGCCCTTTAT 1440

TATTGCTGCA GAAAACAAAA GCCTGGCTGA GTTGATGTTT TACATTCTCC CTTACTGAAA 1500

TCTACATGAC ATGATGCTTC TTGCTGGGTT TTTGTACATG TAAACATTGT CAAGCTGTGA 1560

AAGAAAATGG CTGGAGGTGT GCTTTGTGTG AAAGGTGAGC ACTGAAAGTA TCTGTTAAGT 1620

TCTCCNGAAA AAAAA 1635

(2) INFORMATION FOR SEQ ID NO: 44:

(i) SEQUENCE CHARACTERISTICS:
(A) LENGTH: 780 base pairs
(B) TYPE: nucleic acid
(C) STRANDEDNESS: double
(D) TOPOLOGY: linear

(xi) SEQUENCE DESCRIPTION: SEQ ID NO: 44:

AACATGGTCA TGTCTTTTAG TTTCATTATT TTCCTACTCC TTGTATGTCA AGAAATTACA 60

TTTTGCATGT CTTATGGAGA TGCTGTTAAT TGCTTCAGTG AGTGCTTTTC TAATCTGCAG 120

ACCATTTACA TTTCCTGTTT GCAGCATGCT GTGTGCAAAC AYTCAGTAAT TTGGAGTATT 180

CAATTATTTG TTAGGGCTCT TCCTATTTCC AAATGTGCTG AATTGTCTAT TGATGGGATT 240

TTCAGATCTT TTCATGAGAA CTGGAAATGT AGCTGGGTGG CACCTACCTA GGTTGCTACG 300

TAGTGAGTAG ACTTTCTCTT GGGTATAGTA AGCCTCAGAC AGCTTTCACT TTTATCTACT 360

TTACTTGTGG AAATAAAACA GTCATTTTGT TCTGAAAGAA TAAGATAGCT TTCTGTAGAG 420

AAGGAATTCC TACCTCTAAA AGCTGCCTTG AGAACTCAGA ACTGGCAGTT TTCTGAGGTG 480

ATTTTTAAAT TTCAGTATTA GGGAGAGTCC AGCATTTGCT GACACAGATT CTACATAACT 540

AATGTATGAT AGCAAATGCA AAACTATTAT AATGTGGTGT ATCTTGCGCA TACACAGGTT 600

AGAACAAGTA GACTCTGGCA GCAGATCTCC AGAGACCCAA GTTTAGGTTC TCATAGTGTA 660

TTTGAAGTAG TTATACTCCT GGCTTAAGTA GTTTAGTGCC TGGGAGAATC CATTACTGAA 720

AAGCATTTAA CTTAAAAAAA AAAAAAAAAA AAAACTGAAA AGGTAGTGAA TACAGAATAG 780

(2) INFORMATION FOR SEQ ID NO: 45:

(i) SEQUENCE CHARACTERISTICS:
(A) LENGTH: 2378 base pairs
(B) TYPE: nucleic acid
(C) STRANDEDNESS: double
(D) TOPOLOGY: linear

(xi) SEQUENCE DESCRIPTION: SEQ ID NO: 45:

GCGAAGCAGC TGAAGCCGCC GCCGCGCAGA ATCCACGCTG GCTCCGTGCG CCATGGTCAC 60

CCACAGCAAG TTTCCCGCCG CCGGGATGAG CCGCCCCCTG GACACCAGCC TGCGCCTCAA 120

GACCTTCAGC TCCAAGAGCG AGTACCAGCT GGTGGTGAAC GCAGTGCGCA AGTGCAGGAG 180

AGCGGCTTCT ACTGGAGCGC AGTGACCGGC GGCGAGGCGA ACCTGCTGCT CAGTGCCGAG 240

CCCGCCGGCA CCTTTCTGAT CCGCGACAGC TCGGGACCAG CGCCACTTCT TCACGCTCAG 300

CGTCAAGACC CAGTCTGGGA CCAAGAACCT GCGCATCCAG TGTGAGGGGG GCAGCTTCTC 360

TCTGCAGAGC GATCCCCGGA GCACGCAGCC CGTGSCCCGC TTCGACTGCG TGCTCAAGCT 420

GGTGCACCAC TACATGCCGC CCCCTGGAGC CCCCTCCTTC CCCTCGCCAC CTACTGAACC 480

CTCCTCCGAG GTGCCCGAGC AGCCGTCTGC CCAGCCACTC CCTGGGAGTC CCCCCAGAAG 540

AGCCTATTAC ATCTACTCCG GGGGCGAGAA GATCCCCCTG GTGTTGAGCC GGCCCCTCTC 600

CTCCAACGTG GCCACTCTTC AGCATCTCTG TCGGAAGACC GTCAACGGCC ACCTGGACTC 660

CTATGAGAAA GTCACCCAGC TGCCGGGGCC CATTCGGGAG TTCCTGGACC AGTACGATGC 720

CCCGCTTTAA GGGGTAAAGG GCGCAAAGGG CATGGGTCGG GAGAGGGGAC GCAGGCCCCT 780

CTCCTCCGTG GCACATGGCA CAAGCACAAG AAGCCAACCA GGAGAGAGTC CTGTAGCTCT 840

GGGGGGAAAG AGGGCGGACA GGCCCCTCCC TCTGCCCTCT CCCTGCAGAA TGTGGCAGGC 900

GGACCTGGAA TGTGTTGGAG GGAAGGGGGA GTACCACCTG AGTCTCCAGC TTCTCCGGAG 960

GASCCAGCTG TCCTGGTGGG ACGATAGCAA CCACAAGTGG ATTCTCCTTC AATTCCTCAG 1020

CTTCCCCTCT GCCTCCAAAC AGGGGACACT TCGGGAATGC TGAACTAATG AGAACTGCCA 1080

GGGAATCTTC AAACTTTCCA ACGGAACTTG TTTGCTCTTT GATTTGGTTT AAACCTGAGC 1140

TGGTTGTGGA GCCTGGGAAA GGTGGAAGAG AGAGAGGTCC TGAGGGCCCC AGGGCTGCGG 1200

GCTGGCGAAG GAAATGGTCA CACCCCCCGC CCACCCCAGG CGAGGATCCT GGTGACATGC 1260

TCCTCTCCCT GGCTCCGGGG AGAAGGGCTT GGGGTGACCT GAAAGGGAAC CATCCTGGTG 1320

CCCCACATCC TCTCCTCCGG GACAGTCACC GAAAACACAG GTTCCAAAGT CTACCTGGTG 1380

CCTGAGAGCC CAGGGCCCTT CCTCCGTTTT AAGGGGGAAG CAACATTTGG CACGAGATGG 1440

GCTGGTCAGC TGGTCTCCTT TTCCTACTCA TACTATACCT TCCTGTACCT GGGTGGATGG 1500

AGCGGGAGGA TGGAGAGACG GGACATCTTT CACCTCAGGC TCCTGGTAGA GAATACAGGG 1560

GATTCTACTC TGTGCCTCCT GACTATGTCT GGCTAAGAGA TTCGCCTTAA ATGCTCCCTG 1620

TCCCATGGAG AGGGACCCAG CATAGGAAAG CCACATACTC AGCCTGGATG GGTGGAGAGG 1680

CTGAGGGACT CACTGGAGGG CACCAAGCCA GCCCACAGCC AGGGAAGTGG GGAGGGGGGC 1740

GGAAACCCAT GCCTCCCAGC TGAGCACTGG GAATGTCAGC CCAGTAAGTA TTGGCCAGTC 1800

AGGCGCCTCG TGGTCAGAGC AGAGCCACCA GGTCCCACTG CCCCGAGCCC TGCACAGCCC 1860

TCCCTCCTGC CTGGGTGGGG GAGGCTGGAG GTCATTGGAG AGGCTGGACT GCTGCCACCC 1920

CGGGTGCTCC CGCTCTGCCA TAGCACTGAT CAGTGACAAT TTACAGGAAT GTAGCAGCGA 1980

TGGAATTACC TGGAACAGTT TTTTGTTTTT GTTTTTGTTT TTGTTTTTGT GGGGGGGGGC 2040

AACTAAACAA ACACAAAGTA TTCTGTGTCA GGTATTGGGC TGGACAGGGC AGTTGTGTGT 2100

TGGGGTGGTT TTTTTCTCTA TTTTTTTGTT TGTTTCTTGT TTTTTAATAA TGTTTACAAT 2160

CTGCCTCAAT CACTCTGTCT TTTATAAAGA TTCCACTCCA GTCCTCTCTC CTCCCCCCTA 2220

CTCAGGCCCT TGAGGCTATT AGGAGATGCT TGAAGAACTC AACAAAATCC CAATCCAAGT 2280

CAAACTTTGC ACATATTTAT ATTTATATTC AGAAAAGAAA CATTTCAGTA ATTTATAATA 2340

AAGAGCACTA TTTTTTAATG AAAAAAAAAA AAAAAAAA 2378

(2) INFORMATION FOR SEQ ID NO: 46:

(i) SEQUENCE CHARACTERISTICS:
(A) LENGTH: 1772 base pairs
(B) TYPE: nucleic acid

(C) STRANDEDNESS: double
(D) TOPOLOGY: linear

(xi) SEQUENCE DESCRIPTION: SEQ ID NO: 46:

TCGACCCACG CGTCCGGGAG GATCCCCAGC CGGGTCCCAA GCCTGTGCCT GAGCCTGAGC 60

CTGAGCCTGA GCCGAGCCGG GAGCCGGTCG CGGGGGCTCC GGGCTGTGGG ACCGCTGGGC 120

CCCCAGCGAT GGCGACCCTG TGGGGAGGCC TTCTTCGGCT TGGCTCCTTG CTCAGCCTGT 180

CGTGCCTGGC GCTTTCCGTG CTGCTGCTGG CGCACTGTCA GACGCCGCCA AGAATTTCGA 240

GGATGTCAGA TGTAAATGTA TCTGCCCTCC CTATAAAGAA AAATTCTGGG CATATTTATA 300

ATAAGAACAT ATCTCAGAAA GATTGTGATT GCCTTCATGT TGTGGAGCCC ATGCCTGTGC 360

GGGGGCCTGA TGTAGAAGCA TACTGTCTAC GCTGTGAATG CAAATATGAA GAAAGAAGCT 420

CTGTCACAAT CAAGGTTACC ATTATAATTT ATCTCTCCAT TTTGGGCCTT CTACTTCTGT 480

ACATGGTATA TCTTACTCTG GTTGAGCCCA TACTGAAGAG GCGCCTCTTT GGACATGCAC 540

AGTTGATACA GAGTGATGAT GATATTGGGG ATCACCAGCC TTTTGCAAAT GCACACGATG 600

TGCTAGCCCG CTCCCGCAGT CGAGCCAACG TGCTGAACAA GGTAGAATAT GGCACAGCAG 660

CGCTGGAAGC TTCAAGTCCA AGAGCAGCGA AAAGTCTGTC TTTGACCGGC ATGTTGTCCT 720

CAGCTAATTG GGGAATTGAA TTCAAGGTGA CTAGAAAGAA ACAGGCAGAC AACTGGAAAG 780

GAACTGACTG GGTTTTGCTG GGTTTCATTT TAATACCTTG TTGATTTCAC CAACTGTTGC 840

TGGAAGATTC AAAACTGGAA GKAAAAACTT GCTTGATTTT TTTTTCTTGT TAACGTAATA 900

ATAGAGACAT TTTTAAAAGC ACACAGCTCA AAGTCAGCCA ATAAGTCTTT TCCTATTTGT 960

GACTTTTACT AATAAAAATA AATCTGCCTG TAAAATAAAT TAAAAAATCC TTTACCTGGA 1020

ACAAGCACTC TCTTTTTCAC CACATAGTTT TAACTTGACT TTCCAAGATA ATTTTCAGGG 1080

TTTTTGTTGT TGTTGTTTTT TGTTTGTTTG TTTTGGTGGG AGAGGGGAGG GATGCCTGGG 1140

AAGTGGTTAA CAACTTTTTT CAAGTCACTT TACTAAACAA ACTTTTGTAA ATAGACCTTA 1200

CCTTCTATTT TCGAGTTTCA TTTATATTTT GCAGTGTAGC CAGCCTCATC AAAGAGCTGA 1260

CTTACTCATT TGACTTTTGC ACTGACTGTA TTATCTGGGT ATCTGCTGTG TCTGCACTTC 1320

ATGGTAAACG GGATCTAAAA TGCCTGGTGG CTTTTCACAA AAAGCAGATT TTCTTCATGT 1380

ACTGTGATGT CTGATGCAAT GCATCCTAGA ACAAACTGGC CATTTGCTAG TTTACTCTAA 1440

AGACTAAACA TAGTCTTGGT GTGTGTGGTC TTACTCATCT TCTAGTACCT TTAAGGACAA 1500

ATCCTAAGGA CTTGGACACT TGCAATAAAG AAATTTTATT TTAAACCCAA GCCTCCCTGG 1560

ATTGATAATA TATACACATT TGTCAGCATT TCCGGTCGTG GTGAGAGGCA GCTGTTTGAG 1620

CTCCAATGTG TGCAGCTTTG AACTAGGGCT GGGGTTGTGG GTGCCTCTTC TGAAAGGTCT 1680

AACCATTATT GGATAACTGG CTTTTTTTCT TCCTCTTTGG AATGTAACAA TAAAAATAAT 1740

TTTTGAAACA TCAAAAAAAA AAAAAAAAAA AA 1772

(2) INFORMATION FOR SEQ ID NO: 47:

(i) SEQUENCE CHARACTERISTICS:
(A) LENGTH: 1107 base pairs
(B) TYPE: nucleic acid
(C) STRANDEDNESS: double
(D) TOPOLOGY: linear

(xi) SEQUENCE DESCRIPTION: SEQ ID NO: 47:

CGGGCGAGAA GGGCAGACGG GACATGCAGC CTCTTCCGCC TGAGCCCCGG AAGTGATGTG 60

GCTGCGGCAT CGCGGCCTCG CTATGTCTGC CATTTTCAAT TTTCAGAGTC TATTGACTGT 120

AATCTTGCTG CTTATATGTA CCTGTGCTTA TATTCGATCC TTGGCACCCA GCCTCCTGGA 180

CAGAAATAAA ACTGGATTGT TGGGTATATT TTGGAAGTGT GCCAGAATTG GTGAACGGAA 240

GAGTCCTTAT GTTGCAGTAT GCTGTATAGT AATGGCCTTC AGCATCCTCT TCATACAGTA 300

GCTGGGGAAA ATGCCAGAAT GTAGTTGCCA TCAGATTTGA TTGTGAACAA GGACTGACTG 360

CAGAAAATAA TGGAAAGGAT GTTTAACTCT TTTATCTCCG AACATTGAAT GAGATAAATT 420

TCCAGATGCT GTTCTCTATT TTAATGTTAT TGGACCAATG TTCTGTATAA ACAATTAAGA 480

TGTAACCATT TAATAGTCTG TAACAATCAA CCTCAGTACT GTCACTACAA TATTACATTC 540

TGCAAATGTT ATTCTGTTGT ATCAGATACA AAATTTTAGT GAGGTATCTC TAAGGCACAT 600

AGTAGAAAAC AAAATTGGTT AATTACTCAA GTTCCTTTCA CTGTGATTTG GAAATGATTT 660

AATCTTTATA GAATGAGAAC CTTTTTTGGA CTAGCTTTTT TATTAAAATG GCTCAATTTG 720

TGTTGATAAG GATTGCATTA ATATTTAATA GTGCTTGCTT TTCCTCTGGG CACACCATTT 780

TGATCATTAA CCAGAGTACC TCTACTCTTA GCAAACTCTA GTTTATGACA AGTATTTAAA 840

ATATTTAAAA CAAGCTTATG CAGTTCTTAA GGACGAAGGT AAATGAGATG TAACTTAAAA 900

ATAGTATTGG GAAAATGTTG ATAGTTAACA TTAGTGGATT TAGACTAGCC AAATGACATA 960

GTAGGCTCTG AAACATCTTG TCAAGTATAT GTATTTTGTG CATGAATTTT TGCTGGAAAG 1020

CTGTCTTTCT CTGAAAAACA CAACGTTCTT AGAATGAAAA GAACAATTAT AAAATAAAAA 1080

AAAAATTTAA AAAAAACTGG GCGGGGG 1107

(2) INFORMATION FOR SEQ ID NO: 48:

(i) SEQUENCE CHARACTERISTICS:
(A) LENGTH: 805 base pairs
(B) TYPE: nucleic acid
(C) STRANDEDNESS: double
(D) TOPOLOGY: linear

(xi) SEQUENCE DESCRIPTION: SEQ ID NO: 48:

TGCAGAAGAG ATGGAGTTGC TGTTGGAAAA CTACTACCGA TTGGCTGACG ATCTCTCCAA 60

TGCAGCTCGT GAGCTTAGGG TGCTGATTGA TGATTCACAA AGTATTATTT TCATTAATCT 120

GGACAGCCAC CGAAACGTGA TGATGAGGTT GAATCTACAG CTGACCATGG GAACCTTCTC 180

TCTTTCGCTC TTTGGACTAA TGGGAGTTGC TTTTGGAATG AATTTGGAAT CTTCCCTTGA 240

AGAGGACCAT AGAATTTTTT GGCTGATTAC AGGAATTATG TTCATGGGAA GTGGCCTCAT 300

CTGGAGGCGC CTGCTTTCAT TCCTTGGACG ACAGCTAGAA GCTCCATTGC CTCCTATGGT 360

ATGAAGGATA TGGTTCACGG CGGTATTGTG GAAGGGTTAT GATCATGGGC CCTAAAGTCA 420

GAGCGCCTGG GATTAAGTTG TCACAGGCAC TATGGCCCTT GCGAGTTGCT TTCTCAAACT 480

TCCTTCAGTT TCCCTATCTG TCAGTTAAGT CGGTATTACC TGCTTCATAG GGTTATGGGA 540

AGAATTAAAC AATATGTGTA AAGCACTTAC TAGCACACTG CCTAACACAA TAAGTTAGAA 600

ATATAATTTG TGTAGAACTC TGACAACATA CATTTAAACA GATGTTAGTA ATTCTGGTAT 660

AAGGTTTGTC ATAACCAAAT GGAAATGTAG GAAACATTTA TAATGTTCTT AAAAGATAGR 720

AAATTCACCT CCATTTTCTT TGTACTTGAA GATGGCACCA CTGGAATAAA TACTTAAGAC 780

ACTGAAAAAA AAAAAAAAAA AACTC 805

(2) INFORMATION FOR SEQ ID NO: 49:

(i) SEQUENCE CHARACTERISTICS:
(A) LENGTH: 1408 base pairs
(B) TYPE: nucleic acid
(C) STRANDEDNESS: double
(D) TOPOLOGY: linear

(xi) SEQUENCE DESCRIPTION: SEQ ID NO: 49:

TCATTATTTA TTCATGTGGC TGAAAGAGTA TATTAATTAT GTTTAGATTT TTGGAAAAAG 60

TCTGAACAAA AAAAGGACCT ATACAGTGCT CAAACTATAT TTTTAAAAAT ACTATTTTAT 120

TTTTACTCAC ATATGAAAAA AATGGCTGTA CTATCATGTT TACATACATA CTAACATTGG 180

AAACAGAATA ACGAATTGTA TTTAAATTTT ATGAAGAACA CACAAACATT AAAACACTGA 240

TTGGTTACAG AAAGCAGAGT TTGAGGAAAA AACATTAGCT ATAATTTTCA TTTTCATTAA 300

AGAGCAGCAC CCTCTGAGAA TAATCAAACT GATTAGTAAT ATTCATCTAT ACTGCAAAAT 360

AATATGTACA AAGGAAAGTT AGTGATTGTA CTGATTTTAT TACTTTTACC AAGCCATTTT 420

ATGTTCCTCA CTCAATGCAA AGAAATAAAA CATAATCTGA AGAAAAATAT GTCCTTATTA 480

TTATTCACAA TAAAAAGTTG GCTTTATTCT GCAAGCCTGG GCATATTGTA CAATTGGCAG 540

CACTTAACGG CTCAAGTGGA TCAATGTACC AGTTTGATTC TGATCCACTG AATAGAATCT 600

CTCATCCATA TCTGGTGACC AGACTAACTC CATGGGAGCT GTGATAGACT GAACCATTTC 660

TGTGGTATCC CTAGATCTCA CTAAATAAGA AAGACCCTAC ACCAGAAAAT ATAGCAACTG 720

ATCTATCTAT AAATTACATC TATATGCTAG CTCTTTAGTA TAAGTTGGAA AAAGGGGCCC 780

TTTCTTGAGC ACATGGATAA AAGTATTATT GTAGTCTAAA GATTGCTGGA TTGATATTGT 840

GTTGTTATAA TGAAGATAAG GTACACACTG AAACCACTGT CAGATTAAGA AACTTCCACA 900

ACTTGTCTCA GTTCTTCAAA CAATGGAGCA AGTTCCTTTT CTAGGCTGAC AATTAGTCCT 960

GTATTGGCAC TGCTGCTGGC TATGAAACTC ACCACCAAAG GTAAACGATT AAATTGAACC 1020

ACCTGGTAGG TGTTATAGTA ACAGATGATA CTTTTATTTT TGGAAAGTCC AAGTTTGCTT 1080

CCTTGGTCTG TTGCAAGGGC AAAAGTGGAT AAGAAACCAG GTCGCAAAGC ATGCTCTGGA 1140

GCATTGTCAT TTGCCACTTT AATAACAGGT ACTCCATCTC TATCTGACAC AACAATGGCA 1200

TGGAGCCCTT CAACACTTGG TAACTTTTTA TACAAGAATC GCTTTAGGTC ATCCGCCATG 1260

ATGAACCCCC TTCTCTCGCA GGATCAATCT CCACGCCTGG GGTTTCTGGG CTGCCTGGTT 1320

CTCTCCGCTG TCACTTCAGG GACAGCTTTA AAGACAGGTT CCTCCTCAAG CCACCGTCAC 1380

ATGATTCATG ACCTCGTCTG CGCTCCAG 1408

(2) INFORMATION FOR SEQ ID NO: 50:

(i) SEQUENCE CHARACTERISTICS:
(A) LENGTH: 1813 base pairs
(B) TYPE: nucleic acid
(C) STRANDEDNESS: double
(D) TOPOLOGY: linear

(xi) SEQUENCE DESCRIPTION: SEQ ID NO: 50:

CATGGTGGGG CACGAGATGG CCTCTRACTC TTCWAACACT TCACTGCCAT TCTCAAACAT 60

GGGAAATCCA ATGAACACCA CACAGTTAGG GAAATCACTT TTTCAGTGGC AGGTGGAGCA 120

GGAAGAAAGC AAATTGGCAA ATATTTCCCA AGACCAGTTT CTTTCAAAGG ATGCAGATGG 180

TGACACGTTC CTTCATATTG CTGTTGCCCA AGGGAGAAGG GCACTTTCCT ATGTTCTTGC 240

AAGAAAGATG AATGCACTTC ACATGCTGGA TATTAAAGAG CACAATGGAC AGAGTGCCTT 300

TCAGGTGGCA GTGGCTGCCA ATCAGCATCT CATTGTGCAG GATCTGGTGA ACATCGGGGC 360

ACAGGTGAAC ACCACAGACT GCTGGGGAAG AACACCTCTG CATGTGTGTG CTGAGAAGGG 420

CCACTCCCAG GTGCTTCAGG CGATTCAGAA GGGAGCAGTG GGAAGTAATC AGTTTGTGGA 480

TCTTGAGGCA ACTAACTATG ATGGCCTGAC TCCCCTTCAC TGTGCAGTCA TAGCCCACAA 540

TGCTGTGGTC CATGAACTCC AGAGAAATCA ACAGCCTCAT TCACCTGAAG TTCAGGAGCT 600

TTTACTGAAG AATAAGAGTC TGGTTGATAC CATTAAGTGC CTAATTCAAA TGGGAGCAGC 660

GGTGGAAGCG AAGGATCGCA AAAGTGGCCG CACAGCCCTG CATTTGGCAG CTGAAGAAGC 720

AAATCTGGAA CTCATTCGCC TCTTTTTGGA GCTGCCCAGT TGCCTGTCTT TTGTGAATGC 780

AAAGGCTTAC AATGGCAACA CTGCCCTCCA TGTTGCTGCC AGCTTGCAGT ATCGGTTGAC 840

ACAATTAGAT GCTGTCCGCC TGTTGATGAG GAAGGGAGCA GACCCAAGTA CTCGGAACTT 900

GGAGAACGAA CAGCCAGTGC ATTTGGTTCC CGATGGCCCT GTGGGAGAAC AGATCCGACG 960

TATCCTGAAG GGAAAGTCCA TTCAGCAGAG AGCTCCACCG TATTAGCTCC ATTAGCTTGG 1020

AGCCTGGCTA GCAACACTCA CTGTCAGTTA GGCAGTCCTG ATGTATCTGT ACATAGACCA 1080

TTTGCCTTAT ATTGGCAAAT GTAAGTTGTT TCTATGAAAC AAACATATTT AGTTCACTAT 1140

TATATAGTGG GTTATATTAA AAGAAAAGAA RAAAAATATC TAATTWCTCT TGGCAGATTT 1200

GCATATTTCA TACCCAGGTA TCTGGATCTA GACATCTGAA TTTGATCTCA ATGGTAACAT 1260

TGCCTTCAAT TAACAGTAGC TTTTGAGTAG GAAAGGACTT TGATTTGTGG CACAAAACAT 1320

TATTAATATA GCTATTGACA GTTTCAAAGC AGGTAAATTG TAAATGTTTC TTTAAGAAAA 1380

AGCATGTGAA AGGAAAAAGG TAAATACAGC ATTGAGGCTT CATTTGGCCT TAGTCCCTGG 1440

GAGTTACTGG CGTTGGACAG GCTTCAGTCA TTGGACTAGA TGAAAGGTGT CCATGGTTAG 1500

AATTTGATCT TTGCAAACTG TATATAATTG TTATTTTTGT CCTTAAAAAT ATTGTACATA 1560

CTTGGTTGTT AACATGGTCA TATTTGAAAT GTATAAGTCC ATAAAATAGA AAAGAACAAG 1620

TGAATTGTTG CTATTTAAAA AAATTTTACA ATTCTTACTA AGGAGTTTTT ATTGTGTAAT 1680

CACTAAGTCT TTGTAGATAA AGCAGATGGG GAGTTACGGA GTTGTTCCTT TACTGGCTGA 1740

AAGATATATT CGAATTGTAA AGATGCTTTT YCTCATGCAT TGAAATTATA CATTATTTGT 1800

AGGGAATTGC ATG 1813

(2) INFORMATION FOR SEQ ID NO: 51:

(i) SEQUENCE CHARACTERISTICS:
(A) LENGTH: 2070 base pairs

(B) TYPE: nucleic acid
(C) STRANDEDNESS: double
(D) TOPOLOGY: linear

(xi) SEQUENCE DESCRIPTION: SEQ ID NO: 51:

CCACGCGTCC GGAAGAGCGC GGCACTTCCG CTGGCCGCTG GCTCGCTGGC CGCTCCTGGA 60

GGCGGCGGCG GGAGCGCAGG GGGCGCGCGG CCCGGGGACT CGCATTCCCC GGTTCCCCCT 120

CCACCCCACG CGGCCTGGAC CATGGACGCC AGATGGTGGG CAGTGGTGGT GCTGGCTGCG 180

TTCCCCTCCC TAGGGGCAGG TGGGGAGACT CCCGAAGCCC CTCCGGAGTC ATGGACCCAG 240

CTATGGTTCT TCCGATTTGT GGTGAATGCT GCTGGCTATG CCAGCTTTAT GGTACCAGGC 300

TACCTCCTGG TGCAGTACTT CAGGCGGAAG AACTACCTGG AGACCGGTAG GGGCCTCTGC 360

TTTCCCCTGG TGAAAGCTTG TGTGTTTGGC AATGAGCCCA AGGCCTCTGA TGAGGTTCCC 420

CTGGCGCCCC GAACAGAGGC GGCAGAGACC ACCCCGATGT GGCAGGCCCT GAAGCTGCTC 480

TTCTGTGCCA CAGGGCTCCA GGTGTCTTAT CTGACTTGGG GTGTGCTGCA GGAAAGAGTG 540

ATGACCCGCA GCTATGGGGC CACAGCCACA TCACCGGGTG AGCGCTTTAC GGACTCGCAG 600

TTCCTGGTGC TAATGAACCG AGTGCTGGCA CTGATTGTGG CTGGCCTCTC CTGTGTTCTC 660

TGCAAGCAGC CCCGGCATGG GGCACCCATG TACCGGTACT CCTTTTGCCA GCCTGTCCAA 720

TGTGCTTAGC AGCTGGTGCC AATACGAAGC TCTTAAGTTC GTCAGCTTCC CCACCCAGGT 780

GCTGGCCAAG GCCTCTAAGG TGATCCCTGT CATGCTGATG GGAAAGCTTG TGTCTCGGCG 840

CAGTAACGAA CACTGGGAGT ACCTGACAGC CACCCTCATC TCCATTGGGG TCAGCATGTT 900

TCTGCTATCC AGCGGACCAG AGCCCCGCAG CTCCCCAGCC ACCACACTCT CAGGCCTCAT 960

CTTACTGGCA GGTTATATTG CTTTTGAACA GCTTCACCTC AAACTGGCAG GATGCCCTGT 1020

TTGCCTATAA GATGTCATCG GTGCAGATGA TGTTTGGGGG TCAATTTCTT CTCCTGCCTC 1080

TTCACAGTGG GCTCACTGCT AGAAACAGGG GGCCCTACTG GAGGGAACCC GCTTCATGGG 1140

GCGACACAGT GAGTTTGCTG CCCATGCCCT GCTACTCTCC ATCTGCTCCG CATGTGGCCA 1200

GCTCTTCATC TTTTACACCA TTGGGCAGTT TGGGGCTGCC GTCTTCACCA TCATCATGAC 1260

CCTCCGCCAG GCCTTTGCCA TCCTTCTTTC CTGCCTTCTC TATGGCCACA CTGTCACTGT 1320

GGTGGGAGGG CTGGGGGTGG CTGTGGTCTT TGCTGCCCTC CTGCTCAGAG TCTACGCGCG 1380

GGGCCGTCTA AAGCAACGGG GAAAGAAGGC TGTGCCTGTT GAGTCTCCTG TGCAGAAGGT 1440

TTGAGGGTGG AAAGGGCCTG AGGGGTGAAG TGAAATAGGA CCCTCCCACC ATCCCCTTCT 1500

GCTGTAACCT CTGAGGGAGC TGGCTGAAAG GGCAAAATGC AGGTGTTTTC TCAGTATCAC 1560

AGACCAGCTC TGCAGCAGGG GATTGGGGAG CCCAGGAGGC AGCCTTCCCT TTTGCCTTAA 1620

GTCACCCATC TTCCAGTAAG CAGTTTATTC TGAGCCCCGG GGGTAGACAG TCCTCAGTGA 1680

GGGGTTTTGG GGAGTTTGGG GTCAAGAGAG CATAGGTAGG TTCCACAGTT ACTCTTCCCA 1740

CAAGTTCCCT TAAGTCTTGC CCTAGCTGTG CTCTGCCACC TTCCAGACTC ACTCCCCTCT 1800

GCAAATACCT GCATTTCTTA CCCTGGTGAG AAAAGCACAA GCGGTGTAGG CTCCAATGCT 1860

GCTTTCCCAG GAGGGTGAAG ATGGTGCTGT GCTGAGGAAA GGGGATGCAG AGCCCTGCCC 1920

AGCACCACCA CCTCCTATGC TCCTGGATCC CTAGGCTCTG TTCCATGAGC CTGTTGCAGG 1980

TTTTGGTACT TTAGAAATGT AACTTTTTGC TCTTATAATT TTATTTTATT AAATTAAATT 2040

ACTGCAAAAA AAAAAAAAAA AAAAAAAAAA 2070

(2) INFORMATION FOR SEQ ID NO: 52:

(i) SEQUENCE CHARACTERISTICS:
(A) LENGTH: 1426 base pairs
(B) TYPE: nucleic acid
(C) STRANDEDNESS: double
(D) TOPOLOGY: linear

(xi) SEQUENCE DESCRIPTION: SEQ ID NO: 52:

CCCTCACTAA AGGGAACAAA AGCTGGAGCT CCACCGCGGT GGCGGCCGCT CTAGAACTAG 60

TGGATCCCCC GGGCTGCAGG AATTCGGCAC ACGGATCGGC GTCCGCAGCG GGCGGCTGCT 120

GAGCTGCCTT GAGGTGCAGT GTTGGGGATC CAGAGCCATG TCGGACCTGC TACTACTGGG 180

CCTGATTGGG GGCCTGACTC TCTTACTGCT GCTGACGCTG CTGGCCTTTG CCGGGTACTC 240

AGGGCTACTG GCTGGGGTGG AAGTGAGTGC TGGGTCACCC CCCATCCGCA ACGTCACTGT 300

GGCCTACAAG TTCCACATGG GGCTCTATGG TGAGACTGGG CGGCTTTTCA CTGAGAGCTG 360

CAGCATCTCT CCCAAGCTCC GCTCCATCGC TGTCTACTAT GACAACCCCC ACATGGTGCC 420

CCCTGATAAG TGCCGATGTG CCGTGGGCAG CATCCTGAGT GAAGGTGAGG AATCGCCCTC 480

CCCTGAGCTC ATCGACCTCT ACCAGAAATT TGGCTTCAAG GTGTTCTCCT TCCCGGAACC 540

CAGCCATGTG GTGACAGCCA CCTTTCCCCT AACACCACCA TTCTGTCCCA TCTGGCTGGG 600

CTACCCGCCG TGTCCATCCT GCCTTGGACA CCTACATCAA GGAGCGGAAG CTGTGTGCCT 660

ATCCTCGGCT GGSGATCTAC CAGGAAGACC AGAATCCATT TCATGTGCCC ACTGGCACGG 720

CCAGGGAGAC TTCTATGTGC CTGAGATGAA GGAGACAGAG TGGAAATGGC GGGGGCTTGT 780

GGAGGCCATT GACACCCAGG TGGATGGCAC AGGAGCTGAC ACAATGAGTG ACACGAGTTC 840

TGTAAGCTTG GAAGTGAGCC CTGGCAGCCG GGAGACTTCA GCTGCCACAC TGTCACCTGG 900

GGCGAGCAGC CGTGGCTGGG ATGACGGTGA CACCCGCAGC GAGCACAGCT AACAGCGAGT 960

CAGGTGCCAG CGGCTCCTCT TTTGAGGAGC TGGACTTTGG AGGGCGAGGG GCCCTTAAGG 1020

GGAGTCACGG CTGGACCCTG GGACTTGAGC CCCTGGGGGA CTACCAAGTG GCTCTGGGAG 1080

CCCACTGCCC CTGAGAAGGG CAAGGAGTAA CCCATGGCCT GCACCCTCCT GCAGTGCAGT 1140

TGCTGAGGAA CTGAGCAGAC TCTCCAGCAG ACTCTCCAGC CCTCTTCCTC CTTCCTCTGG 1200

GGGAHGAGGG GTTCCTGAGG GACCTGACTT CCCCTGCTCC AGGCCTCTTG CTAAGCCTTC 1260

TCCTCACTGC CCTTTAGGCT CCCAGGGCCA GAGGAGCCAG GGACTATTTT CTGCACCAGC 1320

CCCCAGGGCT GCCGCCCCTG TTGTGTCTTT TTTTCAGACT CACAGTGGAG CTTCCAGGAC 1380

CCAGAATAAA GCCAATGATT TACTTGTTAA AAAAAAAAAA AAAAAA 1426

(2) INFORMATION FOR SEQ ID NO: 53:

(i) SEQUENCE CHARACTERISTICS:
(A) LENGTH: 1720 base pairs
(B) TYPE: nucleic acid
(C) STRANDEDNESS: double
(D) TOPOLOGY: linear

(xi) SEQUENCE DESCRIPTION: SEQ ID NO: 53:

GGCACGAGTG CGGCCCCAGC CTCTCCTCAC GCTCGCGCAG TCTCCGCCGC AGTCTCAGCT 60

GCAGCTGCAG GACTGAGCCG TGCACCCGGA GGAGACCCCC GGAGGAGGCG ACAAACTTCG 120

CAGTGCCGCG ACCCAACCCC AGCCCTGGGT AGCCTGCAGC ATGGCCCAGC TGTTCCTGCC 180

CCTGCTGGCA GCCCTGGTCC TGGCCCAGGC TCCTGCAGCT TTAGCAGATG TTCTGGAAGG 240

AGACAGCTCA GAGGACCGCG CTTTTCGCGT GCGCATCGCG GGCGACGCGC CACTGCAGGG 300

CGTGCTCGGC GGCGCCCTCA CCATCCCTTG CCACGTCCAC TACCTGCGGC CACCGCCGAG 360

CCGCCGGGCT GTGCTGGGCT CTCCGCGGGT CAAGTGGACT TTCCTGTCCC GGGGCCGGGA 420

GGCAGAAGTG CTGGTGGCGC GGGGAGTGCG CGTCAAGGTG AACGAGGCCT ACCGGTTCCG 480

CGTGGCACTG CCTGCGTACC CAGCGTCGCT CACCGACGTC TCCCCTGGCG CTGAGCGAGC 540

TGCGCCCCAA CGACTCAGGT ATCTATCGCT GTGAGGTCCA GCACGGCATC GATGACAGCA 600

GCGACGCTGT GGAGGTCAAG GTCAAAGGTA TCCCATCCAG ACCCCACGAG AGGCCTGTTA 660

CGGAGACATG GATGGCTTCC CCGGGGTCCG GAACTATGGT GTGGTGGACC CGGATGACCT 720

CTATGATGTG TACTGTTATG CTGAAGACCT AAATGGAGAA CTGTTCCTGG GTGACCCTCC 780

AGAGAAGCTG ACATTGGAGG AAGCACGGGC GTACTGCCAG GAGCGGGGTG CAGAGATTGC 840

CACCACGGGC CAACTGTATG CAGCCTGGGA TGGTGGCCTG GACCACTGCA GCCCAGGGTG 900

GCTAGCTGAT GGCAGTGTGC GCTACCCCAT CGTCACACCC AGCCAGCGCT GTGGTGGGGG 960

CTTGCCTGGT GTCAAGACTC TCTTCCTCTT CCCCAACCAG ACTGGCTTCC CCAATAAGCA 1020

CAGCCGCTTC AACGTCTACT GCTTCCGAGA CTCGGCCCAG CTTCTGCCAT CCCTGAGGCC 1080

TCCAACCCAG CCTCCAACCC AGCTTTGATG GACTAGAGGC TATCGTCACA GTGACAGAGA 1140

CCCTGGAGGA ACTGCAGCTG CCTCAGGAAG CCACAGAGAG TGAATCCCGT GGGGCCATCT 1200

ACTCCATCCC CATCATGGAG GACGGAGGAG GTGGAAGCTC CACTCCAGAA GACCCAGCAG 1260

AGGCCCCTAG GACGCTCCTA GAATTTGAAA CACAATCCAT GGTACCGCCC ACGGGGTTCT 1320

CAGAAGAGGA AGGTAAGGCA TTGGAGGAAG AAGAGAAATA TGAAGATGAA GAAGAGAAAG 1380

AGGAGGAAGA AGAAGAGGAG GAGGTGGAGG ATGAGGCTCT GTGGGCATGG CCCAGCGAGC 1440

TCAGCAGCCC GGGCCCTGAG GCCTCTCTCC CCACTGAGCC AGCAGCCCAG GAGGAGTCAC 1500

TCTCCCAGGC GCCAGCAAGG GCAGTCCTGC AGCCTGGTGC ATCACCACTT CCTGATGGAG 1560

AGTCAGAAGC TTCCAGGCCT CCAAGGGTCC ATGGACCACC TACTGAGACT CTGCCCACTC 1620

CCAGGGAGAG GAACCTAGCA TCCCCATCAC CTTCCACTCT GGTTGAGGCA AGAGAGGTGG 1680

GGGAGGCAAC TGGTGGTCCT GAGCTATCTG GGTCCCTCGA 1720

(2) INFORMATION FOR SEQ ID NO: 54:

(i) SEQUENCE CHARACTERISTICS:
(A) LENGTH: 1117 base pairs
(B) TYPE: nucleic acid
(C) STRANDEDNESS: double
(D) TOPOLOGY: linear

(xi) SEQUENCE DESCRIPTION: SEQ ID NO: 54:

GGCACGAGGC CAAACTTCGG GCGGCTGAGG CGGCGGCCGA GGAGCGGCGG ACTCCGGGCG 60

CGGGGAGTCG AGGCATTTGC GCCTGGGCTT CGGAGCGTAC CCAGGGCCTG AGCCTTTGAA 120

GCAGGAGGAG GGGAGGAGAG AGTGGGGCTC CTCTATCGGG ACCCCCTCCC CATGTGGATC 180

TGCCCAGGCG GCGGCGGCGG AGGAGGCGAC CGAGAAGATG CCCGCCCTGC GCCCCGCTCT 240

GCTGTGGGCG CTGCTGGCGC TCTGGCTGTG CTGCGCGACC CCCGCGCATG CATTGCAGTG 300

TCGAGATGGC TATGAACCCT GTGTAAATGA AGGAATGTGT GTTACCTACC ACAATGGCAC 360

AGGATACTGC AAAGGTCCAG AAGGCTTCTT GGGGGAATAT TGTCAACATC GAGACCCCTG 420

TGAGAAGAAC CGCTGCCAGA ATGGTGGGAC TTGTGTGGCC CAGGCCATGC TGGGGAAAGC 480

CACGTGCCGA TGTGCCTCAG GGTTTACAGG AGAGGACTGC CAGTACTCGA CATCTCATCC 540

ATGCTTTGTG TCTCGACCTT GCCTGAATGG CGGCACATGC CATATGCTCA GCCGGGATAC 600

CTATGAGTGC ACCTGTCAAG TCGGGTTTAC AGGTAAGGAG TGCCAATGGA CCGATGCCTG 660

CCTGTCTCAT CCCTGTGCAA ATGGAAGTAC CTGTACCACT GTGGCCAACC ATTTCCTGCA 720

AATGCCTCAC AGGCTTCACA GGGCAGAAGT GTGAGACTGA TGTCAATGAG TGTGACATTC 780

CAGGACACTG CCAGCATGGT GGCACCTGCC TCAACCTGCC TGGTTCCTAC CAGTGCCAGT 840

GCCTTCAGGG CTTCACAGGC CAGTACTGTG ACAGCCTGTA TGTGCCCTGT GCACCCTCGC 900

CTTGTGTCAA TGGAGGCACC TGTCGGCAGA CTGGTGACTT CACTTTTGAG TGCAACTGCC 960

TTCCAGAAAC AGTGAGAAGA GGAACAGAGC TCTGGGAAAG AGACAGGGAA GTCTGGAATG 1020

GAAAAGAACA CGATGAGAAT TAGACACTGG AAAATATGTA TGTGTGGTTA ATAAAGTGCT 1080

TTAAACTGAA AAAAAAAAAA AAAAAAAAAA AAAAAAA 1117

(2) INFORMATION FOR SEQ ID NO: 55:

(i) SEQUENCE CHARACTERISTICS:
(A) LENGTH: 1903 base pairs
(B) TYPE: nucleic acid
(C) STRANDEDNESS: double
(D) TOPOLOGY: linear

(xi) SEQUENCE DESCRIPTION: SEQ ID NO: 55:

GGCACGAGCT CGGAGAGGCG GCGCCCCTGA GTAGGCCAGG AGCCTCTCTT GCAACTTCTG 60

CCACCGCGGG CCACCGCGGC CGCCTGATCC CGCAGAGGAA GGTCGCGGCC GTGGAGCGAT 120

GACCCGCGGC GGTCCGGGCG GGCGCCCGGG GCTGCCACAG CCGCCGCCGC TTCTGCTGCT 180

GCTGCTGCTG CCGCTGTTGT TAGTCACCGC GGAGCCGCCG AAACCTGCAG GAGTCTACTA 240

TGCAACTGCA TACTGGATGC CTGCTGAAAA GACAGTACAA GTCAAAAATG TAATGGACAA 300

GAATGGGGAC GCCTATGGCT TTTACAATAA CTCTGTGAAA ACCACAGGCT GGGGCATCCT 360

GGAGATCAGA GCTGGCTATG GCTCTCAAAC CCTGAGCAAT GAGATCATCA TGTTTGTGGC 420

TGGCTTTTTG GAGGGTTACC TCATTGCCCC ACACATGAAT GACCACTACA CAAACCTCTA 480

CCCACAGCTG ATCACGAAAC CTTCCATCAT GGATAAAGTG CAGGATTTTA TGGAGAAGCA 540

AGATAAGGTG GACCCGGAAA AATATCAAAG AATACAAGAC TGATTCATTT TGGAGACATA 600

CAGGCTATGT GATGGCACAA ATAGATGGCC TCTATGTAGG AGCAAAGAAG AGGGCTATAT 660

TAGAAGGGAC AAAGCCAATG ACCCTGTTCC AGATTCAGTT CCTGAATAGT GTTGGAGATC 720

TATTGGATCT GATTCCCTCA CTCTCTCCCA CAAAAAACGG CAGCCTAAAG GTTTTTAAGA 780

GATGGGACAT GGGACATTGC TCCGCTCTTA TCAAGGTTCT TCCTGGATTT GAGAACATCC 840

TTTTTGCTCA CTCAAGCTGG TACACGTATG CAGCCATGCT CAGGATATAT AAACACTGGG 900

ACTTCAACAT CATAGATAAA GATACCAGCA GTAGTCGCCT CTCTTTCAGC AGTTACCCAG 960

GGTTTTTGGA GTCTCTGGAT GATTTTTACA TTCTTAGCAG TGGATTGATA TTGCTGCAGA 1020

CCACAAACAG TGTGTTTAAT AAAACCCTGC TAAAGCAGGT AATACCCGAG ACTCTCCTGT 1080

CCTGGCAAAG AGTCCGTGTG GCCAATATGA TGGCAGATAG TGGCAAGAGG TGGGCAGACA 1140

TCTTTTCAAA ATACAACTCT GGCACCTATA ACAATCAATA CATGGTTCTG GACCTGAAGA 1200

AAGTAAAGCT GAACCACAGT CTTGACAAAG GCACTCTGTA CATTGTGGAG CAAATTCCTA 1260

CATATGTAGA ATATTCTGAA CAAACTGATG TTCTACGGAA AGGATATTGG CCCTCCTACA 1320

ATGTTCCTTT CCATGAAAAA ATCTACAACT GGAGTGGCTA TCCACTGTTA GTTCAGAAGC 1380

TGGGCTTGGA CTACTCTTAT GATTTAGCTC CACGAGCCAA AATTTTCCGG CGTGACCAAG 1440

GGAAAGTGAC TGATACGGCA TCCATGAAAT ATATCATGCG ATACAACAAT TATAAGAAGG 1500

ATCCTTACAG TAGAGGTGAC CCCTGTAATA CCATCTGCTG CCGTGAGGAC CCTGAACTCA 1560

CCTAACCCAA GTCCTTGGAG GTTGTTATGA CACAAAAGGT GGCAGATATY TACCTAGCAT 1620

CTCAGTACAC ATCCTATGCC ATAAGTGGTC CCACAGTACA AGGTGGCCTC CCTGTTTTTC 1680

GCTGGGACCG TTTCAACAAA ACTCTACATC AGGGCATGCC AGAGGTCTAC AACTTTGATT 1740

TTATTACCAT GAAACCAATT TTGAAACTTG ATATAAAATG AAGGAGGGAG ATGACGGACT 1800

AGAAGACTGT AAATAAGATA CCAAAGGCAC TATTTTAGCT ATGTTTTTCC CATCAGAATT 1860

ATGCAATAAA ATATATTAAT TTGTCAAAAA AAAAAAAAAA AAA 1903

(2) INFORMATION FOR SEQ ID NO: 56:

(i) SEQUENCE CHARACTERISTICS:
(A) LENGTH: 1869 base pairs
(B) TYPE: nucleic acid
(C) STRANDEDNESS: double
(D) TOPOLOGY: linear

(xi) SEQUENCE DESCRIPTION: SEQ ID NO: 56:

ACAGCTTTTC GGGGCCCGAG TCGCACCCAG CGAAGAGAGC GGGCCCGGGA CAAGCTCGAA 60

CTCCGGCCGC CTCGCCCTTC CCCGGCTCCG CTCCCTCTGC CCCCTCGGGG TCGCGCGCCC 120

ACGATGCTGC AGGGCCCTGG CTCGCTGCTG CTGCTCTTCC TCGCCTCGCA CTGCTGCCTG 180

GGCTCGGCGC GCGGGCTCTT CCTCTTTGGC CAGCCCGACT TCTCCTACAA GCGCANCAAT 240

TGCAAGCCCA TCCCGGTCAA CCTGCAGCTG TGCCACGGCA TCGAATACCA GAACATGCGG 300

CTGCCCAACC TGCTGGGCCA CGAGACCATG AAGGAGGTGC TGGAGCAGGC CGGCGCTTGG 360

ATCCCGCTGG TCATGAAGCA GTGCCACCCG GACACCAAGA AGTTCCTGTG CTCGCTCTTC 420

GCCCCCGTCT GCCTCGATGA CCTAGACGAG ACCATCCAGC CATGCCACTC GCTCTGCGTG 480

CAGGTGAAGG ACCGCTGCGC CCCGGTCATG TCCGCCTTCG GYTTCCCCTG GCCCGACATG 540

CTTGAGTGCG ACCGTTTCCC CCAGGACAAC GACCTTTGCA TCCCCCTCGC TAGCAGCGAC 600

CACCTCCTGC CAGCCACCGA GGAAGCTCCA AAGGTATGTG AAGCCTGCAA AAATAAAAAT 660

GATGATGACA ACGACATAAT GGAAACGCTT TGTAAAAATG ATTTTGCACT GAAAATAAAA 720

GTGAAGGAGA TAACCTACAT CAACCGAGAT ACCAAAATCA TCCTGGAGAC CAAGAGCAAG 780

ACCATTTACA AGCTGAACGG TGTGTCCGAA AGGGACCTGA AGAAATCGGT GCTGTGGCTC 840

AAAGACAGCT TGCAGTGCAC CTGTGAGGAG ATGAACGACA TCAACGCGCC CTATCTGGTC 900

ATGGGACAGA AACAGGGTGG GGAGCTGGTG ATCACCTCGG TGAAGCGGTG GCAGAAGGGG 960

CAGAGAGAGT TCAAGCGCAT CTCCCGCAGC ATCCGCAAGC TGCAGTGCTA GTCCCGGCAT 1020

CCTGATGGCT CCGACAGGCC TGCTCCAGAG CACGGCTGAC CATTTCTGCT CCGGGATCTC 1080

AGCTCCCGTT CCCCAAGCAC ACTCCTAGCT GCTCCAGTCT CAGCCTGGGC AGCTTCCCCC 1140

TGCCTTTTGC ACGTTTGCAT CCCCAGCATT TCCTGAGTTA TAAGGCCACA GGAGTGGATA 1200

GCTGTTTTCA CCTAAAGGAA AAGCCCACCC GAATCTTGTA GAAATATTCA AACTAATAAA 1260

ATCATGAATA TTTTTATGAA GTTTAAAAAT AGCTCACTTT AAAGCTAGTT TTGAATAGGT 1320

GCAACTGTGA CTTGGGTCTG GTTGGTTGTT GTTTGTTGTT TTGAGTCAGC TGATTTTCAC 1380

TTCCCACTGA GGTTGTCATA ACATGCAAAT TGCTTCAATT TTCTCTGTGG CCCAAACTTG 1440

TGGGTCACAA ACCCTGTTGA GATAAAGCTG GCTGTTATCT CAACATCTTC ATCAGCTCCA 1500

GACTGAGACT CAGTGTCTAA GTCTTACAAC AATTCATCAT TTTATACCTT CAATGGGAAC 1560

TTAAACTGTT ACATGTATCA CATTCCAGCT ACAATACTTC CATTTATTAG AAGCACATTA 1620

ACCATTTCTA TAGCATGATT TCTTCAAGTA AAAGGCAAAA GATATAAATT TTATAATTGA 1680

CTTGAGTACT TTAAGCCTTG TTTAAAACAT TTCTTACTTA ACTTTTGCAA ATTAAACCCA 1740

TTGTAGCTTA CCTGTAATAT ACATAGTAGT TTACCTTTAA AAGTTGTAAA AATATTGCTT 1800

TAACCAACAC TGTAAATATT TCAGATAAAC ATTATATTCT TGTATATAAA CTTTACATCC 1860

TGTTTTACC 1869

(2) INFORMATION FOR SEQ ID NO: 57:

(i) SEQUENCE CHARACTERISTICS:
(A) LENGTH: 1259 base pairs

(B) TYPE: nucleic acid
(C) STRANDEDNESS: double
(D) TOPOLOGY: linear

(xi) SEQUENCE DESCRIPTION: SEQ ID NO: 57:

ACCGTGGTCG TGGGCGGACG GCGGCTGCAG CGYGGAGGAG CTGGGGTCGC TGTGGGTCGC 60

GAACAGAGCC CGGGACGTGC GCGCTTGGTG CACGATCCTG AAGGGGAGCT CCGAGGGGCC 120

CGGGTCKCCA GGGCTGCTGC GGCCATTCCC GGAGCCCGGC GCGGGGCCCG NRAGATACTG 180

GTTTAGGCCG TCCCAGGGCT CCGGGCGCAC CCGKTGGCCG CTGCTGCAGC GGAGGGAGCG 240

CGGCGGCGSG NGGGCTCGGA GACAGCGTTT CTCCCGGAAT CTTCCTCGGG CAGCARGTGG 300

GAAGTGGGAG CCGGAGCGGC ACTGGCARCG TTCTCTCCGC ANGTCGGCAC CATGCGCCCT 360

GCAGCCCTGC GCGGGGCCCT GCTGGGCTGC CTCTGCCTGG CGTTGCTTTG CCTGGGCGGT 420

GCGGACAAGC GCCTGCGTGA CAACCATGAG TGGAAAAAAC TAATTATGGT TCAGCACTGG 480

CCTGAGACAG TATGCGAGAA AATTCAAAAC GACTGTAGAG ACCCTCCGGA TTACTGGACA 540

ATACATGGAC TATGGCCCGA TAAAAGTGAA GGATGTAATA GATCGTGGCC CTTCAATTTA 600

GAAGAGATTA AGGATCTTTT GCCAGAAATG AGGGCATACT GGCCTGACGT AATTCACTCG 660

TTTCCCAATC GCAGCCGCTT CTGGAAGCAT GAGTGGGAAA AGCATGGGAC CTGCGCCGCC 720

CAGGTGGATG CGCTCAACTC CCAGAAGAAG TACTTTGGCA GAAGCCTGGA ACTCTACAGG 780

GAGCTGGACC TCAACAGTGT GCTTCTAAAA TTGGGGATAA AACCATCCAT CAATTACTAC 840

CAAGTTGCAG ATTTTAAAGA TGCCCTTGCC AGAGTATATG GAGTGATACC CAAAATCCAG 900

TGCCTTCCAC CAAGCCAGGA TGAGGAAGTA CAGACAATTG GTCAGATAGA ACTGTGCCTC 960

ACTAAGCAAG ACCAGCAGCT GCAAAACTGC ACCGAGCCGG GGGAGCAGCC GTCCCCCAAG 1020

CAGGAAGTCT GGCTGGCAAA TGGGGCCGCC GAGAGCCGGG GTCTGAGAGT CTGTGAAGAT 1080

GGCCCAGTCT TCTATCCCCC ACCTAAAAAG ACCAAGCATT GATGCCCAAG TTTTGGAAAT 1140

ATTCTGTTTT AAAAAGCAAG AGAAATTCAC AAACTGCAGC TTTCTNAAAA AAAAANAAAA 1200

AAAAATTGGG GGGTTTTTTT GGGGSGCCCG GGGCCCTTGG TTTTTCCCCC CGGGGGGGT 1259

(2) INFORMATION FOR SEQ ID NO: 58:

(i) SEQUENCE CHARACTERISTICS:
(A) LENGTH: 1186 base pairs
(B) TYPE: nucleic acid
(C) STRANDEDNESS: double
(D) TOPOLOGY: linear

(xi) SEQUENCE DESCRIPTION: SEQ ID NO: 58:

CGGCATGGAG AATGGCTCCG CTTCTGTTGC AGCTGGCGGT GCTCGGCGCG GCGCTGGCGG 60

CCGCAGCCCT CGTACTGATT TCCATCGTTG CATTTACAAC TGCTACAAAA ATGCCAGCAC 120

TCCATCGACA TGAAGAAGAG AAATTCTTCT TAAATGCCAA AGGCCAGAAA GAAACTTTAC 180

CCAGCATATG GGACTCACCT ACCAAACAAC TTTCTGTCGT TGTGCCTTCA TACAATGAAG 240

AAAAACGGTT GCCTGTGATG ATGGATGAAG CTCTGAGCTA TCTAGAGAAG AGACAGAAAC 300

GAGATCCTGC GTTCACTTAT GAAGTGATAG TAGTTGATGA TGGCAGTAAA GATCAGACCT 360

CAAAGGTAGC TTTTAAATAT TGCCAGAAAT ATGGAAGTGA CAAAGTACGT GTGATAACCC 420

TGGTGAAGAA TCGTGGAAAA GGTGGAGCGA TTAGAATGGG TATATTCAGT TCTCGAGGAG 480

AAAAGATCCT TATGGCAGAT GCTGATGGAG CCACAAAGTT TCCAGATGTT GAGAAATTAG 540

AAAAGGGGCT AAATGATCTA CAGCCTTGGC CTAATCAAAT GGCTATAGCA TGTGGATCTC 600

GAGCTCATTT AGAAAAAGAA TCAATTGCTC AGCGTTCTTA CTTCCGTACT CTTCTCATGT 660

ATGGGTTCCA CTTTCTGGTG TGGTTCCTTT GTGTCAAAGG AATCAGGGAC ACACAGTGTG 720

GGTTCAAATT ATTTACTCGA GAAGCAGCTT CACGGACGTT TTCATCTCTA CACGTTGAAC 780

GATGGGCATT TGATGTAGAA CTACTGTACA TAGCACAGTT CTTTAAAATT CCAATAGCAG 840

AAATTGCTGT CAACTGGACA GAAATTGAAG GTTCTAAATT AGTTCCATTC TGGAGCTGGC 900

TACAAATGGG TAAAGACCTA CTTTTTATAC GACTTCGATA TTTGACTGGT GCCTGGAGGC 960

TTGAGCAAAC TCGGAAAATG AATTAGGTTG TTTGCAGTCT TCAGTTGTGT TCTTATGCTT 1020

CAGTGTCACA TTTCATTTCA TTTGAAACTA AAATTTTAAG TAAAGCTGAA ATAAACTTCT 1080

TGTCATTGTC TGCCTTTTGA TAATTTTAAA GAAATAACTT TCCATAAGTA AAAAATTATA 1140

TATCTCTTTG GATATAAATG ATTTTTAAAA GATGTTTATT TAAAAA 1186

(2) INFORMATION FOR SEQ ID NO: 59:

(i) SEQUENCE CHARACTERISTICS:
(A) LENGTH: 428 base pairs
(B) TYPE: nucleic acid
(C) STRANDEDNESS: double
(D) TOPOLOGY: linear

(xi) SEQUENCE DESCRIPTION: SEQ ID NO: 59:

GATCCCCCGG CTGCAGGATT CGGCACGAGT ACTGATTCTT CACTGAGCTT KGTTAGTATA 60

AGCAGAGTTC CAAGTCTCCC CTAGGGTTGT CTCTACATTT CTTTATCATT CCAGTGGGTA 120

RGGTTTAGCT GGGGGAAGGA CATTTCATAA GGGTTAGTTG GACTGAGCAG TATGGACATT 180

TGCTTTTTTC ATTACGTACT GTTGTTTTTC CTTGTTAGGT GTGCTTTGGT GGTTTTAATA 240

TTATTGTGCC AGGGATGGGG AAATGGGGGG GGTTGTGTGG GAAGAGTACT TATTATTGTG 300

TTTTCTTCAG TGTAATTGTT CTTGGTAATT GATACCTCTC TGTTTTATTT NTCTCATTCT 360

TTCAAAATAA AACTTTTTGA AATTTGAAAA AAAAAAAAAA NAAAAAACTC GGGGGGGGGC 420

CCGGTACC 428

(2) INFORMATION FOR SEQ ID NO: 60:

(i) SEQUENCE CHARACTERISTICS:
(A) LENGTH: 501 base pairs
(B) TYPE: nucleic acid
(C) STRANDEDNESS: double
(D) TOPOLOGY: linear

(xi) SEQUENCE DESCRIPTION: SEQ ID NO: 60:

GGCACGAGCT TTCAGCAGGG GACAGCCCGA TTGGGGACAA TGGCGTCTCT TGGCCACATC 60

TTGGTTTTCT GTGTGGGTCT CCTCACCATG GCCAAGGCAG AAAGTCCAAA GGAACACGAC 120

CCGTTCACTT ACGACTACCA GTCCCTGCAG ATCGGAGGCC TCGTCATCGC CGGGATCCTC 180

TTCATCCTGG GCATCCTCAT CGTGCTGAGC AGAAGATGCC GGTGCAAGTT CAACCAGCAG 240

CAGAGGACTG GGGAACCCGA TGAAGAGGAG GGAACTTTCC GCAGCTCCAT CCGCCGTCTG 300

TCCACCCGCA GGCGGTAGAA ACACCTGGAG CGATGGAATC CGGCCAGGAC TCCCCTGGCA 360

CCTGACATCT CCCACGCTCC AACTGCGCGC CCACCGCCCC CTCCGCCGCC CCTTCCCCAG 420

CCCTGCCCCC GCAGACTCCC CCTGCCGCCA AGACTTCCAA TAAAACGTGC GTTCCTCTCG 480

AAAAAAAAAA AAATAAAAAA A 501

(2) INFORMATION FOR SEQ ID NO: 61:

(i) SEQUENCE CHARACTERISTICS:
(A) LENGTH: 1197 base pairs
(B) TYPE: nucleic acid
(C) STRANDEDNESS: double
(D) TOPOLOGY: linear

(xi) SEQUENCE DESCRIPTION: SEQ ID NO: 61:

ACATGATGGN TACCAAAGAA TTCGGCANAG GGCGCGCAGT GCAGCAGGTG CTCAATATCG 60

AGTGCCTGCG GGACTTCCTG ACGCCCCCGC TGCTGTCCGT GCGCTTCCGG TACGTGGGCG 120

CCCCCCAGGC CCTCACCCTG AAGCTCCCAG TGACCAKCAA CAAGTTCTTC CAGCCCACCG 180

AGATGGCGGC CCAGGATTTC TTCCAGCGCT GGAAGCAGCT GAGCCTCCCT CAACAGGAGG 240

CGCAGAAAAT CTTCAAAGCC AACCACCCCA TGGACGCAGA AGTTACTAAG GCCAAGCTTC 300

TGGGGTTTGG CTCTGCTCTC CTGGACAATG TGGACCCCAA CCCTGAGAAC TTCGTGGGGG 360

CGGGGATCAT CCAGACTAAA GCCCTGCAGG TGGGCTGTCT GCTTCGGCTG GAGCCCAATG 420

CCCAGGCCCA GATGTACCGG CTGACCCTGC GCACCAGCAA GGAGCCCGTC TCCCGTCACC 480

TGTGTGAGCT GCTGGCACAG CAGTTCTGAG CCCTGGACTC TGCCCCGGGG GATGTGGCCG 540

GCACTGGGCA GCCCCTTGGA CTGAGGCAGT TTTGGTGGAT GGGGGACCTC CACTGGTGAC 600

AGAGAAGACA CCAGGGTTTG GGGGATGCCT GGGACTTTCC TCCGGCCTTT TGTATTTTTA 660

TTTTTGTTCA TCTGCTGCTG TTTACATTCT GGGGGGTTAG GGGGAGTCCC CCTCCCTCCC 720

TTTCCCCCCC AAGCACAGAG GGGAGAGGGG CCAGGGAAGT GGATGTCTCC TCCCCTCCCA 780

CCCCACCCTG TTGTAGCCCC TCCTACCCCC TCCCCATCCA GGGGCTGTGT ATTATTGTGA 840

GCGAATAAAC AGAGAGACGC TAACAGCCCC ATGTCTGTGT CCATCACCCA CTGTTAGGTA 900

GTCAAAGAAG TGGGGTGAGG GCATGCAGAG TGTGGGTGGC CAGNTTCGCA GCCCATGGGT 960

GGGACTCTGG GGAGACAGCA GCAGCAGCAG CCGCCGAAGC CCCAGCTGCA AGGCCACCAG 1020

ACGCACTCCT GTGCCTGGTT CCTYAGTCCC CAACACCAGG TAGCAAGCTY TGGGCAGCTG 1080

GGCCTGGTAG ACCTCATCTT CTGTCTTCTY TGGTGGCCCT GGCTCTGGTG GGAAGTGCGT 1140

GGAGGTGACC AGGGTATAGA AGTTTCGGAG CTGATTGGAA GAGGATTAAC TTCCCGC 1197

(2) INFORMATION FOR SEQ ID NO: 62:

(i) SEQUENCE CHARACTERISTICS:
(A) LENGTH: 595 base pairs
(B) TYPE: nucleic acid
(C) STRANDEDNESS: double
(D) TOPOLOGY: linear

(xi) SEQUENCE DESCRIPTION: SEQ ID NO: 62:

ATTNANGACK TKYAGCCTYT WATACMATCA TTATAGGGAR AAGCTGGTAC GCCTGMARGT 60

ACCGGTCYGG AATTCNCGGG TCGACCCACG CGTCCGGCAC AGCGGGAGTT GGTTCTGACA 120

CCAGATGTTC TCTGCTCCTG GTTAATGTCA GTGAGGGCTG GAAGTTGAAT AAATGAGAAC 180

AGGAGTGGTC TGGGCCCATG TAAATGATCC TCCCTTGAAA GGAGGAACAG CTTTCATCAT 240

TTGTTCCAGC TAAGCCTTGC ATGCATTATA GATCTGGTGC TAAGCAGTGG GAAAGATCTC 300

ATAAGTAATG TTTTATGTTC TTTCTGTCTC TCCTCTTCTG TWGTTCTTGG CTTGTGGGTT 360

GTGTTTGTGT GTTAACTGGA AAATTGCTAT AAGCCAGTTG TCTCTAAGTT TTAAAAACGA 420

ATTAGAAAAA CCATAAAATC TCTGGCCTAT GCACATTGTC CCTGTTTTGT GAAAACATTA 480

AAGGGTAAAT AAAAAGGAAG GAGAACAGTC AATAATGTGC ATCAAATATA TTCTGAGTTC 540

TAGAGAAATT AATGACCAAG CATTAGAACT AGAAGCAAAA AAAAAAAAAA AAAAA 595

(2) INFORMATION FOR SEQ ID NO: 63:

(i) SEQUENCE CHARACTERISTICS:
(A) LENGTH: 1478 base pairs
(B) TYPE: nucleic acid
(C) STRANDEDNESS: double
(D) TOPOLOGY: linear

(xi) SEQUENCE DESCRIPTION: SEQ ID NO: 63:

CGGCGCTGAG GACGCACGGA TGCCTTCCGT GCCTTCCATC AAGATCTCAA TTTTGTGCGC 60

AAGTTCCTAC AGCCCCTGTT GATTGGAGAG CTGGCTCCGG AAGAACCCAG CCAGGATGGA 120

CCCCTGAATG CGCATGGTCG AGGACTTCCG AGCCCTGCAC CAGGCAGCCG AGGACATGAA 180

GCTGTTTGAT GCCAGTCCCA CCTTCTTTGC TTTCCTACTG GGCCACATCC TGGCCATGGA 240

GGTGCTGGCC TGGCTCCTTA TCTACCTCCT GGGTCCTGGC TGGGTGCCCA GTGCCCTGGN 300

CCGCCTTCAT CCTGGCCATC TCTCAGGCTC AGTCCTGGTG TCTGCAGCAT GACCTGGGCC 360

ATGCTCCATC TTCAAGAAGW CCTGGTGGAA CCACGTGGCC CAGAAGTTCG TGATGGGGCA 420

GCTAAAGGGC TTCTCCGCCC ACTGGTGGAA CTTCCGCCAC TTCCAGCACC ACGCCAAGCC 480

CAACATCTTC CACAAAGACC CAGACGTGAC GGTGGCGCCC GTCTTCCTCC TGGGGGAGTC 540

ATCCGTCGAG TATGGCAAGA AGAAACGCAG ATACCTACCC TACAACCAGC AGCACCTGTA 600

CTTCTTCCTG ATCGGCCCGC CGCTGCTCAC CCTGGTGAAC TTTGAAGTGG AAAATCTGGC 660

GTACATGCTG GTGTGCATGC AGTGGGCGGA TTTGCTCTGG GCCGCCAGCT TCTATGCCCG 720

CTTCTTCTTA TCCTACCTCC CCTTCTACGG CGTCCCTGGG GTGCTGCTCT TCTTTGTTGC 780

TGTCAGGGTC CTGGAAAGCC ACTGGTTCGT GTGGATCACA CAGATGAACC ACATCCCCAA 840

GGAGATCGGC CACGAGAAGC ACCGGGACTG GGTCAGCTCT CAGCTGGCAG CCACCTGCAA 900

CGTGGAGCCC TCACTTTTCA CCAACTGGTT CAGCGGGCAC CTCAACTTCC AGATCGAGCA 960

CCACCTCTTC CCCAGGATGC CGAGACACAA CTACAGCCGG GTGGCCCCGC TGGTCAAGTC 1020

GCTGTGTGCC AAGCACGGCC TCAGCTACGA ATGAAGCCCT TCCTCACCGC GCTGGTGGAC 1080

ATCGTCAGGT CCCTGAAGAA GTCTGGTGAC ATCTGGCTGG ACGCCTACCT CCATCAGTGA 1140

AGGCAACACC CAGGCGGGCA GAGAAGGGCT CAGGGCACCA GCAACCAAGC CAGCCCCCGG 1200

CGGGATCGAT ACCCCCACCC CTCCACTGGC CAGCCTGGGG GTGCCCTGCC TGCCCTCCTG 1260

GTACTGTTGT CTTCCCCTCG GCCCCCTCAC ATGTGTATTC AGCAGCCCTA TGGCCTTGGC 1320

TCTGGGCCTG ATGGGACAGG GGTAGAGGGA AGGTGAGCAT AGCACATTTT CCTAGAGCGA 1380

GAATTGGGGG AAAGCTGTTA TTTTTATATT AAAATACATT CAGATGTAAA AAAAAAAAAA 1440

AAAAACTCGA GGGGGGGCCC CGGNAACCAA TTCGCCCT 1478

(2) INFORMATION FOR SEQ ID NO: 64:

(i) SEQUENCE CHARACTERISTICS:
(A) LENGTH: 2033 base pairs
(B) TYPE: nucleic acid
(C) STRANDEDNESS: double
(D) TOPOLOGY: linear

(xi) SEQUENCE DESCRIPTION: SEQ ID NO: 64:

GGCACGAGGA AGAACGCAAA GCTGAGAACA TGGACGTTAA TATCGCCCCA CTCCGCGCCT 60

GGGACGATTT CTTCCCGGGT TCCGATCGCT TTGCCCGGCC GGACTTCAGG GACATTTCCA 120

AATGGAACAA CCGCGTAGTG AGCAACCTGC TCTATTACCA GACCAACTAC CTGGTGGTGG 180

CTGCCATGAT GATTTCCATT GTGGGGTTTC TGAGTCCCTT CAACATGATC CTGGGAGGAA 240

TCGTGGTGGT GCTGGTGTTC ACAGGGTTTG TGTGGGCAGC CCACAATAAA GACGTCCTTC 300

GCCGGATGAA GAAGCGCTAC CCCACGACGT TCGTTATGGT GGTCATGTTG GCGAGCTATT 360

TCCTTATCTC CATGTTTGGA GGAGTCATGG TCTTTGTGTT TGGCATTACT TTTCCTTTGC 420

TGTTGATGTT TATCCATGCA TCGTTGAGAC TTCGGAACCT CAAGAACAAA CTGGAGAATA 480

AAATGGAAGG AATAGGTTTG AAGAGGACAC CGATGGGCAT TGTCCTGGAT GCCCTAGAAC 540

AGCAGGAAGA AGGCATCAAC AGACTCACTG ACTATATCAG CAAAGTGAAG GAATAAACAT 600

AACTTACCTG AGCTAGGGTT GCAGCAGAAA TTGAGTTGCA GCTTGCCCTT GTCCAGACCT 660

ATGTTCTGCT TGCGTTTTTG AAACAGGAGG TGCACGTACC ACCCAATTAT CTATGGCAGC 720

ATGCATGTAT AGGCCGAACT ATTATCAGCT CTGATGTTTC AGAGAGAAGA CCTCAGAAAC 780

CGAAAGAAAA CCACCACCCT CCTATTGTGT CTGAAGTTTC ACGTGTGTTT ATGAAATCTA 840

ATGGGAAATG GATCACACGA TTTCTTTAAG GGAATTAAAA AAAATAAAAG AATTACGGCT 900

TTTACAGCAA CAATACGATT ATCTTATAGG AAAAAAAAAT CATTGTAAAG TATCAAGACA 960

ATACGAGTAA ATGAAAAGGC TGTTAAAGTA GATGACATCA TGTGTTAGCC TGTTCCTAAT 1020

CCCCTAGAAT TGTAATGTGT GGGATATAAA TTAGTTTTTA TTATTCTCTT AAAAATCAAA 1080

GATGATCTCT ATCACTTTGC CACCTGTTTG ATGTGCAGTG GAAACTGGTT AAGCCAGTTG 1140

TTCATACTTC CTTTACAAAT ATAAAGATAG CTGTTTAGGA TATTTTGTTA CATTTTTGTA 1200

AATTTTTGAA ATGCTAGTAA TGTGTTTTCA CCAGCAAGTA TTTGTTGCAA ACTTAATGTC 1260

ATTTTCCTTA AGATGGTTAC AGCTATGTAA CCTGTATTAT TCTGGACGGA CTTATTAAAA 1320

TACAAACAGA CAAAAAATAA AACAAAACTT GAGTTCTATT TACCTTGCAC ATTTTTTGTT 1380

GTTACAGTGA AAAAAATGGT CCAAGAAAAT GTTTGCCATT TTTGCATTGT TTCGTTTTTA 1440

ACTGGAACAT TTAGAAAGAA GGAAATGAAT GTGCATTTTA TTAATTCCTT AGGGGCACAA 1500

GGAGGACAAT AATAGCTGAT CTTTTGAAAT TTGAAAAACG TCTTTAGATG ACCAAGCAAA 1560

AAGCTTTAAA AAATGGTAAT GAAAATGGAA TGCAGCTACT GCAGCTAATA AAAAATTTTA 1620

GATAGCAATT GTTACAACCA TATGCCTTTA TAGCTAGACA TTAGAATTAT GATAGCATGA 1680

GTTTATACAT TCTATTATTT TTCCTCCCTT TCTCATGTTT TTATAAATAG GTAATAAAAA 1740

ATGTTTTGCC TGCCAATTGA ATGATTTCGT AGCTGAAGTA GAAACATTTA GGTTTCTGTA 1800

GCATTAAATT GTGAAGACAA CTGGAGTGGT ACTTACTGAA GAAACTCTCT GTATGTCCTA 1860

GAATAAGAAG CAATGATGTG CTGCTTCTGA TTTTTCTTGC ATTTTAAATT CTCAGCCAAC 1920

CTACAGCCAT GATCTTTAGC ACAGTGATAT CACCATGACT TCACAGACAT GGTCTAGAAT 1980

CTGTACCCTT ACCCACATAT GAAGAATAAA ATTGATTAAA GGTTAAAAAA AAA 2033

(2) INFORMATION FOR SEQ ID NO: 65:

(i) SEQUENCE CHARACTERISTICS:
(A) LENGTH: 440 base pairs
(B) TYPE: nucleic acid
(C) STRANDEDNESS: double
(D) TOPOLOGY: linear

(xi) SEQUENCE DESCRIPTION: SEQ ID NO: 65:

ATGTTTCTTA CTAGAATACT GTGTCCAACC TATATAGCCC TAACTTTCCT GGTTTACATT 60

GTGGCCCTAG TATCTGGGCA GCTGTGCATG GAGATAGCCA GAGGAAACAT TTTTTTTCTT 120

AATGAATTGG TGACCACATT TTGTTGTTCT TGCCTCCTAT TATCCGTGCC CTATTTGCAT 180

CCTGGTTTCT TCTACAGTAG TTTATGTAAA TGTTGTTTTG TCCTTGTCGT TCTCAGTAGA 240

ATTGGTTCTG TAAACGAAAC CTGGTCCTGT AATTTCAGTA TATGCTCATA TCTCATCTTT 300

GGCTCTCCCA TTTTCACAGC AGTGATCCCT AAAAGATGTG CCCTAGAGGA TATCCAGAAC 360

AATCCAATTG GATGTCTTCT CCGCTGCACT CCAGCCTGGG AGACAGAGGG AGACTCNATC 420

TCAAAAAAAA TTAAAAAAAA 440

(2) INFORMATION FOR SEQ ID NO: 66:

(i) SEQUENCE CHARACTERISTICS:
(A) LENGTH: 3301 base pairs
(B) TYPE: nucleic acid
(C) STRANDEDNESS: double
(D) TOPOLOGY: linear

(xi) SEQUENCE DESCRIPTION: SEQ ID NO: 66:

GGTCATAAGG GGAGGGTTGN NGTGTGTCCC TCCAGGTTGT GCAGAGGGGA TTAGAAGTAA 60

GTAGGTTAGA GGGGAGGTGG AGGGAGTGTG CTGGGGTGTG AGCTTTTATG ATGCTGAAAG 120

GATCATGATA TGCTAAGGAC AGGATAGTGT TGGGTTGTAC ACACAGGTGT AGGCAATCCT 180

GGTGGCTAGT ATGTAAAAGT GAATGTCCTG ACTCCCTTAG AGGGTACCTG NCAGAGTGCC 240

CTTGGARGGA CTAGTGCTGG AGAAATTAAT AGGAGAGGGG ACGGGCATCC ATTAACCTTT 300

TCTTGCCTGC AGCCTGTAGG GTCCAGCGTC AAAGCGAATC ATGGGGTCCA GGGCTGAGCT 360

GTGCACTCTC TTAGGCGGAT TCTCCTTCCT CCTGCTACTG ATACCAGGCG AGGGGGCCAA 420

GGGTGGATCC CTCAGAGAGA GTCAGGGAGT CTGCTCCAAG CAGACACTGG TGGTCCCGCT 480

CCACTACAAC GAGTCCTACA GCCAACCAGT GTACAAGCCC TACCTGACCT TGTGCGCTGG 540

GAGCGCATCT GCAGCACTTA CAGGACCATG TACCGCGTTA TGTGGCGGGA GGTGAGGCGG 600

GAGGTTCAGC AGACCCATGC AGTGTGCTGC CAGGGCTGGA AGAAGCGGCA CCCGGGGGCG 660

CTCACCTGTG AAGCCATCTG CGCCAAGCCT TGCCTGAACG GAGGCGTCTG CGTTAGGCCT 720

GACCAGTGCG AGTGCGCCCC CGGCTGGGGA GGGAAGCACT GTCATGTGGA CGTGGATGAA 780

TGTAGGACCA GCATCACCCT CTGCTCGCAC CATTGTTTTA ATACGGCARG CAGCTTCAMC 840

TGCGGCTGCC CCATGACCTA GTGCTAGGCG TGGACGGGCG CACCTGCATG GAGGGGTCCC 900

CAGAGCCCCC AACCAGTGCC AGCATACTCA GCGTGGCCST TCGGGARGCG GAAAAAGATG 960

ACGCGCTCTG AAGCAGGAGA TTCACGAGCT GCGAGGCCCT TGAAGCGGCT GGAGCAGTGG 1020

NCCGGTCAGC TGGGCCCTGG NTCAGACGGT GCTGCCCGTG CCGCCTGAAG WGCTGCAGCC 1080

AGAACAGGTG GCTGAGCTGT GGGGCCGGGG TGACCGGATC GAATCTCTCA GCGACCAGGT 1140

GCTGCTGCTG GAGGAGAGGC TAGGTGCCTG CTCCTGTGAG GACAACAGCC TGGGCCTCGG 1200

CGTCAATCAT CGATAAGAAG CCTCTACAGC ACCCCTGCCC CCTAATTTAT ACAGAAACCG 1260

GACCCACTAA TCCTCTGGGA TTGGCCGACT GTGAGCTGCA GATAAGGCTA TCAGCCACCA 1320

AAGAGCAATG AACAATGGAA ACTTCAGAGA GCTGAAGAAA GGGGGAGGCC TGTGTTCTTG 1380

GCCTGCCCCT GAGTCTTCTG GCTGGGGGCA GGTTGCCTGG GCAAGAACTG CTTCTTCAAT 1440

TCCTTAACAA ATGCAACCAC CAACACCCAG ATCTCTCTCT CTCTTTATTT TCAGTTTTTT 1500

TGCTGTTATC CAGATAATTA ATAAAAACCA ACCACGCAAA ACTGGGTCCC ACCCTCTCCT 1560

TTTGCTCCCA GCCTACCTCC CCAGTTGTGG GAACAGGTCT GGAGTGAGAG GCAGGGAGTG 1620

GCTAATGCCN CCAGGAAGAA ATGAAAACTG GCTCAGAGAG GGGGAAGCCT CAACAGAAAA 1680

AGAAATAAAT TAAAAGCCCT CCTATCCCCT CCAGCCAGGG TTCGTTCCTT TCCCCAACTC 1740

CCCAGGGGGC AGAAGTGAGT GCAGCACCTG ATGTCTGCTT CTTCCCCTTG TGTCTGGTGA 1800

GATGGTGCAG CAGGGCTGCA GGGGGCTGGG TGGGGTCATG TCCACTGAAG AACTGTACTA 1860

TGGGGACAGA AAACCAGAAA TGTGGAGACT GAACTGGTAT CCCAGAGAGT GCACGACCCT 1920

GGGCATCTGG GCAAGGGCAG GCATGAGACC TCTGAATTAG AAGGGTCCAG CCCCCACTGA 1980

CAGGAGGCTA CACTGGGAGG GAAGGTGAAG GTGCTGAGGA AAGCTCCCAT GATGAGCCTG 2040

GGAGTGCTTC AGGTATCAGC TTCCAGCCAG AGGGCGAGAA GTCCTCCTCA CAAATGGATG 2100

AGTCCATTGA ATCCATGGAC TTTGGAGTGG GGGGGATTTG TTCCAAAGAA TGGATGAGTC 2160

CACTGGCCAA TGTGGGGTAG AGGGGTAGAG AAGACCACAT AGGAAGAGAC TCCACTGGGG 2220

ATGGAATGTT CCCCTCCCTT GTGTAGGCTG AGTCACTGGA GATGAGGGGG AGGCAACTGT 2280

CCCACAGACA ARACAGTAGG AGGTGGGGGT CAAGAGTGGA GACTGCACCG AGGCAAGAGT 2340

CCATGGATGG GGCCAAGAGG GGGCAGGAGT GGCGCTGTAT CCACATTTCA CTTCAGAAGT 2400

TGAAGATTCC AAAGAGGAGA ATAAGTGGGG AGAGGGGAGA CAAGGAAGAG GGTTTKGCCC 2460

TGCTTCAGGG CCCACTGGGT GGGTAGGTGT GGGGAGGAAG ATGGGGACAG ATGGGAGGAG 2520

AGCTCAGAGC CAGGGTTCAC CCACCGCCCC CAGGCTTCTT CAGATAGTCA CCACCACCCC 2580

GGCCATCAGT GGAGATTTCC CGGAAAACAG TGAAGCATGG AGTGCCGGAC TCTGTCAGCC 2640

AGAGCTGGGA CGTCATCTGG TGTCAGCCCT TCCGTGGGCA CTGGGGGCAG CACCCGCACC 2700

TGACATTGTC CCGAGGTGAA GCGACGCTCC TTCTTGCAGT AGAAGTCTTG GTAGGAGGAC 2760

ATGACTATGG GGACAATGGG AACCTGGGCC TGCACTGCAA GATGGAAGGC GCCACGTTTG 2820

AAGGGCAGCA TGGAGCCATT GTGGTTTCTC GTTCCCTCAG GAAACACCCA GACCYTCACG 2880

TCCTGGGTGA GCAGGGTCTG GGCGACCTCA GACATGACAC TGATGGCATC CCCCGTGCGC 2940

TTCCGGTCGA TGAAGATGAC TCCTGCCAGC CAGCAGGCCA GCCCGCAGAG CCAGCCCACA 3000

GTANTCGCGC TTGGCAATGG GCACACAGCG GCCTGGCAGT ACCTCCATCA TCCCAAGCAG 3060

ATCGAGAGAG CTCTGGTGGT TGGAGACAAC AACATAGGGC TGCGAGGGAG GGAAGTGGTG 3120

AGCCCCTCGC ACCTCCACTC GGATCCCGTA CAGGTATTTG ATGTGGAGCA GCATTAGACG 3180

CAAGATCTTC ATGTTCTCGA CGTTGCGTCC TCGCACGGCA CACACAGGGA TGGCGAGCAC 3240

AGCCAGGAAG AGGATCCAGC CATTGTAGAA GGCCATCTTG AAGAAGTACT TGGCACTGGG 3300

G 3301

(2) INFORMATION FOR SEQ ID NO: 67:

(i) SEQUENCE CHARACTERISTICS:
(A) LENGTH: 1535 base pairs
(B) TYPE: nucleic acid
(C) STRANDEDNESS: double
(D) TOPOLOGY: linear

(xi) SEQUENCE DESCRIPTION: SEQ ID NO: 67:

GGCACGAGGT CAAGCGAAAG GATTTCAAGG AACAGATCAT CCACCATGTG TTCACCATCA 60

TTCTCATCAG CTTTTCCTGG TTTGCCAATT ACATCCGAGC TGGGACTCTA ATCATGGCTC 120

TGCATGGACT CTTCCGATTA CCTGCTGGAG TCAGCCAAGA TGTTTAACTA CGCGGGATGG 180

AAGAACACCT GCAACAACAT CTTCATCGTC TTCGCCATTG TTTTTATCAT CACCCGACTG 240

GTCATCCTGC CCTTCTGGAT CCTGCATTGC ACCCTGGTGT ACCCACTGGA GCTCTATCCT 300

GCCTTCTTTG GCTATTACTT CTTCAATTCC ATGATGGGAG TTCTACAGCT GCTGCATATC 360

TTCTGGGCCT ACCTCATTTT GCGCATGGCC CACAAGTTCA TAACTGGAAA GCTGGTAGAA 420

GATGAACGCA GTACCGGGAA GAAACAGAGA GCTCAGAGGG GGAGGAGGCT GCAGCTGGGG 480

GAGGAGCAAA GAGCCGGCCC CTAGCCAATG GCCACCCCAT CCTCAATAAC AACCATCGTA 540

AGAATGACTG AACCATTATT CCAGCTGCCT CCCAGATTAA TGCATAAAGC CAAGGAACTA 600

CCCCGCTCCC TGCGCTATAG GGTCACTTTA AGCTCTGGGG AAAAAGGAGA AAGTGAGAGG 660

AGAGTTCTCT GCATCCTCCC TCCTTGCTTG TCACCCAGTT GCCTTTAAAC CAAATTCTAA 720

CCAGCCTATC CCCAGGTAGG GGGACGTTGG TTATATTCTG TTAGAGGGGG ACGGTCGTAT 780

TTTCCTCCCT ACCCGCCAAG TCATCCTTTC TACTGCTTTT GAGGCCCTCC CTCAGCTCTC 840

TGTGGGTAGG GGTTACAATT CACATTCCTT ATTCTGAGAA TTTGGCCCCA GCTGTTTGCC 900

TTTGACTCCC TGACCTCCAG AGCCAGGGTT GTGCCTTATT GTCCCATCTG TGGGCCTCAT 960

TCTGCCAAAG CTGGACCAAG GCTAACCTTT CTAAGCTCCC TAACTTGGGC CAGAAACCAA 1020

AGCTGAGCTT TTAACTTTCT CCCTCTATGA CACAAATGAA TTGAGGGTAG GAGGAGGGTG 1080

CACATAACCC TTACCCTACC TCTGCCAAAA AGTGGGGGCT GTACTGGGGA CTGCTCGGAT 1140

GATCTTTCTT AGTGCTACTT CTTTCAGCTG TCCCTGTAGC GACAGGTCTA AGATCTGACT 1200

GCCTCCTCCT TTCTCTGGCC TCTTCCCCCT TCCCTCTTCT CTTCAGCTAG GCTAGCTGGT 1260

TTGGAGTAGA ATGGCAACTA ATTCTAATTT TTATTTATTA AATATTTGGG GTTTTGGTTT 1320

TAAAGCCAGA ATTACGGCTA GCACCTAGCA TTTCAGCAGA GGGACCATTT TAGACCAAAA 1380

TGTACTGTTA ATGGGTTTTT TTTTAAAATT AAAAGATTAA ATAAAAAATA TTAAATAAAA 1440

CATGGCAATA AGTGTCAGAC TATTAGGAAT TGAGAAGGGG GATCAACTAA ATAAACGAAG 1500

AGAGTCTTTC TTATGCAAAA AAAAAAAAAA AAAAA 1535

(2) INFORMATION FOR SEQ ID NO: 68:

(i) SEQUENCE CHARACTERISTICS:
(A) LENGTH: 1244 base pairs
(B) TYPE: nucleic acid
(C) STRANDEDNESS: double
(D) TOPOLOGY: linear

(xi) SEQUENCE DESCRIPTION: SEQ ID NO: 68:

GGGCACCCAC CAGCGGCGCC GACCTCAGCG CGCACCTATG GGCTCGCTAC CAGGACATGC 60

GGAGACTGGT GCACGACCTC CTGCCCCCCG AGGTCTGCAG TCTCCTGAAC CCAGCAGCCA 120

TCTACGCCAA CAACGAGATC AGCCTGCGTG ACGTTGAGGT CTACGGCTTT GACTACGACT 180

ACACCCTGGC CCAGTATGCA GACGCACTGC ACCCCGAGAT CTTCAGTACC GCCCGTGACA 240

TCCTGATCGA GCACTACAAG TACCCAGAAG GGATTCGGAA GTATGACTAC AACCCCAGCT 300

TTGCCATCCG TGGCCTCCAC TATGACATTC AGAAGAGCCT TCTGATGAAG ATTGACGCCT 360

TCCACTACGT GCAGCTGGGG ACAGCCTACA GGGGCCTCCA GCCTGTGCCA GACGAGGAGG 420

TGATTGAGCT GTATGGGGGT ACCCAGCACA TCCCACTATA CCAGATGAGT GGCTTCTATG 480

GCAAGGGTCC CTCCATTAAG CAGTTCATGG ACATCTTCTC GCTACCGGAG ATGGCTCTGC 540

TGTCCTGTGT GGTGGACTAC TTTCTGGGCC ACAGCCTGGA GTTTGACCAA GCACATCTCT 600

ACAAGGACGT GACGGACGCC ATCCGAGACG TGCATGTGAA GGGCCTCATG TACCAGTGGA 660

TCGAGCAGGA CATGGAGAAG TACATCCTGA GAGGGGATGA GACGTTTGCT GTCCTGAGCC 720

GCCTGGTGGC CCATGGGAAA CAGCTGTTCC TCATCACCAA CAGTCCTTTC AGCTTCGTAG 780

ACAAGGGGAT GCGGCACATG GTGGGTCCCG ATTGGCGCCA CTCTTCGATG TGGTCATTGT 840

CCAGGCAGAC AAGCCCAGCT TCTTCACTGA CCGGCGCAAG CTTTNCAGAA AACTCGATGA 900

GAAGGGCTCA CTTCAGTGGG ACCGGATCAC CCGCTTGGAA AAGGGCAAGA TCTATCGGCA 960

GGGAAACCTG TTTGACTTCT TACGCTTGAC GGAATGGCGT GGCCCCCGCG TGCTCTACTT 1020

CGGGGACCAC CTCTATAGTG ATCTGGCGGA TCTCATGCTG CGGCACGGCT GGCGCACAGG 1080

CGCCATCATC CCCGAGCTGG AGCGTGAGAT CCGCATCATC AACACGGAGC AGTACATGCA 1140

CTCGCTKACG TGGCAGCAGG CGCTCACGGG GCTKCTKGAG CGCATKCAGA CCTATCAGGA 1200

CGCGGAGTTG AGGCAGGTCT TGCTTCCTTG ATGAAAGANC GNNT 1244

(2) INFORMATION FOR SEQ ID NO: 69:

(i) SEQUENCE CHARACTERISTICS:
(A) LENGTH: 1292 base pairs
(B) TYPE: nucleic acid
(C) STRANDEDNESS: double
(D) TOPOLOGY: linear

(xi) SEQUENCE DESCRIPTION: SEQ ID NO: 69:

GGCACGAGCA GCGACGCGAC TCTGGTGCGG GCCGTCTTCT TCCCCCCGAG CTGGGCGTGC 60

GCGGCCGCAA TGAACTGGGA GCTGCTGCTG TGGCTGCTGG TGCTGTGCGC GCTGCTCCTG 120

CTCTTGGTGC AGCTGCTGCG CTTCCTGAGG GCTGACGGCG ACCTGACGCT ACTATGGGCC 180

GAGTGGCAGG GACGACGCCC AGAATGGGAG CTGACTGATA TGGTGGTGTG GGTGACTGGA 240

GCCTCGAGTG GAATTGGTGA GGAGCTGGCT TACCAGTTGT CTAAACTAGG AGTTTCTCTT 300

GTGCTGTCAG CCAGAAGAGT GCATGAGCTG GAAAGGGTGA AAAGAAGATG CCTAGAGAAT 360

GGCAATTTAA AAGAAAAAGA TATACTTGTT TTGCCCCTTG ACCTGACCGA CACTGGTTCC 420

CATGAAGCGG CTACCAAAGC TGTTCTCCAG GAGTTTGGTA GAATCGACAT TCTGGTCAAC 480

AATGGTGGAA TGTCCCAGCG TTCTCTGTGC ATGGATACCA GCTTGGATGT CTACAGAAAG 540

CTAATAGAGC TTAACTACTT AGGGACGGTG TCCTTGACAA AATGTGTTCT GCCTCACATG 600

ATCGAGAGGA AGCAAGGAAA GATTGTTACT GTGAATAGCA TCCTGGGTAT CATATCTGTA 660

CCTCTTTCCA TTGGATACTG TGCTAGCAAG CATGCTCTCC GGGGTTTTTT TAATGGCCTT 720

CGAACAGAAC TTGCCACATA CCCAGGTATA ATAGTTTCTA ACATTTGCCC AGGACCTGTG 780

CAATCAAATA TTGTGGAGAA TTCCCTAGCT GGAGAAGTCA CAAAGACTAT AGGCAATAAT 840

GGAGACCAGT CCCACAAGAT GACAACCAGT CGTTGTGTGC GGCTGATGTT AATCAGCATG 900

GCCAATGATT TGAAAGAAGT TTGGATCTCA GAACAACCTT TCTTGTTTAG TAACATATTT 960

GTGGCAATAC ATGCCAACCT GGGCCTGGTG GATAACCAAC AAGATGGGGA AGAAAAGGAT 1020

TGAGAACTTT AAGAGTGGTG TGGATGCAGA CTCTTCTTAT TTTAAAATCT TTAAGACAAA 1080

ACATGACTGA AAAGAGCACC TGTACTTTTC AAGCCACTGG AGGGAGAAAT GGAAAACATG 1140

AAAACAGCAA TCTTCTTATG CTTCTGAATA ATCAAAGACT AATTTGTGAT TTTACTTTTT 1200

AATAGATATG ACTTTGCTTC CAACATGGAA TGAAATAAAA AATAAATAAT AAAAGATTGC 1260

CATGAATCTT GCAAAAAAAA AAAAAAAAAA AA                                    1292

(2) INFORMATION FOR SEQ ID NO: 70:

(i) SEQUENCE CHARACTERISTICS:
(A) LENGTH: 1031 base pairs
(B) TYPE: nucleic acid
(C) STRANDEDNESS: double
(D) TOPOLOGY: linear

(xi) SEQUENCE DESCRIPTION: SEQ ID NO: 70:

GGGCTGTTGC TTTTGAACAG AACCCTATAT TACTCTCCTG GGATCTGAGT TTCTGCAGGT      60

CATTTGTATG TAGGACCAGG AGTATCTCCT CAGGTGACCA GTTTTGGGGA CCCGTATGTG     120

GCAAATTCTA AGCTGCCATA TTGAACATCA TCCCACTGGG AGTGGTTATG TTGTATCCCC     180

ATCTTGGCTG GCTTCAGTTT TTGCTGTAGC CCTAGAGCAC TTTGTTTGTG GGAGGCTGGC     240

CTCTTGCCTA CCTCCTTGCA TGGACAGGGG GATGAATATT TACTTTCCCA CCTCCTTGCT     300

TTTTCTTTCA CTGATACCAC TGAATGGAAC TGGTGCTGTG ACTCCTGCTG CTGGGGATTT     360

ATGTCCCGAG ACCTTAGCCT GGCTGAGTGG AGCCTGAGAC CTGCACAACA GCTCATGGTC     420

ATGCATGARA GAGAAGTGGC TGGCCACAGC AGAGGGAACA GTAACAGCCC AGGGGCCTTT     480

ATTTTGGGAA AGGCTGTCCG GGGCTGTTAC TGTCTCTTCT GGTTATAAAG CAGACATGTG     540

GCCATCTTTT CCGCAGGTTA GAGTGGGCTC CTTTCTTTTT GGAATCCTTT TCTTCTCCTT     600

TGGTAGCAGC TCCCTGCCTC CAGGGCTTCC GCCACCAGCG TCTCTGCTGT GTTGCGCAGT     660

GCAGTGGGGT GCAAGGGCTT TGTTTCTGCC TGCCTGAAAG AGAGGGCTCT GGGGATGGAG     720

ATGAGAAACA ACACGCTCTC CTTCAGACAA TGAGGCATTC TGTCCTCCTG CTGCCATTCT     780

TCATCTCCAC TGAGAGCCAG AGCTGGTAGG AGCCGAGTGC CACAGGCATT CTGCATTGCT     840

CTACTCTTAG GTTTGTGTGT GTGATCCTTC CCCTCCCTGT CGCCCACTCC TCCCTCCTCT     900

GGCTATCCTA CCCTGTCTGT GGGCTCTTTT ACTACCAGCC TATGCTGTGG GACTGTCATG     960

GCATTTAGTT CAGAGTGGAN GGGCTTTGGS CTGAAATAAA ATGCAAGTAT TTAAAAAAAA    1020

AAAAAAAAAA A                                                          1031

(2) INFORMATION FOR SEQ ID NO: 71:

(i) SEQUENCE CHARACTERISTICS:
(A) LENGTH: 855 base pairs
(B) TYPE: nucleic acid
(C) STRANDEDNESS: double

(D) TOPOLOGY: linear

(xi) SEQUENCE DESCRIPTION: SEQ ID NO: 71:

AGCTATTGAC ACTTCCTGGT GGGATCCGAG TGAGGCGACG GGGTAGGGGT TGGCGCTCAG 60

GCGGCGACCA TGGCGTATCA CGGCCTCACT GTGCCTCTCA TTGTGATGAG CGTGTTCTGG 120

GGCTTCGTCG GCTTCTTGGT GCCTTGGTTC ATCCCTAAGG GTCCTAACCG GGGAGTTATC 180

ATTACCATGT TGGTGACCTG TTCAGTTTGC TGCTATCTCT TTTGGCTGAT TGCAATTCTG 240

GCCCAACTCA ACCCTCTCTT TGGACCGCAA TTGAAAAATG AAACCATCTG GTATCTGAAG 300

TATCATTGGC CTTGAGGAAG AAGACATGCT CTACAGTGCT CAGTCTTTGA GGTCACGAGA 360

AGAGAATGCC TTCTAGATGC AAAATCACCT CCAAACCAGA CCACTTTTCT TGACTTGCCT 420

GTTTTGGCCA TTAGCTGCCT TAAACGTTAA CAGCACATTT GAATGCCTTA TTCTACAATG 480

CAGCGTGTTT TCCTTTGCCT TTTTTGCACT TTGGTGAATT ACGTGCCTCC ATAACCTGAA 540

CTGTGCCGAC TCCACAAAAC GATTATGTAC TCTTCTGAGA TAGAAGATGC TGTTCTTCTG 600

AGAGATACGT TACTCTCTCC TTGGAATCTG TGGATTTGAA GATGGCTCCT GCCTTCTCAC 660

GTGGGAATCA GTGAAGTGTT TAGAAACTGC TGCAAGACAA ACAAGACTCC AGTGGGGTGG 720

TCAGTAGGAG AGCACGTTCA GAGGGAAGAG CCATCTCAAC AGAATCGCAC CAAACTATAC 780

TTTCAGGATG AATTTCTTCT TTCTGCCATC TTTTGGAATA AATATTTTCC TCCTTTCTAW 840

RRAAAAAAAA ANANN 855

(2) INFORMATION FOR SEQ ID NO: 72:

(i) SEQUENCE CHARACTERISTICS:
(A) LENGTH: 1274 base pairs
(B) TYPE: nucleic acid
(C) STRANDEDNESS: double
(D) TOPOLOGY: linear

(xi) SEQUENCE DESCRIPTION: SEQ ID NO: 72:

GGCAGAGCTT AGAGTGTGGA AAAGGCAACC AGGTTGGCCG TAAGTGCCTG CTGGAATGCG 60

TGTGCCTCCA CACGGGTCTG GGCATCCGGA CTGATAACCA GCCGGCCAGA CTGAGGGATG 120

GAAGGCACTG AGATGGGGGC CCGTCCAGGC GGACACCCGC AGAAATGGAG CTTTCTGTGG 180

TCTCTTGCAC TCTGGCTGCC TCTTGCCCTC TCTGTGTCTC TCTTTCTTGG TCTCTCCCTC 240

TCTCCTCCTC AGCCTGGTCT TTCTCTTTGG TGCACACTTA GTTATTGTTG TGAGCAATGG 300

AAGTTCAAAG GAACTCCCTC TCCAGCTCTT CTGAATCTTG GGACACAGCC TAAAAAGGAC 360

AAAAAGTTAG AAGACAGCAT AGCAACTCAG CTCAGGGAGC TACCAGAGAA AAATAGCAAC 420

TGATGTGGGT GCTTTTTTTT TTTTTTTAAT TTGAATAAAA AGAATTAGAA GTGATGTCCT 480

TTTATAAAAT GCCTTCTCCC CCTTCCCGCC TACAGTCTCT TCCTCTCCCC TTAGAGGGGG 540

GAAAGTGTAT AAACCTACAG GGTTGTGAGT CTGAAAAGAG GATCCCCCTC ACCCCCACCC 600

TGGGCAGAGC AGTGGGGGTT GGGGGGTGGG AGAGGGGGAC ACAGATCCTG GCACACTGTG 660

GATATTTCTT GCAGATTGCA GTCTCTTGTG GCCCAAACAG GTTAGGTAGA CTATCGCCTC 720

TGGCAGGTGC CACCTTTTGG TACCAACATG TTCTGAGGTG TTAGGATTTG GGTTGGGTTT 780

TTTTTGTTTG TTTTTTTTTT CCTTTTGGTC TTTTTTTTTT TCTCCTTTTA AAGAAAAGCT 840

AAAGGCCGCT GTGAGTCCTG GTGGCAGGCT CTCCATGGAT GTAGCATATC GAAGATAATT 900

TTTATACTGC ATTTTTATGG ATTATTTTGT AATGTGTGAT TCCGTCTGCT GAGGAGGTGG 960

GAGGGGCTCC AGGGAAAGCC ACCCACCTTC AGTGAGGTTG CTCCCCAGCT GAGCGCACCG 1020

GGCATGGGAT GTGGAGGCTG GCGACACACC CTGTGCCTCT CCAAGGCTGG GCGCGTGGGG 1080

CGTCCAGAGT CTCTCTGGGT CTCAGATGTC CATCTGCCAC CTCTTGTTAA GGCTCTAGCC 1140

AGAAGGGAGG GTGAGGGTAG AAGAAAGTTA TTCCCGAAGA AAAAAAGAAT GAAAAGTCAT 1200

TGTACTGAAC TGTTTTTATA TTTTTAAAAG TTACTATTWA AAGGTAAAAA AAAGGGGGGG 1260

CCCGGTACCC AATT 1274

(2) INFORMATION FOR SEQ ID NO: 73:

(i) SEQUENCE CHARACTERISTICS:
(A) LENGTH: 688 base pairs
(B) TYPE: nucleic acid
(C) STRANDEDNESS: double
(D) TOPOLOGY: linear

(xi) SEQUENCE DESCRIPTION: SEQ ID NO: 73:

GGCACGAGTG GAGGCAATGC CAGCTCCAGG ACAGAGGCTC AGGTGCCCAA CGGGCAAGGC 60

AGCCCAGGGG GCTGTGTCTG TTCAAGTCAG GCTTCCCCGG CCCTCGCGCA CAGCGCTTCC 120

ACGGGCAGCC CGGGGCCCCA CCCCACGCAC TGAAGAGGCC GCCTGGGCTG CCATGGCCCT 180

GACCTTCCTG CTGGTGCTGC TCACCCTGGC CACGTCTGCA CACGGCTGCA CAGAAACTTC 240

CGACGCGGGG AGAGCATCTA CTGGGGGCCC ACAGCGGACA GCCAGGACAC AGTGGCTGCT 300

GTGCTGAAGC GGAGGCTGCT GCAGCCCTCG CGCCGGGTCA AGCGCTCGCG CCGGAGACCC 360

CTCTCCCGCC CACGCCGGAC AGCGGCCCGG AAGGCGAGAG CTCGGAGTGA CGGCCTGGGA 420

CCTGCCACTG TGGCGTGCGG CTCCTCCCCG CGCCGCGAGG CCGCGACCTC TGCCACGTGG 480

ACCGCGCGCG GGGCGCTCCC TGGTGGCGAT GGCGCGGCAC TGGCCGAGCA CTGCGGGGGC 540

TTTCCTCCTT GTTGGTTGCT GAGTGGGCGG CCAAGGGGAG AAAAGGAGCC GCTTCTGCCT 600

CCCTTGCCAA AACTCCGTTT CTAATTAAAT TATTTTTAGT AGAAAAAAAA AAAAAAAAAA 660

AAAAAAAAAA AAAAAAAAAA AAAAAAAA 688

(2) INFORMATION FOR SEQ ID NO: 74:

(i) SEQUENCE CHARACTERISTICS:
(A) LENGTH: 1890 base pairs
(B) TYPE: nucleic acid
(C) STRANDEDNESS: double
(D) TOPOLOGY: linear

(xi) SEQUENCE DESCRIPTION: SEQ ID NO: 74:

GAGCAGGAGA GAAGGCACCG CCCCACCCCG CCTCCAAAGC TAACCCTCGG GCTTGAGGGG 60

AAGAGGCTGA CTGTACGTTC CTTCTACTCT GGCACCACTC TCCAGGCTGC CATGGGGCCC 120

AGCACCCCTC TCCTCATCTT GTTCCTTTTG TCATGGTCGG GACCCCTCCA AGGACAGCAG 180

CACCACCTTG TGGAGTACAT GGAACGCCGA CTAGCTGCTT TAGAGGAACG GCTGGCCCAG 240

TGCCAGGACC AGAGTAGTCG GCATGCTGCT GAGCTGCGGG ACTTCAAGAA CAAGATGCTG 300

CCACTGCTGG AGGTGGCAGA GAAGGAGCGG GAGGCACTCA GAACTGAGGC CGACACCATC 360

TCCGGGAGAG TGGATCGTCT GGAGCGGGAG GTAGACTATC TGGAGACCCA GAACCCAGCT 420

CTGCCCTGTG TAGAGTTTGA TGAGAAGGTG ACTGGAGGCC CTGGGACCAA AGGCAAGGGA 480

AGAAGGAATG AGAAGTACGA TATGGTGACA GACTGTGGCT ACACAATCTC TCAAGTGAGA 540

TCAATGAAGA TTCTGAAGCG ATTTGGTGGC CCAGCTGGTC TATGGACCAA GGATCCACTG 600

GGGCAAACAG AGAAGATCTA CGTGTTAGAT GGGACACAGA ATGACACAGC CTTTGTCTTC 660

CCAAGGCTGC GTGACTTCAC CCTTGCCATG GCTGCCCGGA AAGCTTCCCG AGTCCGGGTG 720

CCCTTCCCCT GGGTAGGCAC AGGGCAGCTG GTATATGGTG GCTTTCTTTA TTTTGCTCGG 780

AGGCCTCCTG GAAGACCTGG TGGAGGTGGT GAGATGGAGA ACACTTTGCA GCTAATCAAA 840

TTCCACCTGG CAAACCGAAC AGTGGTGGAC AGCTCAGTAT TCCCAGCAGA GGGGCTGATC 900

CCCCCCTACG GCTTGACAGC AGACACCTAC ATCGACCTGG CAGCTGATGA GGAAGGTCTT 960

TGGGCTGTCT ATGCCACCCG GGAGGATGAC AGGCACTTGT GTCTGGCCAA GTTAGATCCA 1020

CAGACACTGG ACACAGAGCA GCAGTGGGAC ACACCATGTC CCAGAGAGAA TGCTGAGGCT 1080

GCCTTTGTCA TCTGTGGGAC CCTCTATGTC GTCTATAACA CCCGTCCTGC CAGTCGGGCC 1140

CGCATCCAGT GCTCCTTTGA TGCCAGCGGA CCCTGACCCC TGAACGGGCA GCACTCCCTT 1200

ATTTTCCCCG CAGATATGGT GCCCATGCCA GCCTCCGCTA TAACCCCCGA GAACGCCAGC 1260

TCTATGCCTG GGATGATGGC TACCAGATTG TCTATAAGCT GGAGATGAGG AAGAAAGAGG 1320

AGGAGGTTTG AGGAGCTAGC CTTGTTTTTT GCATCTTTCT CACTCCCATA CATTTATATT 1380

ATATCCCCAC TAAATTTCTT GTTCCTCATT CTTCAAATGT GGGCCAGTTG TGGCTCAAAT 1440

CCTCTATATT TTTAGCCAAT GGCAATCAAA TTCTTTCAGC TCCTTTGTTT CATACGGAAC 1500

TCCAGATCCT GAGTAATCCT TTTAGAGCCC GAAGAGTCAA AACCCTCAAT GTTCCCTCCT 1560

GCTCTCCTGC CCCATGTCAA CAAATTTCAG GCTAAGGATG CCCCAGACCC AGGGCTCTAA 1620

CCTTGTATGC GGGCAGGCCC AGGGAGCAGG CAGCAGTGTT CTTCCCCTCA GAGTGACTTG 1680

GGGAGGGAGA AATAGGAGGA GACGTCCAGC TCTGTCCTCT CTTCCTCACT CCTCCCTTCA 1740

GTGTCCTGAG GAACAGGACT TTCTCCACAT TGTTTTGTAT TGCAACATTT TGCATTAAAA 1800

GGAAAATCCA CTGCAAAAAA AAAAAAAAAA AAAAAAAAAA AAAAAAACGG CACGAGGGGG 1860

GGTCCCGTAC CCAATNGCCC TCACATGCAT 1890

(2) INFORMATION FOR SEQ ID NO: 75:

(i) SEQUENCE CHARACTERISTICS:
(A) LENGTH: 1133 base pairs
(B) TYPE: nucleic acid
(C) STRANDEDNESS: double
(D) TOPOLOGY: linear

(xi) SEQUENCE DESCRIPTION: SEQ ID NO: 75:

GCCGGTCTGA GTGCAGAGCT GCTGTCATGG CGGCCGCTCT GTGGGGCTTC TTTCCCGTCC 60

TGCTGCTGCT GCTGCTATCG GGGGATGTCC AGAGCTCGGA GGTGCCCGGG GCTGCTGCTG 120

AGGGATCGGG AGGGAGTGGG GTCGGCATAG GAGATCGCTT CAAGATTGAG GGGCGTGCAG 180

TTGTTCCAGG GGTGAAGCCT CAGGACTGGA TCTCGGCGGC CCGAGTGCTG GTAGACGGAG 240

AAGAGCACGT CGGTTTCCTT AAGACAGATG GGAGTTTTGT GGTTCATGAT ATACCTTCTG 300

GATCTTATGT AGTGGAAGTT GTATCTCCAG CTTACAGATT TGATCCCGTT CGAGTGGATA 360

TCACTTCGAA AGGAAAAATG AGAGCAAGAT ATGTGAATTA CATCAAAACA TCAGAGGTTG 420

TCAGACTGCC CTATCCTCTC CAAATGAAAT CTTCAGGTCC ACCTTCTTAC TTTATTAAAA 480

GGGAATCGTG GGGCTGGACA GACTTTCTAA TGAACCCAAT GGTTATGATG ATGGTTCTTC 540

CTTTATTGAT ATTTGTGCTT CTGCCTAAAG TGGTCAACAC AAGTGATCCT GACATGAGAC 600

GGGAAATGGA GCAGTCAATG AATATGCTGA ATTCCAACCA TGAGTTGCCT GATGTTTCTG 660

AGTTCATGAC AAGACTCTTC TCTTCAAAAT CATCTGGCAA ATCTAGCAGC GGCAGCAGTA 720

AAACAGGCAA AAGTGGGGCT GGCAAAAGGA GGTAGTCAGG CCGTCCAGAG CTGGCATTTG 780

CACAAACACG GCAACACTGG GTGGCATCCA AGTCTTGGAA AACCGTGTGA AGCAACTACT 840

ATAAACTTGA GTCATCCCGA CGTTGATCTC TTACAACTGT GTATGTTAAC TTTTTAGCAC 900

ATGTTTTGTA CTTGGTACAC GAGAAAACCC AGCTTTCATC TTTTGTCTGT ATGAGGTCAA 960

TATTGATGTC ACTGAATTAA TTACAGTGTC CTATAGAAAA TGCCATTAAT AAATTATATG 1020

AACTACTATA CATTATGTAT ATTAATTAAA ACATCTTAAT CCAGAAAAAA AAAAAAARAA 1080

AACTCGAGGG GGGGCCCGGT ACCCAATTTN CCAAATGGGA GTCGTAAAAA ATC 1133

(2) INFORMATION FOR SEQ ID NO: 76:

(i) SEQUENCE CHARACTERISTICS:
(A) LENGTH: 585 base pairs
(B) TYPE: nucleic acid
(C) STRANDEDNESS: double
(D) TOPOLOGY: linear

(xi) SEQUENCE DESCRIPTION: SEQ ID NO: 76:

ATGTTTACAA TGTTGTGTAT AAATGGGACA ACTCCTCGCC CTCTACCTGT CCCCTCCCCC 60

TTTGGTTGTA TGATTTTCTT CTTTTTTAAG AACCCCTGGA AGCAGCGCCT CCTTCAGGGT 120

TGGCTGGGAG CTCGGCCCAT CCACCTCTTG GGGTACCTGC CTCTCTCTCT CCTGTGGTGT 180

CCCTTCCCTC TCCCATGTGC TCGGTGTTCA GTGGTGTATA TTTCTTCTCC CAGACATGGG 240

GCACACGCCC CAAGGGACAT GATCCTCTCC TTAGTCTTAG CTCATGGGGC TCTTTATAAG 300

GAGTTGGGGG GTAGAGGCAG GAAATGGGAA CCGAGCTGAA GCAGAGGCTG AGTTAGGGGG 360

CTAGAGGACA GTGCTCCTGG CCACCCAGCC TCTGCTGAGA ACCATTCCTG GGATTAGAGC 420

TGCCTTTCCC AGGGAAAAAG TGTCGTCTCC CCGACCCTCC CGTGGGCCCT GTGGTGTGAT 480

GCTGTGTCTG TATATTCTAT ACAAAGGTAC TTGTCCTTTC CCTTTGTAAA CTACATTTGA 540

CATGGATTAA ACCAGTATAA ACAGTTAAAA AAAAAAAAAA AAAAA 585

(2) INFORMATION FOR SEQ ID NO: 77:

(i) SEQUENCE CHARACTERISTICS:
(A) LENGTH: 577 base pairs
(B) TYPE: nucleic acid
(C) STRANDEDNESS: double
(D) TOPOLOGY: linear

(xi) SEQUENCE DESCRIPTION: SEQ ID NO: 77:

GGCACGAGGC CTTGCAGAAC TTCTACTTGC CTGCCTCCCT GCCTCTGGCC ATGGCCTGCC 60

GGTGCCTCAG CTTCCTTCTG ATGGGGACCT TCCTGTCAGT TTCCCAGACA GTCCTGGCCC 120

AGCTGGATGC ACTGCTGGTC TTCCCAGGCC AAGTGGCTCA ACTCTCCTGC ACGCTCAGCC 180

CCCAGCACGT CACCATCAGG GACTACGGTG TGTCCTGGTA CCAGCAGCGG GCAGGCAGTG 240

CCCCTCGATA TCTCCTCTAC TACCGCTCGG AGGAGGATCA CCACCGGCCT GCTGACATCC 300

CCGATCGATT CTCGGCAGCC AAGGATGAGG CCCACAATGC CTGTGTCCTC ACCATTAGTC 360

CCGTGCAGCC TGAAGACGAC GCGGATTACT ACTGCTCTGT TGGCTACGGC TTTAGTCCCT 420

AGGGGTGGGG TGTGAGATGG GTGCCTCCCC TCTGCCTCCC ATTTCTGCCC CTGACCTTGG 480

GTCCCTTTTA AACTTTCTCT GAGCCTTGCT TCCCCTCTGT AAAATGGGTT AATAATATTC 540

AACATGTCAA CAACAAAAAA NAAAAAWAAA AACTCGA 577

(2) INFORMATION FOR SEQ ID NO: 78:

(i) SEQUENCE CHARACTERISTICS:
(A) LENGTH: 2278 base pairs
(B) TYPE: nucleic acid
(C) STRANDEDNESS: double
(D) TOPOLOGY: linear

(xi) SEQUENCE DESCRIPTION: SEQ ID NO: 78:

GTAATTCGGC ACGAGGCGCC CAACATGGCG GGTGGGCGCT GCGGCCCGCA SCTAACGGCG 60

CTCCTGGCCG CCTGGATCGC GGCTGTGGCG GCGACGGCAG GCCCCGAGGA GGCCGCGCTG 120

CCGCCGGAGC AGAGCCGGGT CCAGCCCATG ACCGCCTCCA ACTGGACGCT GGTGATGGAG 180

GGCGAGTGGA TGCTGAAATT TTACGCCCCA TGGTGTCCAT CCTGCCAGCA GACTGATTCA 240

GAATGGGAGG CTTTTGCAAA GAATGGTGAA ATACTTCAGA TCAGTGTGGG GAAGGTAGAT 300

GTCATTCAAG AACCAGGTTT GAGTGGCCGC TTCTTTGTCA CCACTCTCCC AGCATTTTTT 360

CATGCAAAGG ATGGGATATT CCGCCGTTAT CGTGGCCCAG GAATCTTCGA AGACCTGCAG 420

AATTATATCT TAGAGAAGAA ATGGCAATCA GTCGAGCCTC TGACTGGCTG GAAATCCCCG 480

GCTTCTCTAA CGATGTCTGG AATGGCTGGT CTTTTTAGCA TCTCTGGCAA GATATGGCAT 540

CTTCACAACT ATTTCACAGT GACTCTTGGA ATTCCTGCTT GGTGTTCTTA TGTCTTTTTC 600

GTCATAGCCA CCTTGGTTTT TGGCCTTTTT ATGGGTCTGG TCTTGGTGGT AATATCAGAA 660

TGTTTCTATG TGCCACTTCC AAGGCATTTA TCTGAGCGTT CTGAGCAGAA TCGGAGATCA 720

GAGGAGGCTC ATAGAGCTGA ACAGTTGCAG GATGCGGAGG AGGAAAAAGA TGATTCAAAT 780

GAAGAAGAAA ACAAAGACAG CCTTGTAGAT GATGAAGAAG AGAAAGAAGA TCTTGGCGAT 840

GAGGATGAAG CAGAGGAAGA AGAGGAGGAG GACAACTTGG CTGCTGGTGT GGATGAGGAG 900

AGAAGTGAGG CCAATGATCA GGGGCCCCCA GGAGAGGACG GTGTGACCCG GGAGGNAAGT 960

AGAGCCTGAG GAGGCTGAAG AAGGCATCTC TGAGCAACCC TGCCCAGCTG ACACAGAGGT 1020

GGTGGAAGAC TCCTTGAGGC AGCGTAAAAG TCAGCATGCT GNCAAGGGAC TGTAGATTTA 1080

ATGATGCGTT TTCAAGAATA CACACCAAAA CAATATGTCA GCTTCCCTTT GGCCTGCAGT 1140

TTGTACCAAA TCCTTAATTT TTCCTGAATG AGCAAGCTTC TCTTAAAAGA TGCTCTCTAG 1200

TCATTTGGTC TCATGGCAGT AAGCCTCATG TATACTAAGG AGAGTCTTCC AGGTGTGACA 1260

ATCAGGATAT AGAAAAACAA ACGTAGTGTN TGGGATCTGT TTGGAGACTG GGATGGGAAC 1320

AAGTTCATTT ACTTAGGGGT CAGAGAGTCT CGACCAGAGG AGGCCATTCC CAGTCCTAAT 1380

CAGCACCTTC CAGAGACAAG GCTGCAGGCC CTGTGAAATG AAAGCCAAGC AGGAGCCTTG 1440

GNTCTGAGGC ATCCCCAAAG TGTAACGTAG AAGCCTTGCA TCCTTTTCTT GTGTAAAGTA 1500

TTTATTTTTG TCAAATTGCA GGAAACATCA GGCACCACAG TGCATGAAAA ATCTTTCACA 1560

GCTAGAAATT GAAAGGGCCT TGGGTATAGA GAGCAGCTCA GAAGTCATCC CAGCCCTCTG 1620

AATCTCCTGT GCTATGTTTT ATTTCTTACC TTTAATTTTT CCAGCATTTC CACCATGGGC 1680

ATTCAGGCTC TCCACACTCT TCACTATTAT CTCTTGGTCA GAGGACTCCA ATAACAGCCA 1740

GGTTTACATG AACTGTGTTT GTTCATTCTG ACCTAAGGGG TTTAGATAAT CAGTAACCAT 1800

AACCCCTGAA GCTGTGACTG CCAAACATCT CAAATGAAAT GTTGTGGCCA TCAGAGACTC 1860

AAAAGGAAGT AAGGATTTTA CAAGACAGAT TAAAAAAAAA TTGTTTTGTC CAAAATATAG 1920

TTGTTGTTGA TTTTTTTTTA AGTTTTCTAA GCAATATTTT TCAAGCCAGA AGTCCTCTAA 1980

GTCTTGCCAG TACAAGGTAG TCTTGTGAAG AAAAGTTGAA TACTGTTTTG TTTTCATCTC 2040

AAGGGGTTCC CTGGGTCTTG AACTACTTTA ATAATAACTA AAAAACCACT TCTGATTTTC 2100

CTTCAGTGAT GTGCTTTTGG TGAAAGAATT AATGAACTCC AGTACCTGAA AGTGAAAGAT 2160

TTGATTTTGT TTCCATCTTC TGTAATCTTC CAAAGAATTA TATCTTTGTA AATCTCTCAA 2220

TACTCAATCT ACTGTAAGTA CCCAGGGAGG CTAATTTCYT TAAAAAAAAA AAAAAAAA 2278

(2) INFORMATION FOR SEQ ID NO: 79:

(i) SEQUENCE CHARACTERISTICS:
(A) LENGTH: 1143 base pairs
(B) TYPE: nucleic acid
(C) STRANDEDNESS: double

(D) TOPOLOGY: linear

(xi) SEQUENCE DESCRIPTION: SEQ ID NO: 79:

CCCCTCCAAC TCTCAACCCA CTTCTCCAGC CAGCGCCCCA GCCCTCCCGC CGCCCGCTCG 60

CAGGTCCCGA GGAGCGCAGA CTGTGTCCCT GACAATGGGA ACAGCCGACA GTGATGAGAT 120

GGCCCCGGAG GCCCCACAGC ACACCCACAT CGATGTGCAC ATCCACCAGG AGTCTGCCCT 180

GGCCAAGCTC CTGCTCACCT GCTGCTCTGC GCTGCGGCCC CGGGCCACCC AGGCCAGGGG 240

CAGCAGCCGG CTGCTGGTGG CCTCGTGGGT GATGCAGATC GTGCTGGGGA TCTTGAGTGC 300

AGTCCTAGGA GGATTTTTCT ACATCCGCGA CTACACCCTC CTCGTCACCT CGGGAGCTGC 360

CATCTGGACA GGGGCTGTGG CTGTGCTGGC TGGAGCTGCT GCCTTCATTT ACGAGAAACG 420

GGGTGGTACA TACTGGGCCC TGCTGAGGAC TCTGCTAGCG CTGGCAGCTT TCTCCACAGC 480

CATCGCTGCC CTCAAACTTT GGAATGAAGA TTTCCGATAT GGCTACTCTT ATTACAACAG 540

TGCCTGCCGC ATCTCCAGCT CGAGTGACTG GAACACTCCA GCCCCCACTC AGAGTCCAGA 600

AGAAGTCAGA AGGCTACACC TATGTACCTC CTTCATGGAC ATGCTGAAGG CCTTGTTCAG 660

AACCCTTCAG GCCATGCTCT TGGGTGTCTG GATTCTGCTG CTTCTGGCAT CTCTGGCCCC 720

TCTGTGGCTG TACTGCTGGA GAATGTTCCC AACCAAAGGG AAAAGAGACC AGAAGGAAAT 780

GTTGGAAGTG AGTGGAATCT AGCCATGCCT CTCCTGATTA TTAGTGCCTG GTGCTTCTGC 840

ACCGGGCGTC CCTGCATCTG ACTGCTGGAA GAAGAACCAG ACTGAGGAAA AGAGGCTCTT 900

CAACAGCCCC AGTTATCCTG GCCCCATGAC CGTGGCCACA GCCCTGCTCC AGCAGCACTT 960

GCCCATTCCT TACACCCCTT CCCCATCCTG CTCCGCTTCA TGTCCCCTCC TGAGTAGTCA 1020

TGTGATAATA AACTCTCATG TTATTGTTNN NAAAAAAAAA AAAAAAAAAA AATTTGGGGG 1080

GGGGCCGGTA CCCATTGGGC CTNNGGGGGN GGTTTAAAAT TAATGGGGGG GGTTTAAAAG 1140

GGN 1143

(2) INFORMATION FOR SEQ ID NO: 80:

(i) SEQUENCE CHARACTERISTICS:
(A) LENGTH: 557 base pairs
(B) TYPE: nucleic acid
(C) STRANDEDNESS: double
(D) TOPOLOGY: linear

(xi) SEQUENCE DESCRIPTION: SEQ ID NO: 80:

GGCAGAGAGC AGATGGCCTT GACACCAGCA GGGTGACATC CGCTATTGCT ACTTCTCTGC 60

TCCCCCACAG TTCCTCTGGA CTTCTCTGGA CCACAGTCCT CTGCCAGACC CCTGCCAGAC 120

CCCAGTCCAC CATGATCCAT CTGGGTCACA TCCTCTTCCT GCTTTTGCTC CCAGTGGCTG 180

CAGCTCAGAC GACTCCAGGA GAGAGATCAT CACTCCCTGC CTTTTACCCT GGCACTTCAG 240

GCTCTTGTTC CGGATGTGGG TCCCTCTCTC TGCCGCTCCT GGCAGGCCTC GTGGCTGCTG 300

ATGCGGTGGC ATCGCTGCTC ATCGTGGGGG CGGTGTTCCT GTGCGCACGC CCACGCCGCA 360

GCCCCGCCCA AGAAGATGGC AAAGTCTACA TCAACATGCC AGGCAGGGGC TGACCCTCCT 420

GCAGCTTGGA CCTTTGACTT CTGACCCTCT CATCCTGGAT GGTGTGTGGT GGCACAGGAA 480

CCCCCGCCCC AACTTTTGGA TTGTAATAAA ACAATTGAAA CACCAAAAAA AAAAAAAAAA 540

AAAAAAAAAA AANTCGA 557

(2) INFORMATION FOR SEQ ID NO: 81:

(i) SEQUENCE CHARACTERISTICS:
(A) LENGTH: 795 base pairs
(B) TYPE: nucleic acid
(C) STRANDEDNESS: double
(D) TOPOLOGY: linear

(xi) SEQUENCE DESCRIPTION: SEQ ID NO: 81:

GCCGGGGCGA TGTGGAGCGC GGGCCGCGGC GGGGCTGCCT GGCCGGTGCT GTTGGGGCTG 60

CTGCTGGCGC TGTTAGTGCC GGGCGGTGGT GCCGCCAAGA CCGGTGCGGA CTCGTGACCT 120

GCGGGTCGGT GCTGAAGCTG CTCAATACGC ACCACCGCGT GCGCTGCACT CGCACGACAT 180

CAAATACGGA TCCGGCAGCG GCCAGCAATC GGTGACCGGC GTAGAGGCGT CGGACGACGC 240

MAATAGCTAC TGGCGGATCC GCGGCGGCTC GGAGGGCGGG TGCCCGCGCG GGTCCCCGGT 300

GCGCTGCGGG CAGGCGGTGA GGCTCACGCA TGTSCTTACG GGCAAGAACY TGCACACGCA 360

CCAYTTCCCG TCGCCGCTGT CCAACAACCA GGAGGTGAGT GCCTTTGGGG AAGACGGCGA 420

GGGCGACGAC CTGGACCTAT GGACAGTGCG CTGCTCTGGA CAGCACTGGG AGCGTGAGGC 480

TGCTGTGCCT TCCAGCATGT GGGCACCTCT GTGTTCCTGT CAGTCACGGG TGAGCAGTAT 540

GGAAGCCCCA TCCGTGGGCA GCATGAGGTC CACGGCATGC CCAGTGCCAA CACGCACAAT 600

ACGTGGAAGG CCATGGAAGG CATCTTCATC AAGCCTAGTG TGGAGCCCTC TGCAGGTCAC 660

GATGAACTCT GAGTGTGTGG ATGGATGGGT GGATGGAGGG TGGCAGGTGG GGCGTCTGCA 720

GGGCCACTCT TGGCAGAGAC TTTGGGTTTG TAGGGGTCCT CAAGTGCCTT TNTGATTAAA 780

GAATGTTGGT CTATG 795

(2) INFORMATION FOR SEQ ID NO: 82:

(i) SEQUENCE CHARACTERISTICS:
(A) LENGTH: 1324 base pairs
(B) TYPE: nucleic acid
(C) STRANDEDNESS: double
(D) TOPOLOGY: linear

(xi) SEQUENCE DESCRIPTION: SEQ ID NO: 82:

NAGGCTTTAA AGCGCCTACC CTGCCTGCAG GTGAGCAGTG GTGTGTGAGA GCCAGGCGTC 60

CCTCTGCCTG CCCACTCAGT GGCAACACCC GGGAGCTGTT TTGTCCTTTG TGGAGCCTCA 120

GCAGTTCCCT CTTTCAGAAC TCACTGCCAA GAGCCCTGAA CAGGAGCCAC CATGCAGTGC 180

TTCAGCTTCA TTAAGACCAT GATGATCCTC TTCAATTTGC TCATCTTTCT GTGTGGTGCA 240

GCCCTGTTGG CAGTGGGCAT CTGGGTGTCA ATCGATGGGG CATCCTTTCT GAAGATCTTC 300

GGGCCACTGT CGTCCAGTGC CATGCAGTTT GTCAACGTGG GCTACTTCCT CATCGCAGCC 360

GGCGTTGTGG TCTTTGCTCT TGGTTTCCTG GGCTGCTATG GTGCTAAGAC TGAGAGCAAG 420

TGTGCCCTCG TGACGTTCTT CTTCATCCTC CTCCTCATCT TCATTGCTGA GGTTGCAGCT 480

GCTGTGGTCG CCTTGGTGTA CACCACAATG GCTGAGCACT TCCTGACGTT GCTGGTAGTG 540

CCTGCCATCA AGAAAGATTA TGGTTCCCAG GAAGACTTCA CTCAAGTGTG GAACACNACC 600

ATGAAAGGGC TCAAGTGCTG TGGCTTCACC AACTATACGG ATTTTGAGGA CTCACCCTAC 660

TTCAAAGAGA ACAGTGCCTT TCCCCCATTC TGTTGCAATG ACAACGTCAC CAACACAGCC 720

AATGAAACCT GCACCAAGCA AAAGGCTCAC GACCAAAAAG TAGAGGGTTG CTTCAATCAG 780

CTTTTGTATG ACATCCGAAC TAATGCAGTC ACCGTGGGTG GTGTGGCAGC TGGAATTGGG 840

GGCCTCGAGC TGGCTGCCAT GATTGTKTCC ATGTATCTGT ACTGCAATCT ACAATAAGTC 900

CACTTCTGCC TCTGCCACTA CTGCTGCCAC ATGGGAACTG TGAAGAGGCA CCCTGGCAAG 960

CAGCAGTGAT TGGGGGAGGG GACAGGATCT AACAATGTCA CTTGGGCCAG AATGGACCTG 1020

CCCTTTCTGC TCCAGACTTG GGGCTAGATA GGGACCACTC CTTTTAGCGA TGCCTGACTT 1080

TCCTTCCATT GGTGGGTGGA TGGGTGGGGG GCATTCCAGA GCCTCTAAGG TAGCCAGTTC 1140

TGTTGCCCAT TCCCCCAGTC TATTAAACCC TTGATATGCC CCCTAGGCCT AGTGGTGATC 1200

CCAGTGCTCT ACTGGGGGAT GAGAGAAAGG CATTTTATAG CCTGGGCATA AGTGAAATCA 1260

GCAGAGCCTC TGGGTGGATG TGTAGAAGGC ACTTCAAAAT GCATAAACCT GTTACAATGT 1320

TAAA 1324

(2) INFORMATION FOR SEQ ID NO: 83:

(i) SEQUENCE CHARACTERISTICS:
(A) LENGTH: 1494 base pairs
(B) TYPE: nucleic acid
(C) STRANDEDNESS: double
(D) TOPOLOGY: linear

(xi) SEQUENCE DESCRIPTION: SEQ ID NO: 83:

CTCAGGCTTC TGTCTCACTT TTCCGGGGGG GGGATTAGGG CAAGGAGGGC ATGAGGGACT 60

GTCTCTCCCT AAAACCCAGA CCCCTGTTCC CCACTCAGTT CTTCTTCATC CTCCTCCTCA 120

TCTTCATTGC TGAGGTTGCA GCTGCTGTGG TCGCCTTGGT GTACACCACA ATGGTGAGAC 180

ACTGGGATGG AGGAAGGGAA GAAGATTGGG CAAAACCCTG GGAGTGGGCT GTGGCCTGTG 240

AATGGCCACC TTCTGTACCA GCCCCTAAAC ACTGGCCTGC CTCACCCAGG CTGAGCACTT 300

CCTGACGTTG CTGGTAGTGC CTGCCATCAA GAAAGATTAT GGTTCCCAGG AAGACTTCAC 360

TCAAGTGTGG AACACCACCA TGAAAGGGGT AAGGTTGGCT GGGGGAGGTT TTAGGGTGGA 420

GAGAAAGAAG CAAGGCCCCA CCTCCACCCT CATCTTGTCT CCAGCTCAAG TGCTGTGGCT 480

TCACCAACTA TACGGATTTT GAGGACTCAC CCTACTTCAA AGAGAACAGT GCCTTTCCCC 540

CATTCTGTTG CAATGACAAC GTCACCCAAC ACAGCCCAAT GAAACCTGCA CCAAGCAAAA 600

GGCTCACSAC CNAAAARTAN AGGTGTGGGC TGGCATGAGT GGGTGGGGAC TGTTTTCATG 660

GCCTCAGAGT GGCAAACGGG GATGGGAGTA GGGCAGCTGC CAACTATAAA TGCTCTTTTC 720

TCTTCCYGAA GGGTTGCTTC AATCAGCTTT TGTATGACAT CCGAACTAAT GCAGTCACCG 780

TGGGTGGTGT GGCAGCTGGA ATTGGGGGCC TCGAGGTAAG CAGATSAGGA GCTGGGACTG 840

GGACATGGGC ATGAGACCAG GGCTGCTCAA CCCATCTGAG GCCTCTCTGG AGGAAACAGA 900

CTTCTAACTG GGCCTCAGGT AGGGTGTCTG TGGGACAGGC TTCAGGATCC CTATCATGTT 960

CCCTCATCTC TCCCTGTTCC TCCCTCTCCA GCTGGCTGCC ATGATTGTGT CCATGTATCT 1020

GTACTGCAAT CTACAATAAG TCCACTTCTG CCTCTGCCAC TACTGCTGCC ACATGGGAAC 1080

TGTGAAGAGG CACCCTGGCA AGCAGCAGTG ATTGGGGGAG GGGACAGGAT CTAACAATGT 1140

CACTTGGGCC AGAATGGACC TGCCCTTTCT GCTCCAGACT TGGGGCTAGA TAGGGACCAC 1200

TCCTTTTAGC GATGCCTGAC TTTCCTTCCA TTGGTGGGTG GATGGGTGGG GGGCATTCCA 1260

GAGCCTCTAA GGTAGCCAGT TCTGTTGCCC ATTCCCCCAG TCTATTAAAC CCTTGATATG 1320

CCCCCTAGGC CTAGTGGTGA TCCCAGTGCT CTACTGGGGG ATGAGAGAAA GGCATTTTAT 1380

AGCCTGGGCA TAAGTGAAAT CAGCAGAGCC TCTGGGTGGA TGTGTAGAAG GCACTTCAAA 1440

ATGCATAAAC CTGTTACAAT GTTAAAAAAA AAAAAAAAAA AACTCGACTC TGCC 1494

(2) INFORMATION FOR SEQ ID NO: 84:

(i) SEQUENCE CHARACTERISTICS:
(A) LENGTH: 1285 base pairs
(B) TYPE: nucleic acid
(C) STRANDEDNESS: double
(D) TOPOLOGY: linear

(xi) SEQUENCE DESCRIPTION: SEQ ID NO: 84:

GCTACGTGGC TGGCATGCAT GGGAACGAGG CCCTGGGGCG GGAGTTGCTT CTGCTCCTGA 60

TGCAGTTCCT GTGCCATGAG TTCCTGCGAG SGAACCCACG GGTGACCCGG CTGCTCTCTG 120

AGATGCGCAT TCACCTGCTG CCCTCCATGA ACCCTGATGG CTATGAGATC GCCTACCACC 180

GGGGTTCAGA RCTGGTGGGC TGGGCCGARG GCCGCTGGAA CAACCAGAGC ATCGATCTTA 240

ACCATAATTT TGCTGAMCTC AACACACCAC TGTGGGAAGC ACAGGACGAT GGGAAGGTGC 300

CCCACATCGT CCCCAACCAT CACCTGCCAT TGCCCACTTA CTACACCCTG CCCAATGCCA 360

CCGTGGCTCC TGAAACGCGG GCAGTAATCA AGTGGATGAA GCGGATCCCC TTTGTGCTAA 420

GTGCCAACCT CCACGGGGGT GAGCTCGTGG TGTCCTACCC ATTCGACATG ACTCGCACCC 480

CGTGGGCTGC CCGCGAGCTC ACGCCCACAC CAGATGATGC TGTGTTTCGC TGGCTCAGCA 540

CTGTCTATGC TGGCAGTAAT CTGGCCATGC AGGACACCAG CCGCCGACCC TGCCACAGCC 600

AGGACTTCTC CGTGCACGGC AACATCATCA ACGGGGCYTG ACTNGGCACA CGGTCCCCGG 660

GANGCATGAA TGAYTTCAGC TACCTACACA CCAACTGCTT TGAGGTCACT GTGGAGCTGT 720

SCTGTGACAA GTTCCCTCAC GAGAATGAAT TGCCCCAGGA GTGGGAGAAC AACAAAGACG 780

CCCTCCTCAC CTACCTGGAG CAGGTGCGCA TGGGCATTGC AGGAGTGGTG AGGGACAAGG 840

ACACGGAGCT TGGGATTGCT GACGCTGTCA TTGCCGTGGA TGGGATTAAC CATGACGTGA 900

CCACGGCGTG GGGCGGGGAT TATTGGCGTC TGCTGACCCC AGGGGACTAC ATGGTGACTG 960

CCAGTKCCGA GGGCTACCAT TCAGTGACAC GGAACTGTCG GGTCACCTTT GAAGAGGGCC 1020

CCTTCCCCTG CAATTTCGTG CTCACCAAGA CTCCCAAACA GAGGCTGCGC GAGCTGCTGG 1080

CAGCTGGGGC CAAGGTGCCC CCGGACCTTC GCAGGCGCCT GGAGCGGCTA AGGGGACAGA 1140

AGGATTGATA CCTGCGGTTT AAGAGCCCTA GGGCAGGCTG GACCTGTCAA GACGGGAAGG 1200

GGAAGAGTAG AGAGGGAGGG ACAAAGTGAG GAAAAGGTGC TCATTAAAGC TACCGGGCAC 1260

CTTAAAAAAA AAAAAAAAAN AAAAA 1285

(2) INFORMATION FOR SEQ ID NO: 85:

(i) SEQUENCE CHARACTERISTICS:
(A) LENGTH: 394 base pairs
(B) TYPE: nucleic acid
(C) STRANDEDNESS: double
(D) TOPOLOGY: linear

(xi) SEQUENCE DESCRIPTION: SEQ ID NO: 85:

GCGCGCTCTA GGAACTAGTG GATCCCCCGG GNCTGCAGGT GTGGAGTGGG CCATCGTAAA 60

TAGTATCTGT GCATAAGGTG GTTGTGCGAT AAATGAGTTA ATGTATGCAA AGCCCTTGGC 120

CCAGAGCCGG CGCAGAGCAT TGTGTAAGTS CTGGCAGGCG TCATGATGGA GATATCATGT 180

CTCCTCTTRT TGATTCAGGA TTCTGATGAG ATGGAGGATG GGCCTGGGGT TCAGGATTAG 240

GCCTTGAGGC ACTGCTCCAG CCTCCTTTGT GGGCCCTGTC ACCCTTGGCT TCATCGGGCC 300

GTARCAAGTC TCCCCTCTCC CACTYTGCAG CAGARGTGTT CAAGAACTGC CTGCTCACGG 360

TTCGTGTTCT GCAAGGCCAT CGCCTAACCT CTAA 394

(2) INFORMATION FOR SEQ ID NO: 86:

(i) SEQUENCE CHARACTERISTICS:
(A) LENGTH: 1925 base pairs
(B) TYPE: nucleic acid
(C) STRANDEDNESS: double
(D) TOPOLOGY: linear

(xi) SEQUENCE DESCRIPTION: SEQ ID NO: 86:

AGTGAAGGGA GCTGGCCGTG CGACTGGGCT TCGGGCCCTG TGCCAGAGGA GCANGCCTTC 60

CTGAGCAGGA GGAAGCAGGT GGTGGCCGCG GCCTTGAGGC AGGCCCTGCA GCTGGATGGA 120

GACCTGCAGG AGGATGAGAT CCCAGTGGTA GCTATTATGG CCACTGGTGG TGGGATCCGG 180

GCAATGACTT CCCTGTATGG GCAGCTGGCT GGCCTGAAGG AGCTGGGCCT CTTGGATTGC 240

KTCTCCTACA TCACCGGGGC CTCGGGCTCC ACCTGGGCCT TGGCCAACCT TTATAAGGAC 300

CCAGAGTGGT CTCAGAAGGA CCTGGCAGGG CCCACTGAGT TGCTGAAGAC CCAGGTGACC 360

AAGAACAAGC TGGGTGTGCT GGCCCCCAGC CAGCTGCAGC GGTACCGGCA GGAGCTGGCC 420

GAGCGTGCCC GCTTGGGCTA CCCAAGCTGC TTCACCAACC TGTGGGCCCT CATCAACGAG 480

GCGCTGCTGC ATGATGAGCC CCATGATCAC AAGCTCTCAG ATCAACGGGA GGCCCTGAGT 540

CATGGCCAGA ACCCTCTGCC CATCTACTGT GCCCTCAACA CCAAAGGGCA GAGCCTGACC 600

ACTTTTGAAT TTGGGGAGTG GTGCGAGTTC TCTCCCTACG AGGTCGGCTT CCCCAAGTAC 660

GGGGCCTTCA TCCCCTCTGA GCTCTTTGGC TCCGAGTTCT TTATGGGGCA GCTGATGAAG 720

AGGCTTCCTG AGTCCCGCAT CTGCTTCTTA GAAGGTATCT GGAGCAACCT GTATGCAGCC 780

AACCTCCAGG ACAGCTTATA CTGGGCCTCA GAGCCCAGCC AGTTCTGGGA CCGCTGGGTC 840

AGGAACCAGG CCAACCTGGA CAAGGAGCAG GTCCCCCTTC TGAAGATAGA AGAACCACCC 900

TCAACAGCCG GCAGAATAGC TGAGTTTTTC ACCGATCTTC TGACGTGGCG TCCACTGGCC 960

CAGGCCACAC ATAATTTCCT GCGTGGCCTC CATTTCCACA AAGACTACTT TCAGCATCCT 1020

CACTTCTCCA CATGGAAAGC TACCACTCTG GATGGGCTCC CCAACCAGCT GACACCCTCG 1080

GAGCCCCACC TGTGCCTGCT GGATGTTGGC TACCTCATCA ATACCAGCTG CCTGCCCCTC 1140

CTGCAGCCCA CTCGGGACGT GGACCTCATC CTGTCATTGG ACTACAACCT CCACGGAGCC 1200

TTCCAGCAGT TGCAGCTCCT GGGCCGGTTC TGCCAGGAGC AGGGGATCCC GTTCCCACCC 1260

ATCTCGCCCA GCCCCGAAGA GCAGCTCCAG CCTCGGGAGT GCCACACCTT CTCCGACCCC 1320

ACCTGCCCCG GAGCCCCTGC GGTGCTGCAC TTTCCTCTGG TCAGCGACTC CTTCCGGGAG 1380

TACTCGGCCC CTGGGGTCCG GCGGACACCC GAGGAGGCGG CAGCTGGGGA GGTGAACCTG 1440

TCTTCATCGG ACTCTCCCTA CCACTACACG AAGGTGACCT ACAGCCAGGA GGACGTGGAC 1500

AAGCTGCTGC ACCTGACACA TTACAATGTC TGCAACAACC AGGAGCAGCT GCTGGAGGCT 1560

CTGCGCCAGG CAGTGCAGCG GAGGCGGCAG CGCAGGCCCC ACTGATGGCC GGGGCCCCTG 1620

CCACCCCTAA CTCTCATTCA TTCCCTGGCT GCTGAGTTGC AGGTGGGAAC TGTCATCACG 1680

CAGTGCTTCA GAGCCTCGGG CTCAGGTGGC ACTGTCCCAG GGTCCAGGCT GAGGGCTGGG 1740

AGCTCCCTTG CGCCTCAGCA GTTTGCAGTG GGGTAAGGAG GCCAAGCCCA TTTGTGTAAT 1800

CACCCAAAAC CCCCCGGCCT GTGCCTGTTT TCCCTTCTGC GCTACCTTGA GTAGTTGGAG 1860

CACTTGATAC ATCACAGACT CATACAAATG TGAGGCGCTG AGAAAAAAAA AAAAAAAAAA 1920

CTCGA 1925

(2) INFORMATION FOR SEQ ID NO: 87:

(i) SEQUENCE CHARACTERISTICS:
(A) LENGTH: 1818 base pairs
(B) TYPE: nucleic acid
(C) STRANDEDNESS: double
(D) TOPOLOGY: linear

(xi) SEQUENCE DESCRIPTION: SEQ ID NO: 87:

CCGGGCCCCC CCNCGNGNTT TTTTTTTTTT TTTTTTTTTK TATGAGTCTG TRATGTATCA 60

AGTGCTCCAA CTACTCAAGG TAGCGCAGAA GGGAAAACAG GCACAGGCCG GGGGGTTTTG 120

GGTGATTACA CAAATGGGCT TGGCCTCCTT ACCCCACTGC AAACTGCTGA GGCGCAAGGG 180

AGCTCCCAGC CCTCAGCCTG GACCCTGGGA CAGTGCCACC TGAGCCCGAG GCTCTGNAAG 240

CACTGCGTGA TGACAGTTCC CACCTGCAAC TCAGCAGCCA GGGAATGAAT GAGAGTTAGG 300

GGTGGCAGGG GCCCCGGCCA TCAGTGGGGC CTGCGCTGCC GCCTCCGCTG CACTGCCTGG 360

CGCAGAGCCT CCAGCAGCTG CTCCTGGTTG TTGCAGACAT TGTAATGTGT CAGGTGCAGC 420

AGCTTGTCCA CGTCCTCCTG GCTGTAGGTC ACCTTCGTGT AGTGGTAGGG AGAGTCCGAT 480

GAAGACAGGT TCACCTCCCC AGCTGCCGCC TCCTCGGGTG TCCGCCGGAC CCCAGGGGCC 540

GAGTACTCCC GGAAGGAGTC GCTGACCAGA GGAAAGTGCA GCACCGCAGG GGCTCCGGGG 600

CAGGTGGGGT CGGAGAAGGT GTGGCACTCC CGAGGCTGGA GCTGCTCTTC GGGGCTGGGC 660

GAGATGGGTG GGAACGGGAT CCCCTGCTCC TGGCAGAACC GGCCCAGGAG CTGCAACTGC 720

TGGAAGGCTC CGTGGAGGTT GTAGTCCAAT GACAGGATGA GGTCCACGTC CCGAGTGGGC 780

TGCAGGAGGG GCAGGCAGCT GGTATTGATG AGGTAGCCAA CATCCAGCAG GCACAGGTGG 840

GGCTCCGAGG GTGTCAGCTG GTTGGGGAGC CCATCCAGAG TGGTAGCTTT CCATGTGGAG 900

AAGTGAGGAT GCTGAAAGTA GTCTTTGTGG AAATGGAGGC CACGCAGGAA ATTATGTGTG 960

GCCTGGGCCA GTGGACGCCA CGTCAGAAGA TCGGTGAAAA ACTCAGCTAT TCTGCCGGCT 1020

GTTGAGGGTG GTTCTTCTAT CTTCAGAAGG GGGACCTGCT CCTTGTCCAG GTTGGCCTGG 1080

TTCCTGACCC AGCGGTCCCA GAACTGGCTG GGCTCTGAGG CCCAGTATAA GCTGTCCTGG 1140

AGGTTGGCTG CATACAGGTT GCTCCAGATA CCTTCTAAGA AGCAGATGCG GGACTCAGGA 1200

AGCCTCTTCA TCAGCTGCCC CATAAAGAAC TCGGAGCCAA AGAGCTCAGA GGGGATGAAG 1260

GCCCCGTACT TGGGGAAGCC GACCTCGTAG GGAGAGAACT CGCACCACTC CCCAAATTCA 1320

AAAGTGGTCA GGCTCTGCCC TTTGGTGTTG AGGGCACAGT AGATGGGCAG AGGGTTCTGG 1380

CCATGACTCA GGGCCTCCCG TTGATCTGAG AGCTTGTGAT CATGGGGCTC ATCATGCAGC 1440

AGCGCCTCGT TGATGAGGGC CCACAGGTTG GTGAAGCAGC TTGGGTAGCC CAAGCGGGCA 1500

CGCTCGGCCA GCTCCTGCCG GTACCGCTGC AGCTGGCTGG GGGCCAGCAC ACCCAGCTTG 1560

TTCTTGGTCA CCTGGGTCTT CAGCAACTCA GTGGGCCCTG CCAGGTCCTT CTGAGACCAC 1620

TCTGGGTCCT YATAAAGGTT GGCCAAGGCC CAGGTGGAGC CCGAGGCCCC GGTGATGTAG 1680

GAGACGCAAT CCAAGAGGCC CCAGCTCCTT TCAGGCCAGC CAGCTGCCCA TACAGGGAAG 1740

TCATTGCCCG GATCCCACCA CCAGTGGCCA TAATAGCTAC CACTGGGATC TCATCCTCCT 1800

GCAGGTCTCC ATCCAGCT 1818

(2) INFORMATION FOR SEQ ID NO: 88:

(i) SEQUENCE CHARACTERISTICS:
(A) LENGTH: 539 base pairs
(B) TYPE: nucleic acid
(C) STRANDEDNESS: double
(D) TOPOLOGY: linear

(xi) SEQUENCE DESCRIPTION: SEQ ID NO: 88:

AGGGTAATTA ATATGAAGTG CAAAAAGTTG AATGTTCCAG TCTAAAAGGC AGTGGGAGAA 60

ATTACATAGC ATGGAAATAA TAAAATGAAY TCTTATTAAT GAGAACGAGG YTCTTGCAGT 120

GGCAAGTTCT GCTGGTCACC CGATGGGGAT GGGAGCCTTT CAAGCTTTTT TTTGGGTAAT 180

ACTCACAGTT TCCAACGTCT GTGTACTTTT CAAAATGAGC TTGTTCTTCC TTCTGACACT 240

CATCTCAAAG CTCCATGGTG ACGCAGAGGT CTGTTGAAGG TCACAGGGTC CTCGCTTGCA 300

TTGGCATACG GTCCTGTAGC ATCACTTGTT AGCCCACTGC TGCTTGAAGG AACTAAGAGT 360

ATTCAGGGAT AGAGAGCTGA AAATAGGATT AATTNNTTCC TTTTGACTCT CCCCTCAAGA 420

TGTCCTTGCT TTGGTCTGAA AACCTCTCCT GACAACTTTT GCCCAAAGCA AACCATCTGC 480

CTTTTCTGAA CTCTGAGTGA ATATATTAGC ATCTTCCCTT CTGAGCCCTC GTACTGCCA 539

(2) INFORMATION FOR SEQ ID NO: 89:

(i) SEQUENCE CHARACTERISTICS:
(A) LENGTH: 855 base pairs
(B) TYPE: nucleic acid
(C) STRANDEDNESS: double
(D) TOPOLOGY: linear

(xi) SEQUENCE DESCRIPTION: SEQ ID NO: 89:

CCTCTGCCCA GGCCGCACCC GAGCTCAGGC TCGTGCCCAC CCACCAAGTT CCAGTGCCGC 60

ACCAGTGGCT TATGCGTGCC CCTCACCTGG CGCTGCGACA GGNACTTGGA CTGCAGCGAT 120

GGCAGCGATG AGGAGGAGTG CAGGATTGAG CCATGTACCC AGAAAGGGCA ATGCCCACCG 180

CCCCCTGGCC TCCCCTGCCC CTGCACCGGC GTCAGTGACT GCTCTGGGGG AACTGACAAG 240

AAACTGCGCA ACTGCAGCCG CCTGGCCTGC CTAGCAGCGG AGCTCCGTTG CACGCTGAGC 300

GATGACTGCA TTCCACTCAC GTGGCGCTGC GACGGCCACC CAGACTGTCC CGACTCCAGC 360

GACGAGCTCG GCTGTGGAAC CAATGAGATC CTCCCGGAAG GGGATGCCAC AACCATGGGG 420

CCCCCTGTGA CCCTGGAGAG TGTCACCTCT CTCAGGAATG CCACAACCAT GGGGCCCCCT 480

GTGAACCCTG GAGAGTGTCC CCTCTGTCGG GAATGCCACA TCCTCCTCTG CCGGAGACCA 540

GTCTGGAAGC CCAACTGCCT ATGGGGTTAT TGCAGCTGCT GCGGTGCTCA GTGCAAGCCT 600

GGTCACCGCC ACCCTCCTCC TTTTGTCCTG GCTCCGAGCC CAGGAGCGCC TCCGCCCACT 660

GGGGTTACTG GTGGCCATGA AGGAGTCCCT GCTGCTGTCA GAACAGAAGA CCTCGCTGCC 720

CTGAGGACAA GCACTTGCCA CCACCGTCAC TCAGCCCTGG GCGTACNGSA CAGGAGGAGA 780

GCAGTGATGC GGATGGGTAC CGGGCACACC AGCCCTTCAG AGACCTGAGC NCTTCTGGCC 840

ACTGGAACTT CGAAC 855

(2) INFORMATION FOR SEQ ID NO: 90:

(i) SEQUENCE CHARACTERISTICS:
(A) LENGTH: 628 base pairs
(B) TYPE: nucleic acid
(C) STRANDEDNESS: double
(D) TOPOLOGY: linear

(xi) SEQUENCE DESCRIPTION: SEQ ID NO: 90:

AAGGACGTGC CGTGCCGCTG GGTTCTGAGC CGGAGTGGTC GGTGGGTGGG ATGGAGGCGA 60

CCTTGGAGCA GCACTTGGAA GACACAATGA AGAATCCCTC CATTGTTGGA GTCCTGTGCA 120

CAGATTCACA AGGACTTAAT CTGGGTTGCC GCGGGACCCT GTCAGATGAG CATGCTGGAG 180

TGATATCTGT TCTAGCCCAG CAAGCAGCTA AGCTAACCTC TGACCCCACT GATATTCCTG 240

TGGTGTGTCT AGAATCAGAT AATGGGAACA TTATGATCCA GAAACACGAT GGCATCACGG 300

TGGCAGTGCA CAAAATGGCC TCTTGATGCT CATATCTGTT CTTCAGCAGC CTGTCATAGG 360

AACTGGATCC TACCTATGTT AATTACCTTA TAGAACTACT AAAGTTCCAG TAGTTAGGCC 420

ATTCATTTAA TGTGCATTAG GCACTTTTCT GTTTATTTAA GAGTCAATTG CTTTCTAATG 480

CTCTATGGAC CGACTATCAA GATATTAGTA AGAAAGGATC ATGTTTTGAA GCAGCAGGTC 540

CAGGTCACTT TGTATATAGA ATTTTGCTGT ATTCAATAAA TCTGTTTGGA GGNAAAAAAA 600

AAAAAARAAA AAMTSGAGGG CCGAAGCT 628

(2) INFORMATION FOR SEQ ID NO: 91:

(i) SEQUENCE CHARACTERISTICS:
(A) LENGTH: 1053 base pairs
(B) TYPE: nucleic acid
(C) STRANDEDNESS: double
(D) TOPOLOGY: linear

(xi) SEQUENCE DESCRIPTION: SEQ ID NO: 91:

CTCTTTTCTG CAGTTCAAGG GAAAGACGAG ATCTTGCACA AGGCACTCTG CTTCTGCCCT 60

TGGCTGGGGA AGGGTGGCAT GGARCCTCTC CGGCTGCTCA TCTTACTCTT TGTCACAGAG 120

CTGTCCGGAG CCCACAACAC CACAGTGTTC CAGGGCGTGG CGGGCCAGTC CCTGCAGGTG 180

TCTTGCCCCT ATGACTCCAT GAAGCACTGG GGGAGGCGCA AGGCCTGGTG CCGCCAGCTG 240

GGAGAGAAGG GCCCATGCCA GCGTGTGGTC AGCACGCACA ACTTGTGGCT GCTGTCCTTC 300

CTGAGGAGGT GGAATGGGAG CACAGCCATC ACAGACGATA CCCTGGGTGG CACTCTCACC 360

ATTACGCTGC GGAATCTACA ACCCCATGAT GCGGGTCTCT ACCAGTGCCA GAGCCTCCAT 420

GGCAGTGAGG CTGACACCCT CAGGAAGGTC CTGGTGGAGG TGCTGGCAGA CCCCCTGGAT 480

CACCGGGATG CTGGAGATCT CTGGTTCCCC GGGGAGTCTG AGAGCTTCGA GGATGCCCAT 540

GTGGAGCACA GCATCTCCAG GAGCCTCTTG GAAGGAGAAA TCCCCTTCCC ACCCACTTCC 600

ATCCTTCTCC TCCTGGCCTG CATCTTTCTC ATCAAGATTC TAGCAGCCAG CGNCCTCTGG 660

GCTGCAGCCT GGCATGGACA GAAGCCAGGG ACACATCCAC CCAGTGAACT GGACTGTGGC 720

CATGACCCAG GGTATCAGCT CCAAACTCTG CCAGGGCTGA GAGACACGTG AAGGAAGATG 780

ATGGGAGGAA AAGCCCAGGA GAAGTCCCAC CAGGGACCAG CCCAGCCTGC ATACTTGCCA 840

CTTGGCCACC AGGACTCCTT GTTCTGCTCT GGCAAGAGAC TACTCTGCCT GAACACTGCT 900

TCTCCTGGAC CCTGGAAGCA GGGACTGGTT GAGGGAGTGG GGAGGTGGTA AGAACACCTG 960

ACAACTTCTG AATATTGGAC ATTTTAAACA CTTACAAATA AATCCAAGAC TGTCATATTT 1020

AAAAAAAAAA AAAAAAAAAA AACNCGAGGG GGG 1053

(2) INFORMATION FOR SEQ ID NO: 92:

(i) SEQUENCE CHARACTERISTICS:
(A) LENGTH: 1075 base pairs
(B) TYPE: nucleic acid
(C) STRANDEDNESS: double
(D) TOPOLOGY: linear

(xi) SEQUENCE DESCRIPTION: SEQ ID NO: 92:

GCACGAGCCT GATCCTCTCT TTTCTGCAGT TCAAGGGAAA GACGAGATCT TGCACAAGGC 60

ACTCTGCTTC TGCCCTTGGC TGGGGAAGGG TGGCATGGAG CCTCTCCGGC TGCTCATCTT 120

ACTCTTTGTC ACAGAGCTGT CCGGAGCCCA CAACACCACA GTGTTCCAGG GCGTGGCGGG 180

CCAGTCCCTG CAGGTGTCTT GCCCCTATGA CTCCATGAAG CACTGGGGGA GGCGCAAGGC 240

CTGGTGCCGC CAGCTGGGAG AGAAGGGCCC ATGCCAGCGT GTGGTCAGCA CGCACAACTT 300

GTGGCTGCTG TCCTTCCTGA GGAGGTGGAA TGGGAGCACA GCCATCACAG ACGATACCCT 360

GGGTGGCACT CTCACCATTA CGCTGCGGAA TCTACAACCC CATGATGCGG GTCTCTACCA 420

GTGCCAGAGC CTCCATGGCA GTGAGGCTGA CACCCTCAGG AAGGTCCTGG TGGAGGTGCT 480

GGCAGACCCC CTGGATCACC GGGATGCTGG AGATCTCTGG TTCCCCGGGG AGTCTGAGAG 540

CTTCGAGGAT GCCCATGTGG AGCACAGCAT CTCCAGGAGC CTCTTGGAAG GAGAAATCCC 600

CTTCCCACCC ACTTCCATCC TTCTCCTCCT GGCCTGCATC TTTCTCATCA AGATTCTAGC 660

AGCCAGCGCC CTCTGGGCTG CAGCCTGGCA TGGACAGAAG CCAGGGACAC ATCCACCCAG 720

TGAACTGGAC TGTGGCCATG ACCCAGGGTA TCAGCTCCAA ACTCTGCCAG GGCTGAGAGA 780

CACGTGAAGG AAGATGATGG GAGGAAAAGC CCAGGAGAAG TCCCACCAGG GACCAGCCCA 840

GCCTGCATAC TTGCCACTTG GCCACCAGGA CTCCTTGTTC TGCTCTGGCA AGAGACTACT 900

GTGCCTGAAC ACTGCTTCTC CTGGACCCTG GAAGCAGGGA CTGGTTGAGG GAGTGGGGAG 960

GTGGTAAGAA CACCTGACAA CTTCTGAATA TTGGACATTT TAAACACTTA CAAATAAATC 1020

CAAGACTGTC ATATTTAAAA AAAAAAAAAA AAAAAAAACN CGAGGGGGGN CCCGG 1075

(2) INFORMATION FOR SEQ ID NO: 93:

(i) SEQUENCE CHARACTERISTICS:
(A) LENGTH: 2492 base pairs
(B) TYPE: nucleic acid
(C) STRANDEDNESS: double
(D) TOPOLOGY: linear

(xi) SEQUENCE DESCRIPTION: SEQ ID NO: 93:

TCCCGACTCA GCTTCCCACC CTGGGCTTTC CGAGGTGCTK TCGCCGCTGT CCCCACCACT 60

GCAGCCATGA TCTCCTTAAC GGACACGCAG AAAATTGGAA TGGGATTAAC AGGATTTGGA 120

GTGTTTTTCC TGTTCTTTGG AATGATTCTC TTTTTTGACA AAGCACTACT GGCTATTGGA 180

AATGTTTTAT TTGTAGCCGG CTTGGCTTTT GTAATTGGTT TAGAAAGAAC ATTCAGATTC 240

TTCTTCCAAA AACATAAAAT GAAAGCTACA GGTTTTTTTC TGGGTGGTGT ATTTGTAGTC 300

CTTATTGGTT GGCCTTTGAT AGGCATGATC TTCGAAATTT ATGGATTTTT TCTCTTGTTC 360

AGGGGCTTCT TTCCTGTCGT TGTTGGCTTT ATTAGAAGAG TGCCAGTCCT TGGATCCCTC 420

CTAAATTTAC CTGGAATTAG ATCATTTGTA GATAAAGTTG GAGAAAGCAA CAATATGGTA 480

TAACAACAAG TGAATTTGAA GACTCATTTA AAATATTGTG TTATTTATAA AGTCATTTGA 540

AGAATATTCA GCACAAAATT AAATTACATG AAATAGCTTG TAATGTTCTT TACAGGAGTT 600

TAAAACGTAT AGCCTACAAA GTACCAGCAG CAAATTAGCA AAGAAGCAGT GAAAACAGGC 660

TTCTACTCAA GTGAACTAAG AAGAAGTCAG CAAGCAAACT GAGAGAGGTG AAATCCATGT 720

TAATGATGCT TAAGAAACTC TTGAAGGCTA TTTGTGTTGT TTTTCCACAA TGTGCGAAAC 780

TCAGCCATCC TTAGAGAACT GTGGTGCCTG TTTCTTTTCT TTTTATTTTG AAGGCTCAGG 840

AGCATCCATA GGCATTTGCT TTTTAGAAAT GTCCACTGCA ATGGCAAAAA TATTTCCAGT 900

TGCACTGTAT CTCTGGAAGT GATGCATGAA TTCGATTGGA TTGTGTCATT TTAAAGTATT 960

AAAACCAAGG AAACCCCAAT TTTGATGTAT GGATTACTTT TTTTTGTAAA CATGGTTAAA 1020

ATAAAACTTC TGTGGTTCTT CTGAATCTTA ATATTTCAAA GCCAGGTGAA AATCTGAACT 1080

AGATATTCTT TGTTGGAATA TGCAAAGGTC ATTCTTTACT AACTTTTAGT TACTAAATTA 1140

TAGCTAAGTT TTGTCAGCAG CATACTCCGG AAAGTCTCAT ACTTCTTGGG AGTCTGCCCT 1200

CCTAAGTATC TGTCTATATC ATTCATTACG TGTAAGTATT TAACAAAAAA GCATTCTTGA 1260

CCATGAATGA AGTAGTTTGT TTCATAGCTT GTCTCATTGA ATAGTATTAT TGAAGATACT 1320

AAATGATGCA AACCAAATGG ATTTTTTCCA TGTCATGATG TAATTTTTCT TTCTTCTTTC 1380

TTTTTTTTAA ATTTTAGCAG TGGCTTATTA TTTGTTTTTC ATAAATTAAA ATAACTTTTG 1440

ATAATGTTTA CTTTAAGACA TGTAACATGT TAAAAGGTTA AACTTATGGC TGTTTTTAAA 1500

GGGCTATTCA TTTAATCTGA GTTTTCCCTT ATTTTCAGCT TTTTCCTAGC ATATAATAGT 1560

CATTAAGCAT GACATATCCT TCATATGATC ACTCATCTTG AGTTAATTAG AAAATACCTG 1620

AGTTCACGTG CTAAAGTCAT TTCACTGTAA TAAACTGACT RTGGTTTCTT AAGAACATGA 1680

CACTAAAAAA AAAGTGGTTT TTTTCCACCG TTGCTGATTA TTAGACAGTA GGAAATAGCT 1740

GTTTTCTTTA GTTTTACAAG ATGTGACAGC TTTAGTGGTA GATGTAGGGA AACATTTCAA 1800

CAGCCATAGT ACTATTTGTT TTACCACTGA TTGCACTGTT TTGTTTTTTT AACAGTTGCA 1860

AAGCTTTTTA ATGCATAAAA GTATAATTGA AATCTGTGGT ATTTATTTAC AAACATGTCT 1920

ACAAAAATAG ATTACAGCTT ATTTTATTTT TAGTTAAATC TCTTAATACA CAGAGNAACT 1980

CCCAATCTTG CTCATCTAAA TAAGGAAAGA CTTGGTGTAT AGTGTGATGG TTTAGTCTTA 2040

AGGATTAAGA CATTTTTGGT ACTTGCATTT GACTTACGAT GTATCTGTGA AAATGGGATG 2100

ATATTGACAA ATGGAGACTC CTACCTCAAT AGTTAATGGA ATAATAAGAG GCTACTGTTG 2160

TGTCTAATGT TCTTCAAAAA AGTAATATCC TCACTTGGAG AGTGTCAAAT ACATACTTTG 2220

AGGATTGACT TTATATAAGG TGCCCTGTAG AAMTCTGTTA CACATATTTT TGACCCATAT 2280

TATTTACAAT GTCTTGATAA TTCTACCTTT TTAGAGCAAG AATAGTATCT GCTAATGTAA 2340

GGGACATCTG TATTTAACTC CTTTGTAGAC ATGAATTTCT ATCAAAATGT TCTTTGCACT 2400

GTAACAGAGA TTCCTTTTTT CAATAATCTT AATTCAAAGC ATTATTAGGM CTTGAAAGGG 2460

TTTGRTAATC TCCCCGTCCT TGGTAAAGGT TG 2492

(2) INFORMATION FOR SEQ ID NO: 94:

(i) SEQUENCE CHARACTERISTICS:
(A) LENGTH: 3058 base pairs
(B) TYPE: nucleic acid
(C) STRANDEDNESS: double
(D) TOPOLOGY: linear

(xi) SEQUENCE DESCRIPTION: SEQ ID NO: 94:

ACCCTAAATC AACAGACAAT GGCATTGTCG AAGAGCAACC TGTTAATGAA ATCATGTTAA 60

AAATCAAGGT TTGGCTTCAG TTTAAATCAC TTGAGGTATG AAGTTTATCC TGTTTTCCAG 120

AGATAAACAT AAGTTGATCT TCCCAAAATA CCATCATTAG GACCTATCAC ACAATATCAC 180

TAGTTTTTTT TGTTTGTTTG TTTTTTGTTT TTTTTCTTGG TAAAGCCATG CACCACAGAC 240

TTCTGGGCAG AGCTGAGAGA CAATGGTCCT GACATAATAA GGATCTTTGA TTAACCCCCA 300

TAAGGCATGT GTGTGTATAC AAATATACTT CTCTTTGGCT TTTCGACATA GAACCTCAGC 360

TGTTAACCAA GGGGAAATAC ATCAGATCTG CAACACAGAA ATGCTCTGCC TGAAATTTCC 420

ACCATGCCTA GGACTCACCC CATTTATCCA GGTCTTTCTG GATCTGTTTA ATCAATAAGC 480

CCTATAATCA CTTGCTAAAC ACTGGGCTTC ATCACCCAGG GATAAAAACA GAGATCATTG 540

TCTTGGACCT CCTGCATCAG CCTATTCAAA ATTATCTCTC TCTCTAGCTT TCCACAAATC 600

CTAAAATTCC TGTCCCAAGC CACCCAAATT CTCAGATCTT TTCTGGAACA AGGCAGAATA 660

TAAAATAAAT ATACATTTAG TGGCTTGGGC TATGGTCTCC AAAGATCCTT CAAAAATACA 720

TCAAGCCAGC TTCATTCACT CACTTTACTT AGAACAGAGA TATAAGGGCC TGGGATGCAT 780

TTATTTTATC AATACCAATT TTTGTGGCCA TGGCAGACAT TGCTAATCAA TCACAGCACT 840

ATTTCCTATT AAGCCCACTG ATTTCTTCAC AATCCTTCTC AAATTACAAT TCCAAAGAGC 900

CGCCACTCAA CAGTCAGATG AACCCAACAG TCAGATGAGA GAAATGAACC CTACTTGCTA 960

TCTCTATCTT AGAAAGCAAA AACAAACAGG AGTTTCCAGG GAGAATGGGA AAGCCAGGGG 1020

GCATAAAAGG TACAGTCAGG GGAAAATAGA TCTAGGCAGA GTGCCTTAGT CAGGGACCAC 1080

GGGCGCTGAA TCTGCAGTGC CAACACCAAA CTGACACATC TCCAGGTGTA CCTCCAACCC 1140

TAGCCTTCTC CCACAGCTGC CTACAACAGA GTCTCCCAGC CTTCTCAGAG AGCTAAAACC 1200

AGAAATTTCC AGACTCATGA AAGCAACCCC CCAGCCTCTC CCCAACCCTG CCGCATTGTC 1260

TAATTTTTAG AACACTAGGC TTCTTCTTTC ATGTAGTTCC TCATAAGCAG GGGCCAGAAT 1320

ATCTCAGCCA CCTGCAGTGA CATTGCTGGA CCCCTGAAAA CCATTCCATA GGAGAATGGG 1380

TTCCCCAGGC TCACAGTGTA GAGACATTGA GCCCATCACA ACTGTTTTGA CTGCTGGCAG 1440

TCTAAAACAG TCCACCCACC CCATGGCACT GCCGCGTGAT TCCCGCGCCA TTCAGAAGTT 1500

CAAGCCGAGA TGCTGACGTT GCTGAGCAAS AGATGGTGAG CATCAGTGCA AATGCACCAT 1560

TCAGCACATC AGTCATATGC CCAGTGCAGT TACAAGATGT TGTTTCGGCA AAGCATTTTG 1620

ATGGAATAGG GAACTGCAAA TGTATGATGA TTTTGAAAAG GCTCAGCAGG ATTTGTTCTT 1680

AAACCGACTC AGTGTGTCAT CCCCGGTTAT TTAGAATTAC AGTTAAGAAG GAGAAACTTC 1740

TATAAGACTG TATGAACAAG GTGATATCTT CATAGTGGGC TATTACAGGC AGGAAAATGT 1800

TTTAACTGGT TTACAAAATC CATCAATACT TGTGTCATTC CCTGTAAAAG GCAGGAGACA 1860

TGTGATTATG ATCAGGAAAC TGCACAAAAT TATTGTTTTC AGCCCCCGTG TTATTGTCCT 1920

TTTGAACTGT TTTTTTTTTA TTAAAGCCAA ATTTGTGTTG TATATATTCG TATTCCATGT 1980

GTTAGATGGA AGCATTTCCT ATCCAGTGTG AATAAAAAGA ACAGTTGTAG TAAATTATTA 2040

TAAAGCCGAT GATATTTCAT GGCAGGTTAT TCTACCAAGC TGTGCTTGTT GGTTTTTCCC 2100

ATGACTGTAT TGCTTTTATA AATGTACAAA TAGTTACTGA AATGACGAGA CCCTTGTTTG 2160

CACAGCATTA ATAAGAACCT TGATAAGAAC CATATTCTGT TGACAGCCAG CTCACAGTTT 2220

CTTGCCTGAA GCTTGGTGCA CCCTCCAGTG AGACACAAGA TCTCTCTTTT ACCAAAGTTG 2280

AGAACAGAGC TGGTGGATTA ATTAATAGTC TTCGATATCT GGCCATGGGT AACCTCATTG 2340

TAACTATCAT CAGAATGGGC AGAGATGATC TTGAAGTGTC ACATACACTA AAGTCCAAAC 2400

ACTATGTCAG ATGGGGGTAA AATCCATTAA AGAACAGGAA AAAATAATTA TAAGATGATA 2460

AGCAAATGTT TCAGCCCAAT GTCAACCCAG TTAAAAAAAA AATTAATGCT GTGTAAAATG 2520

GTTGAATTAG TTTGCAAACT ATATAAAGAC ATATGCAGTA AAAAGTCTGT TAATGCACAT 2580

CCTGTGGGAA TGGAGTGTTC TAACCAATTG CCTTTTCTTG TTATCTGAGC TCTCCTATAT 2640

TATCATACTC AGATAACCAA ATTAAAAGAA TTAGAATATG ATTTTTAATA CACTTAACAT 2700

TAAACTCTTC TAACTTTCTT CTTTCTGTGA TAATTCAGAA GATAGTTATG GATCTTCAAT 2760

GCCTCTGAGT CATTGTTATA AAAAATCAGT TATCACTATA CCATGCTATA GGAGACTGGG 2820

CAAAACCTGT ACAATGACAA CCCTGGAAGT TGCTTTTTTT AAAAAAATAA TAAATTTCTT 2880

AAATCAACTC TTTTTTCTGG TTGTCTGTTT GTTATAAAGT GCAACGKATT CAAGTCCTCA 2940

ATATCCTGAT CATAATACCA TGCTATAGGA GACTGGGCAA AACCTGTACA ATGACAACCC 3000

TGGAAGTTGC TTTTTTAAAA AAATAATAAT TTNTTAATCC AAAAAAANAA AAAAANTT 3058

(2) INFORMATION FOR SEQ ID NO: 95:

(i) SEQUENCE CHARACTERISTICS:
(A) LENGTH: 1099 base pairs
(B) TYPE: nucleic acid
(C) STRANDEDNESS: double
(D) TOPOLOGY: linear

(xi) SEQUENCE DESCRIPTION: SEQ ID NO: 95:

GGCTTTGTAG CTGCTCCGCA GCCCAGCCCG GGCGCGCTCG CAGAGTCCTA GGCGGTGCGC 60

GGCNTCCTGC CTCCTCCCTC CTCGGCGGTC GCGGCCCGCG CCTCCGCGGT GCCTGCCTTC 120

GCTCTCAGGT TGAGGAGCTC AAGCTTGGGA AAATGGTGTG CATTCCTTGT ATCGTCATTC 180

CAGTTCTGCT CTGGATCTAC AAAAAATTCC TGGAGCCATA TATATACCCT CTGGTTTCCC 240

CCTTCGTTAG TCGTATATGG CCTAAGAAAG CAATACAAGA ATCCAATGAT ACAAACAAAG 300

GCAAAGTAAA CTTTAAGGGT GCAGACATGA ATGGATTACC AACAAAAGGA CCAACAGAAA 360

TCTGTGATAA AAAGAAAGAC TAAAGAAATT TTCCTAAAGG ACCCCATCAT TTAAAAAATG 420

GACCTGATAA TATGAAGCAT CTTCCTTGTA ATTGTCTCTG ACCTTTTTAT CTGAGACCGG 480

AATTCAGGAT AGGAGTCTAG ATATTTACCT GATACTAATC AGGAAATATA TGATATCCGT 540

ATTTAAAATG TAGTTAGTTA TATTTAATGA CCTCATTCCT AAGTTCCTTT TTCGTTAATG 600

TAGCTTTCAT TTCTGTTATT GCTGTTTGAA TAATATGATT AAATAGAAGG TTTGTGCCAG 660

TAGACATTAT GTTACTAAAT CAGCACTTTA AAATCTTTGG TTCTCTAATT CATATGAATT 720

TGCTGTTTGC TCTAATTTCT TTGGGCTCTT CTAATTTGAG TGGAGTACAA TTTTGTTGTG 780

AAACAGTCCA GTGAAACTGT GCAGGGAAAT GAAGGTAGAA TTTTGGGAGG TAATAATGAT 840

GTGAAACATA AAGATTTAAT AATTACTGTC CAACACAGTG GAGCAGCTTG TCCACAAATA 900

TAGTAATTAC TATTTATTGC TCTAAGGAAG ATTAAAAAAA GATAGGGAAA AGGGGGAAAC 960

TTCTTTGAAA AATGAAACAT CTGTTACATT AATGTCTAAT TATAAAATTT TAATCCTTAC 1020

TGCATTTCTT CTGTTCCTAC AAATGTATTA AACATTCAGT TTAACTGGTA AAAAAAAAAA 1080

AAAAAAACCC GGGGGGGGG 1099

(2) INFORMATION FOR SEQ ID NO: 96:

(i) SEQUENCE CHARACTERISTICS:
(A) LENGTH: 1580 base pairs
(B) TYPE: nucleic acid
(C) STRANDEDNESS: double
(D) TOPOLOGY: linear

(xi) SEQUENCE DESCRIPTION: SEQ ID NO: 96:

GGCAGAGACT GGAATCTCTC TTCATGAAAA AATGCAGCCC CTTAACTTCA GTTCGACARA 60

GTGCAGCTCC TTCTCTCCAC CCACCACAGT GATTCTCCTT ATCCTGCTGT GCTTTGAGGG 120

CCTGCTCTTC CTCATTTTCA CATCAGTGAT GTTTGGGACC CAGGTGCACT CCATCTGCAC 180

AGATGAGACG GGAATAGAAC AATTGAAAAA GGAAGAGAGA AGATGGGCTA AAAAAACAAA 240

ATGGATGAAC ATGAAAGCCG TTTTTGGCCA CCCCTTCTCT CTAGGCTGGG CCAGCCCCTT 300

TGCCACGCCA GACCAAGGGA AGGCAGACCC GTACCAGTAT GTGGTCTGAA GGACCCCGAC 360

CGGCATGGCC ACTCAGACAC AAGTCCACAC CACAGCACTA CCGTCCCATC CGTTCTCATG 420

AATGTTTAAA TCGAAAAAGC AAAACAACTA CTCTTAAAAC TTTTTTTATG TCTCAAGTAA 480

AATGGCTGAG CATTGCAGAG ARAAAAAAAA GTCCCCACAT TTTATTTTTT AAAAACCATC 540

CTTTCGATTT CTTTTGGTGA CCGAWGCTGC TCTCTTTTCC TTTTAAAATC ACTTCTCTGG 600

CCTCTGGTTT CTCTCTGCTG TCTGTCTGGC ATGACTAATG TAGAGGGCGC TGTCTCGCGC 660

TGTGCCCATT CTACTAACTG AGTGAGACAT GACGCTGTGC TGGATGGAAT AGTCTGGACA 720

CCTGGTGGGG GATGCATGGG AAAGCCAGGA GGGCCCTGAC CTCCCACTGC CCAGGAGGCA 780

GTGGCGGGCT CCCCGATGGG ACATAAAACC TCACCGAAGA TGGATGCTTA CCCCTTGAGG 840

CCTGAGAAGG GCAGGATCAG AAGGGACCTT GGCACAGCGA CCTCATCCCC CAAGTGGACA 900

CGGTTTGCCT GCTAACTCGC AAAGCAATTG CCTGCCTTGT ACTTTATGGG CTTGGGGTGT 960

GTAGAATGAT TTTGCGGGGG AGTGGGGAGA AAGATGAAAG AGGTCTTATT TGTATTCTGA 1020

ATCAGCAATT ATATTCCCTG TGATTATTTG GAAGAGTGTG TAGGAAAGAC GTTTTTCCAG 1080

TTCAAAATGC CTTATACAAT CAAGAGGAAA AAAAATTACA CAATTTCAGG CAAGCTACGT 1140

TTTCCTTTGT TTCATCTGCT TCCTCTCTCA CCACCCCATC TCCCTCTCTT CCCCAGCAAG 1200

ATGTCAATTA AGCAGTGTGA ATTCTGACTG CAATAGGCAC CAGTGCCCAA CACATACAGC 1260

CCCACCATCA TCCCCTTCTC ATTTTATAAA CCTCAAAGTG GATTCACTTT CTGATAGTTA 1320

ACCCCCATAA ATGTGCACGT ACCTGTGTCT TATCTATATT TTAACCKGGG AGACTGTTGT 1380

CCTGGGCATG GGAGATGACC ATGATGCTGG GGTTACCTCA CAGTCCCCAC CCTTTCAAAG 1440

TTNGACATAT GGGCCATCCC ATTGGGCCAG GAATTCCACA GGACACACCT AAGGCTGTGG 1500

GMAYTGGGGG ACAAATAGAT TTTCCATTTT GAGGAGGGCA CTTTCCCTGT TGTTCAGTTC 1560

TTGTTTTGAA GGGAGGTNGG 1580

(2) INFORMATION FOR SEQ ID NO: 97:

(i) SEQUENCE CHARACTERISTICS:
(A) LENGTH: 678 base pairs
(B) TYPE: nucleic acid
(C) STRANDEDNESS: double
(D) TOPOLOGY: linear

(xi) SEQUENCE DESCRIPTION: SEQ ID NO: 97:

ATATTTTTTT AGGCTAATGT CCAAGATACA GCATTGAGGA GGCAGCTATG TCTAATGAGG 60

GCTCTCTTGT TTGCTAGAGA TGAGAGAAAT GTATACTAAT CATTTTAATT TGTACTTAAA 120

ATACATTTTA CTAATCATAT TGATTTTAAA TATGACAAAT TCTTCTAGTA GATACTAATC 180

TTTCTTGTTT ATCATATTGT CCTAGAGAAG CCTAGGTAAA AATGGGTTCC ACCTAGTCTG 240

TTTGTATAAC ACCTTCCCCC GTCCCCTCTC CATCCCTGCC AATTGGGCTC TATGCATATT 300

GACAAGCAAA TAAGAAAACC TTAGGTTTCT TGTATTTGAA TTTCCAAAAC AATAAAAGGT 360

TTTGACTCAA GATTTGCATT CAAGAAGAGG CAGAAATTTT GTCTTATCTT TTTATCATTT 420

TGTGAACTTG TGTTTCTCTG TATGCTTAGA AAATTTTACA CACAAGGAAT GTTTGAAAAA 480

GTGAGAATTT TAGAGTGCTT GGGTGGTTTT TATTTGGTCA GTGCTGATGT GTTARGTGTT 540

TAGGGAAATA ATGCTTCAGG ACCTTTTTGA CAACACAGYT TCATGAATGA CYGGGGGATA 600

TTWAKGTTGT GCTGAGAAAA GGGAGGGAGT GGGCAGTTGG AATGGGGGAC CCTTACCATT 660

GGAAAACATG CATTCNGN 678

(2) INFORMATION FOR SEQ ID NO: 98:

(i) SEQUENCE CHARACTERISTICS:
(A) LENGTH: 1253 base pairs
(B) TYPE: nucleic acid
(C) STRANDEDNESS: double
(D) TOPOLOGY: linear

(xi) SEQUENCE DESCRIPTION: SEQ ID NO: 98:

ACCTCCCTCC CTCTCAGACT GGTCCGAATC CACGCCTAGC CCAGCCACTG CCACTGGGGC 60

CATGGCCACC ACCACTGGGG CACTGCCTGC CCAGCCACTT CCCTTGTCTG TTCCCAGCTC 120

CCTTGCTCAG GCCCAGACCC AGCTGGGGCC CCACCGGNAA GTTACCCCCA AGAGGCAAGT 180

NTTGGCCTGA GACGCTCGTC AGTTCTTAGA TCTTGGGGGC CTAAAGAGAC CCCCGTCCTG 240

CCTCCTTTCT TTCTCTGTCT CTTCCTTCCT TTTAGTCTTT TTCATCCTCT TCTCTTTCCA 300

CCAACCCTCC TGCATCCTTG CCTTGCAGCG TGACCGAGAT AGGTCATCAG CCCAGGGCTT 360

CAGTCTTCCT TTATTTATAA TGGGTGGGGG CTACCACCCA CCCTGCTGCA GTCTTGTGAA 420

GAGTCTGGGA CCTCCTTCTT CCCCACTTCT CTCTTCCCTC ATTCCTTTCT CTCTCCTTCT 480

GGCCTCTCAT TTCCTTACAC TCTGACATGA ATGAATTATT ATTATTTTTC TTTTTCTTTT 540

TTTTTTTACA TTTTGTATAG AAACAAATTC ATTTAAACAA ACTTATTATT ATTATTTTTT 600

ACAAAATATA TATATGGAGA TGCTCCCTCC CCCTGTGAAC CCCCCAGTGC CCCCGTGGGC 660

TGNAGTCTGT GGGCCCATTC GGCCAAGCTG GATTCTGTGT ACCTAGTACA CAGGCATGAC 720

TGGGATCCCG TGTACCGAGT ACACGACCCA GGTATGTACC AAGTAGGCAC CCTTGGGCGC 780

ACCCACTGGG GCCAGGGGTC GGGGGAGTGT TGGGAGCCTC CTCCCCACCC CACCTCCCTC 840

ACTTCACTGC ATTCCAGATT GGACATGTTC CATAGCCTTG CTGGGGAAGG GCCCACTGCC 900

AACTCCCTCT GCCCCAGCCC CACCCTTGGC CATCTCCCTT TGGGAACTAG GGGGCTGCTG 960

GTGGGAAATG GGAGCCAGGG CAGATGTATG CATTCCTTTA TGTCCCTGTA AATGTGGGAC 1020

TACAAGAAGA GGAGCTGCGT GAGTGGTACT TTCTCTTCCT GGTAATCCTC TGGCCCAGCC 1080

TTATGGCAGA ATAGAGGTAT TTTTAGGCTA TTTTTGTAAT ATGGCTTCTG GTCAAAATCC 1140

CTGTGTAGCT GAATTCCCAA GCCCTGCATT GTACAGCCCC CCACTCCCCT CACCACCTAA 1200

TAAAGGAATA GTTAACACTC AAAAAAAAAA AAAAAAAAAA ACTTGAGGGG GGG 1253

(2) INFORMATION FOR SEQ ID NO: 99:

(i) SEQUENCE CHARACTERISTICS:
(A) LENGTH: 447 base pairs
(B) TYPE: nucleic acid
(C) STRANDEDNESS: double
(D) TOPOLOGY: linear

(xi) SEQUENCE DESCRIPTION: SEQ ID NO: 99:

CAAAGAATGA AATTTACCAC TCTCCTCTTC TTGGCAGCTG TAGCAGGGGC CCTGGTCTAT 60

GCTGAAGATG CCTCCTCTGA CTCGACGGGT GCTGATCCTG CCCAGGAAGC TGGGACCTCT 120

AAGCCTAATG AAGAGATCTC AGGTCCAGCA GAACCAGCTT CACCCCCAGA GACAACCACA 180

ACAGCCCAGG AGAYTTCGGC GGCAGCAGTT CAGGGGACAG CCAAGGTCAC CTCAAGCAGG 240

CAGGAACTAA ACCCCCTGAA ATCCATAGTG GAGAAAAGTA TCTTACTAAC AGAACAAGCC 300

CTTGCAAAAG CAGGAAAAGG AATGCACGGA GGCGTGCCAG GTGGAAAACA ATTCATCGAA 360

AATGGAAGTG AATTTGCACA AAAATTACTG AAGAAATTCA GTCTATTAAA ACCATGGGCA 420

TGAGAAGCTG AAAAGAATKG GATCATT 447

(2) INFORMATION FOR SEQ ID NO: 100:

(i) SEQUENCE CHARACTERISTICS:
(A) LENGTH: 611 base pairs
(B) TYPE: nucleic acid
(C) STRANDEDNESS: double
(D) TOPOLOGY: linear

(xi) SEQUENCE DESCRIPTION: SEQ ID NO: 100:

GGTCTGGGGA GGTGACATGT TGGGCTGTGG GATCCCAGCG CTGGGCCTGC TCCTGCTGCT 60

GCAGGSWTCG GCAGACGGAA ATGGAATCCA GGGATTCTTC TACCCATGGA GCTGTGAGGG 120

TGACATATGG GACCGGGAGA GCTGTGGGGG CCAGGCGGCC ATTCGATAGC CCCAACYTCT 180

GCCTGCGTCT CCGGTGCTGC TACCGCAATG GGTCTGCTAC CACCAGCGTC CAGACGAAAA 240

CGTGCGGAGG AAGCACATGT GGGCGCTGGT CTGGACGTGC AGCGGCCTCC TCCTCCTGAG 300

CTGCAGCATC TGCTTGTTMT GGTGGGCCAA GCGCCGGGAC GTGCTGCATA TGCCCGGTTT 360

CCTGGCGGGT CCGTGTGACA TGTCCAAGTC CGTCTCGCTG CTCTCCAAGC ACCGAGGGAC 420

CAAGAAGACG CCGTCCACGG GCAGCGTGCC AGTCGCCCTG TCCAAAGAGT CCAGGGATGT 480

GGAGGGAGGC ACCGAGGGGG AAGGGACGGA GGAGGGTGAG GAGACAGAGG GCGAGGAAGA 540

GGAGGATTAG GGGAGTCCCC GGGGGACTGG TCAATACAGA TACGGTGGAC GGAAAAAAAA 600

AAAAAAAAAA A 611

(2) INFORMATION FOR SEQ ID NO: 101:

(i) SEQUENCE CHARACTERISTICS:
(A) LENGTH: 609 base pairs
(B) TYPE: nucleic acid
(C) STRANDEDNESS: double
(D) TOPOLOGY: linear

(xi) SEQUENCE DESCRIPTION: SEQ ID NO: 101:

GCATTGGTAA AGCTGGCAGT TGAAACCAGT TGGACGGCCC AGCTTGCGTC TCTTCTGCCT 60

GAGTGGGCCT CTCAGGTCAC TCGTGCCCTG CTGGAGGACA GAGGGGCACC TCAGCCGCCC 120

CCAAGCCCAG AGCACAGCAA TAAGGTCGGC CTGCAGGAGC CGGGGTGGGG GTGGGGGTGG 180

GGGGRGCAGG ACCCTRARAT GCCACCAGGA CCTGATGGGC CAGGAAGGGC GTGGACATGG 240

AGGCTGTTTT TACAGTTTTT TTTTTTTTGT TGTTTTGTTT TTAAAGAATA CAGAAGGAGC 300

CAAGCTTTTT TGCACTTTGT ATCCAGCTGC AAGCTCAGGG CAGAGTCAAG GGCCTGGGTT 360

GGAAAAACCT GACTCACAGG AATGCATAAT TGACCCTTGC AGCTACCCAA TAGCCCTTGG 420

AGCTGGCACT GAACCAGGCT GCAAGATTTG ACTGCCTTAA AAACACAAGG CCCTCTAGGC 480

CTGGCAGGGA TGTCCCTGTG CCCAGCACTG GGGGCTCGAA GACTGGTTTC TAGCACTACC 540

GGTCACGGCC ATGTCGTCCT AGAAGGGTCC AGAAGATTAT TTTACGTTGA GTCCATTTTT 600

AATGTTCTG 609

(2) INFORMATION FOR SEQ ID NO: 102:

(i) SEQUENCE CHARACTERISTICS:
(A) LENGTH: 1770 base pairs
(B) TYPE: nucleic acid
(C) STRANDEDNESS: double
(D) TOPOLOGY: linear

(xi) SEQUENCE DESCRIPTION: SEQ ID NO: 102:

ACGGYCCGGA ATCCCGGGTC GACCCACGCG TCCGGGAAAT TGAAACTGAG TGGCCCACGA 60

TGGGAAGAGG GGAAAGCCCA GGGGTACAGG AGGCCTCTGG GTGAAGGCAG AGGCTAACAT 120

GGGGTTCGGA GCGACCTTGG CCGTTGGCCT GACCATCTTT GTGCTGTCTG TCGTCACTAT 180

CATCATCTGC TTCACCTGCT CCTGCTGCTG CCTTTACAAG ACGTGCCGCC GACCACGTCC 240

GGTTGTCACC ACCACCACAT CCACCACTGT GGTGCATGCC CCTTATCCTC AGCCTCCAAG 300

TGTGCCGCCC AGCTACCCTG GACCAAGCTA CCAGGGCTAC CACACCATGC CGCCTCAGCC 360

AGGGATGCCA GCAGCACCCT ACCCAATGCA GTACCCACCA CCTTACCCAG CCCAGCCCAT 420

GGGCCCACCG GCCTACCACG AGACCCTGGC TGGAGGAGCA GCCGCGCCCT ACCCCGCCAG 480

CCAGCCTCCT TACAACCCGG SCTACATGGA TGCCCCGAAG SGGNCCTCTG AGCATTCCCT 540

GGCCTCTYTG GCTGCCACTT GGTTATGTTG TGTGTGTGCG TGARTGGTGT GCAGGCGCGG 600

TTCCTTACGC CCCATGTGTG CTGTGTGTGT CCTGCCTGTA TATGTGGCTT CCTCTGATGC 660

TGACAAGGTG GGGAACAATC CTTGCCAGAG TGGGCTGGGA CCAGACTTTG TTCTCTTCCT 720

CACCTGAAAT TATGCTTCCT AAAATCTCAA GCCAAACTCA AAGAATGGGG TGGTGGGGGG 780

CACCCTGTGA GGTGGCCCCT GAGAGGTGGG GGCCTCTCCA GGGCACATCT GGAGTTCTTC 840

TCCAGCTTAC CCTAGGGTGA CCAAGTAGGG CCTGTCACAC CAGGGTGGCG CAGCTTTCTG 900

TGTGATGCAG ATGTGTCCTG GTTTCGGCAG CGTAGCCAGC TGCTGCTTGA GGCCATGGCT 960

CGTCCCCGGA GTTGGGGGTA CCCGTTGCAG AGCCAGGGAC ATGATGCAGG CGAAGCTTGG 1020

GATCTGGCCA AGTTGGACTT TGATCCTTTG GGCAGATGTC CCATTGCTCC CTGGAGCCTG 1080

TCATGCCTGT TGGGGATCAG GCAGCCTCCT GATGCCAGAA CACCTCAGGC AGAGCCCTAC 1140

TCAGCTGTAC CTGTCTGCCT GGACTGTCCC CTGTCCCCGC ATCTCCCCTG GGACCAGCTG 1200

GAGGGCCACA TGCACACACA GCCTAGCTGC CCCCAGGGAG CTCTGCTGCC CTTGCTGGCC 1260

CTGCCCTTCC CACAGGTGAG CAGGGCTCCT GTCCACCAGC ACACTCAGTT CTCTTCCCTG 1320

CAGTGTTTTC ATTTTATTTT AGCCAAACAT TTTGCCTGTT TTCTGTTTCA AACATGATAG 1380

TTGATATGAG ACTGAAACCC CTGGGTTGTG GAGGGAAATT GGCTCAGAGA TGGACAACCT 1440

GGCAACTGTG AGTCCCTGCT TCCCGACACC AGCCTCATGG AATATGCAAC AACTCCTGTA 1500

CCCCAGTCCA CGGTGTTCTG GCAGCAGGGA CACCTGGGCC AATGGGCCAT CTGGACCAAA 1560

GGTGGGGTGT GGGGCCCTGG ATGGCAGCTC TGGCCCAGAC ATGAATACCT CGTGTTCCTC 1620

CTCCCTCTAT TACTGTTTCA CCAGAGCTGT CTTAGCTCAA ATCTGTTGTG TTTCTGAGTC 1680

TAGGGTCTGT ACACTTGTTT ATAATAAATG CAATCGTTTG GAAAAAAAAA AAAAAAAAAC 1740

TCGTAGGGGG GGCCCGTACC CAATSGCCTA 1770

(2) INFORMATION FOR SEQ ID NO: 103:

(i) SEQUENCE CHARACTERISTICS:
(A) LENGTH: 1832 base pairs
(B) TYPE: nucleic acid
(C) STRANDEDNESS: double
(D) TOPOLOGY: linear

(xi) SEQUENCE DESCRIPTION: SEQ ID NO: 103:

TGTGGCTGAC GTCATCTGGA GGAGATTTGC TTTCTTTTTC TCCAAAAGGG GAGGAAATTG 60

AAACTGCAGT GGCCCACGAT GGGAAGAGGG GAAAGCCCAG GGGTACAGGA GGCCTCTGGG 120

TGAAGGCAGA GGCTAACATG GGGTTCGGAG CGACCTTGGC CGTTGGCTGA CCATCTTTGT 180

GCTGTCTGTC GTCACTATCA TCATCTGCTT CACCTGCTCC TGCTGCTGCC TTTACAAGAC 240

GTGCCGCCGA CCACGTCCGG TTGTCACCAC CACCACATCC ACCACTGTGG TGCATGCCCC 300

TTATCCTCAG CCTCCAAGTG TGCCGCCCAG CTACCCTGGA CCAAGCTACC AGGGCTACCA 360

CACCATGCCG CCTCAGCCAG GGATGCCAGC AGCACCCTAC CCAATGCAGT ACCCACCACC 420

TTACCCAGCC CAGCCCATGG GCCCACCGGC CTACCACGAG ACCCTGGCTG GAGGAGCAGC 480

CGCGCCCTAM CCCGSCAGCC AGCCTCCTTA CAACCCGGCC TACATGGATG CCCGAAGCGG 540

CCCTCTGAGC ATTCCCTGGC CTCTYTGGCT GCCACTTGGT TATGTTGTGT GTGTGCGTRA 600

GTGGTGTGCA GGCGCGGTTC CTTACGCCCC ATGTGTGCTG TGTGTGTCCA GGCACGGTTC 660

CTTACGCCCC ATGTGTGCTG TGTGTGTCCT GCCTGTATAT GTGGCTTCCT CTGATGCTGA 720

CAAGTGGGGA ACAATCCTTG CCAGAGTGGG CTGGGACCAG ACTTTGTTCT CTTCCTCACC 780

TGAAATTATG CTTCCTAAAA TCTCAAGCCA AACTCAAAGA ATGGGGTGGT GGGGGGCACC 840

CTGTGAGGTG GCCCCTGAGA GGTGGGGGCC TCTCCAGGGC ACATCTGGAG TTCTTCTCCA 900

GCTTACCCTA GGGTGACCAA GTAGGGCCTG TCACACCAGG GTGGCGCAST TTCTGTGTGA 960

TGCAGATGTG TCCTGGTTTC GGCAGCGTAG CCAGCTGCTG CTTGAGGCCA TGGCTCGTCC 1020

CCGGAGTTGG GGGTACCCGT TGCAGAGCCA GGGACATGAT GCAGGCGAAG YTTGGGATCT 1080

GGCCAAGTTG GACTTTGATC CTTTGGGCAG ATGTCCCATT GCTCCCTGGA GCCTGTCATG 1140

CCTGTTGGGG ATCAGGCAGC CTCCTGATGC CAGAACACCT CAGGCAGAGC CCTACTCAGC 1200

TGTACCTGTC TGCCTGGACT GTCCCCTGTC CCCGCATCTC CCCTGGGACC AGCTGGAGGG 1260

CCACATGCAC ACACAGCCTA GCTGCCCCCA GGGAGCTCTG CTGCCCTTGC TGGCCCTGCC 1320

CTTCCCACAG GTGAGCAGGG CTCCTGTCCA CCAGCACACT CAGTTCTCTT CCCTGCAGTG 1380

TTTTCATTTT ATTTTAGCCA AACATTTTGC CTGTTTTCTG TTTCAAACAT GATAGTTGAT 1440

ATGAGACTGA AACCCCTGGG TTGTGGAGGG AAATTGGCTC AGAGATGGAC AACCTGGCAA 1500

CTGTGAGTCC CTGCTTCCCG ACACCAGCCT CATGGAATAT GCAACAACTC CTGTACCCCA 1560

GTCCACGGTG TTCTGGCAGC AGGGACACCT GGGCCAATGG GCCATCTGGA CCAAAGGTGG 1620

GGTGTGGGGC CCTGGATGGC AGCTCTGGCC CAGACATGAA TACCTCGTGT TCCTCCTCCC 1680

TCTATTACTG TTTCACCAGA GCTGTCTTAG CTCAAATCTG TTGTGTTTCT GAGTCTAGGG 1740

TCTGTACACT TGTTTATAAT AAATGCAATC GTTTNGGAAA AAAAANANAA AAAAAAAAGG 1800

GGSGGCGCTC TAAAAGGATN CCCCNAAGGG GG 1832

(2) INFORMATION FOR SEQ ID NO: 104:

(i) SEQUENCE CHARACTERISTICS:
(A) LENGTH: 2237 base pairs
(B) TYPE: nucleic acid
(C) STRANDEDNESS: double
(D) TOPOLOGY: linear

(xi) SEQUENCE DESCRIPTION: SEQ ID NO: 104:

AGTTCCCGGT ACTTTATTAC CAAGGTTGCC ATCGGAACCA GGAATGACAT TACTCACTAT 60

CAGAATTGAG AAAATTGGTT TGAAAGATGC TGGGCAGTGC ATCGATCCCT ATATTACAGT 120

TAGTGTAAAG GATCTGAATG GCATAGACTT AACTCCTGTG CAAGATACTC CTGTGGCTTC 180

AAGAAAAGAA GATACATATG TTCATTTTAA TGTGGACATT GAGCTCCAGA AGCATGTTGA 240

AAAATTAACC AAAGGTGCAG CTATCTTCTT TGAATTCAAA CACTACAAGC CTAAAAAAAG 300

GTTTACCAGC ACCAAGTGTT TTGCTTTCAT GGAGATGGAT GAAATTAAAC CTGGGCCAAT 360

TGTAATAGAA CTATACAAGA AACCCACTGA CTTTAAAAGA AAGAAATTGC AATTATTGAC 420

CAAGAAACCA CTTTATCTTC ATCTACATCA AACTTTGCAC AAGGAATGAT CCTGACATGA 480

TGAACCTGGA ACTTCTGTGA ATTTTACCAC TCAGTAGAAA CCATCATAGC TCTGTGTAGC 540

ATATTCACCC TTCAACAGGC AGGAAGCAAG CCGTACCCAG ACCAGTAGGC CGGACGGAGT 600

CAATNGCAAA GCTGTACCAC AGAATTCAGA GTCCAGCACA TCACACTGAC GTATAGGACT 660

CCTTGGGATA CAGGTTTATT GTAGATTTTG AAACATGTTT TTACTTTTCT ATTAATTGTG 720

CAATTAATAG TCTATTTTCT AATTTACCAC TACTCCTACC CTGCTTCCTG GAACAATACT 780

GTTGTGGGTA GGATGTGCTC ATCTTCAGAC TTAATACAGC AATAAGAATG TGCTAGAGTT 840

TACACATCTG TTCACTTTTG CTCCAATATG CTCTTTTGAC TTAACGTCAA GCTTTGGGTT 900

GATGTGGGTA GGGTAGTGTC AAACTGCTTT GAGAGGAATG GGACCAGTTC TGCTGCCTAA 960

GAAGGTCTGT CTGGATGTTT ATAGGCAGCA CCTCTGAAGT GGCCTAAATT CACCCTGATC 1020

TGATAGTTTT CCTGCTTAGA AAGTGTGCCT TGGCCAGATC AGTATCCCAC ATGGGAGTGT 1080

TCCCTAGGTT GTAGCTGTGA TTGTTTCCAG ATGACCAGAT TGTTTTTCTG AAAATGAGCA 1140

TATTTTTAGT CATGTCGATT AGCTGTTCTT CTACATCACA TTGTTACTCT TTCTGATGAT 1200

GATTCTAGGG TTAACATTGG AACCATCTCA AAATAATTAC AAAGTTTTAG ATGGGTTTAC 1260

AATGTCTTCT AAACAATGTA ATCTAAAAAT AATTGAGTCA GATGCTAACG AGATACTGCA 1320

GGCATAACTG CTGTTTTTCT GACAACTGAT TGTGAAACCT TAAAACCTGC ATACCTCTTC 1380

TTACAGTGAG GAGTATGCAA AATCTGGAAA GATATTCTAT TTTTTTTATA TAGGTAGATA 1440

GGATCGCCAT TTATTTCCTA TTTAGATATA CTGACATTCA TCCATATGAA AATATGCAGG 1500

TCATTAGCTT ACTATAATTT ACTTTTGACT TAATGGGGCA TAAATAAAAC TTTCATAGTA 1560

CACATGAGGT GGATATTTGA TACACAGAAC ATTTGCGGTG GGCTTTCTGT GGGTTAGATG 1620

TAAAGCCCAC ATATTTTAAT ATTCACTATT TTAAATGAGC AATGCATGAG GGGAATGCAG 1680

TGTCAGTACC TGGCCTATTT TTAAACTAGT GTAATCACCC TAGTCATACC ATTCAGTATG 1740

TTTGCTTTTT AAAATAAGTA ACCACAATTA AGTTGTTGTA GCCCTTGCAC TTCAAGAGAT 1800

CTAGTCTTTA CTTTCAGTTG TCTGTTAGGT CCATTCTGTT TACTAGACGG ATGTTAATAA 1860

AAACTATGCG AGCCTGAATG AATTCTCAGC CAAATTTAGT CTTGTCTCTC ATCTTGATTG 1920

GATTAATTCC AAATTCTAAA ATGATTCAGT CCACAATAGC TCTAGGGGAT GAAGAATTTG 1980

CCTTACTTTG CCCAGTTCCT AAGACTGTGA GTTGTCAAAT CCCTAGACTG TAAGCTCTTC 2040

AAGGAGCAAG AGGCGCATTT TCTCCGTGTC ATGTAATTTT TCTAAGGTGT TTGGCAGCAC 2100

TCTGTACCCT GTGGAGTACT CAGTACCTTT TGTTTGATGT TGCTGACAAG ACCTGAAAAA 2160

AAATCCCTTA AAAAAAAAAC CCATTAAAGT GTAGCAAAAC CGAAAAAAAA AAAANAAAAA 2220

ACTCGAGACG GGCCCGG 2237

(2) INFORMATION FOR SEQ ID NO: 105:

(i) SEQUENCE CHARACTERISTICS:
(A) LENGTH: 1822 base pairs
(B) TYPE: nucleic acid
(C) STRANDEDNESS: double
(D) TOPOLOGY: linear

(xi) SEQUENCE DESCRIPTION: SEQ ID NO: 105:

GGTCGACCCA CGCGTCCGGA ATTTTCGTAG CAATAAGTTT GTGCATGTAT AGTAATTTGC 60

ATTAGCAAGG TTGTAACCTC TGCCTCTTGG GTTCAAGTGA TTCTCGTGCC CCAGCCTCCC 120

GAGTAGCTGG GACTACAGGC ACGTGCCACC ACGCCCAGCT AATTTTTATA TTTTTAGTAG 180

AGACGGGGTT TTGCTGTGTT GGCCAGGCTG GTCTCAAACT CCTGACCTCA AGTAATCCAC 240

CTGGCCTGCT CTTTTCATGT CTTAACATGG CATGTCTTTT AGTTTCATTA TTTTCCTACT 300

CCTTGTATGT CAAGAAATTA CATTTTGCAT GTCTTATGGA GATGCTGTTA ATTGCTTCAG 360

TGAGTGCTTT TCTAATCTGC AGACCATTTA CATTTCCTGT TTGCAGCATG CTGTGTGCAA 420

ACACTCAGTA ATTTGGAGTA TTCAATTATT TGTTAGGGCT CTTCCTATTT CCAAATGTGC 480

TGAATTGTCT ATTGATGGGA TTTTCAGATC TTTTCATGAG AACTGGAAAT GTAGCTGGGT 540

GGCACCTACC TAGGTTGCTA CGTAGTGAGT AGACTTTCTC TTGGGTATAG TAAGCCTCAG 600

ACAGCTTTCA CTTTTATCTA CTTTACTTGT GGAAATAAAA CAGTCATTTT GTTCTGAAAG 660

AATAAGATAG CTTTCTGTAG AGAAGGAATT CCTACCTCTA AAAGCTGCCT TGAGAACTCA 720

GAACTGGCAG TTTTCTGAGG TGATTTTTAA ATTTCAGTAT TAGGGAGAGT CCAGCATTTG 780

CTGACACAGA TTCTACATAA CTAATGTATG ATAGCAAATG CAAAACTATT ATAATGTGGT 840

GTATCTTGCG CATACACAGG TTAGAACAAG TAGACTCTGG CAGCAGATCT CCAGAGACCC 900

AAGTTTAGGT TCTCATAGTG TATTTGAAGT AGTTATACTC CTGGCTTAAG TAGTTTAGTG 960

CCTGGGAGAA TCCATTACTG AAAAGCATTT AACTTAAAAA AAAAAAAAAA AAAAAAAAAA 1020

AAACCTCGTG CCGAATTCGG CACGAGCTAA CCCAGAAACA TCCAATTCTC AAACTGAAGC 1080

TCGCACTCTC GCCTCCAGCA TGAAAGTCTC TGCCGCCCTT CTGTGCCTGC TGCTCATAGC 1140

AGCCACCTTC ATTCCCCAAG GGCTCGCTCA GCCAGATGCA ATCAATGCCC CAGTCACCTG 1200

CTGYTATAAC TTCACCAATA GGAAGATCTC AGTGCAGAGG CTCGCGAGCT ATAGAAGAAT 1260

CACCAGCAGC AAGTGTCCCA AAGAAGCTGT GATCTTCAAG ACCATTGTGG CCAAGGAGAT 1320

CTGTGCTGAC CCCAAGCAGA AGTGGGTTCA GGATTCCATG GACCACCTGG ACAAGCAAAC 1380

CCAAACTCCG AAGACTTGAA CACTCACTCC ACAACCCAAG AATCTGCAGC TAACTTATTT 1440

TCCCCTAGCT TTCCCCAGAC ACCCTGTTTT ATTTTATTAT AATGAATTTT GTTTGTTGAT 1500

GTGAAACATT ATGCCTTAAG TAATGTTAAT TCTTATTTAA GTTATTGATG TTTTAAGTTT 1560

ATCTTTCATG GTACTAGTGT TTTTTAGATA CAGAGACTTG GGGAAATTGC TTTTCCTCTT 1620

GAACCACAGT TCTACCCCTG GGATGTTTTG AGGGTCTTTG CAAGAATCAT TAATACAAAG 1680

AATTTTTTTT AACATTCCAA TGCATTGCTA AAATATTATT GTGGAAATGA ATATTTTGTA 1740

ACTATTACAC CAAATAAATA TATTTTTGTA CAAAAAAAAA AAAAAAAAAA AAAAAAAAAA 1800

AAGSGGCCGC TCGAATTAAG CC 1822

(2) INFORMATION FOR SEQ ID NO: 106:

(i) SEQUENCE CHARACTERISTICS:
(A) LENGTH: 1712 base pairs
(B) TYPE: nucleic acid
(C) STRANDEDNESS: double
(D) TOPOLOGY: linear

(xi) SEQUENCE DESCRIPTION: SEQ ID NO: 106:

CGTGCCCCAG CCTCCCGAGT AGCTGGRACT ACAGGCACGT SCCACCACGC CCAGCTAATT 60

TTWATATTTT WAGTAGAGAC GGGGTTTTSC TGTKTTGGCC AGGCTGGTCT CAAACTCCTG 120

ACCTCAAGTA ATCCACCTGG CCTGCTCTTT TCATGTCTTA ACATGGCATG TCTTTTAGTT 180

TCATTATTTT CCTACTCCTT GTATGTCAAG AAATTACATT TTGCATGTCT TATGGAGATG 240

CTGTTAATTG CTTCAGTGAG TGCTTTTCTA ATCTGCAGAC CATTTACATT TCCTGTTTGC 300

AGCATGCTGT GTGCAAACAC TCAGTAATTT GGAGTATTCA ATTATTTGTT AGGGCTCTTC 360

CTATTTCCAA ATGTGCTGAA TTGTCTATTG ATGGGATTTT CAGATCTTTT CATGAGAACT 420

GGAAATGTAG CTGGGTGGCA CCTACCTAGG TTGCTACGTA GTGAGTAGAC TTTCTCTTGG 480

GTATAGTAAG CCTCAGACAG CTTTCACTTT TATCTACTTT ACTTGTGGAA ATAAAACAGT 540

CATTTTGTTC TGAAAGAATA AGATAGCTTT CTGTAGAGAA GGAATTCCTA CCTCTAAAAG 600

CTGCCTTGAG AACTCAGAAC TGGCAGTTTT CTGAGGTGAT TTTTAAATTT CAGTATTAGG 660

GAGAGTCCAG CATTTGCTGA CACAGATTCT ACATAACTAA TGTATGATAG CAAATGCAAA 720

ACTATTATAA TGTGGTGTAT CTTGCGCATA CACAGGTTAG AACAAGTAGA CTCTGGCAGC 780

AGATCTCCAG AGACCCAAGT TTAGGTTCTC ATAGTGTATT TGAAGTAGTT ATACTCCTGG 840

CTTAAGTAGT TTAGTGCCTG GGAGAATCCA TTACTGAAAA GCATTTAACT TAAAAAAAAA 900

AAAAAAAAAA AAAAAAAAAC CTCGTGCCGA ATTCGGCACG AGCAGAAACA TCCAATTCTC 960

AAACTGAAGC TCGCACTCTC GCCTCCAGCA TGAAAGTCTC TGCCGCCCTT CTGTGCCTGC 1020

TGCTCATAGC AGCCACCTTC ATTCCCCAAG GGCTCGCTCA GCCAGATGCA ATCAATGCCC 1080

CAGTCACCTG CTGYTATAAC TTCACCAATA GGAAGATCTC AGTGCAGAGG CTCGCGAGCT 1140

ATAGAAGAAT CACCAGCAGC AAGTGTCCCA AAGAAGCTGT GATCTTCAAG ACCATTGTGG 1200

CCAAGGAGAT CTGTGCTGAC CCCAAGCAGA AGTGGGTTCA GGATTCCATG GACCACCTGG 1260

ACAAGCAAAC CCAAACTCCG AAGACTTGAA CACTCACTCC ACAACCCAAG AATCTGCAGC 1320

TAACTTATTT TCCCCTAGCT TTCCCCAGAC ACCCTGTTTT ATTTTATTAT AATGAATTTT 1380

GTTTGTTGAT GTGAAACATT ATGCCTTAAG TAATGTTAAT TCTTATTTAA GTTATTGATG 1440

TTTTAAGTTT ATCTTTCATG GTACTAGTGT TTTTTAGATA CAGAGACTTG GGGAAATTGC 1500

TTTTCCTCTT GAACCACAGT TCTACCCCTG GGATGTTTTG AGGGTCTTTG CAAGAATCAT 1560

TAATACAAAG AATTTTTTTT AACATTCCAA TGCATTGCTA AAATATTATT GTGGAAATGA 1620

ATATTTTGTA ACTATTACAC CAAATAAATA TATTTTTGTA CAAAAAAAAA AAAAAAAAAA 1680

AAAAAAAAAA AAGSGGCCGC TCGAATTAAG CC 1712

(2) INFORMATION FOR SEQ ID NO: 107:

(i) SEQUENCE CHARACTERISTICS:
(A) LENGTH: 1969 base pairs
(B) TYPE: nucleic acid
(C) STRANDEDNESS: double
(D) TOPOLOGY: linear

(xi) SEQUENCE DESCRIPTION: SEQ ID NO: 107:

CCCCTCCTTC CCCTYGCCAC CTACTGAACC CTCCTCCGAG GTGCCCGAGC AGCCGTCTGC 60

CCAGCCACTC CCTGGGAGTC CCCCCAGAAG AGCCTATTAC ATCTACTCCG GGGGCGAGAA 120

GATCCCCCTG GTGTTGAGCC GGCCCCTCTC CTCCAACGTG GCCACTCTTC AGCATCTCTG 180

TCGGAAGACC GTCAACGGCC ACCTGGACTC CTATGAGAAA GTCACCCAGC TGCCGGGGCC 240

CATTCGGRAG TTCCTGGACC AGTACGATGC CCCGMTTTAA GGGGTAAAGG GCGCAAAGGG 300

CATGGGTCGG GAGAGGGGAC GCAGGCCCCT CTCCTCCGTG GCACATGGCA CAAGCACAAG 360

AAGCCAACCA GGAGAGAGTC CTGTAGCTCT GGGGGGAAAG AGGGCGGACA GGCCCCTCCC 420

TCTGCCCTCT CCCTGCAGAA TGTGGCAGGC GGACCTGGAA TGTGTTGGAG GGAAGGGGGA 480

GTACCACCTG AGTCTCCAGC TTCTCCGGAG ACCCAGCTGT CCTGGTGGGA CGATAGCAAC 540

CACAAGTGGA TTCTCCTTCA ATTCCTCAGC TTCCCCTCTG CCTCCAAACA GGGGACACTT 600

CGGGAATGCT GAAYTAATGA GAACTGCCAG GGAATCTTCA AACTTTCCAA CGGAACTTGT 660

TTGCTCTTTG ATTTGGTTTA AACCTGAGCT GGTTGTGGAG CCTGGGAAAG GTGGAAGAGA 720

GAGAGGTCCT GAGGGCCCCA GGGSTGCGGG CTGGCGAAGG AAATGGTCAC ACCCCCCGCC 780

CACCCCAGGC GAGGATCCTG GTGACATGCT CCTCTCCCTG GCTCCGGGGA GAAGGGCTTG 840

GGGTGACCTG AAGGGAACCA TCCTGGTGCC CCACATCCTC TCCTCCGGGN ACAGTCACCG 900

AAAACACAGG TTCCAAAGTC TACCTGGTGC CTGAGAGCCC AGGGCCCTTC CTCCGTTTTA 960

AGGGGGAAGC AACATTTGGA GGGGACGGAT GGGCTGGTCA GCTGGTCTCC TTTTCCTACT 1020

CATACTATAC CTTCCTGTAC CTGGGTGGAT GGAGCGGGAG GATGGAGGAG ACGGGACATC 1080

TTTCACCTCA GGCTCCTGGT AGAGAAGACA GGGGATTCTA CTCTGTGCCT CCTGACTATG 1140

TCTGGCTAAG AGATTCGCCT TAAATGCTCC CTGTCCCATG GAGAGGGACC CAGCATAGGA 1200

AAGCCACATA CTCAGCCTGG ATGGGTGGAG AGGCTGAGGG ACTCACTGGA GGGCACCAAG 1260

CCAGCCCACA GCCAGGGAAG TGGGGAGGGG GGGCGGAAAC CCATGCCTCC CAGCTGAGCA 1320

CTGGGAATGT CAGCCCAGTA AGTATTGGCC AGTCAGGCGC CTCGTGGTCA GAGCAGAGCC 1380

ACCAGGTCCC ACTGCCCCGA GCCCTGCACA GCCCTCCCTC CTGCCTGGGT GGGGGAGGCT 1440

GGAGGTCATT GGAGAGGCTG GACTGCTGCC ACCCCGGGTG CTCCCGCTCT GCCATAGCAC 1500

TGATCAGTGA CAATTTACAG GAATGTAGCA GCGATGGAAT TACCTGGAAC ATTTTTTGTT 1560

TTTGTTTTTG TTTTTGTTTT TGTGGGGGGG GGCAACTAAA CAAACACAAA GTATTCTGTG 1620

TCAGGTATTG GGCTGGACAG GGCAGTTGTG TGTTGGGGTG GTTTTTTTCT CTATTTTTTT 1680

GTTTGTTTCT TGTTTTTTAA TAATGTTTAC AATCTGCCTC AATCACTCTG TCTTTTATAA 1740

AGATTCCACC TCCAGTCCTC TCTCCTCCCC CCTACTCAGG CCCTTGAGGC TATTAGGAGA 1800

TGCTTGAAGA ACTCAACAAA ATCCCAATCC AAGTCAAACT TTGCACATAT TTATATTTAT 1860

ATTCAGAAAA GAAACATTTC AGTAATTTAT AATAAAGAGC ACTATTTTTT AATGAAAAAA 1920

AAAAAAAAAA AAAAAAAAAA CGACGCTGGT GACCGGAATY CGACGTACG 1969

(2) INFORMATION FOR SEQ ID NO: 108:

(i) SEQUENCE CHARACTERISTICS:
(A) LENGTH: 1734 base pairs
(B) TYPE: nucleic acid
(C) STRANDEDNESS: double
(D) TOPOLOGY: linear

(xi) SEQUENCE DESCRIPTION: SEQ ID NO: 108:

CGGGTCCCAA GCCTGTGCCT GAGCCTGAGC CTGAGCCTGA GCCCGAGCCG GGAGCCGGTC 60

GCGGGGGCTC CGGGCTGTGG GACCGCTGGG CCCCCAGCGA TGGCGACCCT GTGGGGAGGC 120

CTTCTTCGGC TTGGCTCCTT GCTCAGCCTG TCGTGCCTGG CGCTTTCCGT GCTGCTGCTG 180

GCGCATGTNC AGACGCCGCC AAGAATTTCG AGGATGTCAG ATGTAAATGT ATCTGCCCTC 240

CCTATAAAGA AAATTCTGGG CATATTTATA ATAAGAACAT ATCTCAGAAA GATTGTGATT 300

GCCTTCATGT TGTGGAGCCC ATGCCTGTGC GGGGGCCTGA TGTAGAAGCA TACTGTCTAC 360

GCTGTGAATG CAAATATGAA GAAAGAAGCT CTGTCACAAT CAAGGTTACC ATTATAATTT 420

ATCTCTCCAT TTTGGGCCTT CTACTTCTGT ACATGGTATA TCTTACTCTG GTTGAGCCCA 480

TACTGAAGAG GCGCCTCTTT GGACATGCAC AGTTGATACA GAGTGATGAT GATATTGGGG 540

ATCACCAGCC TTTTGCAAAT GCACACGATG TGCTAGCCCG CTCCCGCAGT CGAGCCAACG 600

TGCTGAACAA GGTAGAATAT GCACAGCAGC GCTGGAAGCT TCAAGTCCAA GAGCAGCGAA 660

AGTCTGTCTT TGACCGGCAT GTTGTCCTCA GCTAATTGGG GAATTGAATT CAAGGTGACT 720

AGAAAGAAAC AGGCAGACAA CTGGGAAAGA ACTGACTGGG NTTTTGCTGG GTTTCATTTT 780

AATACCTTGT TGATTTCACC AACTGTTGCT GGAAGATTCA AAACTGGAAG CAAAAACTTG 840

CTTGATTTTT TTTTCTTGTT AACGTAATAA TAGAGACATT TTTAAAAGCA CACAGCTCAA 900

AGTCAGCCAA TAAGTCTTTT CCTATTTGTG ACTTTTACTA ATAAAAATAA ATCTGCCTGT 960

AAATTATCTT GAAGTCCTTT ACCTGGAACA AGCACTCTCT TTTTCACCAC ATAGTTTTAA 1020

CTTGACTTTC AAGATAATTT TCAGGGTTTT TGTTGTTGTT GTTTTTTGTT TGTTTGTTTT 1080

GGTGGGAGAG GGGAGGGATG CCTGGGAAGT GGTTAACAAC TTTTTTCAAG TCACTTTACT 1140

AAACAAACTT TTGTAAATAG ACCTTACCTT CTATTTTCGA GTTTCATTTA TATTTTGCAG 1200

TGTAGCCAGC CTCATCAAAG AGCTGACTTA CTCATTTGAC TTTTGCACTG ACTGTATTAT 1260

CTGGGTATCT GCTGTGTCTG CACTTCATGG TAAACGGGAT CTAAAATGCC TGGTGGCTTT 1320

TCACAAAAAG CAGATTTTCT TCATGTACTG TGATGTCTGA TGCAATGCAT CCTAGAACAA 1380

ACTGGCCATT TGCTAGTTTA CTCTAAAGAC TAAACATAGT CTTGGTGTGT GTGGTCTTAC 1440

TCATCTTCTA GTACCTTTAA GGACAAATCC TAAGGACTTG GACACTTGCA ATAAAGAAAT 1500

TTTATTTTAA ACCCAAGCCT CCCTGGATTG ATAATATATA CACATTTGTC AGCATTTCCG 1560

GTCGTGGTGA GAGGCAGCTG TTTGAGCTCC AATGTGTGCA GCTTTGAACT AGGGCTGGGG 1620

TTGTGGGTGC CTCTTCTGAA AGGTCTAACC ATTATTGGAT AACTGGCTTT TTTCTTCCTC 1680

TTTGGAATGT AACAATAAAA ATAATTTTTG AAACATCAAA AAAAAAAAAA AAAA 1734

(2) INFORMATION FOR SEQ ID NO: 109:

(i) SEQUENCE CHARACTERISTICS:
(A) LENGTH: 2003 base pairs
(B) TYPE: nucleic acid
(C) STRANDEDNESS: double
(D) TOPOLOGY: linear

(xi) SEQUENCE DESCRIPTION: SEQ ID NO: 109:
   CGCAGGGGGC GCGCGGCCCG GGGACTCGCA TTCCCCGGTT CCCCCTCCAC CCCACGCGGC 60
   CTGGACCATG GACGCCAGAT GGTGGGCAGT GGTGGTGCTG GCTGCGTTCC CCTCCCTAGG 120
   GGCAGGTGGG GAGACTCCCG AAGCCCCTCC GGAGTCATGG ACCCAGCTAT GGTTCTTCCG 180
   ATTTGTGGTG AATGCTGCTG GCTATGCCAG NTTTATGGTA CCTGGCTACC TCCTGGTGCA 240
   GTACTTCAGG CGGAAGAACT ACCTGGAGAC CGGTAGGGGC CTCTGCTTTC CCCTGGTGAA 300
   AGCTTGTGTG TTTGGCAATG AGCCCAAGGC CTCTGATGAG GTTCCCCTGG CGCCCCGAAC 360
   AGAGGCGGCA GAGACCACCC CGATGTGGCA GGCCCTGAAG CTGCTCTTCT GTGCCACAGG 420
   GCTCCAGGTG TCTTATCTGA CTTGGGGTGT GCTGCAGGAA AGAGTGATGA CCCGCAGCTA 480
   TGGGGCCACA GCCACATCAC CGGGTGAGCG CTTTACGGAC TCGCAGTTCC TGGTGCTAAT 540
   GAACCGAGTG CTGGCACTGA TTGTGGCTGG CCTCTCCTGT GTTCTCTGCA AGCAGCCCCG 600
   GCATGGGGCA CCCATGTACC GGTACTCCTT TGCCAGCCTG TCCAATGTGC TTAGCAGCTG 660
   GTGCCAATAC GAAGCTCTTA AGTTCGTCAG CTTCCCCACC CAGGTGCTGG CCAAGGCCTC 720
   TAAGGTGATC CCTGTCATGC TGATGGGAAA GCTTGTGTCT CGGCGCANTA ACGAACACTG 780
   GGAGTACCTG ACAGCCACCC TCATCTCCAT TGGGGTCAGC ATGTTTCTGC TATCCAGCGG 840
   ACCAGAGCCC CGCAGCTCCC CAGCCACCAC ACTCTCAGGC CTCATCTTAC TGGCAGGTTA 900
   TATTGCTTTT GACAGCTTCA CCTCAAACTG GCAGGATGCC TGTTTGCCTA TAAGATGTCA 960
   TCGGTGCAGA TGATGTTTGG GGTCAATTTC TTCTCCTGCC TCTTCACAGT GGGSTCACTG 1020
   CTAGNAACAG GGGGGMCCTA CTGGAGGGAA CCCGCTTCAT GGGGCGACAC AGTGAGTTTG 1080
   CTGCCCATGC CCTGCTACTC TCCATCTGCT CCGCATGTGG CCAGCTCTTC ATCTTTTACA 1140
   CCATTGGGCA GTTTGGGGCT GCCGTCTTCA CCATCATCAT GACCCTCCGC CAGGCCTTTG 1200
   CCATCCTTCT TTCCTGCCTT CTCTATGGCC ACACTGTCAC TGTGGTGGGA GGGCTGGGGG 1260
   TGGCTGTGGT CTTTGCTGCC CTCCTGCTCA GAGTCTACGC GCGGGGCCGT CTAAAGCAAC 1320
   GGGGAAAGAA GGCTGTGCCT GTTGAGTCTC CTGTGCAGAA GGTTTGAGGG TGGAAAGGGC 1380
   CTGAGGGGTG AAGTGAAATA GGACCCTCCC ACCATCCCCT TCTGCTGTAA CCTCTGAGGG 1440
   AGCTGGCTGA AAGGGCAAAA TGCAGGTGTT TTCTCAGTAT CACAGACCAG CTCTGCAGCA 1500
   GGGGATTGGG GAGCCCAGGA GGCAGCCTTC CCTTTTGCCT TAAGTCACCC ATCTTCCAGT 1560
   AAGCAGTTTA TTCTGAGCCC CGGGGGTAGA CAGTCCTCAG TGAGGGGTTT TGGGGAGTTT 1620
   GGGGTCAAGA GAGCATAGGT AGGTTCCACA GTTACTCTTC CCACAAGTTC CCTTAAGTCT 1680
   TGCCCTAGCT GTGCTCTGCC ACCTTCCAGA CTCACTCCCC TCTGCAAATA CCTGCATTTC 1740
   TTACCCTGGT GAGAAAAGCA CAAGCGGTGT AGGCTCCAAT GCTGCTTTCC CAGGAGGGTG 1800
   AAGATGGTGC TGTGCTGAGG AAAGGGGATG CAGAGCCCTG CCCAGCACCA CCACCTCCTA 1860
   TGCTCCTGGA TCCCTAGGCT CTGTTCCATG AGCCTGTTGC AGGTTTTGGT ACTTTAGAAA 1920
   TGTAACTTTT TGCTCTTATA ATTTTATTTT ATTAAATTAA ATTACTGCAA AAAAAAAAAA 1980
   AAAAAAATCG GGGGGGGCCC CGN 2003

(2) INFORMATION FOR SEQ ID NO: 110:

(i) SEQUENCE CHARACTERISTICS:
(A) LENGTH: 1320 base pairs
(B) TYPE: nucleic acid
(C) STRANDEDNESS: double
(D) TOPOLOGY: linear

(xi) SEQUENCE DESCRIPTION: SEQ ID NO: 110:

GCTGAGCTGC CTTGAGGTGC AGTGTTGGGG ATCCAGAGCC ATGTCGGACC TGCTACTACT 60

GGGCCTGATT GGGGGCCTGA CTCTCTTACT GCTGCTGACG CTGCTGGCCT TTGCCGGGTA 120

CTCAGGGCTA CTGGCTGGGG TGGAAGTGAG TGCTGGGTCA CCCCCCATCC GCAACGTCAC 180

TGTGGCCTAC AAGTTCCACA TGGGGCTCTA TGGTGAGACT GGGCGGCTTT TCACTGAGAG 240

CTGCAGCATC TCTCCCAAGC TCCGCTCCAT CGCTGTCTAC TATGACAACC CCCACATGGT 300

GCCCCCTGAT AAGTGCCGAT GTGCCGTGGG CAGCATCCTG AGTGAAGGTG AGGAATCGCC 360

CTCCCCTGAG CTCATCGACC TCTACCAGAA ATTTGGCTTC AAGGTGTTCT CCTTCCCGGC 420

ACCCAGCCAT GTGGTGACAG CCACCTTCCC CTACACCACC ATTCTGTCCA TCTGGCTGGC 480

TACCCGCCGT GTCCATCCTG CCTTGGACAC CTACATCAAG GAGCGGAAGC TGTGTGCCTA 540

TCCTCGGCTG GAGATCTACC AGGAAGACCA GATCCATTTC ATGTGCCCAC TGGCASGGCA 600

GGGAGACTTC TATGTGCCTG AGATGAAGGA GACAGAGTGG AAATGGCGGG GGCTTGTGGA 660

GGCCATTGAC ACCCAGGTGG ATGGCACAGG AGCTGACACA ATGAGTGACA CGAGTTCTGT 720

AAGCTTGGAA GTGAGCCCTG GCAGCCGGGA GACTTCAGCT GCCACACTGT CACCTGGGGC 780

GAGCAGCCGT GGCTGGGATG ACGGTGACAC CCGCAGCGAG CACAGCTACA GCGAGTCAGG 840

TGCCAGCGGC TCCTCTTTTG AGGAGCTGGA YTTGGAGGGC GAGGGGCCCT TAGGGGAGTC 900

ACGGCTGGAC CCTGGGACTK AGCCCCTGGG GACTACCAAG TGGCTCTGGG AGCCCACTGC 960

CCCTGAGAAG GGCAAGGAGT AACCCATGGC CTGCACCCTC CCTGCAGTGC AGTTGCTGAG 1020

GAACTGAGCA GACTCTCCAG CAGACTCTCC AGCCCTCTTC CTCCTTCCTC TGGGGGAGGA 1080

GGGGTTCCTG AGGGACCTGA CTTCCCCTGC TCCAGGCCTC TTGCTAAGCC TTCTCCTCAC 1140

TGCCCTTTAG GCTCCCAGGG CCAGAGGAGC CAGGGACTAT TTTCTGCAAC CAGCCCCCAG 1200

GGCTGCCNCC CCTGTTGTGT CTTTTTTTCA GACTCACAGT GGAGCTTCCA GGACCCAGAA 1260

TAAAGCCAAT GATTTACTTG TTTCAAAAAA AAAAWAAAAA AAAAAAAAAA AAAAAAAAAA 1320

(2) INFORMATION FOR SEQ ID NO: 111:

(i) SEQUENCE CHARACTERISTICS:
(A) LENGTH: 1962 base pairs
(B) TYPE: nucleic acid
(C) STRANDEDNESS: double
(D) TOPOLOGY: linear

(xi) SEQUENCE DESCRIPTION: SEQ ID NO: 111:

CGGACCCCTT CCTCCTCCTC NAAGCATGTC CCACCATTGT GGCAGGGGCT GGGGANACAG 60

TCACCTGATG CGGGGACCAC GGCCACTCCA CCTCGSTGGC GCTGTCAGTG GGCAGCACTG 120

GCTGGGCCTG CACTGAGGTC CCTGCTGGGG CAGTTCTTCC AGAATTATCT TCAGAGGGGG 180

CCTCCAGCTC CCTGGTACCC TCAGGGGCCC GTGTGGCTGG AAGCAGGGAA GGGGCACCCT 240

CGGAGCTTCC TGTCTCCTCG CTCTCTCCTC GAGGGACCCC AGATAGCTCA GGACCACCAG 300

TTGCCTCCCC CACCTCTCTT GCCTCAACCA GAGTGGAAGG TGATGGGGAT GCTAGGTTCC 360

TCTCCCTGGG AGTGGGCAGA GTCTCAGTAG GTGGTCCATG GACCCTTGGA GGCCTGGAAG 420

CTTCTGACTC TCCATCAGGA AGTGGTGATG CACCAGGCTG CAGGACTGCC CTTGCTGGCG 480

CCTGGGAGAG TGACTCCTCC TGGGCTGCTG GCTCAGTGGG GAGAGAGGCC TCAGGGCCCG 540

GGCTGCTGAG CTCGCTGGGC CATGCCCACA GAGCCTCATC CTCCACCTCC TCCTCTTCTT 600

CTTCCTCCTC TTTCTCTTCT TCATCTTCAT ATTTCTCTTC TTCCTCCAAT GCCTTACCTT 660

CCTCTTYTGR AAACCCCGTG GGCGGTACCA TGGATTGTGT TTCAAATTCT AGGAGCGTCC 720

TAGGGGCCTC TGCTGGGTCT TCTGGAGTGG AGCTTCCACC TCCTCCGTCC TCCATGATGG 780

GGATGGAGTA RATGGCCCCA CGGGATTCAC TCTCTGTGGC TTCCTGAGGC AGCTGCAGTT 840

CCTCCAGGGT CTCTGTCACT GTGACRATAG CCTCTAGTCC ATCAAAAGCT GGGTTGGAGG 900

CTGGGTTGGA GGCCTCAGGG ATGGCAGAAG GCTGGGCCGA GTCTCGGAAG CAGTARACGT 960

TGAAGCGGCT GTGCTTATTG GGGAAGCCAG TCTGGTTGGG GAAGANGAAG AGAGTCTTGA 1020

CACCAGGCAA GCCCCCACCA CAGCGCTGGC TGGGTGTGAC GATGGGGTAG CGCACANTGC 1080

CATCAGCTAG CCACCTGGGC TGCAGTGGTC CAGGCCACCA TCCCAGGCTG CATACAGTTG 1140

GCCCGTGGTG GCAATCTCTG CACCCCGCTC CTGGCAGTAC GCCCGTGCTT CCTCCAATGT 1200

CAGCTTCTCT GGAGGGTCAC CCAGGAACAG TTCTCCATTT AGGTCTTCAG CATAACAGTA 1260

CACATCATAG AGGTCATCCG GGTCCACCAC ACCATAGTTC CGGACCCCGG GGAAGCCATC 1320

CATGTCTCCG TAACAGGCCT CTCGTGGGGT CTGGATGGGA TACCTTTGAC CTTGAMCTCC 1380

ACAGCGTCGC TGCTGTCATC GATGCCGTGC TGGACCTCAC AGCGATAGAT ACCTGAGTCG 1440

TTGGGGCGCA GCTCGCTCAG CGCCAGGGGA GACGTCGGTG AGCGACGCTG GGTACGCAGG 1500

CAGTGCCACG CGGAACCGGT AGGCCTCGTT CACCTTGACG CGCACTCCCC GCGCCACCAG 1560

CACYTCTGCC TCCCGGCCCC GGGACAGGAA AGTCCACTTG ACCCGCGGAG AGCCCAGCAC 1620

AGCCCGGCGG CTCGGCGGTG SCCGCAGGTA GTGGACGTGG CAAGGGATGK TGAGGGCSCC 1680

GCCGAGCAAC GCCYTGCAGT GGCGCGTCGC CCGCGATGCG CACGCGAAAA GCGCGKTCCT 1740

CTGAGCTGTC TCCTTCCAGA ACATCTGCTA AAGCTGCAGG AGCCTGGGCC AGGACCAGGG 1800

CTGCCAGCAG GGGCAGGAAC AGCTGGGCCA TGCTGCAGGC TACCCAGGGC TGGGGTTGGG 1860

TCGCGGCACT GCGAAGTTTG TCGCCTCCTC CGGGGGTCTC CTCCGGGTKC ACGGCTCAGT 1920

NCCTGCAGCT GCAGCTGAGA CTGCGGCGGA GACTGCGCGA GC 1962

(2) INFORMATION FOR SEQ ID NO: 112:

(i) SEQUENCE CHARACTERISTICS:
(A) LENGTH: 1785 base pairs
(B) TYPE: nucleic acid
(C) STRANDEDNESS: double
(D) TOPOLOGY: linear

(xi) SEQUENCE DESCRIPTION: SEQ ID NO: 112:

AAGTTTCAGC CAAACTTCGG GCGGCTGAGG CGGCGGCCGA GGAGCGGCGG ACTCSGGGCG 60

CGGGGAGTCG AGGCATTTGC GCCTGGGCTT CGGAGCGTAC CGCAGGGCCT GAGCCTTTGA 120

AGCAGGAGGA GGGGAGGAGA GAGTGGGGCT CCTCTATCGG GACCCCCTCC CCATGTGGAT 180

CTGCCCAGGC GGCGGCGGCG GCCGAGGAGG CGACCGAGAA GATRCCCGCC CTGCGCCCCG 240

CTCTGCTGTG GGCGCTGCTG GCGCTCTGGC TGTGCTGCGC GACCCCGCGC ATGCATTGCA 300

GTGTCGAGAT GGCTATGAAC CCTGTGTAAA TGAAGGAATG TGTGTTACCT ACCACAATGG 360

CACAGGATAC TGCAAATGTC CAGAAGGCTT CTTGGGGGAA TATTGTCAAC ATCGAGACCC 420

CTGTGAGAAG AACCGCTGCC AGAATGGTGG GACTTGTGTG GCCCAGGCCA TGCTGGGGAA 480

AGCCACGTGC CGATGTGCCT CAGGGTTTAC AGGAGAGGAC TGCCAGTACT CGACATCTCA 540

TCCATGCTTT GTGTCTCGAC CTTGCCTGAA TGGCGGCACA TGCCATATGC TCAGCCGGGA 600

TACCTATGAG TGCACCTGTC AAGTCGGGTT TACAGGTAAG GAGTGCCAAT GGACCGATGC 660

CTGCCTGTCT CATCCCTGTG CAAATGGAAG TACCTGTACC ACTGTGGCCA ACCAGTTCTC 720

CTGCAAATGC CTCACAGGCT TCACAGGGCA GAAGTGTGAG ACTGATGTCA ATGAGTGTGA 780

CATTCCAGGA CACTGCCAGC ATGGTGGCAC CTGCCTCAAC CTGCCTGGTT CCTACCAGTG 840

CCAGTGCCTT CAGGGCTTCA CAGGCCAGTA CTGTGACAGC CTGTATGTGC CCTGTGCACC 900

CTCGCCTTGT GTCAATGGAG GCANCTGTCG GCAGACTGGT GACTTCACTT TTGAGTGCAA 960

CTGCCTTCCA GAAACAGTGA GAAGAGGAAC AGAGCTCTGG GAAAGAGACA GGGAAGTCTG 1020

GAATGGAAAA GAACACGATG AGAATTAGAC ACTGGAAAAT ATGTATGTGT GGTTAATAAA 1080

GTGCTTTAAA CTGAATTGAC ATTAACAGTR GGTGATCAAC TTTMCTATGT GCTTGTGCTT 1140

TTGCTTTTGA TGGAGTAATT CATTGTTTTC TTATCCACCT AAATGCACCC AGCTGCCCTT 1200

GATTTTCTCT GGGCTACTGG CCTTCACAAC CCTCTCCCAT GTACCCTCTC TGACTTTGGG 1260

GTAACCCTCC CCTAACTTAA AGCTAGAGAA TTCTGAAACT GAGGAGGGGA TCCTCTGTTA 1320

ATCAGTGAGC ACTTTTTGAT GAGCTGATAG ATGATATATG AGAGACTATG CGTGGCACAA 1380

TACTTTGTTA CACTCTTCAC TGATACAAGT GTTCTAGAGT GYACACACAA CCCAAAGATA 1440

GAAATAAAAA GAGGAGCAGT GTCGGGGAGC TTGGGGCCTG GTGTTCCATG GAGAGGGAGA 1500

AAGGAACAAG CTTGRCCAAT TCATTCAACT CCTTATAAAA ATGATGAGGA GGCTGAAAAC 1560

CAAGAATTTT GATTGGGAAC AGAATACAAG CAGCTGAAKC AGATGAWTTA CTAAGCAACA 1620

AAGATCCTGT TTTTATACAA ATATCCTTAG TACAAAAACA AAARAAGGAA AACTGTAGGG 1680

GGGAGTAATG TGCTAAGTAA GCAGAATTGC CTCCAAAAGA AGTTGTTTCT AGTTACTCTT 1740

TTCCGGGTNG GGATCTTTAG NTTCCGGTAT TGTGGGTATG GTTCC 1785

(2) INFORMATION FOR SEQ ID NO: 113:

(i) SEQUENCE CHARACTERISTICS:
(A) LENGTH: 1842 base pairs
(B) TYPE: nucleic acid
(C) STRANDEDNESS: double
(D) TOPOLOGY: linear

(xi) SEQUENCE DESCRIPTION: SEQ ID NO: 113:

GGAGCCTCTC TTGCAACTTC TGCCACCGCG GGCCACCGCG GCCGCCTGAT CCCGCAGAGG 60

AAGTCGCGGC CGTGGAGCGA TGACCCGCGG CGGTCCGGGC GGGCGCCCGG GGCTGCCACA 120

GCCGCCGCCG CTTCTGCTGC TGCTGCTGCT GCMGCTGTTG TTAGTCACCG CGGAGCCGCC 180

GAAACCTGCA GGAGTCTACT ATGCAACTGC ATACTGGATG CCTGCTGAAA AGACAGTACA 240

AGTCAAAAAT GTAATGGACA AGAATGGGGA CGCCTATGGC TTTTACAATA ACTCTGTGAA 300

AACCACAGGC TGGGGCATCC TGGAGATCAG AGCTGGCTAT GGCTCTCAAA CCCTGAGCAA 360

TGAGATCATC ATGTTTGTGG CTGGCTTTTT GGAGGGTTAC CTCACTGCCC CACACATGAA 420

TGACCACTAC ACAAACCTCT ACCCACAGCT GATCACGAAA CCTTCCATCA TGGATAAAGT 480

GCAGGATTTT ATGGAGAAGC AAGATAAGTG GACCCGGAAA AATATCAAAG AATACAAGAC 540

TGATTCATTT TGGAGACATA CAGGCTATGT GATGGCACAA ATAGATGGCC TCTATGTAGG 600

AGCAAAGAAG AGGGCTATAT TAGAAGGGAC AAAGCCAATG ACCCTGTTCC AGATTCAGTT 660

CCTGAATAGT GTTGGAGATC TATTGGATCT GATTCCCTCA CTCTCTCCCA CAAAAAACGG 720

CAGCCTAAAG GTTTTTAAGA GATGGGACAT GGGACATTGC TCCGCTCTTA TCAAGGTTCT 780

TCCTGGATTT GAGAACATCC TTTTTGCTCA CTCAAGCTGG TACACGTATG CAGCCATGCT 840

CAGGATATAT AAACACTGGG ACTTCAACRT CATAGATAAA GATACCAGCA GTAGTCGCCT 900

CTCTTTCAGC AGTTACCCAG GGTTTTTGGA GTCTCTGGAT GATTTTTACA TTCTTAGCAG 960

TGGATTGATA TTGCTGCAGA CCACAAACAG TGTGTTTAAT AAAACCCTGC TAAAGCAGTA 1020

ATACCCGAGA CTCTCCTGTC CTGGCAAAGA GTCCGTGTGG CCAATATGAT GGCAGATAGT 1080

GGCAAGAGGT GGGCAGACAT CTTTTCAAAA TACAACTCTG GCACCTATAA CAATCAATAC 1140

ATGGTTCTGG ACCTGAAGAA AGTAAAGCTG AACCACAGTC TTGACAAAGG CACTCTGTAC 1200

ATTGTGGAGC AAATTCCTAC ATATGTAGAA TATTCTGAAC AAACTGATGT TCTACGGAAA 1260

GGATATTGGC CCTCCTACAA TGTTCCTTTC CATGAAAAAA TCTACAACTG GAGTGGCTAT 1320

CCACTGTTAG TTCAGAAGCT GGGCTTGGAC TACTCTTATG ATTTAGCTCC ACGAGCCAAA 1380

ATTTTCCGGC GTGACCAAGG GAAAGTGACT GATACGGCAT CCATGAAATA TATCATGCGA 1440

TACAACAATT ATAAGAAGGA TCCTTACAGT AGAGGTGACC CCTGTAATAC CATCTGCTGC 1500

CGTGAGGACC TGAACTCACC TAACCCAAGT CCTGGAGGTT GTTATGACAC AAAGGTGGCA 1560

GATATCTACC TAGCATCTCA GTACACATCC TATGCCATAA GTGGTCCCAC AGTACAAGGT 1620

GGCCTCCCTG TTTTTCGCTG GGACCGTTTC AACAAAACTC TACATCAGGG CATGSCAGAG 1680

GTCTACAACT TTGATTTTAT TACCATGAAA CCAATTTTGA AACTTGATAT AAAATGAAGG 1740

AGGGAGATGA CGGACTAGAA GACTGTAAAT AAGATACCAA AGGCACTATT TTAGCTATGT 1800

TTTTCCCATC AGAATTATGC AATAAAATAT ATTAATTTGT CA 1842

(2) INFORMATION FOR SEQ ID NO: 114:

(i) SEQUENCE CHARACTERISTICS:
(A) LENGTH: 1960 base pairs
(B) TYPE: nucleic acid
(C) STRANDEDNESS: double
(D) TOPOLOGY: linear

(xi) SEQUENCE DESCRIPTION: SEQ ID NO: 114:

GAATTCGGCA CGAGCTTCTC CGCGCCCCAG CCGCCGGCTG CCAGCTTTTC GGGGCCCCGA 60

GTCGCACCCA GCGAAGAGAG CGGGCCCGGG ACAAGCTCGA ACTCCGGCCG CCTCGCCCTT 120

CCCCGGCTCC GCTCCCTCTG CCCCCTCGGG GTCGCGCGCC CACGATGCTG CAGGGCCCTG 180

GCTCGCTGCT GCTGCTCTTC CTCGCCTCGC ACTGCTGCCT GGGCTCGGCG CGCGGGCTCT 240

TCCTCTTTGG CCAGCCCGAC TTCTCCTACA AGCGCAGMAA TTGCAAGCCC ATCCCGGTCA 300

ACCTGCAGCT GTGCCACGGC ATCGAATACC AGAACATGCG GCTGCCCAAC CTGCTGGGCC 360

ACGAGACCAT GAAGGAGGTG CTGGAGCAGG CCGGCGCTTG GATCCCGCTG GTCATGAAGC 420

AGTGCCACCC GGACACCAAG AAGTTCCTGT GCTCGCTCTT CGCCCCCGTC TGCCTCGATG 480

ACCTAGACGA GACCATCCAG CCATGCCACT CGCTCTGCGT GCAGGTGAAG GACCGCTGCG 540

CCCCGGTCAT GTCCGCCTTC GGNTTCCCCT GGCCCGACAT GCTTGAGTGC GACCGTTTCC 600

CCCAGGACAA CGACCTTTGC ATCCCCCTCG CTAGCAGCGA CCACCTCCTG CCAGCCACCG 660

AGGAAGCTCC AAAGGTATGT GAAGCCTGCA AAAATAAAAA TGATGATGAC AACGACATAA 720

TGGAAACGCT TTGTAAAAAT GATTTTGCAC TGAAAATAAA AGTGAAGGAG ATAACCTACA 780

TCAACCGAGA TACCAAAATC ATCCTGGAGA CCAAGAGCAA GACCATTTAC AAGCTGAACG 840

GTGTGTCCGA AAGGGACCTG AAGAAATCGG TGCTGTGGCT CAAAGACAGC TTGCAGTGCA 900

CCTGTGAGGA GATGAACGAC ATCAACGCGC CCTATCTGGT CATGGGACAG AAACAGGGTG 960

GGGAGCTGGT GATCACCTCG GTGAAGCGGT GGCAGAAGGG GCAGAGAGAG TTCAAGCGCA 1020

TCTCCCGCAG CATCCGCAAG CTGCAGTGCT AGTCCCGGCA TCCTGATGGC TCCGACAGGC 1080

CTGCTCCAGA GCACGGCTGA CCATTTCTGC TCCGGGATCT CAGCTCCCGT TCCCCAAGCA 1140

CACTCCTAGC TGCTCCAGTC TCAGCCTGGG CAGCTTCCCC CTGCCTTTTG CACGTTTGCA 1200

TCCCCAGCAT TTCCTGAGTT ATAAGGCCAC AGGAGTGGAT AGCTGTTTTC ACCTAAAGGA 1260

AAAGCCCACC CGAATCTTGT AGAAATATTC AAACTAATAA AATCATGAAT ATTTTTATGA 1320

AGTTTAAAAA TAGCTCACTT TAAAGCTAGT TTTGAATAGG TGCAACTGTG ACTTGGGTCT 1380

GGTTGGTTGT TGTTTGTTGT TTTGAGTCAG CTGATTTTCA CTTCCCACTG AGGTTGTCAT 1440

AACATGCAAA TTGCTTCAAT TTTCTCTGTG GCCCAAACTT GTGGGTCACA AACCCTGTTG 1500

AGATAAAGCT GGCTGTTATC TCAACATCTT CATCAGCTCC AGACTGAGAC TCAGTGTCTA 1560

AGTCTTACAA CAATTCATCA TTTTATACCT TCAATGGGAA CTTAAACTGT TACATGTATC 1620

ACATTCCAGC TACAATACTT CCATTTATTA GAAGCACATT AACCATTTCT ATAGCATGAT 1680

TTCTTCAAGT AAAAGGCAAA AGATATAAAT TTTATAATTG ACTTGAGTAC TTTAAGCCTT 1740

GTTTAAAACA TTTCTTACTT AACTTTTGCA AATTAAACCC ATTGTAGCTT ACCTGTAATA 1800

TACATAGTAG TTTACCTTTA AAAGTTGTAA AAATATTGCT TTAACCAACA CTGTAAATAT 1860

TTCAGATAAA CATTATATTC TTGTATATAA ACTTTACATC CTGTTTTACC TAAAAAAAAA 1920

AAAAAAAAAA AAAAAACTCG AGGGGGGCCC GGTACCCAAT 1960

(2) INFORMATION FOR SEQ ID NO: 115:

(i) SEQUENCE CHARACTERISTICS:
(A) LENGTH: 536 base pairs
(B) TYPE: nucleic acid
(C) STRANDEDNESS: double
(D) TOPOLOGY: linear

(xi) SEQUENCE DESCRIPTION: SEQ ID NO: 115:

GTGCTCAGCC CCCGGGGCAC AGYAGGACGT TTGGGGGCCT TCTTTCAGCA GGGGACAGCC 60

CGATTGGGGA CAATGGCGTC TCTTGGCCAC ATCTTGGTTT TCTGTGTGGG TCTCCTCACC 120

ATGGCCAAGG CAGAAAGTCC AAAGGAACAC GACCCGTTCA CTTACGACTA CCAGTCCCTG 180

CAGATCGGAG GCCTCGTCAT CGCCGGGATC CTCTTCATCC TGGGCATCCT CATCGTGCTG 240

AGCAGAAGAT GCCGGTGCAA GTTCAACCAG CAGCAGAGGA CTGGGGAACC CGATGAAGAG 300

GAGGGAACTT TCCGCAGCTC CATCCGCCGT CTGTCCAMCC GCANGCGGTA GAAACACCTG 360

GAGCGATGGA ATCCGGCCAG GACTCCCCTG GCACCTGACA TCTCCCACGC TCCACCTGCG 420

CGCCCACCGC CCCCTCCGCC GCCCCTTCCC CAGCCCTGCC CCCGCAGACT CCCCCTGCCG 480

CCAAGACTTC CAATAAAACG TGCGTTCCTC TCGAMAAAAA AAAAAATAAA AAAACT 536

(2) INFORMATION FOR SEQ ID NO: 116:

(i) SEQUENCE CHARACTERISTICS:
(A) LENGTH: 790 base pairs
(B) TYPE: nucleic acid
(C) STRANDEDNESS: double
(D) TOPOLOGY: linear

(xi) SEQUENCE DESCRIPTION: SEQ ID NO: 116:

GTGGGGAGGG GGCGGAGCAA AGCCGCGCCT CTGGGTGGGC GGGTCGGGCC GTCCAGGTCC 60

CTGACTTGAA CCTTCCCGGT CCCCAGCCCT CAACAGGAGG CGCAGAAAAT CTTCAAAGCC 120

AACCACCCCA TGGACGCAGA AGTTACTAAG GCCAAGCTTC TGGGGTTTGG CTCTGCTCTC 180

CTGGACAATG TGGACCCCAA CCCTGAGAAC TTCGTGGGGG CGGGGATCAT CCAGACTAAA 240

GCCCTGCAGG TGGGCTGTCT GCTTCGGCTG GAGCCCAATG CCCAGGCCCA GATGTACCGG 300

CTGACCCTGC GCACCAGCAA GGAGCCCGTC TCCCGTCACC TGTGTGAGCT GCTGGCACAN 360

AGTTCTGAGC CCTGGACTCT GCCCCGGGGG ATGTGGCCGG CACTGGGCAG CCCCTTGGAC 420

TGAGGCAGTT TTGGTGGATG GGGGACCTCC ACTGGTGACA GAGAAGACAC CAGGGTTTGG 480

GGGATGCCTG GGACTTTCCT CCGGCCTTTT GTATTTTTAT TTTTGTTCAT CTGCTGCTGT 540

TTACATTCTG GGGGGTTAGG GGGAGTCCCC CTCCCTCCCT TTCCCCCCCA AGCACAGAGG 600

GGAGAGGGGC CAGGGAAGTG GATGTCTCCT CCCCTCCCAC CCCACCCTGT TGTAGCCCCT 660

CCTACCCCCT CCCCATCCAG GGGCTGTGTA TTATTGTGAG CGAATAAACA GAGAGACGTT 720

AACAGCCCCA TGTCTGTGTC CATCACCCAN TGNTAGGTAG TCAAAGAAGT GGGGTGAGGG 780

CATGCAGAGT 790

(2) INFORMATION FOR SEQ ID NO: 117:

(i) SEQUENCE CHARACTERISTICS:
(A) LENGTH: 776 base pairs
(B) TYPE: nucleic acid
(C) STRANDEDNESS: double
(D) TOPOLOGY: linear

(xi) SEQUENCE DESCRIPTION: SEQ ID NO: 117:

CAGCGCTGGA AGCAGCTGAG CCTGTGAGGG GTGGGGAGGG GGCGGAGCAA AGCCGCGCCT 60

CTGGGTGGGC GGGTCGGGCC GTCCAGGTCC CTGACTTGAA CCTTCCCGGT CCCCAGCCCT 120

CAACAGGAGG CGCAGAAAAT CTTCAAAGCC AACCACCCCA TGGACGCAGA AGTTACTAAG 180

GCCAAGCTTC TGGGGTTTGG CTCTGCTCTC CTGGACAATG TGGACCCCAA CCCTGAGAAC 240

TTCGTGGGGG CGGGGATCAT CCAGACTAAA GCCCTGCAGG TGGGCTGTCT GCTTCGGCTG 300

GAGCCCAATG CCCAGGCCCA GATGTACCGG CTGACCCTGC GCACCAGCAA GGAGCCCGTC 360

TCCCGTCACC TGTGTGAGCT GCTGGCACAG AGTTCTGAGC CCTGGACTCT GCCCCGGGGG 420

ATGTGGCCGG CACTGGGCAG CCCCTTGGAC TGAGGCAGTT TTGGTGGATG GGGGACCTCC 480

ACTGGTGACA GAGAAGACAC CAGGGTTTGG GGGATGCCTG GGACTTTCCT CCGGCCTTTT 540

GTATTTTTAT TTTTGTTCAT CTGCTGCTGT TTACATTCTG GGGGGTTAGG GGGAGTCCCC 600

CTCCCTCCCT TTCCCCCCCA AGCACAGAGG GGAGAGGGGC CAGGGAAGTG GATGTCTCCT 660

CCCCTCCCAC CCCACCCTGT TGTAGCCCCT CCTACCCCCT CCCCATCCAG GGGCTGTGTA 720

TTATTGTGAG CGAATAAACA GAGAGACGCN TAAAAAAAAA AAAAAAAAAT TGAGGG 776

(2) INFORMATION FOR SEQ ID NO: 118:

(i) SEQUENCE CHARACTERISTICS:
(A) LENGTH: 453 base pairs
(B) TYPE: nucleic acid
(C) STRANDEDNESS: double
(D) TOPOLOGY: linear

(xi) SEQUENCE DESCRIPTION: SEQ ID NO: 118:

GGTTCTGACA CCAGATGTTC TCTGCTCCTG GTTAATGTCA GTGAGGGCTG GAAGTTGAAT 60

AAATGAGAAC AGGAGTGGTC TGGGCCCATG TAAATGATCC TCCCTTGAAA GGAGGAACAG 120

CTTTCATCAT TTGTTCCAGC TAAGCCTTGC ATGCATTATA GATCTGGTGC TAAGCAGTGG 180

GAAAGATCTC ATAAGTAATG TTTTATGTTC TTTCKGTCTC TCYTCTTCKG TTGTTCTTGG 240

CTTGTGGGTT GTGTTTGKGG TTGTTAACTG GAAAATTGCT ATAAGCCAGT TGTCYCKAAK 300

TTTWAAAAAC GAATTAGAAA AACCATAAAA TCYTCTGGCC YATGCACATK GTCCCYGTTT 360

TGTGAAAACA TTAAAGGGTA AATAAAAAGG AAGGAGAACA GTCAATAATG TGCATCAAAT 420

ATATTCTGAG TTCTAGAGAA ATTAATGACC AAG 453

(2) INFORMATION FOR SEQ ID NO: 119:

(i) SEQUENCE CHARACTERISTICS:
(A) LENGTH: 2016 base pairs
(B) TYPE: nucleic acid
(C) STRANDEDNESS: double
(D) TOPOLOGY: linear

(xi) SEQUENCE DESCRIPTION: SEQ ID NO: 119:

AGGCTGTTCA CAGGCACCCC GAGACAGCGT CCCCCCTCTG GGCGCACTGG ATTTGACGTT 60

GCAGGACGCG CGGCTGGAAC CCCCAGGCCC CGCTGCTCAC AGACCGGGAC TCCGCCTCCG 120

GTTCCCGAGG GCGTGGCGAG GCGCTGCGGG ANCCCAACAG GATGCCTTCC GTGCCTTCCA 180
TCAAGATCTC AATTTTGTGC GCAATTCCTA CAGCCCCTGT TGATTGGAGA GCTGGCTCCG 240
GAAGAACCCA GCCAKGATGG ACCCCTGAAT GCGCATGGTC GAGGACTTCC GAGCCCTGCA 300
CCAGGCAGCC GAGGACATGA AGCTGTTTGA TGCCAGTCCC ACCTTCTTTG CTTTCCTACT 360
GGGCCACATC CTGGCCATGG AGGTGCTGGC CTGGCTCCTT ATCTACCTCC TGGGTCCTGG 420
CTGGGTGCCC AGTGCCCTGG NCCGCCTTCA TCCTGGCCAT CTCTCAGGCT CAGTCCTGGT 480
GTCTGCAGCA TGACCTGGGC CATGCTCCAT CTTCAAGAAG TCCTGGTGGA ACCACGTGGC 540
CCAGAAGTTC GTGATGGGGC AGCTAAAGGG CTTCTCCGCC CACTGGTGGA ACTTCCGCCA 600
CTTCCAGCAC CACGCCAAGC CCAACATCTT CCACAAAGAC CCAGACGTGA CGGTGGCGCC 660
CGTYTTCCTC CTGGGGGAGT CATCCGTCGA GTATGGNCAA GAAGAAACGC AGATACCTAC 720
CCTACAACCA GCAGCACCTG TACTTCTTCC TGATCGGCCC GCCGCTGCTC ACCCTGGTGA 780
ACTTTGAAGT GGAAAATCTG GCGTACATGC TGGTGTGCAT GCAGTGGGCG GATTTGCTCT 840
GGGCCGCCAG CTTCTATGCC CGCTTCTTCT TATCCTACCT CCCCTTCTAC GGCGTCCCTG 900
GGGTGCTGCT CTTCTTTGTT GCTGTCAGGT ATGGCAGGGA GTGGCGAGGT CACACACAGG 960
CGACAGGTGA CCCCCACTGC AGCCCCCCAC CAGAGCTTCC CTTTTCCCGT CTGCAGAATG 1020
GGGCCAGTGG TACTGCCTCC CTGGCTTGCT GGTGGAATCA CATAAACACA AGYTTCAGGA 1080
GCCCAGGGTC GGTGGGTTTA GGGAGCGTGG CCTGGCTTGT AAGTGGCCCG GTGGGTGTCG 1140
GAGCTGCTCT GGACTCAGCC TCACAGTGGA CACTGCTCCA TTCAGATTCT TTAAACACTG 1200
GCAAGGGGGC GATGGCCACA ATCCTATTGT ACAGATAAGG AAGTCAAGGC CAYTTGGGGA 1260
CAGYTGCTCT TCCAGCCTCC ACTCAGGGTG CCTTAAGTGG TGAGCTGGAC CTAGGGCAGT 1320
GCCGAGCYTC CCCACAGGGT CCTGGAAAGC CACTGGTTCG TGTGGATCAC ACAGATGAAC 1380
CACATCCCCA AGGAGATCGG CCACGAGAAG CACCGGGACT GGGTCAGCTC TCAGCTGGCA 1440
GCCACCTGCA ACGTGGAGCC CTCACTTTTC ACCAACTGGT TCAGCGGGCA CCTCAACTTC 1500
CAGATCGAGC ACCACCTCTT CCCCAGGATG CCGAGACACA ACTACAGCCG GGTGGCCCCG 1560
CTGGTCAAGT CGCTGTGTGC CAAGCACGGC CTCAGCTACG AATGAAGCCC TTCCTCACCG 1620
CGCTGGTGGA CATCGTCAGG TCCCTGAAGA AGTCTGGTGA CATCTGGCTG GACGCCTACC 1680
TCCATCAGTG AAGGCAACAC CCAGGCGGGC AGAGAAGGGC TCAGGGCACC AGCAACCAAG 1740
CCAGCCCCCG GCGGGATCGA TACCCCCAMC CCTCCACTGG CCAGCCTGGG GGTGCCCTGC 1800
CTGCCCTCCT GGTACTGTTG TCTTCCCCTC GGCCCCCTCA CATGTGTATT CAGCAGCCCT 1860
ATGGCCTTGG CTCTGGGCCT GATGGGACAG GGGTAGAGGG AAGGTGAGCA TAGCACATTT 1920

TCCTAGAGCG AGAATTGGGG GAAAGCTGTT ATTTTTATAT TAAAATACAT TCAGATGTAA 1980

AAAAAAAAAA AAAAAAANCT CGAGGGGGGG CCCCGG 2016

(2) INFORMATION FOR SEQ ID NO: 120:

(i) SEQUENCE CHARACTERISTICS:
(A) LENGTH: 2136 base pairs
(B) TYPE: nucleic acid
(C) STRANDEDNESS: double
(D) TOPOLOGY: linear

(xi) SEQUENCE DESCRIPTION: SEQ ID NO: 120:

GGGGACGGAG CCGCTGTCAA CTCTCCAACT CAGCTCAGCT GATCGGTTGC CGCCGCCGCC 60

GCCGCCAGAT TCTGGAGGCG AAGAACGCAA AGCTGAGAAC ATGGACGTTA ATATCGCCCC 120

ACTCCGCGCC TGGGACGATT TCTTCCCGGG TTCCGATCGC TTTGCCCGGC CGGACTTCAG 180

GGACATTTCC AAATGGAACA ACCGCGTAGT GAGCAACCTG CTCTATTACC AGACCAACTA 240

CCTGGTGGTG GCTGCCATGA TGATTTCCAT TGTGGGGTTT CTGAGTCCCT TCAACATGAT 300

CCTGGGAGGA ATCGTGGTGG TGCTGGTGTT CACAGGGTTT GTGTGGGCAG CCCACAATAA 360

AGACGTCCTT CGCCGGATGA AGAAGCGCTA CCCCACGACG TTCGTTATGG TGGTCATGTT 420

GGCGAGCTAT TTCCTTATCT CCATGTTTGG AGGAGTCATG GTCTTTGTGT TTGGCATTAC 480

TTTTCCTTTG CTGTTGATGT TTATCCATGC ATCGTTGAGA CTTCGGAACC TCAAGAACAA 540

ACTGGAGAAT AAAATGGAAG GAATAGGTTT GAAGAGGACA CCGATGGGCA TTGTCCTGGA 600

TGCCCTAGAA CAGCAGGAAG AAGGCATCAA CAGACTCACT GACTATATCA GCAAAGTGAA 660

GGAATAAACA TAACTTACCT GAGCTAGGGT TGCAGCAGAA ATTGAGTTGC AGCTTGCCCT 720

TGTCCAGACC TATKTTCTGC TTGCGTTTTT GAAACAGGAG GTGCACGTAC CACCCAATTA 780

TCTATGGCAG CATGCATGTA TAGGCCGAAC TATTATCAGC TCTGATGTTT CAGAGAGAAG 840

ACCTCAGAAA CCGAAAGAAA ACCACCACCC TCCTATTGTG TCTGAAGTTT CACGTGTGTT 900

TATGAAATCT AATGGGAAAT GGATCACACG ATTTCTTTAA GGGAATTAAA AAAAATAAAA 960

GAATTACGGC TTTTACAGCA ACAATACGAT TATCTTATAG GAAAAAAAAA ATCATTGTAA 1020

AGTATCAAGA CAATACGAGT AAATGAAAAG GCTGTTAAAG TAGATGACAT CATGTGTTAG 1080

CCTGTTCCTA ATCCCCTAGA ATTGTAATGT GTGGGATATA AATTAGTTTT TATTATTCTC 1140

TTAAAAATCA AAGATGATCT CTATCACTTT GCCACCTGTT TGATGTGCAG TGGAAACTGG 1200

TTAAGCCAGT TGTTCATACT TCSTTTACAA ATATAAAGAT AGCTGTTTAG GATATTTTGT 1260

TACATTTTTG TAAATTTTTG AAATGCTAGT AATGTGTTTT CACCAGCAAG TATTTGTTGC 1320

AAACTTAATG TCATTTTCCT TAAGATGGTT ACAGCTATGT AACCTGTATT ATTCTGGACG 1380

GACTTATTAA AATACAAACA GACAAAAAAT AAAACAAAAC TTGAGTTCTA TTTACCTTGC 1440

ACATTTTTTG TTGTTACAGT GAAAAAAATG GTCCAAGAAA ATGTTTGCCA TTTTTGCATT 1500

GTTTCGTTTT TAACTGGAAC ATTTAGAAAG AAGGAAATGA ATGTGCATTT TATTAATTCC 1560

TTAGGGGCAC AAGGAGGACA ATAATAGCTG ATCTTTTGAA ATTTGAAAAA CGTCTTTAGA 1620

TGACCAAGCA AAAAGACTTT AAAAAATGGT AATGAAAATG GAATGCAGCT ACTGCAGCTA 1680

ATAAAAAATT TTAGATAGCA ATTGTTACAA CCATATGCCT TTATAGCTAG ACATTAGAAT 1740

TATGATAGCA TGAGTTTATA CATTCTATTA TTTTTCCTCC CTTTCTCATG TTTTTATAAA 1800

TAGGTAATAA AAAATGTTTT GCCTGCCAAT TGAATGATTT CGTAGCTGAA GTAGAAACAT 1860

TTAGGTTTCT GTAGCATTAA ATTGTGAAGA CAACTGGAGT GGTACTTACT GAAGAAACTC 1920

TCTGTATGTC CTAGAATAAG AAGCAATGAT GTGCTGCTTC TGATTTTTCT TGCATTTTAA 1980

ATTCTCAGCC AACCTACAGC CATGATCTTT AGCACAGTGA TATCACCATG ACTTCACAGA 2040

CATGGTCTAG AATCTGTACC CTTACCCACA TATGAAGAAT AAAATTGATT AAAGGTTAAA 2100

AAAAAAAWAA AAAAAMWAGG GGGGCCCGGT WCCCAG 2136

(2) INFORMATION FOR SEQ ID NO: 121:

(i) SEQUENCE CHARACTERISTICS:
(A) LENGTH: 219 base pairs
(B) TYPE: nucleic acid
(C) STRANDEDNESS: double
(D) TOPOLOGY: linear

(xi) SEQUENCE DESCRIPTION: SEQ ID NO: 121:

GCCCTAGTAT CTGGGCAGCT GTGCATGGAG ATAGCCAGAG GAAACATTTT TTTTCTTAAT 60

GRATTGGTGA CCACATTTTG TTGTTCTTGC CTCCTATTAT CCGTGCSCTA TTTGCATSCT 120

GGTTTCTTCT ACAGTAGTTT ATGTAAATGT TGTTTTGTCC TTGTCGTTCT CAGTAGAATT 180

GGTTCTGTAA ACGAAACCTG GTCCTGTAAT TTCAGTATA 219

(2) INFORMATION FOR SEQ ID NO: 122:

(i) SEQUENCE CHARACTERISTICS:
(A) LENGTH: 1686 base pairs
(B) TYPE: nucleic acid
(C) STRANDEDNESS: double

(D) TOPOLOGY: linear

(xi) SEQUENCE DESCRIPTION: SEQ ID NO: 122:

GCTGGAGATT CACATTTTAC CTGATTGCCT TCATTGCCGG CATGGCCGTC ATTGTGGATA 60

AACCCTGGTT CTATGACATG AAGAAAGTTT GGGAGGGATA TCCCATACAG AGCACTATCC 120

CTTCCCAGTA TTGGTACTAC ATGATTGAAC TTTCCTTCTA CTGGTCCCTG CTCTTCAGCA 180

TTGCCTCTGA TGTCAAGCGA AAGGATTTCA AGGAACAGAT CATCCACCAT GTGRCCACCA 240

TCATTCTCAT CAGCTTTTCC TGGTTTGCCA ATTACATCCG AGCTGGGACT CTAATCATGG 300

CTCTGCATGA CTCTTCCGAT TACCTGCTGG AGTCAGCCAA GATGTTTAAC TACGCGGGAT 360

GGAAGAACAC CTGCAACAAC ATCTTCATCG TCTTCGCCAT TGTTTTTATC ATCACCCGAC 420

TGGTCATCCT GCCCTTCTGG ATCCTGCATT GCACCCTGGT GTACCCACTG GAGCTCTATC 480

CTGCCTTCTT TGGSTATTAC TTCTTCAATT CCATGATGGG AGTTCTACAG CTGCTGCATA 540

TCTTCTGGGC CTACCTCATT TTGCGCATGG CCCACAAGTT CATAACTGGG AAAGCTGGTA 600

GAAGATGAAC GCAWGCRCGG GNAAGAAACA GAGAGCTCAG AGGGGGAGGA GGCTGCAGCT 660

GGGGGAGGAG CAAAGAGCCG GCCCCTAGCC AATGGCCACC CCATCCTCAA TAACAACCAT 720

CGTAAGAATG ACTGAACCAT TATTCCAGCT GCCTCCCAGA TTAATGCATA AAGCCAAGGA 780

ACTACCCYGC TCCCTGCGCT ATAGGGTCAC TTTAAGCTCT GGGGAAAAAG GAGAAAGTGA 840

GAGGAGAGTT CTCTGCATCC TCCCTCCTTG CTTGTCACCC AGTTGCCTTT AAACCAAATT 900

CTAACCAGCC TATCCCCAGG TAGGGGGACG TTGGTTATAT TCTGTTAGAG GGGGACGGTC 960

GTATTTTCCT CCCTACCCGC CAAGTCATCC TTTCTACTGC TTTTGAGGCC CTCCCTCAGC 1020

TCTCTGTGGG TAGGGGTTAC AATTCACATT CCTTATTCTG AGAATTTGGC CCCAGCTGTT 1080

TGCCTTTGAC TCCCTGACCT CCAGAGCCAG GGTTGTGCCT TATTGTCCCA TCTGTGGGCC 1140

TCATTCTGCC AAAGCTGGAC CAAGGCTAAC CTTTCTAAGC TCCCTAACTT GGGCCAGAAA 1200

CCAAAGCTGA GCTTTTAACT TTCTCCCTCT ATGACACAAA TGAATTGAGG GTAGGAGGAG 1260

GGTGCACATA ACCCTTACCC TACCTCTGCC AAAAAGTGGG GGCTGTACTG GGGACTGCTC 1320

GGATGATCTT TCTTAGTGCT ACTTCTTTCA GCTGTCCCTG TAGCGACAGG TCTAAGATCT 1380

GACTGCCTCC TCCTTTCTCT GGCCTCTTCC CCCTTCCCTC TTCTCTTCAG CTAGGCTAGC 1440

TGGTTTGGAG TAGAATGGCA ACTAATTCTA ATTTTTATTT ATTAAATATT TGGGGTTTTG 1500

GTTTTAAAGC CAGAATTACG GCTAGCACCT AGCATTTCAG CAGAGGGACC ATTTTAGACC 1560

AAAATGTACT GTTAATGGGT TTTTTTTTAA AATTAAAAGA TTAAATAAAA AATATTAAAT 1620

AAAACATGGC AATAAGTGTC AGACTATTAG GAATTGAGAA GGGGGATCAA CTAAATAAAC 1680

GAAGAG 1686

(2) INFORMATION FOR SEQ ID NO: 123:

(i) SEQUENCE CHARACTERISTICS:
(A) LENGTH: 1211 base pairs
(B) TYPE: nucleic acid
(C) STRANDEDNESS: double
(D) TOPOLOGY: linear

(xi) SEQUENCE DESCRIPTION: SEQ ID NO: 123:

CAGCCTGTGC CAGACGAGGA GGTGATTGAG CTGTATGGGG GTACCCAGCA CATCCCACTA 60

TACCAGATGA GTGGCTTCTA TGGCAAGGGT CCCTCCATTA AGCAGTTCAT GGACATCTTC 120

TCGCTACCGG AGATGGCTCT GCTGTCCTGT GTGGTGGACT ACTTTCTGGG CCACAGCCTG 180

GAGTTTGACC AAACATCTCT ACAAGGACGT GACGGACGCC ATCCGAGACG TGCATGTGAA 240

GGGCCTCATG TACCAGTGGA TCGAGCAGGA CATGGAGAAG TACATCCTGA GAGGGGATGA 300

GACGTTTGCT GTCCTGAGCC GCCTGGTGGC CCATGGAAAA CAGCTGTTCC TCATCACCAA 360

CAGTCCTTTC AGCTTCGTAG ACAAGGGGAT GCGGCACATG GTGGGTCCCG ATTGGCGCCA 420

CTCTTCGATG TGGTCATTGT CCAGGCAGAC AAGCCCAGCT TCTTCACTGA CCGGCGCAAC 480

TTTCAGAAAA CTCGATGAGA AGGGCTCACT TCAGTGGGAC CGGATCACCC GCTTGGAAAA 540

GGGCAAGATC TATCGGCAGG GAAACCTGTT TGACTTCTTA CGCTTGACGG AATGGCGTGG 600

CCCCCGCGTG CTCTACTTCG GGGACCACCT CTATAGTGAT CTGGCGGATC TCATGCTGCG 660

GCACGGCTGG CGCACAGGCG CCATCATCCC CGAGCTGGAG CGTGAGATCC GCATCATCAA 720

CACGGAGCAG TACATGCACT CGCTGACGTG GCAGCAGGCG CTCACGGGGC TGCTGGAGCG 780

CATGCAGACC TATCAGGACG CGGAGTCGAG GCAGGTGCTG GCTGCCTGGA TGAAAGAGCG 840

GCAGGAGCTG AGGTGCATCA CCAAGGCCCT GTTCAATGCG CAGTTCGGCA GCATCTTCCG 900

CACCTTCCAC AACCCCACCT ACTTCTCAAG GCGCCTCGTG CGCTTCTCTG ACCTCTACAT 960

GGCCTCCCTC AGCTGCCTGC TCAACTACCG CGTGGACTTC ACCTTCTACC CACGCCGTAC 1020

GCCGCTGCAG CACGAGGCAC CCCTCTGGAT GGACCAGCTT CTGCACCGGC TGCATGAAGA 1080

CCCCCTTCCT TGGTGACATG GCCCACATCC GCTGAGGGCA CCTTTATTGT CTGGGACAGG 1140

CCCTCAGCCC CTCCTGCCCC ATCCACCCAG ACAAGCAATA AAAGTGGTCT CCTCCCTGAA 1200

AAAAAAAAAA A 1211

(2) INFORMATION FOR SEQ ID NO: 124:

(i) SEQUENCE CHARACTERISTICS:
(A) LENGTH: 1804 base pairs
(B) TYPE: nucleic acid
(C) STRANDEDNESS: double
(D) TOPOLOGY: linear

(xi) SEQUENCE DESCRIPTION: SEQ ID NO: 124:

CGCACCTATG GGCTCGCTAC CAGGACATGC GGAGACTGGT GCACGACCTC CTGCCCCCCG 60

AGGTCTGCAG TCTCCTGAAC CCAGCAGCCA TCTACGCCAA CAACGAGATC AGCCTGCGTG 120

ACGTTGAGGT CTACGGCTTT GACTACGACT ACACCCTGGC CCAGTATGCA GACGCACTGC 180

ACCCCGAGAT CTTCAGTACC GCCCGTGACA TCCTGATCGA GCACTACAAG TACCCAGAAG 240

GGATTCGGAA GTATGACTAC AACCCCAGCT TTGCCATCCG TGGCCTCCAC TATGACATTC 300

AGAAGAGCCT TCTGATGAAG ATTGACGCCT TCCACTACGT GCAGCTGGGG ACAGCCTACA 360

GGGGCCTCCA GCCTGTGCCA GACGAGGAGG TGATTGAGCT GTATGGGGGT ACCCAGCACA 420

TCCCACTATA CCAGATGAGT GGCTTCTATG GCAAGGGTCC CTCCATTAAG CAGTTCATGG 480

ACATCTTCTC GCTACCGGAG ATGGCTCTGC TGTCCTGTGT GGTGGACTAC TTTCTGGGCC 540

ACAGCCTGGN AGTTTGACCA AGCACATCTC TACAAGGACG TGACGGACGC CATCCGAGAC 600

GTGCATGTGA AGGGCCTCAT GTACCAGTGG ATCGAGCAGG ACATGGAGAA GTACATCCTG 660

AGAGGGGATG AGACGTTTGC TGTCCTGAGC CGCCTGGTGG CCCATGGGAA ACAGCTGTTC 720

CTCATCACCA ACAGTCCTTT CAGCTTCGTA GACAAGGGGA TGCGGCACAT GGTGGGTCCC 780

GATTGGCGCC ACTCTTCGAT GTGGTCATTG TCCAGGCAGA CAAGCCCAGC TTCTTCACTG 840

ACCGGCGCAA GCTTTTCAGA AAACTCGATG AGAAGGGCTC ACTTCAGTGG GACCGGATCA 900

CCCGCTTGGA AAAGGGCAAG ATCTATCGGC AGGGAAACCT GTTTGACTTC TTACGCTTGA 960

CGGAATGGCG TGGCCCCCGC GTGCTCTACT TCGGGGACCA CCTCTATAGT GATCTGGCGG 1020

ATCTCATGCT GCGGCACGGC TGGCGCACAG GCGCCATCAT CCCCGAGCTG GAGCGTGAGA 1080

TCCGCATCAT CAACACGGAG CAGTACATGC ACTCGCTGAC GTGGCAGCAG GCGCTCACGG 1140

GGCTGCTGGA GCGCATGCAG ACCTATCAGG ACGCGGAGTC GAGGCAGGTG CTGGCTGCCT 1200

GGATGAAAGA GCGGCAGGAG CTGAGGTGCA TCACCAAGGC CCTGTTCAAT GCGCAGTTCG 1260

GCAGCATCTT CCGCACCTTC CACAACCCCA CCTACTTCTC AAAGGCGCCT CGTGCGCTTC 1320

TCTGACCTCT ACATGGCCTC CCTCAGCTGC CTGCTCAACT ACCGCGTGGA CTTCACCTTC 1380

TACCCACGCC GTACGCCGCT GCAGCACGAG GCACCCCTCT GGATGGACCA GCTCTGCACC 1440

GGCTGCATGA AGACCCCCTT CCTTGGTGAC ATGGCCCACA TCCGCTGAGG GCACCTTTAT 1500

TGTCTGGGAC AGGCCCTCAG CCCCTCCTGC CCCATCCACC CAGACAAGCA ATAAAAGTGG 1560

TCTCCTCCCT GTGCATGCTT CTGCTTTCAG CCCCAGCCTC GTCACTTGAC TGTGAGGATC 1620

CTCTGGGTGT CAGGGAAGTC CTCCTCCAGC AGTGAGTCAT CGAAGGGTTC ACAAAAGGTG 1680

TCGCTGCCAA AGACAGGGTT GGGGACAGAG ACCAGGGTGG GGTTGGTCCC TTCTTGCCAC 1740

GGTGAGAAGT CGTCGTCAGC CGGACGCGTG GGTCGACCCG GGAATTCCGG ACCGGTACCT 1800

GCAG 1804

(2) INFORMATION FOR SEQ ID NO: 125:

(i) SEQUENCE CHARACTERISTICS:
(A) LENGTH: 1282 base pairs
(B) TYPE: nucleic acid
(C) STRANDEDNESS: double
(D) TOPOLOGY: linear

(xi) SEQUENCE DESCRIPTION: SEQ ID NO: 125:

CCGCAGGNCA GCGACGCGAC TCTGGTGCGG GCCGTCTTCT TCCCCCCGAG CTGGGCGTGC 60

GCGGCCGCAA TGAACTGGGA GCTGCTGCTG TGGCTGCTGG TGCTGTGCGC GCTGCTCCTG 120

CTCTTGGTGC AGCTGCTGCG CTTCCTGAGG GCTGACGGCG ACCTGACGCT ACTATGGGCC 180

GAGTGGCAGG GACGACGCCC AGAATGGGAG CTGACTGATA TGGTGGTGTG GGTGACTGGA 240

GCCTCGAGTG GAATTGGTGA GGAGCTGGCT TACCAGTTGT CTAAACTAGG AGTTTCTCTT 300

GTGCTGTCAG CCAGAAGAGT GCATGAGCTG GAAAGGGTGA AAAGAAGATG CCTAGAGAAT 360

GGCAATTTAA AAGAAAAAGA TATACTTGTT TTGCCCCTTG ACCTGACCGA CACTGGTTCC 420

CATGAAGCGG CTACCAAAGC TGTTCTCCAG GAGTTTGGTA GAATCGACAT TCTGGTCAAC 480

AATGGTGGAA TGTCCCAGCG TTCTCTGTGC ATGGATACCA GCTTGGATGT CTACAGAAAG 540

CTAATAGAGC TTAACTACTT AGGGACGGTG TCCTTGACAA AATGTGTTCT GCCTCACATG 600

ATCGAGAGGA AGCAAGGAAA GATTGTTACT GTGAATAGCA TCCTGGGTAT CATATCTGTA 660

CCTCTTTCCA TTGGATACTG TGCTAGCAAG CATGCTCTCC GGGGTTTTTT TAATGGCCTT 720

CGAACAGAAC TTGCCACATA CCCAGGTATA ATAGTTTCTA ACATTTGCCC AGGACCTGTG 780

CAATCAAATA TTGTGGAGAA TTCCCTAGCT GGAGAAGTCA CAAAGACTAT AGGCAATAAT 840

GGAGACCAGT CCCACAAGAT GACAACCAGT CGTTGTGTGC GGCTGATGTT AATCAGCATG 900

GCCAATGATT TGAAAGAAGT TTGGATCTCA GAACAACCTT TCTTGTTAGT AACATATTTG 960

TGGCAATACA TGCCAACCTG GGCCTGGTGG ATAACCAACA AGATGGGGAA GAAAAGGATT 1020

GAGAACTTTA AGAGTGGTGT GGATGCAGAC TCTTCTTATT TTAAAATCTT TAAGACAAAA 1080

CATGACTGAA AAGAGCAYCT GTACTTTTCA AGCCACTGGA GGGARAAATG GAAAACATGA 1140

AAACAGCAAT CTTCTTATGC TTCTGAATAA TCAAAGACTA ATTTGTGRTT TTACTTTTTA 1200

ATAGATATGA CTTTGCTTCC AACATGGAAT GAAATAAAAA ATAAATAATA AAAGATTGCC 1260

ATGGAAAAAA AAAAGNNGGG AN 1282

(2) INFORMATION FOR SEQ ID NO: 126:

(i) SEQUENCE CHARACTERISTICS:
(A) LENGTH: 1296 base pairs
(B) TYPE: nucleic acid
(C) STRANDEDNESS: double
(D) TOPOLOGY: linear

(xi) SEQUENCE DESCRIPTION: SEQ ID NO: 126:

GGCAGAGCTT AGAGTGTGGA AAAGGCAACC AGGTTGGCCG TAAGTGCCTG CTGGAATGCG 60

TGTGCCTCCA CASGGRTCTG GGCATCCGGA CTGATAACCA GCCGGCCAGA CTGAGGGATG 120

GAAGGCACTG AGATGGGGGC CCGTCCAGGC GGACACCCGC AGAAATGGAG CTTTCTGTGG 180

TCTCTTGCAC TCTGGCTGCC TCTTGCCCTC TCTGTGTCTC TCTTTCTTGG TCTCTCCCTC 240

TCTCCTCCTC AGCCTGGTCT TTCTCTTTGG TGCACACTTA GTTATTGTTG TGAGCAATGG 300

AAGTTCAAAG GAACTCCCTC TCCAGCTCTT CTGAATCTTG GGACACAGCC TAAAAAGGAC 360

AAAAAGTTAG AAGACAGCAT AGCAACTCAG CTCAGGGRGC TACCAGAGAA AAATAGCAAC 420

TGATGTGGGT GCTTTTTTTT TTTTTTTAAT TTGAATAAAA AGAATTAGAA GTGATGTCCT 480

TTTATAAAAT GCCTTCTCCC CCTTCCCGCC TACAGTCTCT TCCTCTCCCC TTAGAGGGGG 540

GAAAGTGTAT AAACCTACAG GGTTGTGAGT CTGAAAAGAG GATCCCCCTC ACCCCCACCC 600

TGGGCAGAGC AGTGGGGGTT GGGGGGTGGG AGAGGGGGAC ACAGATCCTG GCACACTGTG 660

GATATTTCTT GCAGATTGCA GTCTCTTGTG GCCCAAACAG GTTAGGTAGA CTATCGCCTC 720

TGGCAGGTGC CACCTTTTGG TACCAACATG TTCTGAGGTG TTAGGATTTG GGTTGGGTTT 780

TTTTTGTTTG TTTTTTTTTT CCNTTTGGTC TTTTTTTTTT TCYCCTTKTA AAGAAAAGCT 840

AAAGGCCGCT GTGAGTCCTG GTGGCAGGCT CTCCATGGAT GTAGCATATC GAAGATAATT 900

TTTATACTGC ATTTTTATGG ATTATTTTGT AATGTGTGAT TCCGTCTGCT GAGGAGGTGG 960

GAGGGGCTCC AGGGAAAGCC ACCCACCTTC AGTGAGGTTG CTCCCCAGCT GAGCGCACCG 1020

GGCATGGGAT GTGGAGGCTG GCGACACACC CTGTGCCTCT CCAAGGCTGG GCGCGTGGGG 1080

CGTCCAGAGT CTCTCTGGGT CTCAGATGTC CATCTGCCAC CTCTTGTTAA GGCTCTAGCC 1140

AGAAGGGAGG GTGAGGGTAG AAGAAAGTTA TTCCCGAAGA AAAAAAGAAT GAAAAGTCAT 1200

TGTACTGAAC TGTTTTTATA TTTTTAAAAG TTACTATTTA AAGCGGACGT CGTGGGTCGA 1260

CCCGGGAATT CCCGGACCGG TACTGTCAGG TCTAAC 1296

(2) INFORMATION FOR SEQ ID NO: 127:

(i) SEQUENCE CHARACTERISTICS:
(A) LENGTH: 737 base pairs
(B) TYPE: nucleic acid
(C) STRANDEDNESS: double
(D) TOPOLOGY: linear

(xi) SEQUENCE DESCRIPTION: SEQ ID NO: 127:

GGCANAGTGG AGGCAATGCC AGCTCCAGGA CAGAGGCTCA GGTGCCCAAC GGGCAAGGCA 60

GCCCAGGGGG CTGTGTCTGT TCAAGTCAGG CTTCCCCGGC CCYTCGCGCA NCAGCGCTTC 120

CACGGGCAGC CCGGGGCCCC ACCCCACGCA CTGAAGAGGC CGCCTGGGCT GCCATGGCCC 180

TGACCTTCCT GCTGGTGCTG CTCACCCTGG CCACGCTCTG CACACGGCTG CACAGAAACT 240

TCCGACGCGG GGAGAGCATC TACTGGGGGC CCACAGCGGA CAGCCAGGAC ACAGTGGCTG 300

CTGTGCTGAA GCGGAGGCTG CTGCAGCCCT CGCGCCGGGT CAAGCGCTCG CGCCGGAGAC 360

CCYTCYTCCC GCCCACGCCG GACAGCGGCC CGGAAGGCGA GAGCTCGGAG TGACGGCCTG 420

GGACCTGCCA CTGTGGCGTG CGGTCTCCCC GCGCCGCGAG GCCGCGAMCT NTGCCACGTG 480

GACCGCGCGC NGGGCGCTMC CCTGGTGGCG ATGGCGCGGC ACTGGCGAGC ACTGCGKGGG 540

CTTTCCTCCT TGTTGGTTGC TGAGTGGGCG GCCAAGGGGA GAAAAGGAGC CGCTTYTGCC 600

TCCCTTGCCA AAACTCCGTT TCTAATTAAA TTATTTTTAG TAGAAAAAAA AAAAAAAAAA 660

AAAAAAAAAA AAAAAAAAAA AAAAAAAAAC TCGAGGGGGG GCCCGGTACC CAATTNGCCA 720

AATAGCGATC GTATNAA 737

(2) INFORMATION FOR SEQ ID NO: 128:

(i) SEQUENCE CHARACTERISTICS:
(A) LENGTH: 1925 base pairs
(B) TYPE: nucleic acid
(C) STRANDEDNESS: double
(D) TOPOLOGY: linear

(xi) SEQUENCE DESCRIPTION: SEQ ID NO: 128:

CCCCGCCTCC AAAGCTAACC CTCGGGCTTG AGGGGAAGAR GCTGACTGTA CGTTCCTTCT 60

ACTCTGGCAC CACTCTCCAG GCTGCCATGG GGCCCAGCAC CCCTCTCCTC ATCTTGTTCC 120

TTTTGTCATG GTCGGGACCC CTCCAAGGAC AGCAGCACCA CCTTGTGGAG TACATGGAAC 180

GCCGACTAGC TGCTTTAGAG GAACGGCTGG CCCAGTGCCA GGACCAGAGT AGTCGGCATG 240

CTGCTGAGCT GCGGGACTTC AAGAACAAGA TGCTGCCACT GCTGGAGGTG GCAGAGAAGG 300

AGCGGGAGGC ACTCAGAACT GAGGCCGACA CCATCTCCGG GAGAGTGGAT CGTCTGGAGC 360

GGGAGGTAGA CTATCTGGAG ACCCAGAACC CAGCTCTGCC CTGTGTAGAG TTTGATGAGA 420

AGGTGACTGG AGGCCCTGGG ACCAAAGGCA AGGGAAGAAG GAATGAGAAG TACGATATGG 480

TGACAGACTG TGGCTACACA ATCTCTCAAG TGAGATCAAT GAAGATTCTG AAGCGATTTG 540

GTGGCCCAGC TGGTCTATGG ACCAAGGATC CACTGGGGCA AACAGAGAAG ATCTACGTGT 600

TAGATGGGAC ACAGAATGAC ACAGCCTTTG TCTTCCCAAG GCTGCGTGAC TTCACCCTTG 660

CCATGGCTGC CCGGAAAGCT TCCCGAGTCC GGGTGCCCTT CCCCTGGGTA GGCACAGGGC 720

AGCTGGTATA TGGTGGCTTT CTTTATTTTG CTCGGAGGCC TCCTGGAAGA CCTGGTGGAG 780

GTGGTGAGAT GGAGAACACT TTGCAGCTAA TCAAATTCCA CCTGGCAAAC CGAACAGTGG 840

TGGACAGCTC AGTATTCCCA GCAGAGGGGC TGATCCCCCC CTACGGCTTG ACAGCAGACA 900

CCTACATCGA CCTGGCAGCT GATGAGGAAG GTCTTTGGGC TGTCTATGCC ACCCGGGAGG 960

ATGACAGGCA CTTGTGTCTG GCCAAGTTAG ATCCACAGAC ACTGGACACA GAGCAGCAGT 1020

GGGACACACC ATGTCCCAGA GAGAATGCTG AGGCTGCCTT TKTCATCTGT GGGACCCTCT 1080

ATGTCGTCTA TAACACCCGT CCTGCCAGTC GGGCCCGCAT CCAGTGCTCC TTTGATGCCA 1140

GCGGACCCTG ACCCCTGAAC GGGCAGCACT CCCTTATTTT CCCCGCAGAT ATGGTGCCCA 1200

TGCCAGCCTC CGCTATAACC CCCGAGAACG CCAGCTCTAT GCCTGGGATG ATGGCTACCA 1260

GATTGTCTAT AAGCTGGAGA TGAGGAAGAA AGAGGAGGAG GTTTGAGGAG CTAGCCTTGT 1320

TTTTTGCATC TTTCTCACTC CCATACATTT ATATTATATC CCCACTAAAT TTCTTGTTCC 1380

TCATTCTTCA AATGTGGGCC AGTTGTGGCT CAAATCCTCT ATATTTTTAG CCAATGGCAA 1440

TCAAATTCTT TCAGCTCCTT TGTTTCATAC GGAACTCCAG ATCCTGAGTA ATCCTTTTAG 1500

AGCCCGAAGA GTCAAAACCC TCAATGTTCC CTCCTGCTCT CCTGCCCCAT GTCAACAAAT 1560

TTCAGGCTAA GGATGCCCCA GACCCAGGGC TCTAACCTTG TATGCGGGCA GGCCCAGGGA 1620

GCAGGCAGCA GTGTTCTTCC CCTCAGAGTG ACTTGGGGAG GGAGAAATAG GAGGAGACGT 1680

CCAGCTCTGT CCTCTCTTCC TCACTCCTCC CTTCAGTGTC CTGAGGAACA GGACTTTCTC 1740

CACATTGTTT TGTATTGCAA CATTTTGCAT TAAAAGGAAA ATCCAMAAAA AAAAAAAAAA 1800

AAAAAAAAAA AAAAAAAAAA AAAAAAAAAA AAAAAAAAAA AAAAAAAAAA AAAAAAAAAA 1860

ACTGCGGCCG CTGTCCCTTC TGTCGTCTTC TCGCAGCCGT ACCCTTCTGT CGTCTTCTCG 1920

CAGCC 1925

(2) INFORMATION FOR SEQ ID NO: 129:

(i) SEQUENCE CHARACTERISTICS:
(A) LENGTH: 2713 base pairs
(B) TYPE: nucleic acid
(C) STRANDEDNESS: double
(D) TOPOLOGY: linear

(xi) SEQUENCE DESCRIPTION: SEQ ID NO: 129:

TCCTACCTTC CCAACCCTCT GGCATCCCCA GCACTGATGG TCCTGGCATC CACGGCTGAG 60

GCCAGCCGTG ACTGCTTCCA TCCCTTGTCA GCAGCCACGA CCCTTTGGTG TACCTGTYTC 120

AGTTGACAAG GACGTGCATA TTCCTTTCAC CAACGGTTCC TATACCTTTG CCTCTATGTA 180

CCATCGGCAA GGTGGGGTGC CAGGCACTTT TGCCAATCGT GATTTCCCCC CTTCTCTACT 240

ACACCTCCAC CCTCAATTTG CTCCCCCAAA TCTAGATTGC ACCCCAATCA GTATGCTGAA 300

TCATAAGTGG TGTGGGGGTT TCCGGCCTTT GSCTCCACCC GRGGACCGGG RGAGYTATCA 360

GTCAGCTTTA CGCCGGCCAA GCGACTTAAG AACTGCCATG ACACAGAGTC TCCCCACTTG 420

CGCNTCTCAG ATGCAGATGG GAANGAATAT GACTTTGGGA CACAGCTGCM ATCTAGCTCC 480

CCCGGTTCAC TAAAGGTTGA TGACACTGGG AAGAAGATTT TTGCTGTCTC TGGCCTCATT 540

TCTGATCGGG AAGCCTCATC TAGCCCAGAG GNTCGGNAAT GACAGATGTA AGAAGAAAGC 600

AGCGGCATTG TTCGACAGCC AGGCCCCAAT TTGCCCCATC TGCCAGGTCC TGCTGAGGCC 660

CAGTGAGCTG CAGGAGCATA TGGAGCAGGA ACTGGAGCAG CTAGCCCAAC TGCCCTCGAG 720

CAAGAATTCC CTTCTGAAGG ATGCCATGGC TCCAGGCACC CCAAAGTCCC TCCTGTTGTC 780

TGCTTCCATC AAGAGGGAAG GAGAGTCTCC AACGGCATCA CCCCACTCAT CTGCCACCGA 840

TGACCTCCAC CATTCAGACA GATACCAGAC CTTTCTGCGA GTACGAGCCA ACCGGCAGAC 900

CCGAYTGAAT GYTCGGATTG GGAAAATGAA ACGGAGGAAG CAAGATGAAG GGCAGGTATG 960

TCCCCTGTGC AACCGCCCCC TGGCAGGATC GGAGCAGGAG ATGAGTAGGC ATGTGGAGCA 1020

TTGCCTTTCT AAGAGGGAAG GCTCCTGCAT GGCTGAGGAT GATGCTGTGG ACATCGAGCA 1080

TGAGAACAAC AACCGCTTTG AGGAGTATGA GTGGTGTGGA CAGAAGCGGA TACGGGCCAC 1140

CACTCTCCTG GAAGGTGGCT TCCGAGGCTC TGGCTTCATC ATGTGCAGCG GCAAAGAGAA 1200

CCCGGACAGT GATGCTGACT TGGATGTGGA TGGGGATGAC ACTCTGGAGT ATGGGAAGCC 1260

ACAATACACA GAGGCTGATG TCATCCCCTG CACAGGCGAG GAGCCTGGTG AAGCCAAGGA 1320

GAGAGAGGCA CTTCGGGGCG CAGTCCTAAA TGGCGGCCCT CCCAGCACGC GCATCACACC 1380

TGAGTTCTCT AAATGGGCCA GTGATGAGAT GCCATCCACC AGCAATGGTG AAAGCAGCAA 1440

GCAGGAGGCC ATGCAGAAGA CCTGCAAGAA CAGCGACATC GAGAAAATCA CCGAAGATTC 1500

AGCTGTGACC ACGTTTGAGG CTCTGAAGGC TCGGGTCAGA GAACTTGAAC GGCAGCTATC 1560

TCGTGGGGAC CGTTACAAAT GCCTCATCTG CATGGACTCG TACTCGATGC CCCTAACGTC 1620

CATCCAGTGT TGGCACGTGC ACTGCGAGGA GTGCTGGCTG CGGACCCTGG GTGCCAAGAA 1680

GCTCTGCCCT CAGTGCAACA CGATCACAGC GCCCGGAGAC CTGCGGAGGA TCTACTTGTG 1740

AGCTATCTGC CCCAGGCAGG CCTCGCCTCC AGCAGCCCCA CCTGCCCCCA GCCTCTGTGA 1800

CAGTGACCGT YTCCCTTTGT ACATACTTGC ACACAGGTTC CCCATGTACA TACATGCACA 1860

TACTCAAACA TGCGTACACA CACACACATT TACACACGCA GGACTCTGGA GCCAGAGTAG 1920

AGGCTGTGGC CCAGGCACTA CCTGCTGGCT CCCACCTATG GTTTGGGGGC CATACCTGTT 1980

CCAGCTCTGT TCCCAGGGTG GGGCAGGGAG GTGGGGGTTG GGGGAGTAGT GGGGCACGGC 2040

TCCTAAGATC CAGCCCCCAT ACTGACAGAC GGACAGACAG ACATGCAAAC ACCAGACTGA 2100

AGCACATGTA ATATAGACCG TGTATGTTTA CAATGTTGTG TATAAATGGG ACAACTCCTC 2160

GCCCTCTACC TGTCCCCTCC CCCTTTGGTT GTATGATTTT CTTCTTTTTT AAGAACCCCT 2220

GGAAGCAGCG CCTCCTTCAG GGTTGGCTGG GAGCTCGGCC CATCCACCTC TTGGGGTAYC 2280

TGCCTCTCTC TCTCCTGTGG TGTCCCTTCC CTCTCCCATG TGCTCGGTGT TCAGTGGTGT 2340

ATATTTCTTC TCCCAGACAT GGGGCACACG CCCCAAGGGA CATGATCCTC TCCTTAGTCT 2400

TAGCTCATGG GGCTCTTTAT AAGGAGTTGG GGGGTAGAGG CAGGAAATGG GAACCGAGCT 2460

GAAGCAGAGG CTGAGTTAGG GGGCTAGAGG ACAGTGCTCC TGGCCACCCA GCCTCTGCTG 2520

AGAACCATTC CTGGGATTAG AGCTGCCTTT CCCAGGGAAA AAGTGTCGTC TCCCCGACCC 2580

TCCCGTGGGC CCTGTGGTGT GATGCTGTGT CTGTATATTC TATACAAAGG TACTTGTCCT 2640

TTCCCTTTGT AAACTACATT TGACATGGAT TAAACCAGTA TAAACAGTTA AAAAAAAAAA 2700

AAAAAAAACT CGA 2713

(2) INFORMATION FOR SEQ ID NO: 130:

(i) SEQUENCE CHARACTERISTICS:
(A) LENGTH: 1011 base pairs
(B) TYPE: nucleic acid
(C) STRANDEDNESS: double
(D) TOPOLOGY: linear

(xi) SEQUENCE DESCRIPTION: SEQ ID NO: 130:

AGAGGACGGT GTGACCCGGG AGGAAGTAGA GCCTGAGGAG GCTGAAGAAG GCATCTCTGA 60

GCAACCCTGC CCAGCTGACA CAGAGGTGGT GGAAGACTCC TTGAGGCAGC GTAAAAGTCA 120

GCATGCTGAC AAGGGACTGT AGATTTAATG ATGCGTTTTC AAGAATACAC ACCAAAACAA 180

TATGTCAGCT TCCCTTTGGC CTGCAGTTTG TACCAAATCC TTAATTTTTY YTGAATGAGC 240

AAGCTTCTCT TAAAAGATGC TCTCTAGTCA TTTGGTCTCA TGGCAGTAAG CCTCATGTAT 300

ACTAAGGAGA GTCTTCCAGG TGTGACAATC AGGATATAGA AAAACAAACG TAGTGTNTGG 360

GATCTGTTTG GAGACTGGGA TGGGAACAAG TTCATTTACT TAGGGGTCAG AGAGTCTCGA 420

CCAGAGGAGG CCATTCCCAG TCCTAATCAG CACCTTCCAG AGACAAGGCT GCAGGCCCTG 480

TGAAATGAAA GCCAAGCAGG AGCCTTGGCT CTGAGNCATC CCCAAAGTGT AACGTAGAAG 540

CCTTGCATCC TTTTCTTGTG TAAAGTATTT ATTTTTGTCA AATTGCAGGA AACATCAGGC 600

ACCACAGTGC ATGAAAAATC TTTCACAGCT AGAAATTGAA AGGGCCTTGG GTATAGAGAG 660

CAGCTCAGAA GTCATCCCAG CCCTCTGAAT CTCCTGTGCT ATGTTTTATT TCTTACCTTT 720

AATTTTTCCA GCATTTCCAC CATGGGCATT CAGGCTCTCC ACACTCTTCA CTATTATCTC 780

TTGGTCAGAG GACTCCAATA ACAGCCAGGT TTACATGAAC TGTGTTTGTT CATTCTGACC 840

TAAGGGGTTT AGATAATCAG TAACCATAAC CCCTGAAGCT GTGACTGCCA AACATCTCAA 900

ATGAAATGTT GTRGCCATCA GAGACTCAAA AGGAAGTAAG GATTTTACAA GACAGATTAA 960

AAAAAAATTG TTTTGTCCAA AAAANAAAAA AAAAAAACTC GAAGGGGGGG C 1011

(2) INFORMATION FOR SEQ ID NO: 131:

(i) SEQUENCE CHARACTERISTICS:
(A) LENGTH: 2278 base pairs
(B) TYPE: nucleic acid
(C) STRANDEDNESS: double
(D) TOPOLOGY: linear

(xi) SEQUENCE DESCRIPTION: SEQ ID NO: 131:

GTAATTCGGC ACGAGGCGCC CAACATGGCG GGTGGGCGCT GCGGCCCGCA SCTAACGGCG 60

CTCCTGGCCG CCTGGATCGC GGCTGTGGCG GCGACGGCAG GCCCCGAGGA GGCCGCGCTG 120

CCGCCGGAGC AGAGCCGGGT CCAGCCCATG ACCGCCTCCA ACTGGACGCT GGTGATGGAG 180

GGCGAGTGGA TGCTGAAATT TTACGCCCCA TGGTGTCCAT CCTGCCAGCA GACTGATTCA 240

GAATGGGAGG CTTTTGCAAA GAATGGTGAA ATACTTCAGA TCAGTGTGGG GAAGGTAGAT 300

GTCATTCAAG AACCAGGTTT GAGTGGCCGC TTCTTTGTCA CCACTCTCCC AGCATTTTTT 360

CATGCAAAGG ATGGGATATT CCGCCGTTAT CGTGGCCCAG GAATCTTCGA AGACCTGCAG 420

AATTATATCT TAGAGAAGAA ATGGCAATCA GTCGAGCCTC TGACTGGCTG GAAATCCCCG 480

GCTTCTCTAA CGATGTCTGG AATGGCTGGT CTTTTTAGCA TCTCTGGCAA GATATGGCAT 540

CTTCACAACT ATTTCACAGT GACTCTTGGA ATTCCTGCTT GGTGTTCTTA TGTCTTTTTC 600

GTCATAGCCA CCTTGGTTTT TGGCCTTTTT ATGGGTCTGG TCTTGGTGGT AATATCAGAA 660

TGTTTCTATG TGCCACTTCC AAGGCATTTA TCTGAGCGTT CTGAGCAGAA TCGGAGATCA 720

GAGGAGGCTC ATAGAGCTGA ACAGTTGCAG GATGCGGAGG AGGAAAAAGA TGATTCAAAT 780

GAAGAAGAAA ACAAAGACAG CCTTGTAGAT GATGAAGAAG AGAAAGAAGA TCTTGGCGAT 840

GAGGATGAAG CAGAGGAAGA AGAGGAGGAG GACAACTTGG CTGCTGGTGT GGATGAGGAG 900

AGAAGTGAGG CCAATGATCA GGGGCCCCCA GGAGAGGACG GTGTGACCCG GGAGGNAAGT 960

AGAGCCTGAG GAGGCTGAAG AAGGCATCTC TGAGCAACCC TGCCCAGCTG ACACAGAGGT 1020

GGTGGAAGAC TCCTTGAGGC AGCGTAAAAG TCAGCATGCT GNCAAGGGAC TGTAGATTTA 1080

ATGATGCGTT TTCAAGAATA CACACCAAAA CAATATGTCA GCTTCCCTTT GGCCTGCAGT 1140

TTGTACCAAA TCCTTAATTT TTCCTGAATG AGCAAGCTTC TCTTAAAAGA TGCTCTCTAG 1200

TCATTTGGTC TCATGGCAGT AAGCCTCATG TATACTAAGG AGAGTCTTCC AGGTGTGACA 1260

ATCAGGATAT AGAAAAACAA ACGTAGTGTN TGGGATCTGT TTGGAGACTG GGATGGGAAC 1320

AAGTTCATTT ACTTAGGGGT CAGAGAGTCT CGACCAGAGG AGGCCATTCC CAGTCCTAAT 1380

CAGCACCTTC CAGAGACAAG GCTGCAGGCC TGTGAAATGA AAGCCAAGCA GGAGCCTTGG 1440

CTCTGAGGCA TCCCCAAAGT GTAACGTAGA AGCCTTGCAT CCTTTTCTTG TGTAAAGTAT 1500

TTATTTTTGT CAAATTGCAG GAAACATCAG GCACCACAGT GCATGAAAAA TCTTTCACAG 1560

CTAGAAATTG AAAGGGCCTT GGGTATAGAG AGCAGCTCAG AAGTCATCCC AGCCCTCTGA 1620

ATCTCCTGTG CTATGTTTTA TTTCTTACCT TTAATTTTTC CAGCATTTCC ACCATGGGCA 1680

TTCAGGCTCT CCACACTCTT CACTATTATC TCTTGGTCAG AGGACTCCAA TAACAGCCAG 1740

GTTTACATGA ACTGTGTTTG TTCATTCTGA CCTAAGGGGT TTAGATAATC AGTAACCATA 1800

ACCCCTGAAG CTGTGACTGC CAAACATCTC AAATGAAATG TTGTRGCCAT CAGAGACTCA 1860

AAAGGAAGTA AGGATTTTAC AAGACAGATT AAAAAAAAAT TGTTTTGTCC NAAAATATAG 1920

TTGTTGTTGA TTTTTTTTTA AGTTTTCTAA GCAATATTTT TCAAGCCAGA AGTCCTCTAA 1980

GTCTTGCCAG TACAAGGTAG TCTTGTGAAG AAAAGTTGAA TACTGTTTTG TTTTCATCTC 2040

AAGGGGTTCC CTGGGTCTTG AACTACTTTA ATAATAACTA AAAAACCACT TCTGATTTTC 2100

CTTCAGTGAT GTGCTTTTGG TGAAAGAATT AATGAACTCC AGTACCTGAA AGTGAAAGAT 2160

TTGATTTTGT TTCCATCTTC TGTAATCTTC CAAAGAATTA TATCTTTGTA AATCTCTCAA 2220

TACTCAATCT ACTGTAAGTA CCCAGGGRGG STAATTTCYT TAAAAAAAAA AAAAAAAA 2278

(2) INFORMATION FOR SEQ ID NO: 132:

(i) SEQUENCE CHARACTERISTICS:
(A) LENGTH: 1088 base pairs
(B) TYPE: nucleic acid
(C) STRANDEDNESS: double
(D) TOPOLOGY: linear

(xi) SEQUENCE DESCRIPTION: SEQ ID NO: 132:

GGCAGGGGCG GCGTGAACCC GTCGGGCACT GTGTCCCTGA CAATGGGAAC AGCCGACAGT 60

GATGAGATGG CCCCGGAGCC CCACAGCACA CCCACATCGA TGTGCACATC CACCAGGAGT 120

CTGCCCTGGC CAAGCTCCTG CTCACCTGCT GCTCTGCGCT GCGGCCCCGG GCCACCCAGG 180

CCAGGGGCAG CANCCGGCTG CTGGTGGCCT CGTGGGTGAT GCAGATCGTG CTGGGGATCT 240

TGAGTGCAGT CCTAGGAGGA TTTTTCTACA TCCGCGACTA CACCCTCCTC GTCACCTCGG 300

GAGCTGCCAT CTGGACAGGG GCTGTGGCTG TGCTGGCTGG AGCTGCTGCC TTCATTTAYG 360

AGAAACGGGG TGGTACATAC TGGGCCCTGC TGAGGACTCT GCTARCGCTG GCAGCTTTCT 420

CCACAGCCAT CGCTGCCCTC AAACTTTGGA ATGAAGATTT CCGATATGGC TACTCTTATT 480

ACAACAGTGC CTGCCGCATC TCCAGCTCGA GTGACTGGAA CACTCCAGCC CCCACTCAGA 540

GTCCAGAAGA AGTCAGAAGG CTACACCTAT GTACCTCCTT CATGGACATG CTGAAGGCCT 600

TGTTCAGAAC CCTTCAGGCC ATGCTCTTGG GTGTCTGGAT TCTGCTGCTT CTGGCATCTC 660

TGGCCCCTCT GTGGCTGTAC TGCTGGAGAA TGTTCCCAAC CAAAGGGAAA AGAGACCAGA 720

AGGAAATGTT GGAAGTGAGT GGAATCTAGC CATGCCTCTC CTGATTATTA GTGCCTGGTG 780

CTTCTGCACC GGGCGTCCCT GCATCTGACT GCTGGAAGAA GAACCAGACT GAGGAAAAGA 840

GGCTCTTCAA CAGCCCCAGT TATCCTGGCC CCATGACCGT GGCCACAGCC CTGCTCCAGC 900

AGCACTTGCC CATTCCTTAC ACCCCTTCCC CATCCTGCTC CGCTTCATGT CCCCTCCTGA 960

GTAGTCATGT GATAATAAAC TCTCATGTTA TTGTTCCNAA AAAAAAAAAA AAAAAAAAAT 1020

TGGGGGGGGG CCGGTACCCA TTGGGCCTNN GGGGGNGGTT TAAAATTAAT GGGGGGGGTT 1080

TAAAAGGG 1088

(2) INFORMATION FOR SEQ ID NO: 133:

(i) SEQUENCE CHARACTERISTICS:
(A) LENGTH: 553 base pairs
(B) TYPE: nucleic acid
(C) STRANDEDNESS: double
(D) TOPOLOGY: linear

(xi) SEQUENCE DESCRIPTION: SEQ ID NO: 133:

GGCAGAGAGC AGATGGCCTT GACACCAGCA GGGTGACATC CGCTATTGCT ACTTCTCTGC 60

TCCCCCACAG TTCCTCTGGA CTTCTCTGGA CCACAGTCCT CTGCCAGACC CCTGCCAGAC 120

CCCAGTCCAC CATGATCCAT CTGGGTCACA TCCTCTTCCT GCTTTTGCTC CCAGTGGCTG 180

CAGCTCAGAC GACTCCAGGA GAGAGATCAT CACTCCCTGC CTTTTACCCT GGCACTTCAG 240

GCTCTTGTTC CGGATGTGGG TCCCTCTCTC TGCCGCTCCT GGCAGGCCTC GTGGCTGCTG 300

ATGCGGTGGC ATCGCTGCTC ATCGTGGGGG CGGTGTTCCT GTGCGCACGC CCACGCCGCA 360

GCCCCGCCCA AGATGGCAAA GTCTACATCA ACATGCCAGG CAGGGGCTGA CCCTCCTGCA 420

GCTTGGACCT TTGACTTCTG ACCCTCTCAT CCTGGATGGT GTGTGGTGGC ACAGGAACCC 480

CCGCCCCAAC TTTTGGATTG TAATAAAACA ATTGAAACAC CAAAAAAAAA AAAAAAAAAA 540

AAAAAAAAAA AAA 553

(2) INFORMATION FOR SEQ ID NO: 134:

(i) SEQUENCE CHARACTERISTICS:
(A) LENGTH: 467 amino acids
(B) TYPE: amino acid
(D) TOPOLOGY: linear
(xi) SEQUENCE DESCRIPTION: SEQ ID NO: 134:

Met Arg Pro Gln Glu Leu Pro Arg Leu Ala Phe Pro Leu Leu Leu Leu
1               5                   10                  15

Leu Leu Leu Leu Leu Pro Pro Pro Pro Cys Pro Ala His Ser Ala Thr
            20                  25                  30

Arg Phe Asp Pro Thr Trp Glu Ser Leu Asp Ala Arg Gln Leu Pro Ala
        35                  40                  45

Trp Phe Asp Gln Ala Lys Phe Gly Ile Phe Ile His Trp Gly Val Phe
    50                  55                  60

Ser Val Pro Ser Phe Gly Ser Glu Trp Phe Trp Trp Tyr Trp Gln Lys
65                  70                  75                  80

Glu Lys Ile Pro Lys Tyr Val Glu Phe Met Lys Asp Asn Tyr Pro Pro
                85                  90                  95

Xaa Phe Lys Tyr Glu Asp Phe Gly Pro Leu Phe Thr Ala Lys Phe Phe
            100                 105                 110

Asn Ala Asn Gln Trp Ala Xaa Ile Phe Gln Ala Ser Gly Ala Lys Tyr
        115                 120                 125

Ile Val Leu Thr Ser Lys His His Glu Gly Phe Thr Leu Trp Gly Ser
    130                 135                 140

Glu Tyr Ser Trp Asn Trp Asn Ala Ile Asp Glu Gly Pro Lys Arg Asp
145                 150                 155                 160

Ile Val Lys Glu Leu Glu Val Ala Ile Arg Asn Arg Thr Asp Leu Arg
                165                 170                 175

Phe Gly Leu Tyr Tyr Ser Leu Phe Glu Trp Phe His Pro Leu Phe Leu
            180                 185                 190

Glu Asp Glu Ser Ser Ser Phe His Lys Arg Gln Phe Pro Val Ser Lys
        195                 200                 205

Thr Leu Pro Glu Leu Tyr Glu Leu Val Asn Asn Tyr Gln Pro Glu Val
    210                 215                 220

Leu Trp Ser Asp Gly Asp Gly Gly Ala Pro Asp Gln Tyr Trp Asn Xaa
225                 230                 235                 240

Thr Gly Phe Leu Ala Trp Leu Tyr Asn Glu Ser Pro Val Arg Gly Thr
                245                 250                 255

Val Val Thr Asn Asp Arg Trp Gly Ala Gly Ser Ile Cys Lys His Gly
            260                 265                 270

Gly Phe Tyr Thr Cys Ser Asp Arg Tyr Asn Pro Gly His Leu Leu Pro
        275                 280                 285

His Lys Trp Glu Asn Cys Met Thr Ile Asp Lys Leu Ser Trp Gly Tyr
    290                 295                 300

Arg Arg Glu Ala Gly Ile Ser Asp Tyr Leu Thr Ile Glu Glu Leu Val
305                 310                 315                 320

Lys Gln Leu Val Glu Thr Val Ser Cys Gly Gly Asn Leu Leu Met Asn
                325                 330                 335

Ile Gly Pro Thr Leu Asp Gly Thr Ile Ser Val Val Phe Glu Glu Arg
            340                 345                 350

Leu Arg Gln Met Gly Ser Trp Leu Lys Val Asn Gly Glu Ala Ile Tyr
        355                 360                 365

Glu Thr His Thr Trp Arg Ser Gln Asn Asp Thr Val Thr Pro Asp Val
    370                 375                 380

Trp Tyr Thr Ser Lys Pro Lys Glu Lys Leu Val Tyr Ala Ile Phe Leu
385                 390                 395                 400

Lys Trp Pro Thr Ser Gly Gln Leu Phe Leu Gly His Pro Lys Ala Ile
                405                 410                 415

Leu Gly Ala Thr Glu Val Lys Leu Leu Gly His Gly Gln Pro Leu Asn
            420                 425                 430

Trp Ile Ser Leu Glu Gln Asn Gly Ile Met Val Glu Leu Pro Gln Leu
        435                 440                 445

Thr Ile His Gln Met Pro Cys Lys Trp Gly Trp Ala Leu Ala Leu Thr
    450                 455                 460

Asn Val Ile
465

(2) INFORMATION FOR SEQ ID NO: 135:

(i) SEQUENCE CHARACTERISTICS:
(A) LENGTH: 222 amino acids
(B) TYPE: amino acid
(D) TOPOLOGY: linear
(xi) SEQUENCE DESCRIPTION: SEQ ID NO: 135:

Met Trp Ser Ala Gly Arg Gly Gly Ala Ala Trp Pro Val Leu Leu Gly
1               5                   10                  15

Leu Leu Leu Ala Leu Leu Val Pro Gly Gly Gly Ala Ala Lys Thr Gly
            20                  25                  30

Ala Glu Leu Val Thr Cys Gly Ser Val Leu Lys Leu Leu Asn Thr His
        35                  40                  45

His Arg Val Arg Leu His Ser His Asp Ile Lys Tyr Gly Ser Gly Ser
    50                  55                  60

Gly Gln Gln Ser Val Thr Gly Val Glu Ala Ser Asp Asp Ala Asn Ser
65                  70                  75                  80

Tyr Trp Arg Ile Arg Gly Gly Ser Glu Gly Gly Cys Arg Arg Gly Ser
                85                  90                  95

Pro Val Arg Cys Gly Gln Ala Val Arg Leu Thr His Val Leu Thr Gly
            100                 105                 110

Lys Asn Leu His Thr His His Phe Pro Ser Pro Leu Ser Asn Asn Gln
        115                 120                 125

Glu Val Ser Ala Phe Gly Glu Asp Gly Glu Gly Asp Asp Leu Asp Leu
    130                 135                 140

Trp Thr Val Arg Cys Ser Gly Gln His Trp Glu Arg Glu Ala Ala Val
145                 150                 155                 160

Arg Phe Gln His Val Gly Thr Ser Val Phe Leu Ser Val Thr Gly Glu
                165                 170                 175

Gln Tyr Gly Ser Pro Ile Arg Gly Gln His Glu Val His Gly Met Pro
            180                 185                 190

Ser Ala Asn Thr His Asn Thr Trp Lys Ala Met Glu Gly Ile Phe Ile
        195                 200                 205

Lys Pro Ser Val Glu Pro Ser Ala Gly His Asp Glu Leu Xaa

210                215                220

(2) INFORMATION FOR SEQ ID NO: 136:

(i) SEQUENCE CHARACTERISTICS:
(A) LENGTH: 156 amino acids
(B) TYPE: amino acid
(D) TOPOLOGY: linear
(xi) SEQUENCE DESCRIPTION: SEQ ID NO: 136:

Met Val Ile Glu Ile Ser Asn Lys Thr Ser Ser Ser Ser Thr Cys Ile
1               5                   10                  15

Leu Val Leu Leu Val Ser Phe Cys Leu Leu Leu Val Pro Ala Met Tyr
            20                  25                  30

Ser Ser Asp Thr Arg Gly Ser Leu Pro Ala Glu His Gly Val Leu Ser
        35                  40                  45

Arg Gln Leu Arg Ala Leu Pro Ser Glu Asp Pro Tyr Gln Leu Glu Leu
    50                  55                  60

Pro Ala Leu Gln Ser Glu Val Pro Lys Asp Ser Thr His Gln Trp Leu
65                  70                  75                  80

Asp Gly Ser Asp Cys Val Leu Gln Ala Pro Gly Asn Thr Ser Cys Leu
                85                  90                  95

Leu His Tyr Met Pro Gln Ala Pro Ser Ala Glu Pro Pro Leu Glu Trp
            100                 105                 110

Pro Phe Pro Asp Leu Phe Ser Glu Pro Leu Cys Arg Gly Pro Ile Leu
        115                 120                 125

Pro Leu Gln Ala Asn Leu Thr Arg Lys Gly Gly Trp Leu Pro Thr Gly
    130                 135                 140

Ser Pro Ser Val Ile Leu Gln Asp Arg Tyr Ser Gly
145                 150                 155

(2) INFORMATION FOR SEQ ID NO: 137:

(i) SEQUENCE CHARACTERISTICS:
(A) LENGTH: 233 amino acids
(B) TYPE: amino acid
(D) TOPOLOGY: linear
(xi) SEQUENCE DESCRIPTION: SEQ ID NO: 137:

Met Met Ile Leu Phe Asn Leu Leu Ile Phe Leu Cys Gly Ala Ala Leu
1               5                   10                  15

Leu Ala Val Gly Ile Trp Val Ser Ile Asp Gly Ala Ser Phe Leu Lys
            20                  25                  30

Ile Phe Gly Pro Leu Ser Ser Ser Ala Met Gln Phe Val Asn Val Gly
        35                  40                  45

Tyr Phe Leu Ile Ala Ala Gly Val Val Val Phe Ala Leu Gly Phe Leu
    50                  55                  60

Gly Cys Tyr Gly Ala Lys Thr Glu Ser Lys Cys Ala Leu Val Thr Phe
65                  70                  75                  80

Phe Phe Ile Leu Leu Leu Ile Phe Ile Ala Glu Val Ala Ala Ala Val
                85                  90                  95

Val Ala Leu Val Tyr Thr Thr Met Ala Glu His Phe Leu Thr Leu Leu
            100                 105                 110

Val Val Pro Ala Ile Lys Lys Asp Tyr Gly Ser Gln Glu Asp Phe Thr
        115                 120                 125

Gln Val Trp Asn Thr Thr Met Lys Gly Leu Lys Cys Cys Gly Phe Thr
    130                 135                 140

Asn Tyr Thr Asp Phe Glu Asp Ser Pro Tyr Phe Lys Glu Asn Ser Ala
145                 150                 155                 160

Phe Pro Pro Phe Cys Cys Asn Asp Asn Val Thr Asn Thr Ala Asn Glu
                165                 170                 175

Thr Cys Thr Lys Gln Lys Ala His Asp Gln Lys Val Glu Gly Cys Phe
            180                 185                 190

Asn Gln Leu Leu Tyr Asp Ile Arg Thr Asn Ala Val Thr Val Gly Gly
        195                 200                 205

Val Ala Ala Gly Ile Gly Gly Leu Glu Leu Ala Ala Met Ile Val Ser
    210                 215                 220

Met Tyr Leu Tyr Cys Asn Leu Gln Xaa
225                 230

(2) INFORMATION FOR SEQ ID NO: 138:

     (i) SEQUENCE CHARACTERISTICS:
          (A) LENGTH: 61 amino acids
          (B) TYPE: amino acid
          (D) TOPOLOGY: linear
     (xi) SEQUENCE DESCRIPTION: SEQ ID NO: 138:

Met Gly Ser Ser Arg Trp Ser Val Ala Cys Pro Thr Gly Leu Gly Val
 1               5                  10                  15

Leu Met Leu Gly Leu Gly Gly Asp His Pro Pro Gly Ser Gln Val Asp
            20                  25                  30

Pro Leu Leu Met Gly Xaa Cys Val Arg Pro Xaa Leu Pro Glu Leu Thr
        35                  40                  45

Ala Xaa Trp Arg Glu Xaa Gln Xaa Arg Ser Ala Ser Ala
    50                  55                  60

(2) INFORMATION FOR SEQ ID NO: 139:

(i) SEQUENCE CHARACTERISTICS:
(A) LENGTH: 73 amino acids
(B) TYPE: amino acid
(D) TOPOLOGY: linear
(xi) SEQUENCE DESCRIPTION: SEQ ID NO: 139:

Met Gly Trp Leu Phe Leu Lys Val Leu Leu Ala Gly Val Ser Phe Ser
1               5                   10                  15

Gly Phe Leu Tyr Pro Leu Val Asp Phe Cys Ile Ser Gly Lys Thr Arg
            20                  25                  30

Gly Gln Lys Pro Asn Phe Val Ile Ile Leu Ala Asp Asp Met Gly Trp
        35                  40                  45

Gly Asp Trp Gly Ala Asn Trp Ala Glu Thr Lys Asp Thr Ala Asn Leu
    50                  55                  60

Asp Lys Met Ala Ser Glu Gly Met Xaa
65                  70

(2) INFORMATION FOR SEQ ID NO: 140:

(i) SEQUENCE CHARACTERISTICS:
(A) LENGTH: 377 amino acids
(B) TYPE: amino acid
(D) TOPOLOGY: linear
(xi) SEQUENCE DESCRIPTION: SEQ ID NO: 140:

Met His Gly Asn Glu Ala Leu Gly Arg Glu Leu Leu Leu Leu Leu Met
1               5                   10                  15

Gln Phe Leu Cys His Glu Phe Leu Arg Gly Asn Pro Arg Val Thr Arg
            20                  25                  30

Leu Leu Ser Glu Met Arg Ile His Leu Leu Pro Ser Met Asn Pro Asp
        35                  40                  45

Gly Tyr Glu Ile Ala Tyr His Arg Gly Ser Glu Leu Val Gly Trp Ala
    50                  55                  60

Glu Gly Arg Trp Asn Asn Gln Ser Ile Asp Leu Asn His Asn Phe Ala
65                  70                  75                  80

Asp Leu Asn Thr Pro Leu Trp Glu Ala Gln Asp Asp Gly Lys Val Pro
                85                  90                  95

His Ile Val Pro Asn His His Leu Pro Leu Pro Thr Tyr Tyr Thr Leu
            100                 105                 110

Pro Asn Ala Thr Val Ala Pro Glu Thr Arg Ala Val Ile Lys Trp Met
        115                 120                 125

Lys Arg Ile Pro Phe Val Leu Ser Ala Asn Leu His Gly Gly Glu Leu
    130                 135                 140

Val Val Ser Tyr Pro Phe Asp Met Thr Arg Thr Pro Trp Ala Ala Arg
145                 150                 155                 160

Glu Leu Thr Pro Thr Pro Asp Asp Ala Val Phe Arg Trp Leu Ser Thr
                165                 170                 175

Val Tyr Ala Gly Ser Asn Leu Ala Met Gln Asp Thr Ser Arg Arg Pro
            180                 185                 190

Cys His Ser Gln Asp Phe Ser Val His Gly Asn Ile Ile Asn Gly Ala
        195                 200                 205

Asp Trp His Thr Val Pro Gly Ser Met Asn Asp Phe Ser Tyr Leu His
    210                 215                 220

Thr Asn Cys Phe Glu Val Thr Val Glu Leu Ser Cys Asp Lys Phe Pro
225                 230                 235                 240

His Glu Asn Glu Leu Pro Gln Glu Trp Glu Asn Asn Lys Asp Ala Leu
                245                 250                 255

Leu Thr Tyr Leu Glu Gln Val Arg Met Gly Ile Ala Gly Val Val Arg
            260                 265                 270

Asp Lys Asp Thr Glu Leu Gly Ile Ala Asp Ala Val Ile Ala Val Asp
        275                 280                 285

Gly Ile Asn His Asp Val Thr Thr Ala Trp Gly Gly Asp Tyr Trp Arg
    290                 295                 300

Leu Leu Thr Pro Gly Asp Tyr Met Val Thr Ala Ser Ala Glu Gly Tyr
305                 310                 315                 320

His Ser Val Thr Arg Asn Cys Arg Val Thr Phe Glu Glu Gly Pro Phe
                325                 330                 335

Pro Cys Asn Phe Val Leu Thr Lys Thr Pro Lys Gln Arg Leu Arg Glu
            340                 345                 350

Leu Leu Ala Ala Gly Ala Lys Val Pro Pro Asp Leu Arg Arg Arg Leu
        355                 360                 365

Glu Arg Leu Arg Gly Gln Lys Asp Xaa
    370                 375

(2) INFORMATION FOR SEQ ID NO: 141:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 141:

Met Ile Cys Leu Ile Leu Leu Leu Gln Ala Val Val Phe Leu Arg Ser
  1               5                  10                  15

Leu His Val Val His Asn Phe Gln Ile Leu Asp Leu Ser Gly Thr Ser

            20                  25                  30

Tyr Pro Lys Phe Tyr Gln Thr Leu His Arg Gln
        35                  40

(2) INFORMATION FOR SEQ ID NO: 142:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 142:

(2) INFORMATION FOR SEQ ID NO: 143:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 143:

Met Gly Ser Cys Ser Lys Asn Arg Ser Phe Phe Trp Met Thr Gly Leu
1               5                   10                  15

Leu Val Phe Ile Ser Leu Leu Leu Ser Glu Trp Gln Gly Pro Trp Glu
            20                  25                  30

Gly Arg Ala Ile Gly Glu Gly Trp Ala Ser Trp Ala Leu Thr Asn Gly
        35                  40                  45

Trp Ala Val Gln Leu Leu Met Ser Leu Gly Asn Asn Thr Glu Lys His
    50                  55                  60

Ser Val Met Ile Tyr Glu
65                  70

(2) INFORMATION FOR SEQ ID NO: 144:

(i) SEQUENCE CHARACTERISTICS:
(A) LENGTH: 483 amino acids
(B) TYPE: amino acid
(D) TOPOLOGY: linear
(xi) SEQUENCE DESCRIPTION: SEQ ID NO: 144:

Met Ala Thr Gly Gly Gly Ile Arg Ala Met Thr Ser Leu Tyr Gly Gln

1               5                   10                  15

Leu Ala Gly Leu Lys Glu Leu Gly Leu Leu Asp Cys Xaa Ser Tyr Ile
                20                  25                  30

Thr Gly Ala Ser Gly Ser Thr Trp Ala Leu Ala Asn Leu Tyr Lys Asp
            35                  40                  45

Pro Glu Trp Ser Gln Lys Asp Leu Ala Gly Pro Thr Glu Leu Leu Lys
    50                  55                  60

Thr Gln Val Thr Lys Asn Lys Leu Gly Val Leu Ala Pro Ser Gln Leu
65                  70                  75                  80

Gln Arg Tyr Arg Gln Glu Leu Ala Glu Arg Ala Arg Leu Gly Tyr Pro
                85                  90                  95

Ser Cys Phe Thr Asn Leu Trp Ala Leu Ile Asn Glu Ala Leu Leu His
                100                 105                 110

Asp Glu Pro His Asp His Lys Leu Ser Asp Gln Arg Glu Ala Leu Ser
            115                 120                 125

His Gly Gln Asn Pro Leu Pro Ile Tyr Cys Ala Leu Asn Thr Lys Gly
    130                 135                 140

Gln Ser Leu Thr Thr Phe Glu Phe Gly Glu Trp Cys Glu Phe Ser Pro
145                 150                 155                 160

Tyr Glu Val Gly Phe Pro Lys Tyr Gly Ala Phe Ile Pro Ser Glu Leu
                165                 170                 175

Phe Gly Ser Glu Phe Phe Met Gly Gln Leu Met Lys Arg Leu Pro Glu
                180                 185                 190

Ser Arg Ile Cys Phe Leu Glu Gly Ile Trp Ser Asn Leu Tyr Ala Ala
            195                 200                 205

Asn Leu Gln Asp Ser Leu Tyr Trp Ala Ser Glu Pro Ser Gln Phe Trp
    210                 215                 220

Asp Arg Trp Val Arg Asn Gln Ala Asn Leu Asp Lys Glu Gln Val Pro
225                 230                 235                 240

Leu Leu Lys Ile Glu Glu Pro Pro Ser Thr Ala Gly Arg Ile Ala Glu
                245                 250                 255

Phe Phe Thr Asp Leu Leu Thr Trp Arg Pro Leu Ala Gln Ala Thr His
                260                 265                 270

Asn Phe Leu Arg Gly Leu His Phe His Lys Asp Tyr Phe Gln His Pro
            275                 280                 285

His Phe Ser Thr Trp Lys Ala Thr Thr Leu Asp Gly Leu Pro Asn Gln
    290                 295                 300

Leu Thr Pro Ser Glu Pro His Leu Cys Leu Leu Asp Val Gly Tyr Leu
305                 310                 315                 320

Ile Asn Thr Ser Cys Leu Pro Leu Leu Gln Pro Thr Arg Asp Val Asp

325                 330                 335

Leu Ile Leu Ser Leu Asp Tyr Asn Leu His Gly Ala Phe Gln Gln Leu
            340                 345                 350

Gln Leu Leu Gly Arg Phe Cys Gln Glu Gln Gly Ile Pro Phe Pro Pro
        355                 360                 365

Ile Ser Pro Ser Pro Glu Glu Gln Leu Gln Pro Arg Glu Cys His Thr
    370                 375                 380

Phe Ser Asp Pro Thr Cys Pro Gly Ala Pro Ala Val Leu His Phe Pro
385                 390                 395                 400

Leu Val Ser Asp Ser Phe Arg Glu Tyr Ser Ala Pro Gly Val Arg Arg
                405                 410                 415

Thr Pro Glu Glu Ala Ala Ala Gly Glu Val Asn Leu Ser Ser Ser Asp
            420                 425                 430

Ser Pro Tyr His Tyr Thr Lys Val Thr Tyr Ser Gln Glu Asp Val Asp
        435                 440                 445

Lys Leu Leu His Leu Thr His Tyr Asn Val Cys Asn Asn Gln Glu Gln
    450                 455                 460

Leu Leu Glu Ala Leu Arg Gln Ala Val Gln Arg Arg Arg Gln Arg Arg
465                 470                 475                 480

Pro His Xaa

(2) INFORMATION FOR SEQ ID NO: 145:

     (i) SEQUENCE CHARACTERISTICS:
          (A) LENGTH: 226 amino acids
          (B) TYPE: amino acid
          (D) TOPOLOGY: linear
     (xi) SEQUENCE DESCRIPTION: SEQ ID NO: 145:

Met Glu Gly Ala Pro Pro Gly Ser Leu Ala Leu Arg Leu Leu Leu Phe
  1               5                  10                  15

Val Ala Leu Pro Ala Ser Gly Trp Leu Thr Thr Gly Ala Pro Glu Pro
            20                  25                  30

Pro Pro Leu Ser Gly Ala Pro Gln Asp Gly Ile Arg Ile Asn Val Thr
        35                  40                  45

Thr Leu Lys Asp Asp Gly Asp Ile Ser Lys Gln Gln Val Val Leu Asn
    50                  55                  60

Ile Thr Tyr Glu Ser Gly Gln Val Tyr Val Asn Asp Leu Pro Val Asn
 65                  70                  75                  80

Ser Gly Val Thr Arg Ile Ser Cys Gln Thr Leu Ile Val Lys Asn Glu
                85                  90                  95

Asn Leu Glu Asn Leu Glu Glu Lys Glu Tyr Phe Gly Ile Val Ser Val
            100                 105                 110

Arg Ile Leu Val His Glu Trp Pro Met Thr Ser Gly Ser Ser Leu Gln
        115                 120                 125

Leu Ile Val Ile Gln Glu Glu Val Val Glu Ile Asp Gly Lys Gln Val
    130                 135                 140

Gln Gln Lys Asp Val Thr Glu Ile Asp Ile Leu Val Lys Asn Arg Gly
145                 150                 155                 160

Val Leu Arg His Ser Asn Tyr Thr Leu Pro Leu Glu Glu Ser Met Leu
                165                 170                 175

Tyr Ser Ile Ser Arg Asp Ser Asp Ile Leu Phe Thr Leu Pro Asn Leu
            180                 185                 190

Ser Lys Lys Glu Ser Val Ser Ser Leu Gln Thr Thr Ser Gln Tyr Leu
        195                 200                 205

Ile Arg Asn Val Glu Thr Thr Val Asp Glu Asp Val Leu Pro Gly Gln
    210                 215                 220

Val Thr
225

(2) INFORMATION FOR SEQ ID NO: 146:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 146:

Met Gly Met Gly Ala Phe Gln Ala Phe Phe Trp Val Ile Leu Thr Val
1               5                   10                  15

Ser Asn Val Cys Val Leu Phe Lys Met Ser Leu Phe Phe Leu Leu Thr
            20                  25                  30

Leu Ile Ser Lys Leu His Gly Asp Ala Glu Val Cys Xaa
        35                  40                  45

(2) INFORMATION FOR SEQ ID NO: 147:

(i) SEQUENCE CHARACTERISTICS:
(A) LENGTH: 132 amino acids
(B) TYPE: amino acid
(D) TOPOLOGY: linear
(xi) SEQUENCE DESCRIPTION: SEQ ID NO: 147:

Met Ser Gly Gly Trp Met Ala Gln Val Gly Ala Trp Arg Thr Gly Ala
1               5                   10                  15

Leu Gly Leu Ala Leu Leu Leu Leu Leu Gly Leu Gly Leu Gly Leu Glu

            20                  25                  30

Ala Pro Arg Ala Arg Phe Pro Pro Arg Pro Leu Pro Arg Pro His Pro
        35                  40                  45

Ser Ser Gly Ser Cys Pro Pro Thr Lys Phe Gln Cys Arg Thr Ser Gly
    50                  55                  60

Leu Cys Val Pro Leu Thr Trp Arg Cys Asp Arg Thr Trp Thr Ala Ala
65                  70                  75                  80

Met Ala Ala Met Arg Arg Ser Ala Gly Leu Ser His Val Pro Arg Lys
                85                  90                  95

Gly Asn Ala His Arg Pro Leu Ala Ser Pro Ala Pro Ala Pro Ala Ser
            100                 105                 110

Val Thr Ala Leu Gly Glu Leu Thr Arg Asn Cys Ala Thr Ala Ala Ala
        115                 120                 125

Trp Pro Ala Xaa
    130

(2) INFORMATION FOR SEQ ID NO: 148:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 148:

Met Glu Ala Thr Leu Glu Gln His Leu Glu Asp Thr Met Lys Asn Pro
1               5                   10                  15

Ser Ile Val Gly Val Leu Cys Thr Asp Ser Gln Gly Leu Asn Leu Gly
            20                  25                  30

Cys Arg Gly Thr Leu Ser Asp Glu His Ala Gly Val Ile Ser Val Leu
        35                  40                  45

Ala Gln Gln Ala Ala Lys Leu Thr Ser Asp Pro Thr Asp Ile Pro Val
    50                  55                  60

Val Cys Leu Glu Ser Asp Asn Gly Asn Ile Met Ile Gln Lys His Asp
65                  70                  75                  80

Gly Ile Thr Val Ala Val His Lys Met Ala Ser Xaa
                85                  90

(2) INFORMATION FOR SEQ ID NO: 149:

(i) SEQUENCE CHARACTERISTICS:
(A) LENGTH: 165 amino acids
(B) TYPE: amino acid
(D) TOPOLOGY: linear
(xi) SEQUENCE DESCRIPTION: SEQ ID NO: 149:

Met Glu Pro Leu Arg Leu Leu Ile Leu Leu Phe Val Thr Glu Leu Ser
1               5                   10                  15

Gly Ala His Asn Thr Thr Val Phe Gln Gly Val Ala Gly Gln Ser Leu
            20                  25                  30

Gln Val Ser Cys Pro Tyr Asp Ser Met Lys His Trp Gly Arg Arg Lys
        35                  40                  45

Ala Trp Cys Arg Gln Leu Gly Glu Lys Gly Pro Cys Gln Arg Val Val
    50                  55                  60

Ser Thr His Asn Leu Trp Leu Leu Ser Phe Leu Arg Arg Trp Asn Gly
65                  70                  75                  80

Ser Thr Ala Ile Thr Asp Asp Thr Leu Gly Gly Thr Leu Thr Ile Thr
                85                  90                  95

Leu Arg Asn Leu Gln Pro His Asp Ala Gly Leu Tyr Gln Cys Gln Ser
            100                 105                 110

Leu His Gly Ser Glu Ala Asp Thr Leu Arg Lys Val Leu Val Glu Val
        115                 120                 125

Leu Ala Asp Pro Leu Asp His Arg Asp Ala Gly Asp Leu Trp Phe Pro
    130                 135                 140

Gly Glu Ser Glu Ser Phe Glu Asp Ala His Val Glu His Ser Ile Ser
145                 150                 155                 160

Arg Ser Ser Ser Xaa
                165

(2) INFORMATION FOR SEQ ID NO: 150:

(i) SEQUENCE CHARACTERISTICS:
(A) LENGTH: 139 amino acids
(B) TYPE: amino acid
(D) TOPOLOGY: linear
(xi) SEQUENCE DESCRIPTION: SEQ ID NO: 150:

Met Ile Ser Leu Thr Asp Thr Gln Lys Ile Gly Met Gly Leu Thr Gly
1               5                   10                  15

Phe Gly Val Phe Phe Leu Phe Phe Gly Met Ile Leu Phe Phe Asp Lys
            20                  25                  30

Ala Leu Leu Ala Ile Gly Asn Val Leu Phe Val Ala Gly Leu Ala Phe
        35                  40                  45

Val Ile Gly Leu Glu Arg Thr Phe Arg Phe Phe Phe Gln Lys His Lys
    50                  55                  60

Met Lys Ala Thr Gly Phe Phe Leu Gly Gly Val Phe Val Val Leu Ile
65                  70                  75                  80

Gly Trp Pro Leu Ile Gly Met Ile Phe Glu Ile Tyr Gly Phe Phe Leu

                85                  90                  95

Leu Phe Arg Gly Phe Phe Pro Val Val Val Gly Phe Ile Arg Arg Val
            100                 105                 110

Pro Val Leu Gly Ser Leu Leu Asn Leu Pro Gly Ile Arg Ser Phe Val
        115                 120                 125

Asp Lys Val Gly Glu Ser Asn Asn Met Val Xaa
    130                 135

(2) INFORMATION FOR SEQ ID NO: 151:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 151:

Met Ser Ala Pro Gln Thr Arg Ile Ser Arg Ala Leu Val Leu Leu Phe
1               5                   10                  15

Leu Ala Pro Thr Leu Leu Ser Leu Gly His Gly Ile His Pro Ile Asn
            20                  25                  30

Thr Ala Thr Pro Tyr Xaa Thr Asp Gln Ala Lys Leu Ala Pro Gly Thr
        35                  40                  45

Lys Glu Leu Asn His Asp Gln Ser Val Thr
    50                  55

(2) INFORMATION FOR SEQ ID NO: 152:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 152:

Met Ile Arg Lys Leu His Lys Ile Ile Val Phe Ser Pro Arg Val Ile
1               5                   10                  15

Val Leu Leu Asn Cys Phe Phe Phe Ile Lys Ala Lys Phe Val Leu Tyr
            20                  25                  30

Ile Phe Val Phe His Val Leu Asp Gly Ser Ile Ser Tyr Pro Val Xaa
        35                  40                  45

(2) INFORMATION FOR SEQ ID NO: 153:

(i) SEQUENCE CHARACTERISTICS:

(A) LENGTH: 42 amino acids
(B) TYPE: amino acid
(D) TOPOLOGY: linear
(xi) SEQUENCE DESCRIPTION: SEQ ID NO: 153:

Met Leu Leu Asn Gln His Phe Lys Ile Phe Gly Ser Leu Ile His Met
1               5                   10                  15

Asn Leu Leu Phe Ala Leu Ile Ser Leu Gly Ser Ser Asn Leu Ser Gly
            20                  25                  30

Val Gln Phe Cys Cys Glu Thr Val Gln Xaa
        35                  40

(2) INFORMATION FOR SEQ ID NO: 154:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 154:

Met Leu Ser Leu Ser Phe Leu Leu Arg Arg Val Leu Phe Leu Gly Phe
1               5                   10                  15

Leu Gln Ala Ser Val Gly Glu Lys Lys Ser Leu Arg Xaa Leu Asn Tyr
            20                  25                  30

Ser Val Pro His Pro Met Leu Xaa His Pro Pro Pro Asp Thr Ala Gln
        35                  40                  45

Val Pro Pro Arg Leu Glu Arg Ser Leu Leu Gln Gln Glu Leu Trp Thr
    50                  55                  60

Pro Gly Pro His His Ser Asn Ile
65                  70

(2) INFORMATION FOR SEQ ID NO: 155:

(i) SEQUENCE CHARACTERISTICS:
(A) LENGTH: 106 amino acids
(B) TYPE: amino acid
(D) TOPOLOGY: linear
(xi) SEQUENCE DESCRIPTION: SEQ ID NO: 155:

Met Gln Pro Leu Asn Phe Ser Ser Thr Glu Cys Ser Ser Phe Ser Pro
1               5                   10                  15

Pro Thr Thr Val Ile Leu Leu Ile Leu Leu Cys Phe Glu Gly Leu Leu
            20                  25                  30

Phe Leu Ile Phe Thr Ser Val Met Phe Gly Thr Gln Val His Ser Ile
        35                  40                  45

Cys Thr Asp Glu Thr Gly Ile Glu Gln Leu Lys Lys Glu Glu Arg Arg
    50                  55                  60

Trp Ala Lys Lys Thr Lys Trp Met Asn Met Lys Ala Val Phe Gly His
65                  70                  75                  80

Pro Phe Ser Leu Gly Trp Ala Ser Pro Phe Ala Thr Pro Asp Gln Gly
                85                  90                  95

Lys Ala Asp Pro Tyr Gln Tyr Val Val Xaa
            100                 105

(2) INFORMATION FOR SEQ ID NO: 156:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 156:

Met Tyr Thr Asn His Phe Asn Leu Tyr Leu Lys Tyr Ile Leu Leu Ile
1               5                   10                  15

Ile Leu Ile Leu Asn Met Thr Asn Ser Ser Ser Arg Tyr
            20                  25

(2) INFORMATION FOR SEQ ID NO: 157:

(i) SEQUENCE CHARACTERISTICS:
(A) LENGTH: 53 amino acids
(B) TYPE: amino acid
(D) TOPOLOGY: linear
(xi) SEQUENCE DESCRIPTION: SEQ ID NO: 157:

Met Asn Glu Leu Leu Leu Phe Phe Phe Phe Phe Phe Phe Phe Thr Phe
1               5                   10                  15

Cys Ile Glu Thr Asn Ser Phe Lys Gln Thr Tyr Tyr Tyr Tyr Phe Leu
            20                  25                  30

Gln Asn Ile Tyr Met Glu Met Leu Pro Pro Pro Val Asn Pro Pro Val
        35                  40                  45

Pro Pro Trp Gly Xaa
    50

(2) INFORMATION FOR SEQ ID NO: 158:

(i) SEQUENCE CHARACTERISTICS:
(A) LENGTH: 75 amino acids
(B) TYPE: amino acid
(D) TOPOLOGY: linear
(xi) SEQUENCE DESCRIPTION: SEQ ID NO: 158:

(2) INFORMATION FOR SEQ ID NO: 159:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 159:

Met Trp Pro Pro Leu Leu Leu Leu Leu Leu Leu Leu Pro Ala Ala Pro
1               5                   10                  15

Val Pro Thr Ala Lys Ala Ala Pro His Pro Asp Ala Asn Thr Gln Glu
            20                  25                  30

Gly Leu Gln Asn Leu Leu Gln Gly Val Gly Ala Gly Gly Asp Gly Glu
        35                  40                  45

Leu Arg Ala Asp Ser His Leu Ala Pro Gly Ser Gly Cys Ile Asp Gly
    50                  55                  60

Ala Val Val Ala Thr Arg Pro Glu Ser Arg Gly Gly Arg Pro Ala Val
65                  70                  75                  80

Pro

(2) INFORMATION FOR SEQ ID NO: 160:

(i) SEQUENCE CHARACTERISTICS:
(A) LENGTH: 139 amino acids
(B) TYPE: amino acid
(D) TOPOLOGY: linear
(xi) SEQUENCE DESCRIPTION: SEQ ID NO: 160:

Met Lys Phe Thr Thr Leu Leu Phe Leu Ala Ala Val Ala Gly Ala Leu
1               5                   10                  15

Val Tyr Ala Glu Asp Ala Ser Ser Asp Ser Thr Gly Ala Asp Pro Ala
            20                  25                  30

Gln Glu Ala Gly Thr Ser Lys Pro Asn Glu Glu Ile Ser Gly Pro Ala
        35                  40                  45

Glu Pro Ala Ser Pro Pro Glu Thr Thr Thr Thr Ala Gln Glu Thr Ser
    50                  55                  60

Ala Ala Ala Val Gln Gly Thr Ala Lys Val Thr Ser Ser Arg Gln Glu
65                  70                  75                  80

Leu Asn Pro Leu Lys Ser Ile Val Glu Lys Ser Ile Leu Leu Thr Glu
                85                  90                  95

Gln Ala Leu Ala Lys Ala Gly Lys Gly Met His Gly Gly Val Pro Gly
            100                 105                 110

Gly Lys Gln Phe Ile Glu Asn Gly Ser Glu Phe Ala Gln Lys Leu Leu
        115                 120                 125

Lys Lys Phe Ser Leu Leu Lys Pro Trp Ala Xaa
    130                 135

(2) INFORMATION FOR SEQ ID NO: 161:

(i) SEQUENCE CHARACTERISTICS:
(A) LENGTH: 178 amino acids
(B) TYPE: amino acid
(D) TOPOLOGY: linear
(xi) SEQUENCE DESCRIPTION: SEQ ID NO: 161:

Met Leu Gly Cys Gly Ile Pro Ala Leu Gly Leu Leu Leu Leu Leu Gln
1               5                   10                  15

Gly Ser Ala Asp Gly Asn Gly Ile Gln Gly Phe Phe Tyr Pro Trp Ser
            20                  25                  30

Cys Glu Gly Asp Ile Trp Asp Arg Glu Ser Cys Gly Gly Gln Ala Ala
        35                  40                  45

Ile Asp Ser Pro Asn Leu Cys Leu Arg Leu Arg Cys Cys Tyr Arg Asn
    50                  55                  60

Gly Val Cys Tyr His Gln Arg Pro Asp Glu Asn Val Arg Arg Lys His
65                  70                  75                  80

Met Trp Ala Leu Val Trp Thr Cys Ser Gly Leu Leu Leu Leu Ser Cys
                85                  90                  95

Ser Ile Cys Leu Phe Trp Trp Ala Lys Arg Arg Asp Val Leu His Met
            100                 105                 110

Pro Gly Phe Leu Ala Gly Pro Cys Asp Met Ser Lys Ser Val Ser Leu
        115                 120                 125

Leu Ser Lys His Arg Gly Thr Lys Lys Thr Pro Ser Thr Gly Ser Val
    130                 135                 140

Pro Val Ala Leu Ser Lys Glu Ser Arg Asp Val Glu Gly Gly Thr Glu
145                 150                 155                 160

Gly Glu Gly Thr Glu Glu Gly Glu Glu Thr Glu Gly Glu Glu Glu Glu
                165                 170                 175

Asp Xaa

(2) INFORMATION FOR SEQ ID NO: 162:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 162:

Met Glu Ala Val Phe Thr Val Phe Phe Phe Val Val Val Leu Phe Leu
1               5                   10                  15

Lys Asn Thr Glu Gly Ala Lys Leu Phe Cys Thr Leu Tyr Pro Ala Ala
            20                  25                  30

Ser Ser Gly Gln Ser Gln Gly Pro Gly Leu Glu Lys Pro Asp Ser Gln
        35                  40                  45

Glu Cys Ile Ile Asp Pro Cys Ser Tyr Pro Ile Ala Leu Gly Ala Gly
    50                  55                  60

Thr Glu Pro Gly Cys Lys Ile Xaa
65                  70

(2) INFORMATION FOR SEQ ID NO: 163:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 163:

Met Trp Phe Tyr Phe Leu Ser Val Ser Phe Pro Leu Leu Pro Val Xaa
1               5                   10                  15

Ala Pro Xaa Pro Pro Pro Ala Pro Thr Thr Leu Cys Leu Leu Leu Phe
            20                  25                  30

Leu Gly Xaa Leu Tyr Asn Ser Thr Cys Ile His Cys Val His Thr Thr
        35                  40                  45

Ser Xaa Thr Gln Asn Pro Thr Ala Asn Thr Leu Lys Lys Lys Lys Lys
    50                  55                  60

Asn Trp Gly
65

(2) INFORMATION FOR SEQ ID NO: 164:

(i) SEQUENCE CHARACTERISTICS:
(A) LENGTH: 155 amino acids

(B) TYPE: amino acid
(D) TOPOLOGY: linear
(xi) SEQUENCE DESCRIPTION: SEQ ID NO: 164:

Met Gly Phe Gly Ala Thr Leu Ala Val Gly Leu Thr Ile Phe Val Leu
1               5                   10                  15

Ser Val Val Thr Ile Ile Ile Cys Phe Thr Cys Ser Cys Cys Cys Leu
            20                  25                  30

Tyr Lys Thr Cys Arg Arg Pro Arg Pro Val Val Thr Thr Thr Thr Ser
        35                  40                  45

Thr Thr Val Val His Ala Pro Tyr Pro Gln Pro Pro Ser Val Pro Pro
    50                  55                  60

Ser Tyr Pro Gly Pro Ser Tyr Gln Gly Tyr His Thr Met Pro Pro Gln
65                  70                  75                  80

Pro Gly Met Pro Ala Ala Pro Tyr Pro Met Gln Tyr Pro Pro Pro Tyr
                85                  90                  95

Pro Ala Gln Pro Met Gly Pro Pro Ala Tyr His Glu Thr Leu Ala Gly
            100                 105                 110

Glu Gln Pro Arg Pro Thr Pro Pro Ala Ser Leu Leu Thr Thr Arg Pro
        115                 120                 125

Thr Trp Met Pro Arg Arg Arg Pro Ser Glu His Ser Leu Ala Ser Leu
    130                 135                 140

Ala Ala Thr Trp Leu Cys Cys Val Cys Ala Xaa
145                 150                 155

(2) INFORMATION FOR SEQ ID NO: 165:

(i) SEQUENCE CHARACTERISTICS:
(A) LENGTH: 104 amino acids
(B) TYPE: amino acid
(D) TOPOLOGY: linear
(xi) SEQUENCE DESCRIPTION: SEQ ID NO: 165:

Met Ile Ile Leu Val Phe Ile Ala Phe Phe Ile Pro Leu Gln Lys Thr
1               5                   10                  15

Ile Gly Lys Ile Ala Thr Cys Leu Glu Leu Arg Ser Ala Ala Leu Gln
            20                  25                  30

Ser Thr Gln Ser Gln Glu Glu Phe Lys Leu Glu Asp Leu Lys Lys Leu
        35                  40                  45

Glu Pro Ile Leu Lys Asn Ile Leu Thr Tyr Asn Lys Glu Phe Pro Phe
    50                  55                  60

Asp Val Gln Pro Val Pro Leu Arg Arg Ile Leu Ala Pro Gly Glu Glu
65                  70                  75                  80

Glu Asn Leu Glu Phe Glu Glu Asp Glu Glu Glu Gly Gly Ala Gly Ala

                85                  90                  95

Gly Leu Leu Ile Leu Ser Cys Xaa
            100

(2) INFORMATION FOR SEQ ID NO: 166:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 166:

Met Ala Gly Thr Met Val Ile Val Val Val Val Val Val Gly Glu Val
1               5                   10                  15

Val Val Glu Ala Glu Val Val Val Gln Ala Arg Glu Glu Ala Gly Glu
            20                  25                  30

Glu Glu Gly Ala Arg Ile Ile Thr Lys Gly Val Asn Leu Asn Ser Ile
        35                  40                  45

Ser Ser Met Glu Val Ile Ser Ile Ile Ile Leu Asp Leu Asp Arg Glu
    50                  55                  60

Asp Ile Thr Leu Val Glu Ala Thr Glu Pro Tyr Ile Leu Leu Glu Leu
65                  70                  75                  80

Lys

(2) INFORMATION FOR SEQ ID NO: 167:

(i) SEQUENCE CHARACTERISTICS:
(A) LENGTH: 93 amino acids
(B) TYPE: amino acid
(D) TOPOLOGY: linear
(xi) SEQUENCE DESCRIPTION: SEQ ID NO: 167:

Met Ser Phe Ser Phe Ile Ile Phe Leu Leu Leu Val Cys Gln Glu Ile
1               5                   10                  15

Thr Phe Cys Met Ser Tyr Gly Asp Ala Val Asn Cys Phe Ser Glu Cys
            20                  25                  30

Phe Ser Asn Leu Gln Thr Ile Tyr Ile Ser Cys Leu Gln His Ala Val
        35                  40                  45

Cys Lys His Ser Val Ile Trp Ser Ile Gln Leu Phe Val Arg Ala Leu
    50                  55                  60

Pro Ile Ser Lys Cys Ala Glu Leu Ser Ile Asp Gly Ile Phe Arg Ser
65                  70                  75                  80

Phe His Glu Asn Trp Lys Cys Ser Trp Val Ala Pro Thr
                85                  90

(2) INFORMATION FOR SEQ ID NO: 168:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 168:

Met Gly Trp Ser Ala Gly Leu Leu Phe Leu Leu Ile Leu Tyr Leu Pro
1               5                   10                  15

Val Pro Gly Trp Met Glu Arg Glu Asp Gly Glu Thr Gly His Leu Ser
            20                  25                  30

Pro Gln Ala Pro Gly Arg Glu Tyr Arg Gly Phe Tyr Ser Val Pro Pro
        35                  40                  45

Asp Tyr Val Trp Leu Arg Asp Ser Pro Xaa
    50                  55

(2) INFORMATION FOR SEQ ID NO: 169:

(i) SEQUENCE CHARACTERISTICS:
(A) LENGTH: 232 amino acids
(B) TYPE: amino acid
(D) TOPOLOGY: linear
(xi) SEQUENCE DESCRIPTION: SEQ ID NO: 169:

Met Ala Thr Leu Trp Gly Gly Leu Leu Arg Leu Gly Ser Leu Leu Ser
1               5                   10                  15

Leu Ser Cys Leu Ala Leu Ser Val Leu Leu Leu Ala His Cys Gln Thr
            20                  25                  30

Pro Pro Arg Ile Ser Arg Met Ser Asp Val Asn Val Ser Ala Leu Pro
        35                  40                  45

Ile Lys Lys Asn Ser Gly His Ile Tyr Asn Lys Asn Ile Ser Gln Lys
    50                  55                  60

Asp Cys Asp Cys Leu His Val Val Glu Pro Met Pro Val Arg Gly Pro
65                  70                  75                  80

Asp Val Glu Ala Tyr Cys Leu Arg Cys Glu Cys Lys Tyr Glu Glu Arg
                85                  90                  95

Ser Ser Val Thr Ile Lys Val Thr Ile Ile Ile Tyr Leu Ser Ile Leu
            100                 105                 110

Gly Leu Leu Leu Leu Tyr Met Val Tyr Leu Thr Leu Val Glu Pro Ile
        115                 120                 125

Leu Lys Arg Arg Leu Phe Gly His Ala Gln Leu Ile Gln Ser Asp Asp
    130                 135                 140

Asp Ile Gly Asp His Gln Pro Phe Ala Asn Ala His Asp Val Leu Ala
145                 150                 155                 160

Arg Ser Arg Ser Arg Ala Asn Val Leu Asn Lys Val Glu Tyr Gly Thr
                165                 170                 175

Ala Ala Leu Glu Ala Ser Ser Pro Arg Ala Ala Lys Ser Leu Ser Leu
            180                 185                 190

Thr Gly Met Leu Ser Ser Ala Asn Trp Gly Ile Glu Phe Lys Val Thr
        195                 200                 205

Arg Lys Lys Gln Ala Asp Asn Trp Lys Gly Thr Asp Trp Val Leu Leu
    210                 215                 220

Gly Phe Ile Leu Ile Pro Cys Xaa
225                 230

(2) INFORMATION FOR SEQ ID NO: 170:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 170:

Met Ser Ala Ile Phe Asn Phe Gln Ser Leu Leu Thr Val Ile Leu Leu
  1               5                  10                  15

Leu Ile Cys Thr Cys Ala Tyr Ile Arg Ser Leu Ala Pro Ser Leu Leu
             20                  25                  30

Asp Arg Asn Lys Thr Gly Leu Leu Gly Ile Phe Trp Lys Cys Ala Arg
         35                  40                  45

Ile Gly Glu Arg Lys Ser Pro Tyr Val Ala Val Cys Cys Ile Val Met
     50                  55                  60

Ala Phe Ser Ile Leu Phe Ile Gln
 65                  70

(2) INFORMATION FOR SEQ ID NO: 171:

(i) SEQUENCE CHARACTERISTICS:
(A) LENGTH: 65 amino acids
(B) TYPE: amino acid
(D) TOPOLOGY: linear
(xi) SEQUENCE DESCRIPTION: SEQ ID NO: 171:

Met Gly Thr Phe Ser Leu Ser Leu Phe Gly Leu Met Gly Val Ala Phe
  1               5                  10                  15

Gly Met Asn Leu Glu Ser Ser Leu Glu Glu Asp His Arg Ile Phe Trp
             20                  25                  30

Leu Ile Thr Gly Ile Met Phe Met Gly Ser Gly Leu Ile Trp Arg Arg

35                  40                  45

Leu Leu Ser Phe Leu Gly Arg Gln Leu Glu Ala Pro Leu Pro Pro Met
    50                  55                  60

Val
65

(2) INFORMATION FOR SEQ ID NO: 172:

(i) SEQUENCE CHARACTERISTICS:
(A) LENGTH: 75 amino acids
(B) TYPE: amino acid
(D) TOPOLOGY: linear
(xi) SEQUENCE DESCRIPTION: SEQ ID NO: 172:

Met Tyr Lys Gly Lys Leu Val Ile Val Leu Ile Leu Leu Leu Leu Pro
1               5                   10                  15

Ser His Phe Met Phe Leu Thr Gln Cys Lys Glu Ile Lys His Asn Leu
            20                  25                  30

Lys Lys Asn Met Ser Leu Leu Leu Phe Thr Ile Lys Ser Trp Leu Tyr
        35                  40                  45

Ser Ala Ser Leu Gly Ile Leu Tyr Asn Trp Gln His Leu Thr Ala Gln
    50                  55                  60

Val Asp Gln Cys Thr Ser Leu Ile Leu Ile His
65                  70                  75

(2) INFORMATION FOR SEQ ID NO: 173:

(i) SEQUENCE CHARACTERISTICS:
(A) LENGTH: 334 amino acids
(B) TYPE: amino acid
(D) TOPOLOGY: linear
(xi) SEQUENCE DESCRIPTION: SEQ ID NO: 173:

Met Val Gly His Glu Met Ala Ser Xaa Ser Ser Asn Thr Ser Leu Pro
1               5                   10                  15

Phe Ser Asn Met Gly Asn Pro Met Asn Thr Thr Gln Leu Gly Lys Ser
            20                  25                  30

Leu Phe Gln Trp Gln Val Glu Gln Glu Glu Ser Lys Leu Ala Asn Ile
        35                  40                  45

Ser Gln Asp Gln Phe Leu Ser Lys Asp Ala Asp Gly Asp Thr Phe Leu
    50                  55                  60

His Ile Ala Val Ala Gln Gly Arg Arg Ala Leu Ser Tyr Val Leu Ala
65                  70                  75                  80

Arg Lys Met Asn Ala Leu His Met Leu Asp Ile Lys Glu His Asn Gly
                85                  90                  95

Gln Ser Ala Phe Gln Val Ala Val Ala Ala Asn Gln His Leu Ile Val
            100                 105                 110

Gln Asp Leu Val Asn Ile Gly Ala Gln Val Asn Thr Thr Asp Cys Trp
        115                 120                 125

Gly Arg Thr Pro Leu His Val Cys Ala Glu Lys Gly His Ser Gln Val
    130                 135                 140

Leu Gln Ala Ile Gln Lys Gly Ala Val Gly Ser Asn Gln Phe Val Asp
145                 150                 155                 160

Leu Glu Ala Thr Asn Tyr Asp Gly Leu Thr Pro Leu His Cys Ala Val
                165                 170                 175

Ile Ala His Asn Ala Val Val His Glu Leu Gln Arg Asn Gln Gln Pro
            180                 185                 190

His Ser Pro Glu Val Gln Glu Leu Leu Leu Lys Asn Lys Ser Leu Val
        195                 200                 205

Asp Thr Ile Lys Cys Leu Ile Gln Met Gly Ala Ala Val Glu Ala Lys
    210                 215                 220

Asp Arg Lys Ser Gly Arg Thr Ala Leu His Leu Ala Ala Glu Glu Ala
225                 230                 235                 240

Asn Leu Glu Leu Ile Arg Leu Phe Leu Glu Leu Pro Ser Cys Leu Ser
                245                 250                 255

Phe Val Asn Ala Lys Ala Tyr Asn Gly Asn Thr Ala Leu His Val Ala
            260                 265                 270

Ala Ser Leu Gln Tyr Arg Leu Thr Gln Leu Asp Ala Val Arg Leu Leu
        275                 280                 285

Met Arg Lys Gly Ala Asp Pro Ser Thr Arg Asn Leu Glu Asn Glu Gln
    290                 295                 300

Pro Val His Leu Val Pro Asp Gly Pro Val Gly Glu Gln Ile Arg Arg
305                 310                 315                 320

Ile Leu Lys Gly Lys Ser Ile Gln Gln Arg Ala Pro Pro Tyr
                325                 330

(2) INFORMATION FOR SEQ ID NO: 174:

        (i) SEQUENCE CHARACTERISTICS:
                (A) LENGTH: 196 amino acids
                (B) TYPE: amino acid
                (D) TOPOLOGY: linear
        (xi) SEQUENCE DESCRIPTION: SEQ ID NO: 174:

Met Asp Ala Arg Trp Trp Ala Val Val Val Leu Ala Ala Phe Pro Ser
  1               5                  10                  15

Leu Gly Ala Gly Gly Glu Thr Pro Glu Ala Pro Pro Glu Ser Trp Thr

20                  25                  30

Gln Leu Trp Phe Phe Arg Phe Val Val Asn Ala Ala Gly Tyr Ala Ser
        35                  40                  45

Phe Met Val Pro Gly Tyr Leu Leu Val Gln Tyr Phe Arg Arg Lys Asn
    50                  55                  60

Tyr Leu Glu Thr Gly Arg Gly Leu Cys Phe Pro Leu Val Lys Ala Cys
65                  70                  75                  80

Val Phe Gly Asn Glu Pro Lys Ala Ser Asp Glu Val Pro Leu Ala Pro
                85                  90                  95

Arg Thr Glu Ala Ala Glu Thr Thr Pro Met Trp Gln Ala Leu Lys Leu
            100                 105                 110

Leu Phe Cys Ala Thr Gly Leu Gln Val Ser Tyr Leu Thr Trp Gly Val
        115                 120                 125

Leu Gln Glu Arg Val Met Thr Arg Ser Tyr Gly Ala Thr Ala Thr Ser
    130                 135                 140

Pro Gly Glu Arg Phe Thr Asp Ser Gln Phe Leu Val Leu Met Asn Arg
145                 150                 155                 160

Val Leu Ala Leu Ile Val Ala Gly Leu Ser Cys Val Leu Cys Lys Gln
                165                 170                 175

Pro Arg His Gly Ala Pro Met Tyr Arg Tyr Ser Phe Cys Gln Pro Val
            180                 185                 190

Gln Cys Ala Xaa
        195

(2) INFORMATION FOR SEQ ID NO: 175:

    (i) SEQUENCE CHARACTERISTICS:
        (A) LENGTH: 265 amino acids
        (B) TYPE: amino acid
        (D) TOPOLOGY: linear
    (xi) SEQUENCE DESCRIPTION: SEQ ID NO: 175:

Met Ser Asp Leu Leu Leu Leu Gly Leu Ile Gly Gly Leu Thr Leu Leu
1               5                   10                  15

Leu Leu Leu Thr Leu Leu Ala Phe Ala Gly Tyr Ser Gly Leu Leu Ala
            20                  25                  30

Gly Val Glu Val Ser Ala Gly Ser Pro Pro Ile Arg Asn Val Thr Val
        35                  40                  45

Ala Tyr Lys Phe His Met Gly Leu Tyr Gly Glu Thr Gly Arg Leu Phe
    50                  55                  60

Thr Glu Ser Cys Ser Ile Ser Pro Lys Leu Arg Ser Ile Ala Val Tyr
65                  70                  75                  80

Tyr Asp Asn Pro His Met Val Pro Pro Asp Lys Cys Arg Cys Ala Val
                85                  90                  95

Gly Ser Ile Leu Ser Glu Gly Glu Glu Ser Pro Ser Pro Glu Leu Ile
            100                 105                 110

Asp Leu Tyr Gln Lys Phe Gly Phe Lys Val Phe Ser Phe Pro Glu Pro
        115                 120                 125

Ser His Val Val Thr Ala Thr Phe Pro Leu Thr Pro Pro Phe Cys Pro
    130                 135                 140

Ile Trp Leu Gly Tyr Pro Pro Cys Pro Ser Cys Leu Gly His Leu His
145                 150                 155                 160

Gln Gly Ala Glu Ala Val Cys Leu Ser Ser Ala Gly Asp Leu Pro Gly
                165                 170                 175

Arg Pro Glu Ser Ile Ser Cys Ala His Trp His Gly Gln Gly Asp Phe
            180                 185                 190

Tyr Val Pro Glu Met Lys Glu Thr Glu Trp Lys Trp Arg Gly Leu Val
        195                 200                 205

Glu Ala Ile Asp Thr Gln Val Asp Gly Thr Gly Ala Asp Thr Met Ser
    210                 215                 220

Asp Thr Ser Ser Val Ser Leu Glu Val Ser Pro Gly Ser Arg Glu Thr
225                 230                 235                 240

Ser Ala Ala Thr Leu Ser Pro Gly Ala Ser Ser Arg Gly Trp Asp Asp
                245                 250                 255

Gly Asp Thr Arg Ser Glu His Ser Xaa
            260                 265

(2) INFORMATION FOR SEQ ID NO: 176:

    (i) SEQUENCE CHARACTERISTICS:
        (A) LENGTH: 138 amino acids
        (B) TYPE: amino acid
        (D) TOPOLOGY: linear
    (xi) SEQUENCE DESCRIPTION: SEQ ID NO: 176:

Met Ala Gln Leu Phe Leu Pro Leu Leu Ala Ala Leu Val Leu Ala Gln
  1               5                  10                  15

Ala Pro Ala Ala Leu Ala Asp Val Leu Glu Gly Asp Ser Ser Glu Asp
            20                  25                  30

Arg Ala Phe Arg Val Arg Ile Ala Gly Asp Ala Pro Leu Gln Gly Val
        35                  40                  45

Leu Gly Gly Ala Leu Thr Ile Pro Cys His Val His Tyr Leu Arg Pro
    50                  55                  60

Pro Pro Ser Arg Arg Ala Val Leu Gly Ser Pro Arg Val Lys Trp Thr
 65                  70                  75                  80

Phe Leu Ser Arg Gly Arg Glu Ala Glu Val Leu Val Ala Arg Gly Val
                85                  90                  95

Arg Val Lys Val Asn Glu Ala Tyr Arg Phe Arg Val Ala Leu Pro Ala
            100                 105                 110

Tyr Pro Ala Ser Leu Thr Asp Val Ser Pro Gly Ala Glu Arg Ala Ala
        115                 120                 125

Pro Gln Arg Leu Arg Tyr Leu Ser Leu Xaa
    130                 135

(2) INFORMATION FOR SEQ ID NO: 177:

        (i) SEQUENCE CHARACTERISTICS:
                (A) LENGTH: 179 amino acids
                (B) TYPE: amino acid
                (D) TOPOLOGY: linear
        (xi) SEQUENCE DESCRIPTION: SEQ ID NO: 177:

Met Pro Ala Leu Arg Pro Ala Leu Leu Trp Ala Leu Leu Ala Leu Trp
  1               5                  10                  15

Leu Cys Cys Ala Thr Pro Ala His Ala Leu Gln Cys Arg Asp Gly Tyr
            20                  25                  30

Glu Pro Cys Val Asn Glu Gly Met Cys Val Thr Tyr His Asn Gly Thr
        35                  40                  45

Gly Tyr Cys Lys Gly Pro Glu Gly Phe Leu Gly Glu Tyr Cys Gln His
    50                  55                  60

Arg Asp Pro Cys Glu Lys Asn Arg Cys Gln Asn Gly Gly Thr Cys Val
65                  70                  75                  80

Ala Gln Ala Met Leu Gly Lys Ala Thr Cys Arg Cys Ala Ser Gly Phe
                85                  90                  95

Thr Gly Glu Asp Cys Gln Tyr Ser Thr Ser His Pro Cys Phe Val Ser
            100                 105                 110

Arg Pro Cys Leu Asn Gly Gly Thr Cys His Met Leu Ser Arg Asp Thr
        115                 120                 125

Tyr Glu Cys Thr Cys Gln Val Gly Phe Thr Gly Lys Glu Cys Gln Trp
    130                 135                 140

Thr Asp Ala Cys Leu Ser His Pro Cys Ala Asn Gly Ser Thr Cys Thr
145                 150                 155                 160

Thr Val Ala Asn His Phe Leu Gln Met Pro His Arg Leu His Arg Ala
                165                 170                 175

Glu Val Xaa

(2) INFORMATION FOR SEQ ID NO: 178:

(i) SEQUENCE CHARACTERISTICS:
(A) LENGTH: 155 amino acids
(B) TYPE: amino acid
(D) TOPOLOGY: linear
(xi) SEQUENCE DESCRIPTION: SEQ ID NO: 178:

Met Thr Arg Gly Gly Pro Gly Gly Arg Pro Gly Leu Pro Gln Pro Pro
1               5                   10                  15

Pro Leu Leu Leu Leu Leu Leu Leu Pro Leu Leu Leu Val Thr Ala Glu
            20                  25                  30

Pro Pro Lys Pro Ala Gly Val Tyr Tyr Ala Thr Ala Tyr Trp Met Pro
        35                  40                  45

Ala Glu Lys Thr Val Gln Val Lys Asn Val Met Asp Lys Asn Gly Asp
    50                  55                  60

Ala Tyr Gly Phe Tyr Asn Asn Ser Val Lys Thr Thr Gly Trp Gly Ile
65                  70                  75                  80

Leu Glu Ile Arg Ala Gly Tyr Gly Ser Gln Thr Leu Ser Asn Glu Ile
                85                  90                  95

Ile Met Phe Val Ala Gly Phe Leu Glu Gly Tyr Leu Ile Ala Pro His
            100                 105                 110

Met Asn Asp His Tyr Thr Asn Leu Tyr Pro Gln Leu Ile Thr Lys Pro
        115                 120                 125

Ser Ile Met Asp Lys Val Gln Asp Phe Met Glu Lys Gln Asp Lys Val
    130                 135                 140

Asp Pro Glu Lys Tyr Gln Arg Ile Gln Asp Xaa
145                 150                 155


(2) INFORMATION FOR SEQ ID NO: 179:

(i) SEQUENCE CHARACTERISTICS:
(A) LENGTH: 295 amino acids
(B) TYPE: amino acid
(D) TOPOLOGY: linear
(xi) SEQUENCE DESCRIPTION: SEQ ID NO: 179:

Met Leu Gln Gly Pro Gly Ser Leu Leu Leu Leu Phe Leu Ala Ser His
1               5                   10                  15

Cys Cys Leu Gly Ser Ala Arg Gly Leu Phe Leu Phe Gly Gln Pro Asp
            20                  25                  30

Phe Ser Tyr Lys Arg Xaa Asn Cys Lys Pro Ile Pro Val Asn Leu Gln
        35                  40                  45

Leu Cys His Gly Ile Glu Tyr Gln Asn Met Arg Leu Pro Asn Leu Leu
    50                  55                  60

Gly His Glu Thr Met Lys Glu Val Leu Glu Gln Ala Gly Ala Trp Ile
65                  70                  75                  80

Pro Leu Val Met Lys Gln Cys His Pro Asp Thr Lys Lys Phe Leu Cys
                85                  90                  95

Ser Leu Phe Ala Pro Val Cys Leu Asp Asp Leu Asp Glu Thr Ile Gln
            100                 105                 110

Pro Cys His Ser Leu Cys Val Gln Val Lys Asp Arg Cys Ala Pro Val
        115                 120                 125

Met Ser Ala Phe Gly Phe Pro Trp Pro Asp Met Leu Glu Cys Asp Arg
    130                 135                 140

Phe Pro Gln Asp Asn Asp Leu Cys Ile Pro Leu Ala Ser Ser Asp His
145                 150                 155                 160

Leu Leu Pro Ala Thr Glu Glu Ala Pro Lys Val Cys Glu Ala Cys Lys
                165                 170                 175

Asn Lys Asn Asp Asp Asp Asn Asp Ile Met Glu Thr Leu Cys Lys Asn
            180                 185                 190

Asp Phe Ala Leu Lys Ile Lys Val Lys Glu Ile Thr Tyr Ile Asn Arg
        195                 200                 205

Asp Thr Lys Ile Ile Leu Glu Thr Lys Ser Lys Thr Ile Tyr Lys Leu
    210                 215                 220

Asn Gly Val Ser Glu Arg Asp Leu Lys Lys Ser Val Leu Trp Leu Lys
225                 230                 235                 240

Asp Ser Leu Gln Cys Thr Cys Glu Glu Met Asn Asp Ile Asn Ala Pro
                245                 250                 255

Tyr Leu Val Met Gly Gln Lys Gln Gly Gly Glu Leu Val Ile Thr Ser
            260                 265                 270

Val Lys Arg Trp Gln Lys Gly Gln Arg Glu Phe Lys Arg Ile Ser Arg
        275                 280                 285

Ser Ile Arg Lys Leu Gln Cys
    290                 295

(2) INFORMATION FOR SEQ ID NO: 180:

(i) SEQUENCE CHARACTERISTICS:
(A) LENGTH: 256 amino acids
(B) TYPE: amino acid
(D) TOPOLOGY: linear
(xi) SEQUENCE DESCRIPTION: SEQ ID NO: 180:

Met Arg Pro Ala Ala Leu Arg Gly Ala Leu Leu Gly Cys Leu Cys Leu
1               5                   10                  15

Ala Leu Leu Cys Leu Gly Gly Ala Asp Lys Arg Leu Arg Asp Asn His

            20                  25                  30

Glu Trp Lys Lys Leu Ile Met Val Gln His Trp Pro Glu Thr Val Cys
        35                  40                  45

Glu Lys Ile Gln Asn Asp Cys Arg Asp Pro Pro Asp Tyr Trp Thr Ile
    50                  55                  60

His Gly Leu Trp Pro Asp Lys Ser Glu Gly Cys Asn Arg Ser Trp Pro
65                  70                  75                  80

Phe Asn Leu Glu Glu Ile Lys Asp Leu Leu Pro Glu Met Arg Ala Tyr
                85                  90                  95

Trp Pro Asp Val Ile His Ser Phe Pro Asn Arg Ser Arg Phe Trp Lys
            100                 105                 110

His Glu Trp Glu Lys His Gly Thr Cys Ala Ala Gln Val Asp Ala Leu
        115                 120                 125

Asn Ser Gln Lys Lys Tyr Phe Gly Arg Ser Leu Glu Leu Tyr Arg Glu
    130                 135                 140

Leu Asp Leu Asn Ser Val Leu Leu Lys Leu Gly Ile Lys Pro Ser Ile
145                 150                 155                 160

Asn Tyr Tyr Gln Val Ala Asp Phe Lys Asp Ala Leu Ala Arg Val Tyr
                165                 170                 175

Gly Val Ile Pro Lys Ile Gln Cys Leu Pro Pro Ser Gln Asp Glu Glu
            180                 185                 190

Val Gln Thr Ile Gly Gln Ile Glu Leu Cys Leu Thr Lys Gln Asp Gln
        195                 200                 205

Gln Leu Gln Asn Cys Thr Glu Pro Gly Glu Gln Pro Ser Pro Lys Gln
    210                 215                 220

Glu Val Trp Leu Ala Asn Gly Ala Ala Glu Ser Arg Gly Leu Arg Val
225                 230                 235                 240

Cys Glu Asp Gly Pro Val Phe Tyr Pro Pro Pro Lys Lys Thr Lys His
                245                 250                 255

(2) INFORMATION FOR SEQ ID NO: 181:

(i) SEQUENCE CHARACTERISTICS:
(A) LENGTH: 324 amino acids
(B) TYPE: amino acid
(D) TOPOLOGY: linear
(xi) SEQUENCE DESCRIPTION: SEQ ID NO: 181:

Met Ala Pro Leu Leu Leu Gln Leu Ala Val Leu Gly Ala Ala Leu Ala
1               5                   10                  15

Ala Ala Ala Leu Val Leu Ile Ser Ile Val Ala Phe Thr Thr Ala Thr
            20                  25                  30

Lys Met Pro Ala Leu His Arg His Glu Glu Glu Lys Phe Phe Leu Asn
        35                  40                  45

Ala Lys Gly Gln Lys Glu Thr Leu Pro Ser Ile Trp Asp Ser Pro Thr
    50                  55                  60

Lys Gln Leu Ser Val Val Val Pro Ser Tyr Asn Glu Glu Lys Arg Leu
65                  70                  75                  80

Pro Val Met Met Asp Glu Ala Leu Ser Tyr Leu Glu Lys Arg Gln Lys
                85                  90                  95

Arg Asp Pro Ala Phe Thr Tyr Glu Val Ile Val Val Asp Asp Gly Ser
            100                 105                 110

Lys Asp Gln Thr Ser Lys Val Ala Phe Lys Tyr Cys Gln Lys Tyr Gly
        115                 120                 125

Ser Asp Lys Val Arg Val Ile Thr Leu Val Lys Asn Arg Gly Lys Gly
    130                 135                 140

Gly Ala Ile Arg Met Gly Ile Phe Ser Ser Arg Gly Glu Lys Ile Leu
145                 150                 155                 160

Met Ala Asp Ala Asp Gly Ala Thr Lys Phe Pro Asp Val Glu Lys Leu
                165                 170                 175

Glu Lys Gly Leu Asn Asp Leu Gln Pro Trp Pro Asn Gln Met Ala Ile
            180                 185                 190

Ala Cys Gly Ser Arg Ala His Leu Glu Lys Glu Ser Ile Ala Gln Arg
        195                 200                 205

Ser Tyr Phe Arg Thr Leu Leu Met Tyr Gly Phe His Phe Leu Val Trp
    210                 215                 220

Phe Leu Cys Val Lys Gly Ile Arg Asp Thr Gln Cys Gly Phe Lys Leu
225                 230                 235                 240

Phe Thr Arg Glu Ala Ala Ser Arg Thr Phe Ser Ser Leu His Val Glu
                245                 250                 255

Arg Trp Ala Phe Asp Val Glu Leu Leu Tyr Ile Ala Gln Phe Phe Lys
            260                 265                 270

Ile Pro Ile Ala Glu Ile Ala Val Asn Trp Thr Glu Ile Glu Gly Ser
        275                 280                 285

Lys Leu Val Pro Phe Trp Ser Trp Leu Gln Met Gly Lys Asp Leu Leu
    290                 295                 300

Phe Ile Arg Leu Arg Tyr Leu Thr Gly Ala Trp Arg Leu Glu Gln Thr
305                 310                 315                 320

Arg Lys Met Asn

(2) INFORMATION FOR SEQ ID NO: 182:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 182:

Met Asp Ile Cys Phe Phe His Tyr Val Leu Leu Phe Phe Leu Val Arg
1               5                   10                  15

Cys Ala Leu Val Val Leu Ile Leu Leu Cys Gln Gly Trp Gly Asn Gly
            20                  25                  30

Gly Gly Cys Val Gly Arg Val Leu Ile Ile Val Phe Ser Ser Val
        35                  40                  45

(2) INFORMATION FOR SEQ ID NO: 183:

(i) SEQUENCE CHARACTERISTICS:
(A) LENGTH: 93 amino acids
(B) TYPE: amino acid
(D) TOPOLOGY: linear
(xi) SEQUENCE DESCRIPTION: SEQ ID NO: 183:

Met Ala Ser Leu Gly His Ile Leu Val Phe Cys Val Gly Leu Leu Thr
1               5                   10                  15

Met Ala Lys Ala Glu Ser Pro Lys Glu His Asp Pro Phe Thr Tyr Asp
            20                  25                  30

Tyr Gln Ser Leu Gln Ile Gly Gly Leu Val Ile Ala Gly Ile Leu Phe
        35                  40                  45

Ile Leu Gly Ile Leu Ile Val Leu Ser Arg Arg Cys Arg Cys Lys Phe
    50                  55                  60

Asn Gln Gln Gln Arg Thr Gly Glu Pro Asp Glu Glu Glu Gly Thr Phe
65                  70                  75                  80

Arg Ser Ser Ile Arg Arg Leu Ser Thr Arg Arg Arg Xaa
                85                  90

(2) INFORMATION FOR SEQ ID NO: 184:

(i) SEQUENCE CHARACTERISTICS:
(A) LENGTH: 168 amino acids
(B) TYPE: amino acid
(D) TOPOLOGY: linear
(xi) SEQUENCE DESCRIPTION: SEQ ID NO: 184:

Met Xaa Thr Lys Glu Phe Gly Xaa Gly Arg Ala Val Gln Gln Val Leu
1               5                   10                  15

Asn Ile Glu Cys Leu Arg Asp Phe Leu Thr Pro Pro Leu Leu Ser Val
            20                  25                  30

Arg Phe Arg Tyr Val Gly Ala Pro Gln Ala Leu Thr Leu Lys Leu Pro
        35                  40                  45

Val Thr Xaa Asn Lys Phe Phe Gln Pro Thr Glu Met Ala Ala Gln Asp
    50                  55                  60

Phe Phe Gln Arg Trp Lys Gln Leu Ser Leu Pro Gln Gln Glu Ala Gln
65                  70                  75                  80

Lys Ile Phe Lys Ala Asn His Pro Met Asp Ala Glu Val Thr Lys Ala
                85                  90                  95

Lys Leu Leu Gly Phe Gly Ser Ala Leu Leu Asp Asn Val Asp Pro Asn
            100                 105                 110

Pro Glu Asn Phe Val Gly Ala Gly Ile Ile Gln Thr Lys Ala Leu Gln
        115                 120                 125

Val Gly Cys Leu Leu Arg Leu Glu Pro Asn Ala Gln Ala Gln Met Tyr
    130                 135                 140

Arg Leu Thr Leu Arg Thr Ser Lys Glu Pro Val Ser Arg His Leu Cys
145                 150                 155                 160

Glu Leu Leu Ala Gln Gln Phe Xaa
                165

(2) INFORMATION FOR SEQ ID NO: 185:

      (i) SEQUENCE CHARACTERISTICS:
            (A) LENGTH: 43 amino acids
            (B) TYPE: amino acid
            (D) TOPOLOGY: linear
      (xi) SEQUENCE DESCRIPTION: SEQ ID NO: 185:

Met Phe Tyr Val Leu Ser Val Ser Pro Leu Leu Xaa Phe Leu Ala Cys
  1               5                  10                  15

Gly Leu Cys Leu Cys Val Asn Trp Lys Ile Ala Ile Ser Gln Leu Ser
            20                  25                  30

Leu Ser Phe Lys Asn Glu Leu Glu Lys Pro Xaa
        35                  40

(2) INFORMATION FOR SEQ ID NO: 186:

      (i) SEQUENCE CHARACTERISTICS:
            (A) LENGTH: 59 amino acids
            (B) TYPE: amino acid
            (D) TOPOLOGY: linear
      (xi) SEQUENCE DESCRIPTION: SEQ ID NO: 186:

Met Lys Leu Phe Asp Ala Ser Pro Thr Phe Phe Ala Phe Leu Leu Gly

1               5                   10                  15

His Ile Leu Ala Met Glu Val Leu Ala Trp Leu Leu Ile Tyr Leu Leu
            20                  25                  30

Gly Pro Gly Trp Val Pro Ser Ala Leu Xaa Arg Leu His Pro Gly His
        35                  40                  45

Leu Ser Gly Ser Val Leu Val Ser Ala Ala Xaa
    50                  55

(2) INFORMATION FOR SEQ ID NO: 187:

(i) SEQUENCE CHARACTERISTICS:
(A) LENGTH: 189 amino acids
(B) TYPE: amino acid
(D) TOPOLOGY: linear
(xi) SEQUENCE DESCRIPTION: SEQ ID NO: 187:

Met Asp Val Asn Ile Ala Pro Leu Arg Ala Trp Asp Asp Phe Phe Pro
1               5                   10                  15

Gly Ser Asp Arg Phe Ala Arg Pro Asp Phe Arg Asp Ile Ser Lys Trp
            20                  25                  30

Asn Asn Arg Val Val Ser Asn Leu Leu Tyr Tyr Gln Thr Asn Tyr Leu
        35                  40                  45

Val Val Ala Ala Met Met Ile Ser Ile Val Gly Phe Leu Ser Pro Phe
    50                  55                  60

Asn Met Ile Leu Gly Gly Ile Val Val Val Leu Val Phe Thr Gly Phe
65                  70                  75                  80

Val Trp Ala Ala His Asn Lys Asp Val Leu Arg Arg Met Lys Lys Arg
                85                  90                  95

Tyr Pro Thr Thr Phe Val Met Val Val Met Leu Ala Ser Tyr Phe Leu
            100                 105                 110

Ile Ser Met Phe Gly Gly Val Met Val Phe Val Phe Gly Ile Thr Phe
        115                 120                 125

Pro Leu Leu Leu Met Phe Ile His Ala Ser Leu Arg Leu Arg Asn Leu
    130                 135                 140

Lys Asn Lys Leu Glu Asn Lys Met Glu Gly Ile Gly Leu Lys Arg Thr
145                 150                 155                 160

Pro Met Gly Ile Val Leu Asp Ala Leu Glu Gln Gln Glu Glu Gly Ile
                165                 170                 175

Asn Arg Leu Thr Asp Tyr Ile Ser Lys Val Lys Glu Xaa
            180                 185

(2) INFORMATION FOR SEQ ID NO: 188:

(i) SEQUENCE CHARACTERISTICS:
(A) LENGTH: 146 amino acids
(B) TYPE: amino acid
(D) TOPOLOGY: linear
(xi) SEQUENCE DESCRIPTION: SEQ ID NO: 188:

(2) INFORMATION FOR SEQ ID NO: 189:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 189:

Met Gly Ser Arg Ala Glu Leu Cys Thr Leu Leu Gly Gly Phe Ser Phe
1               5                   10                  15

Leu Leu Leu Leu Ile Pro Gly Glu Gly Ala Lys Gly Gly Ser Leu Arg
            20                  25                  30

Glu Ser Gln Gly Val Cys Ser Lys Gln Thr Leu Val Val Pro Leu His
        35                  40                  45

Tyr Asn Glu Ser Tyr Ser Gln Pro Val Tyr Lys Pro Tyr Leu Thr Leu
    50                  55                  60

Cys Ala Gly Ser Ala Ser Ala Ala Leu Thr Gly Pro Cys Thr Ala Leu

65                  70                  75                  80

Cys Gly Gly Arg

(2) INFORMATION FOR SEQ ID NO: 190:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 190:

Met Met Gly Val Leu Gln Leu Leu His Ile Phe Trp Ala Tyr Leu Ile
1               5                   10                  15

Leu Arg Met Ala His Lys Phe Ile Thr Gly Lys Leu Val Glu Asp Glu
            20                  25                  30

Arg Ser Thr Gly Lys Lys Gln Arg Ala Gln Arg Gly Arg Arg Leu Gln
        35                  40                  45

Leu Gly Glu Glu Gln Arg Ala Gly Pro Xaa
    50                  55

(2) INFORMATION FOR SEQ ID NO: 191:

(i) SEQUENCE CHARACTERISTICS:
(A) LENGTH: 311 amino acids
(B) TYPE: amino acid
(D) TOPOLOGY: linear
(xi) SEQUENCE DESCRIPTION: SEQ ID NO: 191:

Met Arg Arg Leu Val His Asp Leu Leu Pro Pro Glu Val Cys Ser Leu
1               5                   10                  15

Leu Asn Pro Ala Ala Ile Tyr Ala Asn Asn Glu Ile Ser Leu Arg Asp
            20                  25                  30

Val Glu Val Tyr Gly Phe Asp Tyr Asp Tyr Thr Leu Ala Gln Tyr Ala
        35                  40                  45

Asp Ala Leu His Pro Glu Ile Phe Ser Thr Ala Arg Asp Ile Leu Ile
    50                  55                  60

Glu His Tyr Lys Tyr Pro Glu Gly Ile Arg Lys Tyr Asp Tyr Asn Pro
65                  70                  75                  80

Ser Phe Ala Ile Arg Gly Leu His Tyr Asp Ile Gln Lys Ser Leu Leu
                85                  90                  95

Met Lys Ile Asp Ala Phe His Tyr Val Gln Leu Gly Thr Ala Tyr Arg
            100                 105                 110

Gly Leu Gln Pro Val Pro Asp Glu Glu Val Ile Glu Leu Tyr Gly Gly
        115                 120                 125

Thr Gln His Ile Pro Leu Tyr Gln Met Ser Gly Phe Tyr Gly Lys Gly
    130                 135                 140

Pro Ser Ile Lys Gln Phe Met Asp Ile Phe Ser Leu Pro Glu Met Ala
145                 150                 155                 160

Leu Leu Ser Cys Val Val Asp Tyr Phe Leu Gly His Ser Leu Glu Phe
                165                 170                 175

Asp Gln Ala His Leu Tyr Lys Asp Val Thr Asp Ala Ile Arg Asp Val
            180                 185                 190

His Val Lys Gly Leu Met Tyr Gln Trp Ile Glu Gln Asp Met Glu Lys
        195                 200                 205

Tyr Ile Leu Arg Gly Asp Glu Thr Phe Ala Val Leu Ser Arg Leu Val
    210                 215                 220

Ala His Gly Lys Gln Leu Phe Leu Ile Thr Asn Ser Pro Phe Ser Phe
225                 230                 235                 240

Val Asp Lys Gly Met Arg His Met Val Gly Pro Asp Trp Arg His Ser
                245                 250                 255

Ser Met Trp Ser Leu Ser Arg Gln Thr Ser Pro Ala Ser Ser Leu Thr
            260                 265                 270

Gly Ala Ser Phe Xaa Glu Asn Ser Met Arg Arg Ala His Phe Ser Gly
        275                 280                 285

Thr Gly Ser Pro Ala Trp Lys Arg Ala Arg Ser Ile Gly Arg Glu Thr
    290                 295                 300

Cys Leu Thr Ser Tyr Ala Xaa
305                 310

(2) INFORMATION FOR SEQ ID NO: 192:

        (i) SEQUENCE CHARACTERISTICS:
                (A) LENGTH: 318 amino acids
                (B) TYPE: amino acid
                (D) TOPOLOGY: linear
        (xi) SEQUENCE DESCRIPTION: SEQ ID NO: 192:

Met Asn Trp Glu Leu Leu Leu Trp Leu Leu Val Leu Cys Ala Leu Leu
  1               5                  10                  15

Leu Leu Leu Val Gln Leu Leu Arg Phe Leu Arg Ala Asp Gly Asp Leu
             20                  25                  30

Thr Leu Leu Trp Ala Glu Trp Gln Gly Arg Arg Pro Glu Trp Glu Leu
         35                  40                  45

Thr Asp Met Val Val Trp Val Thr Gly Ala Ser Ser Gly Ile Gly Glu
     50                  55                  60

Glu Leu Ala Tyr Gln Leu Ser Lys Leu Gly Val Ser Leu Val Leu Ser

65                  70                  75                  80

Ala Arg Arg Val His Glu Leu Glu Arg Val Lys Arg Arg Cys Leu Glu
                85                  90                  95

Asn Gly Asn Leu Lys Glu Lys Asp Ile Leu Val Leu Pro Leu Asp Leu
            100                 105                 110

Thr Asp Thr Gly Ser His Glu Ala Ala Thr Lys Ala Val Leu Gln Glu
        115                 120                 125

Phe Gly Arg Ile Asp Ile Leu Val Asn Asn Gly Gly Met Ser Gln Arg
    130                 135                 140

Ser Leu Cys Met Asp Thr Ser Leu Asp Val Tyr Arg Lys Leu Ile Glu
145                 150                 155                 160

Leu Asn Tyr Leu Gly Thr Val Ser Leu Thr Lys Cys Val Leu Pro His
                165                 170                 175

Met Ile Glu Arg Lys Gln Gly Lys Ile Val Thr Val Asn Ser Ile Leu
            180                 185                 190

Gly Ile Ile Ser Val Pro Leu Ser Ile Gly Tyr Cys Ala Ser Lys His
        195                 200                 205

Ala Leu Arg Gly Phe Phe Asn Gly Leu Arg Thr Glu Leu Ala Thr Tyr
    210                 215                 220

Pro Gly Ile Ile Val Ser Asn Ile Cys Pro Gly Pro Val Gln Ser Asn
225                 230                 235                 240

Ile Val Glu Asn Ser Leu Ala Gly Glu Val Thr Lys Thr Ile Gly Asn
                245                 250                 255

Asn Gly Asp Gln Ser His Lys Met Thr Thr Ser Arg Cys Val Arg Leu
            260                 265                 270

Met Leu Ile Ser Met Ala Asn Asp Leu Lys Glu Val Trp Ile Ser Glu
        275                 280                 285

Gln Pro Phe Leu Phe Ser Asn Ile Phe Val Ala Ile His Ala Asn Leu
    290                 295                 300

Gly Leu Val Asp Asn Gln Gln Asp Gly Glu Glu Lys Asp Xaa
305                 310                 315

(2) INFORMATION FOR SEQ ID NO: 193:

        (i) SEQUENCE CHARACTERISTICS:
                (A) LENGTH: 53 amino acids
                (B) TYPE: amino acid
                (D) TOPOLOGY: linear
        (xi) SEQUENCE DESCRIPTION: SEQ ID NO: 193:

Met Trp Pro Ser Phe Pro Gln Val Arg Val Gly Ser Phe Leu Phe Gly
  1               5                  10                  15

Ile Leu Phe Phe Ser Phe Gly Ser Ser Ser Leu Pro Pro Gly Leu Pro
            20                  25                  30

Pro Pro Ala Ser Leu Leu Cys Cys Ala Val Gln Trp Gly Ala Arg Ala
        35                  40                  45

Leu Phe Leu Pro Ala
    50

(2) INFORMATION FOR SEQ ID NO: 194:

        (i) SEQUENCE CHARACTERISTICS:
                (A) LENGTH: 42 amino acids
                (B) TYPE: amino acid
                (D) TOPOLOGY: linear
        (xi) SEQUENCE DESCRIPTION: SEQ ID NO: 194:

Met Leu Val Thr Cys Ser Val Cys Cys Tyr Leu Phe Trp Leu Ile Ala
  1               5                  10                  15

Ile Leu Ala Gln Leu Asn Pro Leu Phe Gly Pro Gln Leu Lys Asn Glu
            20                  25                  30

Thr Ile Trp Tyr Leu Lys Tyr His Trp Pro
        35                  40

(2) INFORMATION FOR SEQ ID NO: 195:

        (i) SEQUENCE CHARACTERISTICS:
                (A) LENGTH: 102 amino acids
                (B) TYPE: amino acid
                (D) TOPOLOGY: linear
        (xi) SEQUENCE DESCRIPTION: SEQ ID NO: 195:

Met Glu Gly Thr Glu Met Gly Ala Arg Pro Gly Gly His Pro Gln Lys
  1               5                  10                  15

Trp Ser Phe Leu Trp Ser Leu Ala Leu Trp Leu Pro Leu Ala Leu Ser
            20                  25                  30

Val Ser Leu Phe Leu Gly Leu Ser Leu Ser Pro Pro Gln Pro Gly Leu
        35                  40                  45

Ser Leu Trp Cys Thr Leu Ser Tyr Cys Cys Glu Gln Trp Lys Phe Lys
    50                  55                  60

Gly Thr Pro Ser Pro Ala Leu Leu Asn Leu Gly Thr Gln Pro Lys Lys
 65                  70                  75                  80

Asp Lys Lys Leu Glu Asp Ser Ile Ala Thr Gln Leu Arg Glu Leu Pro
                85                  90                  95

Glu Lys Asn Ser Asn Xaa
            100

(2) INFORMATION FOR SEQ ID NO: 196:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 196:

Met Ala Leu Thr Phe Leu Leu Val Leu Leu Thr Leu Ala Thr Ser Ala
1               5                   10                  15

His Gly Cys Thr Glu Thr Ser Asp Ala Gly Arg Ala Ser Thr Gly Gly
            20                  25                  30

Pro Gln Arg Thr Ala Arg Thr Gln Trp Leu Leu Cys Xaa
        35                  40                  45

(2) INFORMATION FOR SEQ ID NO: 197:

(i) SEQUENCE CHARACTERISTICS:
(A) LENGTH: 355 amino acids
(B) TYPE: amino acid
(D) TOPOLOGY: linear
(xi) SEQUENCE DESCRIPTION: SEQ ID NO: 197:

Met Gly Pro Ser Thr Pro Leu Leu Ile Leu Phe Leu Leu Ser Trp Ser
1               5                   10                  15

Gly Pro Leu Gln Gly Gln Gln His His Leu Val Glu Tyr Met Glu Arg
            20                  25                  30

Arg Leu Ala Ala Leu Glu Glu Arg Leu Ala Gln Cys Gln Asp Gln Ser
        35                  40                  45

Ser Arg His Ala Ala Glu Leu Arg Asp Phe Lys Asn Lys Met Leu Pro
    50                  55                  60

Leu Leu Glu Val Ala Glu Lys Glu Arg Glu Ala Leu Arg Thr Glu Ala
65                  70                  75                  80

Asp Thr Ile Ser Gly Arg Val Asp Arg Leu Glu Arg Glu Val Asp Tyr
                85                  90                  95

Leu Glu Thr Gln Asn Pro Ala Leu Pro Cys Val Glu Phe Asp Glu Lys
            100                 105                 110

Val Thr Gly Gly Pro Gly Thr Lys Gly Lys Gly Arg Arg Asn Glu Lys
        115                 120                 125

Tyr Asp Met Val Thr Asp Cys Gly Tyr Thr Ile Ser Gln Val Arg Ser
    130                 135                 140

Met Lys Ile Leu Lys Arg Phe Gly Gly Pro Ala Gly Leu Trp Thr Lys
145                 150                 155                 160

Asp Pro Leu Gly Gln Thr Glu Lys Ile Tyr Val Leu Asp Gly Thr Gln
                165                 170                 175

Asn Asp Thr Ala Phe Val Phe Pro Arg Leu Arg Asp Phe Thr Leu Ala
            180                 185                 190

Met Ala Ala Arg Lys Ala Ser Arg Val Arg Val Pro Phe Pro Trp Val
        195                 200                 205

Gly Thr Gly Gln Leu Val Tyr Gly Gly Phe Leu Tyr Phe Ala Arg Arg
    210                 215                 220

Pro Pro Gly Arg Pro Gly Gly Gly Gly Glu Met Glu Asn Thr Leu Gln
225                 230                 235                 240

Leu Ile Lys Phe His Leu Ala Asn Arg Thr Val Val Asp Ser Ser Val
                245                 250                 255

Phe Pro Ala Glu Gly Leu Ile Pro Pro Tyr Gly Leu Thr Ala Asp Thr
            260                 265                 270

Tyr Ile Asp Leu Ala Ala Asp Glu Glu Gly Leu Trp Ala Val Tyr Ala
        275                 280                 285

Thr Arg Glu Asp Asp Arg His Leu Cys Leu Ala Lys Leu Asp Pro Gln
    290                 295                 300

Thr Leu Asp Thr Glu Gln Gln Trp Asp Thr Pro Cys Pro Arg Glu Asn
305                 310                 315                 320

Ala Glu Ala Ala Phe Val Ile Cys Gly Thr Leu Tyr Val Val Tyr Asn
                325                 330                 335

Thr Arg Pro Ala Ser Arg Ala Arg Ile Gln Cys Ser Phe Asp Ala Ser
            340                 345                 350

Gly Pro Xaa
        355

(2) INFORMATION FOR SEQ ID NO: 198:

(i) SEQUENCE CHARACTERISTICS:
(A) LENGTH: 74 amino acids
(B) TYPE: amino acid
(D) TOPOLOGY: linear
(xi) SEQUENCE DESCRIPTION: SEQ ID NO: 198:

Met Val Leu Pro Leu Leu Ile Phe Val Leu Leu Pro Lys Val Val Asn
1               5                   10                  15

Thr Ser Asp Pro Asp Met Arg Arg Glu Met Glu Gln Ser Met Asn Met
            20                  25                  30

Leu Asn Ser Asn His Glu Leu Pro Asp Val Ser Glu Phe Met Thr Arg
        35                  40                  45

Leu Phe Ser Ser Lys Ser Ser Gly Lys Ser Ser Ser Gly Ser Ser Lys
    50                  55                  60

Thr Gly Lys Ser Gly Ala Gly Lys Arg Arg

65                  70

(2) INFORMATION FOR SEQ ID NO: 199:

(i) SEQUENCE CHARACTERISTICS:
(A) LENGTH: 113 amino acids
(B) TYPE: amino acid
(D) TOPOLOGY: linear
(xi) SEQUENCE DESCRIPTION: SEQ ID NO: 199:

Met Phe Thr Met Leu Cys Ile Asn Gly Thr Thr Pro Arg Pro Leu Pro
1               5                   10                  15

Val Pro Ser Pro Phe Gly Cys Met Ile Phe Phe Phe Phe Lys Asn Pro
            20                  25                  30

Trp Lys Gln Arg Leu Leu Gln Gly Trp Leu Gly Ala Arg Pro Ile His
        35                  40                  45

Leu Leu Gly Tyr Leu Pro Leu Ser Leu Leu Trp Cys Pro Phe Pro Leu
    50                  55                  60

Pro Cys Ala Arg Cys Ser Val Val Tyr Ile Ser Ser Pro Arg His Gly
65                  70                  75                  80

Ala His Ala Pro Arg Asp Met Ile Leu Ser Leu Val Leu Ala His Gly
                85                  90                  95

Ala Leu Tyr Lys Glu Leu Gly Gly Arg Gly Arg Lys Trp Glu Pro Ser
            100                 105                 110

Xaa

(2) INFORMATION FOR SEQ ID NO: 200:

(i) SEQUENCE CHARACTERISTICS:
(A) LENGTH: 123 amino acids
(B) TYPE: amino acid
(D) TOPOLOGY: linear
(xi) SEQUENCE DESCRIPTION: SEQ ID NO: 200:

Met Ala Cys Arg Cys Leu Ser Phe Leu Leu Met Gly Thr Phe Leu Ser
1               5                   10                  15

Val Ser Gln Thr Val Leu Ala Gln Leu Asp Ala Leu Leu Val Phe Pro
            20                  25                  30

Gly Gln Val Ala Gln Leu Ser Cys Thr Leu Ser Pro Gln His Val Thr
        35                  40                  45

Ile Arg Asp Tyr Gly Val Ser Trp Tyr Gln Gln Arg Ala Gly Ser Ala
    50                  55                  60

Pro Arg Tyr Leu Leu Tyr Tyr Arg Ser Glu Glu Asp His His Arg Pro
65                  70                  75                  80

Ala Asp Ile Pro Asp Arg Phe Ser Ala Ala Lys Asp Glu Ala His Asn
                85                  90                  95

Ala Cys Val Leu Thr Ile Ser Pro Val Gln Pro Glu Asp Asp Ala Asp
            100                 105                 110

Tyr Tyr Cys Ser Val Gly Tyr Gly Phe Ser Pro
        115                 120

(2) INFORMATION FOR SEQ ID NO: 201:

    (i) SEQUENCE CHARACTERISTICS:
        (A) LENGTH: 315 amino acids
        (B) TYPE: amino acid
        (D) TOPOLOGY: linear
    (xi) SEQUENCE DESCRIPTION: SEQ ID NO: 201:

Met Ala Gly Gly Arg Cys Gly Pro Xaa Leu Thr Ala Leu Leu Ala Ala
  1               5                  10                  15

Trp Ile Ala Ala Val Ala Ala Thr Ala Gly Pro Glu Glu Ala Ala Leu
            20                  25                  30

Pro Pro Glu Gln Ser Arg Val Gln Pro Met Thr Ala Ser Asn Trp Thr
        35                  40                  45

Leu Val Met Glu Gly Glu Trp Met Leu Lys Phe Tyr Ala Pro Trp Cys
    50                  55                  60

Pro Ser Cys Gln Gln Thr Asp Ser Glu Trp Glu Ala Phe Ala Lys Asn
 65                  70                  75                  80

Gly Glu Ile Leu Gln Ile Ser Val Gly Lys Val Asp Val Ile Gln Glu
                85                  90                  95

Pro Gly Leu Ser Gly Arg Phe Phe Val Thr Thr Leu Pro Ala Phe Phe
            100                 105                 110

His Ala Lys Asp Gly Ile Phe Arg Arg Tyr Arg Gly Pro Gly Ile Phe
        115                 120                 125

Glu Asp Leu Gln Asn Tyr Ile Leu Glu Lys Lys Trp Gln Ser Val Glu
    130                 135                 140

Pro Leu Thr Gly Trp Lys Ser Pro Ala Ser Leu Thr Met Ser Gly Met
145                 150                 155                 160

Ala Gly Leu Phe Ser Ile Ser Gly Lys Ile Trp His Leu His Asn Tyr
                165                 170                 175

Phe Thr Val Thr Leu Gly Ile Pro Ala Trp Cys Ser Tyr Val Phe Phe
            180                 185                 190

Val Ile Ala Thr Leu Val Phe Gly Leu Phe Met Gly Leu Val Leu Val
        195                 200                 205

Val Ile Ser Glu Cys Phe Tyr Val Pro Leu Pro Arg His Leu Ser Glu

210                 215                 220

Arg Ser Glu Gln Asn Arg Arg Ser Glu Glu Ala His Arg Ala Glu Gln
225                 230                 235                 240

Leu Gln Asp Ala Glu Glu Glu Lys Asp Asp Ser Asn Glu Glu Glu Asn
                245                 250                 255

Lys Asp Ser Leu Val Asp Asp Glu Glu Glu Lys Glu Asp Leu Gly Asp
            260                 265                 270

Glu Asp Glu Ala Glu Glu Glu Glu Glu Glu Asp Asn Leu Ala Ala Gly
        275                 280                 285

Val Asp Glu Glu Arg Ser Glu Ala Asn Asp Gln Gly Pro Pro Gly Glu
    290                 295                 300

Asp Gly Val Thr Arg Glu Xaa Ser Arg Ala Xaa
305                 310                 315

(2) INFORMATION FOR SEQ ID NO: 202:

        (i) SEQUENCE CHARACTERISTICS:
                (A) LENGTH: 236 amino acids
                (B) TYPE: amino acid
                (D) TOPOLOGY: linear
        (xi) SEQUENCE DESCRIPTION: SEQ ID NO: 202:

Met Gly Thr Ala Asp Ser Asp Glu Met Ala Pro Glu Ala Pro Gln His
  1               5                  10                  15

Thr His Ile Asp Val His Ile His Gln Glu Ser Ala Leu Ala Lys Leu
             20                  25                  30

Leu Leu Thr Cys Cys Ser Ala Leu Arg Pro Arg Ala Thr Gln Ala Arg
         35                  40                  45

Gly Ser Ser Arg Leu Leu Val Ala Ser Trp Val Met Gln Ile Val Leu
     50                  55                  60

Gly Ile Leu Ser Ala Val Leu Gly Gly Phe Phe Tyr Ile Arg Asp Tyr
 65                  70                  75                  80

Thr Leu Leu Val Thr Ser Gly Ala Ala Ile Trp Thr Gly Ala Val Ala
                 85                  90                  95

Val Leu Ala Gly Ala Ala Ala Phe Ile Tyr Glu Lys Arg Gly Gly Thr
            100                 105                 110

Tyr Trp Ala Leu Leu Arg Thr Leu Leu Ala Leu Ala Ala Phe Ser Thr
        115                 120                 125

Ala Ile Ala Ala Leu Lys Leu Trp Asn Glu Asp Phe Arg Tyr Gly Tyr
    130                 135                 140

Ser Tyr Tyr Asn Ser Ala Cys Arg Ile Ser Ser Ser Ser Asp Trp Asn
145                 150                 155                 160

Thr Pro Ala Pro Thr Gln Ser Pro Glu Glu Val Arg Arg Leu His Leu
                165                 170                 175

Cys Thr Ser Phe Met Asp Met Leu Lys Ala Leu Phe Arg Thr Leu Gln
            180                 185                 190

Ala Met Leu Leu Gly Val Trp Ile Leu Leu Leu Leu Ala Ser Leu Ala
        195                 200                 205

Pro Leu Trp Leu Tyr Cys Trp Arg Met Phe Pro Thr Lys Gly Lys Arg
    210                 215                 220

Asp Gln Lys Glu Met Leu Glu Val Ser Gly Ile Xaa
225                 230                 235

(2) INFORMATION FOR SEQ ID NO: 203:

(i) SEQUENCE CHARACTERISTICS:
(A) LENGTH: 93 amino acids
(B) TYPE: amino acid
(D) TOPOLOGY: linear
(xi) SEQUENCE DESCRIPTION: SEQ ID NO: 203:

Met Ile His Leu Gly His Ile Leu Phe Leu Leu Leu Leu Pro Val Ala
1               5                   10                  15

Ala Ala Gln Thr Thr Pro Gly Glu Arg Ser Ser Leu Pro Ala Phe Tyr
            20                  25                  30

Pro Gly Thr Ser Gly Ser Cys Ser Gly Cys Gly Ser Leu Ser Leu Pro
        35                  40                  45

Leu Leu Ala Gly Leu Val Ala Ala Asp Ala Val Ala Ser Leu Leu Ile
    50                  55                  60

Val Gly Ala Val Phe Leu Cys Ala Arg Pro Arg Arg Ser Pro Ala Gln
65                  70                  75                  80

Glu Asp Gly Lys Val Tyr Ile Asn Met Pro Gly Arg Gly
                85                  90

(2) INFORMATION FOR SEQ ID NO: 204:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 204:

Met Trp Ser Ala Gly Arg Gly Gly Ala Ala Trp Pro Val Leu Leu Gly
1               5                   10                  15

Leu Leu Leu Ala Leu Leu Val Pro Gly Gly Gly Ala Ala Lys Thr Gly
            20                  25                  30

Ala Asp Ser

        35

(2) INFORMATION FOR SEQ ID NO: 205:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 205:

Asp Cys Xaa His Val Ser Val Leu Gln Ser Thr Ile Ser Pro Leu Leu
1               5                   10                  15

Pro Leu Pro Leu Leu Leu Pro His Gly Asn Cys Glu Glu Ala Pro Trp
            20                  25                  30

Gln Ala Ala Val Ile Gly Gly Gly Asp Arg Ile
        35                  40

(2) INFORMATION FOR SEQ ID NO: 206:

(i) SEQUENCE CHARACTERISTICS:
(A) LENGTH: 85 amino acids
(B) TYPE: amino acid
(D) TOPOLOGY: linear
(xi) SEQUENCE DESCRIPTION: SEQ ID NO: 206:

Met Arg Asp Cys Leu Ser Leu Lys Pro Arg Pro Leu Phe Pro Thr Gln
1               5                   10                  15

Phe Phe Phe Ile Leu Leu Leu Ile Phe Ile Ala Glu Val Ala Ala Ala
            20                  25                  30

Val Val Ala Leu Val Tyr Thr Thr Met Val Arg His Trp Asp Gly Gly
        35                  40                  45

Arg Glu Glu Asp Trp Ala Lys Pro Trp Glu Trp Ala Val Ala Cys Glu
    50                  55                  60

Trp Pro Pro Ser Val Pro Ala Pro Lys His Trp Pro Ala Ser Pro Arg
65                  70                  75                  80

Leu Ser Thr Ser Xaa
                85

(2) INFORMATION FOR SEQ ID NO: 207:

(i) SEQUENCE CHARACTERISTICS:
(A) LENGTH: 208 amino acids
(B) TYPE: amino acid
(D) TOPOLOGY: linear
(xi) SEQUENCE DESCRIPTION: SEQ ID NO: 207:

Met His Gly Asn Glu Ala Leu Gly Arg Glu Leu Leu Leu Leu Leu Met

1                   5                   10                  15

Gln Phe Leu Cys His Glu Phe Leu Arg Xaa Asn Pro Arg Val Thr Arg
                20                  25                  30

Leu Leu Ser Glu Met Arg Ile His Leu Leu Pro Ser Met Asn Pro Asp
            35                  40                  45

Gly Tyr Glu Ile Ala Tyr His Arg Gly Ser Glu Leu Val Gly Trp Ala
    50                  55                  60

Glu Gly Arg Trp Asn Asn Gln Ser Ile Asp Leu Asn His Asn Phe Ala
65                  70                  75                  80

Xaa Leu Asn Thr Pro Leu Trp Glu Ala Gln Asp Asp Gly Lys Val Pro
                85                  90                  95

His Ile Val Pro Asn His His Leu Pro Leu Pro Thr Tyr Tyr Thr Leu
            100                 105                 110

Pro Asn Ala Thr Val Ala Pro Glu Thr Arg Ala Val Ile Lys Trp Met
        115                 120                 125

Lys Arg Ile Pro Phe Val Leu Ser Ala Asn Leu His Gly Gly Glu Leu
    130                 135                 140

Val Val Ser Tyr Pro Phe Asp Met Thr Arg Thr Pro Trp Ala Ala Arg
145                 150                 155                 160

Glu Leu Thr Pro Thr Pro Asp Asp Ala Val Phe Arg Trp Leu Ser Thr
                165                 170                 175

Val Tyr Ala Gly Ser Asn Leu Ala Met Gln Asp Thr Ser Arg Arg Pro
            180                 185                 190

Cys His Ser Gln Asp Phe Ser Val His Gly Asn Ile Ile Asn Gly Ala
        195                 200                 205

(2) INFORMATION FOR SEQ ID NO: 208:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 208:

Met Glu Ile Ser Cys Leu Leu Leu Leu Ile Gln Asp Ser Asp Glu Met
1               5                   10                  15

Glu Asp Gly Pro Gly Val Gln Asp
            20

(2) INFORMATION FOR SEQ ID NO: 209:

(i) SEQUENCE CHARACTERISTICS:
(A) LENGTH: 483 amino acids
(B) TYPE: amino acid
(D) TOPOLOGY: linear
(xi) SEQUENCE DESCRIPTION: SEQ ID NO: 209:

Met Ala Thr Gly Gly Gly Ile Arg Ala Met Thr Ser Leu Tyr Gly Gln
1               5                   10                  15

Leu Ala Gly Leu Lys Glu Leu Gly Leu Leu Asp Cys Xaa Ser Tyr Ile
            20                  25                  30

Thr Gly Ala Ser Gly Ser Thr Trp Ala Leu Ala Asn Leu Tyr Lys Asp
        35                  40                  45

Pro Glu Trp Ser Gln Lys Asp Leu Ala Gly Pro Thr Glu Leu Leu Lys
    50                  55                  60

Thr Gln Val Thr Lys Asn Lys Leu Gly Val Leu Ala Pro Ser Gln Leu
65                  70                  75                  80

Gln Arg Tyr Arg Gln Glu Leu Ala Glu Arg Ala Arg Leu Gly Tyr Pro
                85                  90                  95

Ser Cys Phe Thr Asn Leu Trp Ala Leu Ile Asn Glu Ala Leu Leu His
            100                 105                 110

Asp Glu Pro His Asp His Lys Leu Ser Asp Gln Arg Glu Ala Leu Ser
        115                 120                 125

His Gly Gln Asn Pro Leu Pro Ile Tyr Cys Ala Leu Asn Thr Lys Gly
    130                 135                 140

Gln Ser Leu Thr Thr Phe Glu Phe Gly Glu Trp Cys Glu Phe Ser Pro
145                 150                 155                 160

Tyr Glu Val Gly Phe Pro Lys Tyr Gly Ala Phe Ile Pro Ser Glu Leu
                165                 170                 175

Phe Gly Ser Glu Phe Phe Met Gly Gln Leu Met Lys Arg Leu Pro Glu
            180                 185                 190

Ser Arg Ile Cys Phe Leu Glu Gly Ile Trp Ser Asn Leu Tyr Ala Ala
        195                 200                 205

Asn Leu Gln Asp Ser Leu Tyr Trp Ala Ser Glu Pro Ser Gln Phe Trp
    210                 215                 220

Asp Arg Trp Val Arg Asn Gln Ala Asn Leu Asp Lys Glu Gln Val Pro
225                 230                 235                 240

Leu Leu Lys Ile Glu Glu Pro Pro Ser Thr Ala Gly Arg Ile Ala Glu
                245                 250                 255

Phe Phe Thr Asp Leu Leu Thr Trp Arg Pro Leu Ala Gln Ala Thr His
            260                 265                 270

Asn Phe Leu Arg Gly Leu His Phe His Lys Asp Tyr Phe Gln His Pro
        275                 280                 285

His Phe Ser Thr Trp Lys Ala Thr Thr Leu Asp Gly Leu Pro Asn Gln
    290                 295                 300

Leu Thr Pro Ser Glu Pro His Leu Cys Leu Leu Asp Val Gly Tyr Leu
305                 310                 315                 320

Ile Asn Thr Ser Cys Leu Pro Leu Leu Gln Pro Thr Arg Asp Val Asp
                325                 330                 335

Leu Ile Leu Ser Leu Asp Tyr Asn Leu His Gly Ala Phe Gln Gln Leu
            340                 345                 350

Gln Leu Leu Gly Arg Phe Cys Gln Glu Gln Gly Ile Pro Phe Pro Pro
        355                 360                 365

Ile Ser Pro Ser Pro Glu Glu Gln Leu Gln Pro Arg Glu Cys His Thr
    370                 375                 380

Phe Ser Asp Pro Thr Cys Pro Gly Ala Pro Ala Val Leu His Phe Pro
385                 390                 395                 400

Leu Val Ser Asp Ser Phe Arg Glu Tyr Ser Ala Pro Gly Val Arg Arg
                405                 410                 415

Thr Pro Glu Glu Ala Ala Ala Gly Glu Val Asn Leu Ser Ser Ser Asp
            420                 425                 430

Ser Pro Tyr His Tyr Thr Lys Val Thr Tyr Ser Gln Glu Asp Val Asp
        435                 440                 445

Lys Leu Leu His Leu Thr His Tyr Asn Val Cys Asn Asn Gln Glu Gln
    450                 455                 460

Leu Leu Glu Ala Leu Arg Gln Ala Val Gln Arg Arg Arg Gln Arg Arg
465                 470                 475                 480

Pro His Xaa

(2) INFORMATION FOR SEQ ID NO: 210:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 210:

Leu Glu Val Gly Cys Ile Gln Val Ala Pro Asp Thr Phe
  1               5                  10

(2) INFORMATION FOR SEQ ID NO: 211:

(i) SEQUENCE CHARACTERISTICS:
(A) LENGTH: 20 amino acids
(B) TYPE: amino acid

(D) TOPOLOGY: linear
(xi) SEQUENCE DESCRIPTION: SEQ ID NO: 211:

Met Ser Leu Phe Phe Leu Leu Thr Leu Ile Ser Lys Leu His Gly Asp
1               5                   10                  15

Ala Glu Val Cys
            20

(2) INFORMATION FOR SEQ ID NO: 212:

(i) SEQUENCE CHARACTERISTICS:
(A) LENGTH: 55 amino acids
(B) TYPE: amino acid
(D) TOPOLOGY: linear
(xi) SEQUENCE DESCRIPTION: SEQ ID NO: 212:

Met Pro His Pro Pro Leu Pro Glu Thr Ser Leu Glu Ala Gln Leu Pro
1               5                   10                  15

Met Gly Leu Leu Gln Leu Leu Arg Cys Ser Val Gln Ala Trp Ser Pro
            20                  25                  30

Pro Pro Ser Ser Phe Cys Pro Gly Ser Glu Pro Arg Ser Ala Ser Ala
        35                  40                  45

His Trp Gly Tyr Trp Trp Pro
    50                  55

(2) INFORMATION FOR SEQ ID NO: 213:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 213:

Asp Pro Glu Thr Arg Trp His His Gly Gly Ser Ala Gln Asn Gly Leu
1               5                   10                  15

Leu Met Leu Ile Ser Val Leu Gln Gln Pro Val Ile Gly Thr Gly Ser
            20                  25                  30

Tyr Leu Cys
        35

(2) INFORMATION FOR SEQ ID NO: 214:

(i) SEQUENCE CHARACTERISTICS:
(A) LENGTH: 230 amino acids
(B) TYPE: amino acid
(D) TOPOLOGY: linear
(xi) SEQUENCE DESCRIPTION: SEQ ID NO: 214:

Met Glu Pro Leu Arg Leu Leu Ile Leu Leu Phe Val Thr Glu Leu Ser
1               5                   10                  15

Gly Ala His Asn Thr Thr Val Phe Gln Gly Val Ala Gly Gln Ser Leu
            20                  25                  30

Gln Val Ser Cys Pro Tyr Asp Ser Met Lys His Trp Gly Arg Arg Lys
        35                  40                  45

Ala Trp Cys Arg Gln Leu Gly Glu Lys Gly Pro Cys Gln Arg Val Val
    50                  55                  60

Ser Thr His Asn Leu Trp Leu Leu Ser Phe Leu Arg Arg Trp Asn Gly
65                  70                  75                  80

Ser Thr Ala Ile Thr Asp Asp Thr Leu Gly Gly Thr Leu Thr Ile Thr
                85                  90                  95

Leu Arg Asn Leu Gln Pro His Asp Ala Gly Leu Tyr Gln Cys Gln Ser
            100                 105                 110

Leu His Gly Ser Glu Ala Asp Thr Leu Arg Lys Val Leu Val Glu Val
        115                 120                 125

Leu Ala Asp Pro Leu Asp His Arg Asp Ala Gly Asp Leu Trp Phe Pro
    130                 135                 140

Gly Glu Ser Glu Ser Phe Glu Asp Ala His Val Glu His Ser Ile Ser
145                 150                 155                 160

Arg Ser Leu Leu Glu Gly Glu Ile Pro Phe Pro Pro Thr Ser Ile Leu
                165                 170                 175

Leu Leu Leu Ala Cys Ile Phe Leu Ile Lys Ile Leu Ala Ala Ser Xaa
            180                 185                 190

Leu Trp Ala Ala Ala Trp His Gly Gln Lys Pro Gly Thr His Pro Pro
        195                 200                 205

Ser Glu Leu Asp Cys Gly His Asp Pro Gly Tyr Gln Leu Gln Thr Leu
    210                 215                 220

Pro Gly Leu Arg Asp Thr
225                 230

(2) INFORMATION FOR SEQ ID NO: 215:

(i) SEQUENCE CHARACTERISTICS:
(A) LENGTH: 231 amino acids
(B) TYPE: amino acid
(D) TOPOLOGY: linear
(xi) SEQUENCE DESCRIPTION: SEQ ID NO: 215:

Met Glu Pro Leu Arg Leu Leu Ile Leu Leu Phe Val Thr Glu Leu Ser
1               5                   10                  15

Gly Ala His Asn Thr Thr Val Phe Gln Gly Val Ala Gly Gln Ser Leu
            20                  25                  30

Gln Val Ser Cys Pro Tyr Asp Ser Met Lys His Trp Gly Arg Arg Lys
        35                  40                  45

Ala Trp Cys Arg Gln Leu Gly Glu Lys Gly Pro Cys Gln Arg Val Val
    50                  55                  60

Ser Thr His Asn Leu Trp Leu Leu Ser Phe Leu Arg Arg Trp Asn Gly
65                  70                  75                  80

Ser Thr Ala Ile Thr Asp Asp Thr Leu Gly Gly Thr Leu Thr Ile Thr
                85                  90                  95

Leu Arg Asn Leu Gln Pro His Asp Ala Gly Leu Tyr Gln Cys Gln Ser
            100                 105                 110

Leu His Gly Ser Glu Ala Asp Thr Leu Arg Lys Val Leu Val Glu Val
        115                 120                 125

Leu Ala Asp Pro Leu Asp His Arg Asp Ala Gly Asp Leu Trp Phe Pro
    130                 135                 140

Gly Glu Ser Glu Ser Phe Glu Asp Ala His Val Glu His Ser Ile Ser
145                 150                 155                 160

Arg Ser Leu Leu Glu Gly Glu Ile Pro Phe Pro Pro Thr Ser Ile Leu
                165                 170                 175

Leu Leu Leu Ala Cys Ile Phe Leu Ile Lys Ile Leu Ala Ala Ser Ala
            180                 185                 190

Leu Trp Ala Ala Ala Trp His Gly Gln Lys Pro Gly Thr His Pro Pro
        195                 200                 205

Ser Glu Leu Asp Cys Gly His Asp Pro Gly Tyr Gln Leu Gln Thr Leu
    210                 215                 220

Pro Gly Leu Arg Asp Thr Xaa
225                 230

(2) INFORMATION FOR SEQ ID NO: 216:

        (i) SEQUENCE CHARACTERISTICS:
                (A) LENGTH: 127 amino acids
                (B) TYPE: amino acid
                (D) TOPOLOGY: linear
        (xi) SEQUENCE DESCRIPTION: SEQ ID NO: 216:

Met Gly Leu Thr Gly Phe Gly Val Phe Phe Leu Phe Phe Gly Met Ile
1               5                   10                  15

Leu Phe Phe Asp Lys Ala Leu Leu Ala Ile Gly Asn Val Leu Phe Val
            20                  25                  30

Ala Gly Leu Ala Phe Val Ile Gly Leu Glu Arg Thr Phe Arg Phe Phe
        35                  40                  45

Phe Gln Lys His Lys Met Lys Ala Thr Gly Phe Phe Leu Gly Gly Val

50                  55                  60

Phe Val Val Leu Ile Gly Trp Pro Leu Ile Gly Met Ile Phe Glu Ile
65                  70                  75                  80

Tyr Gly Phe Phe Leu Leu Phe Arg Gly Phe Phe Pro Val Val Val Gly
                85                  90                  95

Phe Ile Arg Arg Val Pro Val Leu Gly Ser Leu Leu Asn Leu Pro Gly
            100                 105                 110

Ile Arg Ser Phe Val Asp Lys Val Gly Glu Ser Asn Asn Met Val
        115                 120                 125

(2) INFORMATION FOR SEQ ID NO: 217:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 217:

Met Ile Arg Lys Leu His Lys Ile Ile Val Phe Ser Pro Arg Val Ile
1               5                   10                  15

Val Leu Leu Asn Cys Phe Phe Phe Ile Lys Ala Lys Phe Val Leu Tyr
            20                  25                  30

Ile Phe Val Phe His Val Leu Asp Gly Ser Ile Ser Tyr Pro Val
        35                  40                  45

(2) INFORMATION FOR SEQ ID NO: 218:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 218:

(2) INFORMATION FOR SEQ ID NO: 219:

(i) SEQUENCE CHARACTERISTICS:
(A) LENGTH: 105 amino acids
(B) TYPE: amino acid
(D) TOPOLOGY: linear

(xi) SEQUENCE DESCRIPTION: SEQ ID NO: 219:

Met Gln Pro Leu Asn Phe Ser Ser Thr Xaa Cys Ser Ser Phe Ser Pro
1               5                   10                  15

Pro Thr Thr Val Ile Leu Leu Ile Leu Leu Cys Phe Glu Gly Leu Leu
            20                  25                  30

Phe Leu Ile Phe Thr Ser Val Met Phe Gly Thr Gln Val His Ser Ile
        35                  40                  45

Cys Thr Asp Glu Thr Gly Ile Glu Gln Leu Lys Lys Glu Glu Arg Arg
    50                  55                  60

Trp Ala Lys Lys Thr Lys Trp Met Asn Met Lys Ala Val Phe Gly His
65                  70                  75                  80

Pro Phe Ser Leu Gly Trp Ala Ser Pro Phe Ala Thr Pro Asp Gln Gly
                85                  90                  95

Lys Ala Asp Pro Tyr Gln Tyr Val Val
            100                 105

(2) INFORMATION FOR SEQ ID NO: 220:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 220:

Met Tyr Thr Asn His Phe Asn Leu Tyr Leu Lys Tyr Ile Leu Leu Ile
1               5                   10                  15

Ile Leu Ile Leu Asn Met Thr Asn Ser Ser Ser Arg Tyr
            20                  25

(2) INFORMATION FOR SEQ ID NO: 221:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 221:

Met Asn Glu Leu Leu Leu Phe Phe Phe Phe Phe Phe Phe Leu His Phe
1               5                   10                  15

Val

(2) INFORMATION FOR SEQ ID NO: 222:

(i) SEQUENCE CHARACTERISTICS:

(A) LENGTH: 138 amino acids
(B) TYPE: amino acid
(D) TOPOLOGY: linear
(xi) SEQUENCE DESCRIPTION: SEQ ID NO: 222:

Met Lys Phe Thr Thr Leu Leu Phe Leu Ala Ala Val Ala Gly Ala Leu
1               5                   10                  15

Val Tyr Ala Glu Asp Ala Ser Ser Asp Ser Thr Gly Ala Asp Pro Ala
            20                  25                  30

Gln Glu Ala Gly Thr Ser Lys Pro Asn Glu Glu Ile Ser Gly Pro Ala
        35                  40                  45

Glu Pro Ala Ser Pro Pro Glu Thr Thr Thr Thr Ala Gln Glu Xaa Ser
    50                  55                  60

Ala Ala Ala Val Gln Gly Thr Ala Lys Val Thr Ser Ser Arg Gln Glu
65                  70                  75                  80

Leu Asn Pro Leu Lys Ser Ile Val Glu Lys Ser Ile Leu Leu Thr Glu
                85                  90                  95

Gln Ala Leu Ala Lys Ala Gly Lys Gly Met His Gly Gly Val Pro Gly
            100                 105                 110

Gly Lys Gln Phe Ile Glu Asn Gly Ser Glu Phe Ala Gln Lys Leu Leu
        115                 120                 125

Lys Lys Phe Ser Leu Leu Lys Pro Trp Ala
    130                 135

(2) INFORMATION FOR SEQ ID NO: 223:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 223:

Met Leu Gly Cys Gly Ile Pro Ala Leu Gly Leu Leu Leu Leu Leu Gln
1               5                   10                  15

Xaa Ser Ala Asp Gly Asn Gly Ile Gln Gly Phe Phe Tyr Pro Trp Ser
            20                  25                  30

Cys Glu Gly Asp Ile Trp Asp Arg Glu Ser Cys Gly Gly Gln Ala Ala
        35                  40                  45

Ile Arg
    50

(2) INFORMATION FOR SEQ ID NO: 224:

(i) SEQUENCE CHARACTERISTICS:
(A) LENGTH: 15 amino acids

(B) TYPE: amino acid
(D) TOPOLOGY: linear
(xi) SEQUENCE DESCRIPTION: SEQ ID NO: 224:

Met Glu Ala Val Phe Thr Val Phe Phe Phe Leu Leu Phe Cys Phe
1               5                   10                  15

(2) INFORMATION FOR SEQ ID NO: 225:

(i) SEQUENCE CHARACTERISTICS:
(A) LENGTH: 155 amino acids
(B) TYPE: amino acid
(D) TOPOLOGY: linear
(xi) SEQUENCE DESCRIPTION: SEQ ID NO: 225:

Met Gly Phe Gly Ala Thr Leu Ala Val Gly Leu Thr Ile Phe Val Leu
1               5                   10                  15

Ser Val Val Thr Ile Ile Ile Cys Phe Thr Cys Ser Cys Cys Cys Leu
            20                  25                  30

Tyr Lys Thr Cys Arg Arg Pro Arg Pro Val Val Thr Thr Thr Thr Ser
        35                  40                  45

Thr Thr Val Val His Ala Pro Tyr Pro Gln Pro Pro Ser Val Pro Pro
    50                  55                  60

Ser Tyr Pro Gly Pro Ser Tyr Gln Gly Tyr His Thr Met Pro Pro Gln
65                  70                  75                  80

Pro Gly Met Pro Ala Ala Pro Tyr Pro Met Gln Tyr Pro Pro Pro Tyr
                85                  90                  95

Pro Ala Gln Pro Met Gly Pro Pro Ala Tyr His Glu Thr Leu Ala Gly
            100                 105                 110

Gly Ala Ala Ala Pro Tyr Pro Ala Ser Gln Pro Pro Tyr Asn Pro Xaa
        115                 120                 125

Tyr Met Asp Ala Pro Lys Xaa Xaa Ser Glu His Ser Leu Ala Ser Leu
    130                 135                 140

Ala Ala Thr Trp Leu Cys Cys Val Cys Ala Xaa
145                 150                 155

(2) INFORMATION FOR SEQ ID NO: 226:

(i) SEQUENCE CHARACTERISTICS:
(A) LENGTH: 10 amino acids
(B) TYPE: amino acid
(D) TOPOLOGY: linear
(xi) SEQUENCE DESCRIPTION: SEQ ID NO: 226:

Met Gly Phe Gly Ala Thr Leu Ala Val Gly
1               5                   10

(2) INFORMATION FOR SEQ ID NO: 227:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 227:

Met Ser Ile Phe Leu Val Met Ser Ile Ser Cys Ser Ser Thr Ser His
1               5                   10                  15

Cys Tyr Ser Phe
            20

(2) INFORMATION FOR SEQ ID NO: 228:

(i) SEQUENCE CHARACTERISTICS:
(A) LENGTH: 94 amino acids
(B) TYPE: amino acid
(D) TOPOLOGY: linear
(xi) SEQUENCE DESCRIPTION: SEQ ID NO: 228:

Met Ser Phe Ser Phe Ile Ile Phe Leu Leu Leu Val Cys Gln Glu Ile
1               5                   10                  15

Thr Phe Cys Met Ser Tyr Gly Asp Ala Val Asn Cys Phe Ser Glu Cys
            20                  25                  30

Phe Ser Asn Leu Gln Thr Ile Tyr Ile Ser Cys Leu Gln His Ala Val
        35                  40                  45

Cys Lys His Ser Val Ile Trp Ser Ile Gln Leu Phe Val Arg Ala Leu
    50                  55                  60

Pro Ile Ser Lys Cys Ala Glu Leu Ser Ile Asp Gly Ile Phe Arg Ser
65                  70                  75                  80

Phe His Glu Asn Trp Lys Cys Ser Trp Val Ala Pro Thr Xaa
                85                  90

(2) INFORMATION FOR SEQ ID NO: 229:

(i) SEQUENCE CHARACTERISTICS:
(A) LENGTH: 94 amino acids
(B) TYPE: amino acid
(D) TOPOLOGY: linear
(xi) SEQUENCE DESCRIPTION: SEQ ID NO: 229:

Met Ser Phe Ser Phe Ile Ile Phe Leu Leu Leu Val Cys Gln Glu Ile
1               5                   10                  15

Thr Phe Cys Met Ser Tyr Gly Asp Ala Val Asn Cys Phe Ser Glu Cys
            20                  25                  30

Phe Ser Asn Leu Gln Thr Ile Tyr Ile Ser Cys Leu Gln His Ala Val
        35                  40                  45

Cys Lys His Ser Val Ile Trp Ser Ile Gln Leu Phe Val Arg Ala Leu
    50                  55                  60

Pro Ile Ser Lys Cys Ala Glu Leu Ser Ile Asp Gly Ile Phe Arg Ser
65                  70                  75                  80

Phe His Glu Asn Trp Lys Cys Ser Trp Val Ala Pro Thr Xaa
                85                  90

(2) INFORMATION FOR SEQ ID NO: 230:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 230:

Met Gly Trp Ser Ala Gly Leu Leu Phe Leu Leu Ile Leu Tyr Leu Pro
  1               5                  10                  15

Val Pro Gly Trp Met Glu Arg Glu Asp Gly Gly Asp Gly Thr Ser Phe
             20                  25                  30

Thr Ser Gly Ser Trp
        35

(2) INFORMATION FOR SEQ ID NO: 231:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 231:

(2) INFORMATION FOR SEQ ID NO: 232:

(i) SEQUENCE CHARACTERISTICS:
(A) LENGTH: 301 amino acids
(B) TYPE: amino acid
(D) TOPOLOGY: linear
(xi) SEQUENCE DESCRIPTION: SEQ ID NO: 232:

Met Asp Ala Arg Trp Trp Ala Val Val Val Leu Ala Ala Phe Pro Ser
1               5                   10                  15

Leu Gly Ala Gly Gly Glu Thr Pro Glu Ala Pro Pro Glu Ser Trp Thr
            20                  25                  30

Gln Leu Trp Phe Phe Arg Phe Val Val Asn Ala Ala Gly Tyr Ala Xaa
        35                  40                  45

Phe Met Val Pro Gly Tyr Leu Leu Val Gln Tyr Phe Arg Arg Lys Asn
    50                  55                  60

Tyr Leu Glu Thr Gly Arg Gly Leu Cys Phe Pro Leu Val Lys Ala Cys
65                  70                  75                  80

Val Phe Gly Asn Glu Pro Lys Ala Ser Asp Glu Val Pro Leu Ala Pro
                85                  90                  95

Arg Thr Glu Ala Ala Glu Thr Thr Pro Met Trp Gln Ala Leu Lys Leu
            100                 105                 110

Leu Phe Cys Ala Thr Gly Leu Gln Val Ser Tyr Leu Thr Trp Gly Val
        115                 120                 125

Leu Gln Glu Arg Val Met Thr Arg Ser Tyr Gly Ala Thr Ala Thr Ser
    130                 135                 140

Pro Gly Glu Arg Phe Thr Asp Ser Gln Phe Leu Val Leu Met Asn Arg
145                 150                 155                 160

Val Leu Ala Leu Ile Val Ala Gly Leu Ser Cys Val Leu Cys Lys Gln
                165                 170                 175

Pro Arg His Gly Ala Pro Met Tyr Arg Tyr Ser Phe Ala Ser Leu Ser
            180                 185                 190

Asn Val Leu Ser Ser Trp Cys Gln Tyr Glu Ala Leu Lys Phe Val Ser
        195                 200                 205

Phe Pro Thr Gln Val Leu Ala Lys Ala Ser Lys Val Ile Pro Val Met
    210                 215                 220

Leu Met Gly Lys Leu Val Ser Arg Arg Xaa Asn Glu His Trp Glu Tyr
225                 230                 235                 240

Leu Thr Ala Thr Leu Ile Ser Ile Gly Val Ser Met Phe Leu Leu Ser
                245                 250                 255

Ser Gly Pro Glu Pro Arg Ser Ser Pro Ala Thr Thr Leu Ser Gly Leu
            260                 265                 270

Ile Leu Leu Ala Gly Tyr Ile Ala Phe Asp Ser Phe Thr Ser Asn Trp
        275                 280                 285

Gln Asp Ala Cys Leu Pro Ile Arg Cys His Arg Cys Arg
    290                 295                 300

(2) INFORMATION FOR SEQ ID NO: 233:

(i) SEQUENCE CHARACTERISTICS:
(A) LENGTH: 313 amino acids
(B) TYPE: amino acid
(D) TOPOLOGY: linear
(xi) SEQUENCE DESCRIPTION: SEQ ID NO: 233:

Met Ser Asp Leu Leu Leu Leu Gly Leu Ile Gly Gly Leu Thr Leu Leu
1               5                   10                  15

Leu Leu Leu Thr Leu Leu Ala Phe Ala Gly Tyr Ser Gly Leu Leu Ala
            20                  25                  30

Gly Val Glu Val Ser Ala Gly Ser Pro Pro Ile Arg Asn Val Thr Val
        35                  40                  45

Ala Tyr Lys Phe His Met Gly Leu Tyr Gly Glu Thr Gly Arg Leu Phe
    50                  55                  60

Thr Glu Ser Cys Ser Ile Ser Pro Lys Leu Arg Ser Ile Ala Val Tyr
65                  70                  75                  80

Tyr Asp Asn Pro His Met Val Pro Pro Asp Lys Cys Arg Cys Ala Val
                85                  90                  95

Gly Ser Ile Leu Ser Glu Gly Glu Glu Ser Pro Ser Pro Glu Leu Ile
            100                 105                 110

Asp Leu Tyr Gln Lys Phe Gly Phe Lys Val Phe Ser Phe Pro Ala Pro
        115                 120                 125

Ser His Val Val Thr Ala Thr Phe Pro Tyr Thr Thr Ile Leu Ser Ile
    130                 135                 140

Trp Leu Ala Thr Arg Arg Val His Pro Ala Leu Asp Thr Tyr Ile Lys
145                 150                 155                 160

Glu Arg Lys Leu Cys Ala Tyr Pro Arg Leu Glu Ile Tyr Gln Glu Asp
                165                 170                 175

Gln Ile His Phe Met Cys Pro Leu Ala Xaa Gln Gly Asp Phe Tyr Val
            180                 185                 190

Pro Glu Met Lys Glu Thr Glu Trp Lys Trp Arg Gly Leu Val Glu Ala
        195                 200                 205

Ile Asp Thr Gln Val Asp Gly Thr Gly Ala Asp Thr Met Ser Asp Thr
    210                 215                 220

Ser Ser Val Ser Leu Glu Val Ser Pro Gly Ser Arg Glu Thr Ser Ala
225                 230                 235                 240

Ala Thr Leu Ser Pro Gly Ala Ser Ser Arg Gly Trp Asp Asp Gly Asp
                245                 250                 255

Thr Arg Ser Glu His Ser Tyr Ser Glu Ser Gly Ala Ser Gly Ser Ser
            260                 265                 270

Phe Glu Glu Leu Asp Leu Glu Gly Glu Gly Pro Leu Gly Glu Ser Arg
        275                 280                 285

Leu Asp Pro Gly Thr Xaa Pro Leu Gly Thr Thr Lys Trp Leu Trp Glu
    290                 295                 300

Pro Thr Ala Pro Glu Lys Gly Lys Glu
305                 310

(2) INFORMATION FOR SEQ ID NO: 234:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 234:

Pro Gln Ser Leu Ile Leu His Leu Leu Leu Phe Phe Phe Leu Leu Phe
1               5                   10                  15

Leu Phe Phe Ile Phe Ile Phe Leu Phe Phe Leu Gln Cys Leu Thr Phe
            20                  25                  30

Leu Phe Xaa Lys Pro Arg Gly Arg Tyr His Gly Leu Cys Phe Lys Phe
        35                  40                  45

(2) INFORMATION FOR SEQ ID NO: 235:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 235:

Pro Ala Leu Arg Pro Ala Leu Leu Trp Ala Leu Leu Ala Leu Trp Leu
1               5                   10                  15

Cys Cys Ala Thr Pro Arg Met His Cys Ser Val Glu Met Ala Met Asn
            20                  25                  30

Pro Val

(2) INFORMATION FOR SEQ ID NO: 236:

(i) SEQUENCE CHARACTERISTICS:
(A) LENGTH: 313 amino acids
(B) TYPE: amino acid
(D) TOPOLOGY: linear
(xi) SEQUENCE DESCRIPTION: SEQ ID NO: 236:

Met Thr Arg Gly Gly Pro Gly Gly Arg Pro Gly Leu Pro Gln Pro Pro
1               5                   10                  15

Pro Leu Leu Leu Leu Leu Leu Leu Xaa Leu Leu Leu Val Thr Ala Glu
            20                  25                  30

Pro Pro Lys Pro Ala Gly Val Tyr Tyr Ala Thr Ala Tyr Trp Met Pro
        35                  40                  45

Ala Glu Lys Thr Val Gln Val Lys Asn Val Met Asp Lys Asn Gly Asp
    50                  55                  60

Ala Tyr Gly Phe Tyr Asn Asn Ser Val Lys Thr Thr Gly Trp Gly Ile
65                  70                  75                  80

Leu Glu Ile Arg Ala Gly Tyr Gly Ser Gln Thr Leu Ser Asn Glu Ile
                85                  90                  95

Ile Met Phe Val Ala Gly Phe Leu Glu Gly Tyr Leu Thr Ala Pro His
            100                 105                 110

Met Asn Asp His Tyr Thr Asn Leu Tyr Pro Gln Leu Ile Thr Lys Pro
        115                 120                 125

Ser Ile Met Asp Lys Val Gln Asp Phe Met Glu Lys Gln Asp Lys Trp
    130                 135                 140

Thr Arg Lys Asn Ile Lys Glu Tyr Lys Thr Asp Ser Phe Trp Arg His
145                 150                 155                 160

Thr Gly Tyr Val Met Ala Gln Ile Asp Gly Leu Tyr Val Gly Ala Lys
                165                 170                 175

Lys Arg Ala Ile Leu Glu Gly Thr Lys Pro Met Thr Leu Phe Gln Ile
            180                 185                 190

Gln Phe Leu Asn Ser Val Gly Asp Leu Leu Asp Leu Ile Pro Ser Leu
        195                 200                 205

Ser Pro Thr Lys Asn Gly Ser Leu Lys Val Phe Lys Arg Trp Asp Met
    210                 215                 220

Gly His Cys Ser Ala Leu Ile Lys Val Leu Pro Gly Phe Glu Asn Ile
225                 230                 235                 240

Leu Phe Ala His Ser Ser Trp Tyr Thr Tyr Ala Ala Met Leu Arg Ile
                245                 250                 255

Tyr Lys His Trp Asp Phe Asn Xaa Ile Asp Lys Asp Thr Ser Ser Ser
            260                 265                 270

Arg Leu Ser Phe Ser Ser Tyr Pro Gly Phe Leu Glu Ser Leu Asp Asp
        275                 280                 285

Phe Tyr Ile Leu Ser Ser Gly Leu Ile Leu Leu Gln Thr Thr Asn Ser
    290                 295                 300

Val Phe Asn Lys Thr Leu Leu Lys Gln
305                 310

(2) INFORMATION FOR SEQ ID NO: 237:

        (i) SEQUENCE CHARACTERISTICS:
                (A) LENGTH: 296 amino acids
                (B) TYPE: amino acid
                (D) TOPOLOGY: linear
        (xi) SEQUENCE DESCRIPTION: SEQ ID NO: 237:

Met Leu Gln Gly Pro Gly Ser Leu Leu Leu Leu Phe Leu Ala Ser His
  1               5                  10                  15

Cys Cys Leu Gly Ser Ala Arg Gly Leu Phe Leu Phe Gly Gln Pro Asp
             20                  25                  30

Phe Ser Tyr Lys Arg Xaa Asn Cys Lys Pro Ile Pro Val Asn Leu Gln
         35                  40                  45

Leu Cys His Gly Ile Glu Tyr Gln Asn Met Arg Leu Pro Asn Leu Leu
     50                  55                  60

Gly His Glu Thr Met Lys Glu Val Leu Glu Gln Ala Gly Ala Trp Ile
 65                  70                  75                  80

Pro Leu Val Met Lys Gln Cys His Pro Asp Thr Lys Lys Phe Leu Cys
                 85                  90                  95

Ser Leu Phe Ala Pro Val Cys Leu Asp Asp Leu Asp Glu Thr Ile Gln
            100                 105                 110

Pro Cys His Ser Leu Cys Val Gln Val Lys Asp Arg Cys Ala Pro Val
        115                 120                 125

Met Ser Ala Phe Gly Phe Pro Trp Pro Asp Met Leu Glu Cys Asp Arg
    130                 135                 140

Phe Pro Gln Asp Asn Asp Leu Cys Ile Pro Leu Ala Ser Ser Asp His
145                 150                 155                 160

Leu Leu Pro Ala Thr Glu Glu Ala Pro Lys Val Cys Glu Ala Cys Lys
                165                 170                 175

Asn Lys Asn Asp Asp Asp Asn Asp Ile Met Glu Thr Leu Cys Lys Asn
            180                 185                 190

Asp Phe Ala Leu Lys Ile Lys Val Lys Glu Ile Thr Tyr Ile Asn Arg
        195                 200                 205

Asp Thr Lys Ile Ile Leu Glu Thr Lys Ser Lys Thr Ile Tyr Lys Leu
    210                 215                 220

Asn Gly Val Ser Glu Arg Asp Leu Lys Lys Ser Val Leu Trp Leu Lys

225                 230                 235                 240

Asp Ser Leu Gln Cys Thr Cys Glu Glu Met Asn Asp Ile Asn Ala Pro
                245                 250                 255

Tyr Leu Val Met Gly Gln Lys Gln Gly Gly Glu Leu Val Ile Thr Ser
            260                 265                 270

Val Lys Arg Trp Gln Lys Gly Gln Arg Glu Phe Lys Arg Ile Ser Arg
        275                 280                 285

Ser Ile Arg Lys Leu Gln Cys Xaa
    290                 295

(2) INFORMATION FOR SEQ ID NO: 238:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 238:

Met Ala Ser Leu Gly His Ile Leu Val Phe Cys Val Gly Leu Leu Thr
  1               5                  10                  15

Met Ala Lys Ala Glu Ser Pro Lys Glu His Asp Pro Phe Thr Tyr Asp
             20                  25                  30

Tyr Gln Ser Leu Gln Ile Gly Gly Leu Val Ile Ala Gly Ile Leu Phe
         35                  40                  45

Ile Leu Gly Ile Leu Ile Val Leu Ser Arg Arg Cys Arg Cys Lys Phe
     50                  55                  60

Asn Gln Gln Gln Arg Thr Gly Glu Pro Asp Glu Glu Glu Gly Thr Phe
 65                  70                  75                  80

Arg Ser Ser Ile Arg Arg Leu Ser Xaa Arg Xaa Arg
                 85                  90

(2) INFORMATION FOR SEQ ID NO: 239:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 239:

Met Pro Gly Thr Phe Leu Arg Pro Phe Val Phe Leu Phe Leu Phe Ile
  1               5                  10                  15

Cys Cys Cys Leu His Ser Gly Gly Leu Gly Gly Val Pro Leu Pro Pro
             20                  25                  30

Phe Pro Pro Gln Ala Gln Arg Gly Glu Gly Pro Gly Lys Trp Met Ser
         35                  40                  45

Pro Pro Leu Pro Pro His Pro Val Val Ala Pro Pro Thr Pro Ser Pro
    50                  55                  60

Ser Arg Gly Cys Val Leu Leu
65                  70

(2) INFORMATION FOR SEQ ID NO: 240:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 240:

Met Pro Gly Thr Phe Leu Arg Pro Phe Val Phe Leu Phe Leu Phe Ile
1               5                   10                  15

Cys Cys Cys Leu His Ser Gly Gly Leu Gly Gly Val Pro Leu Pro Pro
            20                  25                  30

Phe Pro Pro Gln Ala Gln Arg Gly Glu Gly Pro Gly Lys Trp Met Ser
        35                  40                  45

Pro Pro Leu Pro Pro His Pro Val Val Ala Pro Pro Thr Pro Ser Pro
    50                  55                  60

Ser Arg Gly Cys Val Leu Leu
65                  70

(2) INFORMATION FOR SEQ ID NO: 241:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 241:

Met Phe Tyr Val Leu Ser Val Ser Xaa Leu Xaa Leu Phe Leu Ala Cys
1               5                   10                  15

Gly Leu Cys Leu Xaa Leu Leu Thr Gly Lys Leu Leu
            20                  25

(2) INFORMATION FOR SEQ ID NO: 242:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 242:

Met Lys Leu Phe Asp Ala Ser Pro Thr Phe Phe Ala Phe Leu Leu Gly
1               5                   10                  15

His Ile Leu Ala Met Glu Val Leu Ala Trp Leu Leu Ile Tyr Leu Leu
            20                  25                  30

Gly Pro Gly Trp Val Pro Ser Ala Leu Xaa Arg Leu His Pro Gly His
        35                  40                  45

Leu Ser Gly Ser Val Leu Val Ser Ala Ala
    50                  55

(2) INFORMATION FOR SEQ ID NO: 243:

      (i) SEQUENCE CHARACTERISTICS:
            (A) LENGTH: 123 amino acids
            (B) TYPE: amino acid
            (D) TOPOLOGY: linear
      (xi) SEQUENCE DESCRIPTION: SEQ ID NO: 243:

(2) INFORMATION FOR SEQ ID NO: 244:

      (i) SEQUENCE CHARACTERISTICS:
            (A) LENGTH: 73 amino acids
            (B) TYPE: amino acid
            (D) TOPOLOGY: linear
      (xi) SEQUENCE DESCRIPTION: SEQ ID NO: 244:

Ala Leu Val Ser Gly Gln Leu Cys Met Glu Ile Ala Arg Gly Asn Ile
 1               5                  10                  15

Phe Phe Leu Asn Xaa Leu Val Thr Thr Phe Cys Cys Ser Cys Leu Leu
            20                  25                  30

Leu Ser Val Xaa Tyr Leu His Xaa Gly Phe Phe Tyr Ser Ser Leu Cys
        35                  40                  45

Lys Cys Cys Phe Val Leu Val Val Leu Ser Arg Ile Gly Ser Val Asn
    50                  55                  60

Glu Thr Trp Ser Cys Asn Phe Ser Ile
65                  70

(2) INFORMATION FOR SEQ ID NO: 245:

(i) SEQUENCE CHARACTERISTICS:
(A) LENGTH: 49 amino acids
(B) TYPE: amino acid
(D) TOPOLOGY: linear
(xi) SEQUENCE DESCRIPTION: SEQ ID NO: 245:

Thr Pro Ala Thr Thr Ser Ser Ser Ser Ser Pro Leu Phe Leu Ser Ser
1               5                   10                  15

Pro Asp Trp Ser Ser Cys Pro Ser Gly Ser Cys Ile Ala Pro Trp Cys
            20                  25                  30

Thr His Trp Ser Ser Ile Leu Pro Ser Leu Xaa Ile Thr Ser Ser Ile
        35                  40                  45

Pro

(2) INFORMATION FOR SEQ ID NO: 246:

(i) SEQUENCE CHARACTERISTICS:
(A) LENGTH: 339 amino acids
(B) TYPE: amino acid
(D) TOPOLOGY: linear
(xi) SEQUENCE DESCRIPTION: SEQ ID NO: 246:

Met Ala Arg Val Pro Pro Leu Ser Ser Ser Trp Thr Ser Ser Arg Tyr
1               5                   10                  15

Arg Arg Trp Leu Cys Cys Pro Val Trp Trp Thr Thr Phe Trp Ala Thr
            20                  25                  30

Ala Trp Ser Leu Thr Lys His Leu Tyr Lys Asp Val Thr Asp Ala Ile
        35                  40                  45

Arg Asp Val His Val Lys Gly Leu Met Tyr Gln Trp Ile Glu Gln Asp
    50                  55                  60

Met Glu Lys Tyr Ile Leu Arg Gly Asp Glu Thr Phe Ala Val Leu Ser
65                  70                  75                  80

Arg Leu Val Ala His Gly Lys Gln Leu Phe Leu Ile Thr Asn Ser Pro
                85                  90                  95

Phe Ser Phe Val Asp Lys Gly Met Arg His Met Val Gly Pro Asp Trp

            100                 105                 110

Arg His Ser Ser Met Trp Ser Leu Ser Arg Gln Thr Ser Pro Ala Ser
        115                 120                 125

Ser Leu Thr Gly Ala Thr Phe Arg Lys Leu Asp Glu Lys Gly Ser Leu
    130                 135                 140

Gln Trp Asp Arg Ile Thr Arg Leu Glu Lys Gly Lys Ile Tyr Arg Gln
145                 150                 155                 160

Gly Asn Leu Phe Asp Phe Leu Arg Leu Thr Glu Trp Arg Gly Pro Arg
                165                 170                 175

Val Leu Tyr Phe Gly Asp His Leu Tyr Ser Asp Leu Ala Asp Leu Met
            180                 185                 190

Leu Arg His Gly Trp Arg Thr Gly Ala Ile Ile Pro Glu Leu Glu Arg
        195                 200                 205

Glu Ile Arg Ile Ile Asn Thr Glu Gln Tyr Met His Ser Leu Thr Trp
    210                 215                 220

Gln Gln Ala Leu Thr Gly Leu Leu Glu Arg Met Gln Thr Tyr Gln Asp
225                 230                 235                 240

Ala Glu Ser Arg Gln Val Leu Ala Ala Trp Met Lys Glu Arg Gln Glu
                245                 250                 255

Leu Arg Cys Ile Thr Lys Ala Leu Phe Asn Ala Gln Phe Gly Ser Ile
            260                 265                 270

Phe Arg Thr Phe His Asn Pro Thr Tyr Phe Ser Arg Arg Leu Val Arg
        275                 280                 285

Phe Ser Asp Leu Tyr Met Ala Ser Leu Ser Cys Leu Leu Asn Tyr Arg
    290                 295                 300

Val Asp Phe Thr Phe Tyr Pro Arg Arg Thr Pro Leu Gln His Glu Ala
305                 310                 315                 320

Pro Leu Trp Met Asp Gln Leu Leu His Arg Leu His Glu Asp Pro Leu
                325                 330                 335

Pro Trp Xaa

(2) INFORMATION FOR SEQ ID NO: 247:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 247:

Met Ala Leu Leu Ser Cys Val Val Asp Tyr Phe Leu Gly His Ser Leu
1               5                   10                  15

Xaa Val

(2) INFORMATION FOR SEQ ID NO: 248:

(i) SEQUENCE CHARACTERISTICS:
(A) LENGTH: 339 amino acids
(B) TYPE: amino acid
(D) TOPOLOGY: linear
(xi) SEQUENCE DESCRIPTION: SEQ ID NO: 248:

Met Asn Trp Glu Leu Leu Leu Trp Leu Leu Val Leu Cys Ala Leu Leu
1               5                   10                  15

Leu Leu Leu Val Gln Leu Leu Arg Phe Leu Arg Ala Asp Gly Asp Leu
            20                  25                  30

Thr Leu Leu Trp Ala Glu Trp Gln Gly Arg Arg Pro Glu Trp Glu Leu
        35                  40                  45

Thr Asp Met Val Val Trp Val Thr Gly Ala Ser Ser Gly Ile Gly Glu
    50                  55                  60

Glu Leu Ala Tyr Gln Leu Ser Lys Leu Gly Val Ser Leu Val Leu Ser
65                  70                  75                  80

Ala Arg Arg Val His Glu Leu Glu Arg Val Lys Arg Arg Cys Leu Glu
                85                  90                  95

Asn Gly Asn Leu Lys Glu Lys Asp Ile Leu Val Leu Pro Leu Asp Leu
            100                 105                 110

Thr Asp Thr Gly Ser His Glu Ala Ala Thr Lys Ala Val Leu Gln Glu
        115                 120                 125

Phe Gly Arg Ile Asp Ile Leu Val Asn Asn Gly Gly Met Ser Gln Arg
    130                 135                 140

Ser Leu Cys Met Asp Thr Ser Leu Asp Val Tyr Arg Lys Leu Ile Glu
145                 150                 155                 160

Leu Asn Tyr Leu Gly Thr Val Ser Leu Thr Lys Cys Val Leu Pro His
                165                 170                 175

Met Ile Glu Arg Lys Gln Gly Lys Ile Val Thr Val Asn Ser Ile Leu
            180                 185                 190

Gly Ile Ile Ser Val Pro Leu Ser Ile Gly Tyr Cys Ala Ser Lys His
        195                 200                 205

Ala Leu Arg Gly Phe Phe Asn Gly Leu Arg Thr Glu Leu Ala Thr Tyr
    210                 215                 220

Pro Gly Ile Ile Val Ser Asn Ile Cys Pro Gly Pro Val Gln Ser Asn
225                 230                 235                 240

Ile Val Glu Asn Ser Leu Ala Gly Glu Val Thr Lys Thr Ile Gly Asn
                245                 250                 255

Asn Gly Asp Gln Ser His Lys Met Thr Thr Ser Arg Cys Val Arg Leu
            260                 265                 270

Met Leu Ile Ser Met Ala Asn Asp Leu Lys Glu Val Trp Ile Ser Glu
        275                 280                 285

Gln Pro Phe Leu Leu Val Thr Tyr Leu Trp Gln Tyr Met Pro Thr Trp
    290                 295                 300

Ala Trp Trp Ile Thr Asn Lys Met Gly Lys Lys Arg Ile Glu Asn Phe
305                 310                 315                 320

Lys Ser Gly Val Asp Ala Asp Ser Ser Tyr Phe Lys Ile Phe Lys Thr
                325                 330                 335

Lys His Asp

(2) INFORMATION FOR SEQ ID NO: 249:

(i) SEQUENCE CHARACTERISTICS:
(A) LENGTH: 96 amino acids
(B) TYPE: amino acid
(D) TOPOLOGY: linear
(xi) SEQUENCE DESCRIPTION: SEQ ID NO: 249:

Met Gly Ala Arg Pro Gly Gly His Pro Gln Lys Trp Ser Phe Leu Trp
1               5                   10                  15

Ser Leu Ala Leu Trp Leu Pro Leu Ala Leu Ser Val Ser Leu Phe Leu
            20                  25                  30

Gly Leu Ser Leu Ser Pro Pro Gln Pro Gly Leu Ser Leu Trp Cys Thr
        35                  40                  45

Leu Ser Tyr Cys Cys Glu Gln Trp Lys Phe Lys Gly Thr Pro Ser Pro
    50                  55                  60

Ala Leu Leu Asn Leu Gly Thr Gln Pro Lys Lys Asp Lys Lys Leu Glu
65                  70                  75                  80

Asp Ser Ile Ala Thr Gln Leu Arg Xaa Leu Pro Glu Lys Asn Ser Asn
                85                  90                  95

(2) INFORMATION FOR SEQ ID NO: 250:

(i) SEQUENCE CHARACTERISTICS:
(A) LENGTH: 79 amino acids
(B) TYPE: amino acid
(D) TOPOLOGY: linear
(xi) SEQUENCE DESCRIPTION: SEQ ID NO: 250:

Met Ala Leu Thr Phe Leu Leu Val Leu Leu Thr Leu Ala Thr Leu Cys
1               5                   10                  15

Thr Arg Leu His Arg Asn Phe Arg Arg Gly Glu Ser Ile Tyr Trp Gly
            20                  25                  30

Pro Thr Ala Asp Ser Gln Asp Thr Val Ala Ala Val Leu Lys Arg Arg
        35                  40                  45

Leu Leu Gln Pro Ser Arg Arg Val Lys Arg Ser Arg Arg Arg Pro Xaa
    50                  55                  60

Xaa Pro Pro Thr Pro Asp Ser Gly Pro Glu Gly Glu Ser Ser Glu
65                  70                  75

(2) INFORMATION FOR SEQ ID NO: 251:

(i) SEQUENCE CHARACTERISTICS:
(A) LENGTH: 354 amino acids
(B) TYPE: amino acid
(D) TOPOLOGY: linear
(xi) SEQUENCE DESCRIPTION: SEQ ID NO: 251:

Met Gly Pro Ser Thr Pro Leu Leu Ile Leu Phe Leu Leu Ser Trp Ser
1               5                   10                  15

Gly Pro Leu Gln Gly Gln Gln His His Leu Val Glu Tyr Met Glu Arg
            20                  25                  30

Arg Leu Ala Ala Leu Glu Glu Arg Leu Ala Gln Cys Gln Asp Gln Ser
        35                  40                  45

Ser Arg His Ala Ala Glu Leu Arg Asp Phe Lys Asn Lys Met Leu Pro
    50                  55                  60

Leu Leu Glu Val Ala Glu Lys Glu Arg Glu Ala Leu Arg Thr Glu Ala
65                  70                  75                  80

Asp Thr Ile Ser Gly Arg Val Asp Arg Leu Glu Arg Glu Val Asp Tyr
                85                  90                  95

Leu Glu Thr Gln Asn Pro Ala Leu Pro Cys Val Glu Phe Asp Glu Lys
            100                 105                 110

Val Thr Gly Gly Pro Gly Thr Lys Gly Lys Gly Arg Arg Asn Glu Lys
        115                 120                 125

Tyr Asp Met Val Thr Asp Cys Gly Tyr Thr Ile Ser Gln Val Arg Ser
    130                 135                 140

Met Lys Ile Leu Lys Arg Phe Gly Gly Pro Ala Gly Leu Trp Thr Lys
145                 150                 155                 160

Asp Pro Leu Gly Gln Thr Glu Lys Ile Tyr Val Leu Asp Gly Thr Gln
                165                 170                 175

Asn Asp Thr Ala Phe Val Phe Pro Arg Leu Arg Asp Phe Thr Leu Ala
            180                 185                 190

Met Ala Ala Arg Lys Ala Ser Arg Val Arg Val Pro Phe Pro Trp Val
        195                 200                 205

Gly Thr Gly Gln Leu Val Tyr Gly Gly Phe Leu Tyr Phe Ala Arg Arg
    210                 215                 220

Pro Pro Gly Arg Pro Gly Gly Gly Gly Glu Met Glu Asn Thr Leu Gln
225                 230                 235                 240

Leu Ile Lys Phe His Leu Ala Asn Arg Thr Val Val Asp Ser Ser Val
                245                 250                 255

Phe Pro Ala Glu Gly Leu Ile Pro Pro Tyr Gly Leu Thr Ala Asp Thr
            260                 265                 270

Tyr Ile Asp Leu Ala Ala Asp Glu Glu Gly Leu Trp Ala Val Tyr Ala
        275                 280                 285

Thr Arg Glu Asp Asp Arg His Leu Cys Leu Ala Lys Leu Asp Pro Gln
    290                 295                 300

Thr Leu Asp Thr Glu Gln Gln Trp Asp Thr Pro Cys Pro Arg Glu Asn
305                 310                 315                 320

Ala Glu Ala Ala Phe Xaa Ile Cys Gly Thr Leu Tyr Val Val Tyr Asn
                325                 330                 335

Thr Arg Pro Ala Ser Arg Ala Arg Ile Gln Cys Ser Phe Asp Ala Ser
            340                 345                 350

Gly Pro

(2) INFORMATION FOR SEQ ID NO: 252:

(i) SEQUENCE CHARACTERISTICS:
(A) LENGTH: 109 amino acids
(B) TYPE: amino acid
(D) TOPOLOGY: linear
(xi) SEQUENCE DESCRIPTION: SEQ ID NO: 252:

Met Leu Cys Ile Asn Gly Thr Thr Pro Arg Pro Leu Pro Val Pro Ser
1               5                   10                  15

Pro Phe Gly Cys Met Ile Phe Phe Phe Phe Lys Asn Pro Trp Lys Gln
            20                  25                  30

Arg Leu Leu Gln Gly Trp Leu Gly Ala Arg Pro Ile His Leu Leu Gly
        35                  40                  45

Tyr Leu Pro Leu Ser Leu Leu Trp Cys Pro Phe Pro Leu Pro Cys Ala
    50                  55                  60

Arg Cys Ser Val Val Tyr Ile Ser Ser Pro Arg His Gly Ala His Ala
65                  70                  75                  80

Pro Arg Asp Met Ile Leu Ser Leu Val Leu Ala His Gly Ala Leu Tyr

85                    90                    95

Lys Glu Leu Gly Gly Arg Gly Arg Lys Trp Glu Pro Ser
            100                 105

(2) INFORMATION FOR SEQ ID NO: 253:

        (i) SEQUENCE CHARACTERISTICS:
                (A) LENGTH: 45 amino acids
                (B) TYPE: amino acid
                (D) TOPOLOGY: linear
        (xi) SEQUENCE DESCRIPTION: SEQ ID NO: 253:

Met Phe Tyr Phe Leu Pro Leu Ile Phe Pro Ala Phe Pro Pro Trp Ala
  1               5                  10                  15

Phe Arg Leu Ser Thr Leu Phe Thr Ile Ile Ser Trp Ser Glu Asp Ser
             20                  25                  30

Asn Asn Ser Gln Val Tyr Met Asn Cys Val Cys Ser Phe
         35                  40                  45

(2) INFORMATION FOR SEQ ID NO: 254:

        (i) SEQUENCE CHARACTERISTICS:
                (A) LENGTH: 315 amino acids
                (B) TYPE: amino acid
                (D) TOPOLOGY: linear
        (xi) SEQUENCE DESCRIPTION: SEQ ID NO: 254:

Met Ala Gly Gly Arg Cys Gly Pro Xaa Leu Thr Ala Leu Leu Ala Ala
  1               5                  10                  15

Trp Ile Ala Ala Val Ala Ala Thr Ala Gly Pro Glu Glu Ala Ala Leu
             20                  25                  30

Pro Pro Glu Gln Ser Arg Val Gln Pro Met Thr Ala Ser Asn Trp Thr
         35                  40                  45

Leu Val Met Glu Gly Glu Trp Met Leu Lys Phe Tyr Ala Pro Trp Cys
     50                  55                  60

Pro Ser Cys Gln Gln Thr Asp Ser Glu Trp Glu Ala Phe Ala Lys Asn
 65                  70                  75                  80

Gly Glu Ile Leu Gln Ile Ser Val Gly Lys Val Asp Val Ile Gln Glu
                 85                  90                  95

Pro Gly Leu Ser Gly Arg Phe Phe Val Thr Thr Leu Pro Ala Phe Phe
            100                 105                 110

His Ala Lys Asp Gly Ile Phe Arg Arg Tyr Arg Gly Pro Gly Ile Phe
        115                 120                 125

Glu Asp Leu Gln Asn Tyr Ile Leu Glu Lys Lys Trp Gln Ser Val Glu
    130                 135                 140

Pro Leu Thr Gly Trp Lys Ser Pro Ala Ser Leu Thr Met Ser Gly Met
145                 150                 155                 160

Ala Gly Leu Phe Ser Ile Ser Gly Lys Ile Trp His Leu His Asn Tyr
                165                 170                 175

Phe Thr Val Thr Leu Gly Ile Pro Ala Trp Cys Ser Tyr Val Phe Phe
            180                 185                 190

Val Ile Ala Thr Leu Val Phe Gly Leu Phe Met Gly Leu Val Leu Val
        195                 200                 205

Val Ile Ser Glu Cys Phe Tyr Val Pro Leu Pro Arg His Leu Ser Glu
    210                 215                 220

Arg Ser Glu Gln Asn Arg Arg Ser Glu Glu Ala His Arg Ala Glu Gln
225                 230                 235                 240

Leu Gln Asp Ala Glu Glu Glu Lys Asp Asp Ser Asn Glu Glu Glu Asn
                245                 250                 255

Lys Asp Ser Leu Val Asp Asp Glu Glu Glu Lys Glu Asp Leu Gly Asp
            260                 265                 270

Glu Asp Glu Ala Glu Glu Glu Glu Glu Glu Asp Asn Leu Ala Ala Gly
        275                 280                 285

Val Asp Glu Glu Arg Ser Glu Ala Asn Asp Gln Gly Pro Pro Gly Glu
    290                 295                 300

Asp Gly Val Thr Arg Glu Xaa Ser Arg Ala Xaa
305                 310                 315

(2) INFORMATION FOR SEQ ID NO: 255:

        (i) SEQUENCE CHARACTERISTICS:
                (A) LENGTH: 53 amino acids
                (B) TYPE: amino acid
                (D) TOPOLOGY: linear
        (xi) SEQUENCE DESCRIPTION: SEQ ID NO: 255:

Met Leu Lys Ala Leu Phe Arg Thr Leu Gln Ala Met Leu Leu Gly Val
  1               5                  10                  15

Trp Ile Leu Leu Leu Leu Ala Ser Leu Ala Pro Leu Trp Leu Tyr Cys
            20                  25                  30

Trp Arg Met Phe Pro Thr Lys Gly Lys Arg Asp Gln Lys Glu Met Leu
        35                  40                  45

Glu Val Ser Gly Ile
    50

(2) INFORMATION FOR SEQ ID NO: 256:

(i) SEQUENCE CHARACTERISTICS:
(A) LENGTH: 93 amino acids
(B) TYPE: amino acid
(D) TOPOLOGY: linear
(xi) SEQUENCE DESCRIPTION: SEQ ID NO: 256:

Met Ile His Leu Gly His Ile Leu Phe Leu Leu Leu Leu Pro Val Ala
1               5                   10                  15

Ala Ala Gln Thr Thr Pro Gly Glu Arg Ser Ser Leu Pro Ala Phe Tyr
            20                  25                  30

Pro Gly Thr Ser Gly Ser Cys Ser Gly Cys Gly Ser Leu Ser Leu Pro
        35                  40                  45

Leu Leu Ala Gly Leu Val Ala Ala Asp Ala Val Ala Ser Leu Leu Ile
    50                  55                  60

Val Gly Ala Val Phe Leu Cys Ala Arg Pro Arg Arg Ser Pro Ala Gln
65                  70                  75                  80

Asp Gly Lys Val Tyr Ile Asn Met Pro Gly Arg Gly Xaa
                85                  90

(2) INFORMATION FOR SEQ ID NO: 257:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 257:

Pro Gly His Leu Leu Pro His Lys Trp Glu Asn Cys
1               5                   10

(2) INFORMATION FOR SEQ ID NO: 258:

(i) SEQUENCE CHARACTERISTICS:
(A) LENGTH: 1852 base pairs
(B) TYPE: nucleic acid
(C) STRANDEDNESS: double
(D) TOPOLOGY: linear

(xi) SEQUENCE DESCRIPTION: SEQ ID NO: 258:

TGGCATCTGT GAGCAGCTGC CAGGCTCCGG CCAGGATCCC TTCCTTCTCC TCATTGGCTG 60

ATGGATCCCA AGGGGCTCCT CTCCTTGACC TTCGTGCTGT TTCTCTCCCT GGCTTTTGGG 120

GCAAGCTACG GAACAGGTGG GCGCATGATG AACTGCCCAA AGATTCTCCG GCAGTTGGGA 180

AGCAAAGTGC TGCTGCCCCT GACATATGAA AGGATAAATA AGAGCATGAA CAAAAGCATC 240

CACATTGTCG TCACAATGGC AAAATCACTG GAGAACAGTG TCGAGAACAA AATAGTGTCT 300

CTTGATCCAT CCGAAGCAGG CCCTCCACGT TATCTAGGAG ATCGCTACAA GTTTTATCTG 360

GAGAATCTCA CCCTGGGGAT ACGGGAAAGC AGGAAGGAGG ATGAGGGATG GTACCTTATG 420

ACCCTGGAGA AAAATGTTTC AGTTCAGCGC TTTTGCCTGC AGTTGAGGCT TTATGAGCAG 480

GTCTCCACTC CAGAAATTAA AGTTTTAAAC AAGACCCAGG AGAACGGGAC CTGCACCTTG 540

ATACTGGGCT GCACAGTGGA GAAGGGGGAC CATGTGGCTT ACAGCTGGAG TGAAAAGGCG 600

GGCACCCACC CACTGAACCC AGCCAACAGC TCCCACCTCC TGTCCCTCAC CCTCGGCCCC 660

CAGCATGCTG ACAATATCTA CATCTGCACC GTGAGCAACC CTATCAGCAA CAATTCCCAG 720

ACCTTCAGCC CGTGGCCCGG ATGCAGGACA GACCCCTCAG AAACAAAACC ATGGGCAGTG 780

TATGCTGGGC TGTTAGGGGG TGTCATCATG ATTCTCATCA TGGTGGTAAT ACTACAGTTG 840

AGAAGAAGAG GTAAAACGAA CCATTACCAG ACAACAGTGG AAAAAAAAAG CCTTACGATC 900

TATGCCCAAG TCCAGAAACC AGGTGACACT CATCATCAGA CTTCGGACTT ATTCTAATCC 960

AGGATGACCT TATTTTGAAA TCCTTATCTT GACATCTGTG AAGACCTTTA TTCAAATAAA 1020

GTCACATTTT GACATTCTGC GAGGGGCTGG AGCCGGGCCG GGGCGATGTG GAGCGCGGGC 1080

CGCGGCGGGG CTGCCTGGCC GGTGCTGTTG GGGCTGCTGC TGGCGCTGTT AGTGCCGGGC 1140

GGTGGTGCCG CCAAGACCGG TGCGGAGCTC GTGACTGCGG GTCGGTGCTG AAGCTGCTCA 1200

ATACGCACCA CCGGTGCGGC TGCACTCGCA CGACATCAAA TACGGATCCG GCAGCGGCCA 1260

GCAATCGGTG ACCGGCGTAG AGGTCGGAGC GACGAATAGC TACTGGCGGA TCCGCGGCGG 1320

CTCGGAGGGG GGTGCCCGCG CGGGTCCCCG GTGCGCTGCG GGCAGGCGGT GAGGTCACAC 1380

ATGTGCTTAC GGGCAAGAAC CTGCACACGC ACCACTTCCC GTCGCCGCTG TCCAACAACC 1440

AGGAAGTGAG TGCCAAAGGG GAAGACGGCG AGGGCGACGA CCTGGACCTA TGGACAGTGC 1500

GCTGCTCTGC TCTGGACAGC ACTGGAGCGG TGAGGCTGCT GTGGCGCCTT CCAGCATGTG 1560

GCACCTCTGT GGTTCCTGTC AGTCACGGTA GCAGTATGGA AGCCCCATCC GTGGGCAGCA 1620

TGAGGTCCAC GCATGCCCAG TGCCAACACG CACAATACGT GGAAGGCCAT GGAAGGCATC 1680

TTCATCAAGC CTAGTGTGGA GCCCTCTGCA GGTCACGATG AACTCTGAGT GTGTGGATGG 1740

ATGGGTGGAT GGAGGGTGGC AGGTGGGGCG TCTGCAGGGC CACTCTTGGC AGAGACTTTG 1800

GGTTTGTAGG GGTCCTCAAG TGCCTTTGTG ATTAAAGAAT GTTGGTCTAT GA 1852

(2) INFORMATION FOR SEQ ID NO: 259:

(i) SEQUENCE CHARACTERISTICS:
(A) LENGTH: 371 amino acids
(B) TYPE: amino acid
(D) TOPOLOGY: linear

(xi) SEQUENCE DESCRIPTION: SEQ ID NO: 259:

(2) INFORMATION FOR SEQ ID NO: 260:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 260:

Cys Arg Cys Ala Ser Gly Phe Thr Gly Glu Asp Cys
  1               5                  10

(2) INFORMATION FOR SEQ ID NO: 261:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 261:

Cys Thr Cys Gln Val Gly Phe Thr Gly Lys Glu Cys
  1               5                  10

(2) INFORMATION FOR SEQ ID NO: 262:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 262:

Cys Leu Asn Leu Pro Gly Ser Tyr Gln Cys Gln Cys
  1               5                  10

(2) INFORMATION FOR SEQ ID NO: 263:

(i) SEQUENCE CHARACTERISTICS:
(A) LENGTH: 12 amino acids
(B) TYPE: amino acid

(D) TOPOLOGY: linear
(xi) SEQUENCE DESCRIPTION: SEQ ID NO: 263:

Cys Lys Cys Leu Thr Gly Phe Thr Gly Gln Lys Cys
1               5                   10

(2) INFORMATION FOR SEQ ID NO: 264:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 264:

Cys Gln Cys Leu Gln Gly Phe Thr Gly Gln Tyr Cys
1               5                   10

(2) INFORMATION FOR SEQ ID NO: 265:

(i) SEQUENCE CHARACTERISTICS:
(A) LENGTH: 127 amino acids
(B) TYPE: amino acid
(D) TOPOLOGY: linear
(xi) SEQUENCE DESCRIPTION: SEQ ID NO: 265:

Gly Leu Ala Cys Trp Leu Ala Gly Val Ile Phe Ile Asp Arg Lys Arg
1               5                   10                  15

Thr Gly Asp Ala Ile Ser Val Met Ser Glu Val Ala Gln Thr Leu Leu
            20                  25                  30

Thr Gln Asp Val Xaa Val Trp Val Phe Pro Glu Gly Thr Arg Asn His
        35                  40                  45

Asn Gly Ser Met Leu Pro Phe Lys Arg Gly Ala Phe His Leu Ala Val
    50                  55                  60

Gln Ala Gln Val Pro Ile Val Pro Ile Val Met Ser Ser Tyr Gln Asp
65                  70                  75                  80

Phe Tyr Cys Lys Lys Glu Arg Arg Phe Thr Ser Gly Gln Cys Gln Val
                85                  90                  95

Arg Val Leu Pro Pro Val Pro Thr Glu Gly Leu Thr Pro Asp Asp Val
            100                 105                 110

Pro Ala Leu Ala Asp Arg Val Arg His Ser Met Leu His Cys Phe
        115                 120                 125

(2) INFORMATION FOR SEQ ID NO: 266:

(i) SEQUENCE CHARACTERISTICS:
(A) LENGTH: 98 amino acids
(B) TYPE: amino acid

(D) TOPOLOGY: linear
(xi) SEQUENCE DESCRIPTION: SEQ ID NO: 266:

Pro Ser Ala Lys Tyr Phe Phe Lys Met Ala Phe Tyr Asn Gly Trp Ile
1               5                   10                  15

Leu Phe Leu Ala Val Leu Ala Ile Pro Val Cys Ala Val Arg Gly Arg
            20                  25                  30

Asn Val Glu Asn Met Lys Ile Leu Arg Leu Met Leu Leu His Ile Lys
        35                  40                  45

Tyr Leu Tyr Gly Ile Arg Val Glu Val Arg Gly Ala His His Phe Pro
    50                  55                  60

Pro Ser Gln Pro Tyr Val Val Val Ser Asn His Gln Ser Ser Leu Asp
65                  70                  75                  80

Leu Leu Gly Met Met Glu Val Leu Pro Gly Arg Cys Val Pro Ile Ala
                85                  90                  95

Lys Arg

(2) INFORMATION FOR SEQ ID NO: 267:

(i) SEQUENCE CHARACTERISTICS:
(A) LENGTH: 9 amino acids
(B) TYPE: amino acid
(D) TOPOLOGY: linear
(xi) SEQUENCE DESCRIPTION: SEQ ID NO: 267:

Thr Val Phe Arg Glu Ile Ser Thr Asp
1               5

(2) INFORMATION FOR SEQ ID NO: 268:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 268:

Leu Trp Ala Gly Ser Ala Gly Trp Pro Ala Gly
1               5                   10

(2) INFORMATION FOR SEQ ID NO: 269:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 269:

Ser Ile Leu Gly Ile Ile Ser Val Pro Leu Ser Ile Gly Tyr Cys Ala
1               5                   10                  15

Ser Lys His Ala Leu Arg Gly Phe Phe Asn Gly Leu Arg
            20                  25

(2) INFORMATION FOR SEQ ID NO: 270:

(i) SEQUENCE CHARACTERISTICS:
(A) LENGTH: 8 amino acids
(B) TYPE: amino acid
(D) TOPOLOGY: linear
(xi) SEQUENCE DESCRIPTION: SEQ ID NO: 270:

Met Ala Tyr His Gly Leu Thr Val
1               5

(2) INFORMATION FOR SEQ ID NO: 271:

(i) SEQUENCE CHARACTERISTICS:
(A) LENGTH: 6 amino acids
(B) TYPE: amino acid
(D) TOPOLOGY: linear
(xi) SEQUENCE DESCRIPTION: SEQ ID NO: 271:

Ile Ser Ala Ala Arg Val
1               5

(2) INFORMATION FOR SEQ ID NO: 272:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 272:

Pro Asp Val Ser Glu Phe Met Thr Arg Leu Phe
1               5                   10

(2) INFORMATION FOR SEQ ID NO: 273:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 273:

Phe Asp Pro Val Arg Val Asp Ile Thr Ser Lys Gly Lys Met Arg Ala
1               5                   10                  15

Arg